# EUROPEAN PATENT SPECIFICATION

(11) **EP 2 992 013 B1**
(45) Date of publication and mention of the grant of the patent: **04.12.2019**
(21) Application number: 13721243.7
(22) Date of filing: 29.04.2013
(51) Int. Cl.: C07K 16/28, C07K 14/00

(54) **ANTI-CD38 ANTIBODIES AND FUSIONS TO ATTENUATED INTERFERON ALPHA-2B**
ANTI-CD38-ANTIKÖRPER UND FUSIONEN DAVON MIT ABGESCHWÄCHTEM INTERFERON-ALPHA-2B
ANTICORPS ANTI-CD38 ET FUSIONS SUR UN INTERFÉRON ALPHA-2B ATTÉNUÉ

(43) Date of publication of application: 09.03.2016
(73) Proprietor: Teva Pharmaceuticals Australia Pty Ltd, Macquarie Park, NSW 2113 (AU)
(72) Inventor: CLARKE, Adam, Macquarie Park, NSW 2113 (AU); POLLARD, Matthew, Macquarie Park, NSW 2113 (AU); DOYLE, Anthony, Gerard, Macquarie Park, NSW 2113 (AT); BEHRENS, Collette, Macquarie Park, NSW 2113 (AU); YAMAGISHI, Tetsuo, Macquarie Park, NSW 2113 (AU); WILSON, David, S. Jr., Redwood City, CA 94063 (US); POGUE, Sarah, L., Freemont, CA 94555 (US); TAURA, Tetsuya, Palo Alto, CA 94306 (US)
(74) Representative: Lee, Nicholas John
(86) International application number: PCT/US2013/038659
(87) International publication number: WO 2014/178820

(56) References cited:
- WO-A1-01/97844
- WO-A1-2011/154453
- WO-A1-2012/092612
- WO-A2-2005/103083
- WO-A2-2007/042309
- WO-A2-2013/059885
- US-A1- 2002 164 788

## Description

### FIELD OF THE DISCLOSURE

This disclosure relates generally to the field of antibody engineering. More specifically, this disclosure relates to antibodies that bind specifically to CD38, as well as constructs comprising such antibodies and attenuated interferon-alpha ligands, and methods of treatment using these constructs. In these constructs, the antibodies direct the ligands to cells that express both CD38 and receptors for the ligands, and the attenuated interferon-alpha reduces interferon signaling in cells that do not express CD38.

### BACKGROUND OF THE DISCLOSURE

Various publications, including patents, published applications, technical articles, scholarly articles, and gene or protein accession numbers are cited throughout the specification.

CD38 is a 46kDa type II transmembrane glycoprotein. It has a short N-terminal cytoplasmic tail of 20 amino acids, a single transmembrane helix and a long extracellular domain of 256 amino acids. It is expressed on the surface of many immune cells including CD4 and CD8 positive T cells, B cells, NK cells, monocytes, plasma cells and on a significant proportion of normal bone marrow precursor cells. In some instances, the expression of CD38 in lymphocytes may be dependent on the differentiation and activation state of the cell, for example, resting T and B cells may be negative while immature and activated lymphocytes may be predominantly positive for CD38 expression. CD38 mRNA expression has been detected in non-hemopoeitic organs such as the pancreas, brain, spleen and liver (Koguma, T. (1994) Biochim. Biophys. Acta 1223:160).

CD38 is a multifunctional ectoenzyme that is involved in transmembrane signaling and cell adhesion. It is also known as cyclic ADP ribose hydrolase because it can transform NAD⁺ and NADP⁺ into cADPR, ADPR and NAADP, depending on extracellular pH. These products induce Ca²⁺ -mobilization inside the cell, which can lead to tyrosine phosphorylation and activation of the cell. CD38 is also a receptor that can interact with a ligand, CD31. Activation of receptor via CD31 leads to intracellular events including Ca²⁺ mobilization, cell activation, proliferation, differentiation and migration.

CD38 is expressed at high levels on multiple myeloma cells, in most cases of T- and B-lineage acute lymphoblastic leukemias, some acute myelocyticleukemias, follicular center cell lymphomas and T lymphoblastic lymphomas. CD38 is also expressed on B-lineage chronic lymphoblastic leukemia (B-CLL) cells. In some cases, B-CLL patients presenting with a CD38+ clone are characterized by an unfavorable clinical course with a more advanced stage of disease, poor responsiveness to chemotherapy and shorter survival time. The use of antibodies to CD38 has been proposed for the treatment of CD38-expressing cancers and hematological malignancies. It may therefore be advantageous to provide alternative antibodies to CD38 which have desirable manufacturing, stability and immunogenic properties.

Numerous peptide and polypeptide ligands have been described to function by interacting with a receptor on a cell surface, and thereby stimulating, inhibiting, or otherwise modulating a biological response, usually involving signal transduction pathways inside the cell that bears the said receptor. Examples of such ligands include peptide and polypeptide hormones, cytokines, chemokines, growth factors, and apoptosis-inducing factors.

Due to the biological activities of such ligands, many have potential uses as therapeutics. Several peptide or polypeptide ligands have been approved by regulatory agencies as therapeutic products including, for example, human growth hormone, insulin, interferon (IFN)-alpha2b, IFN-alpha2a, IFNβ, erythropoietin, G-CSF and GM-CSF.

While these and other ligands have demonstrated potential in therapeutic applications, they may also exhibit toxicity when administered to human patients. One reason for toxicity is that most of these ligands trigger receptors on a variety of cells, including cells other than those that mediate the desired therapeutic effect. A consequence of such "off target" activity of ligands is that many ligands are currently not suitable for use as therapeutic agents because the ligands cannot be administered at sufficiently high dosages to produce maximal or optimal therapeutic effects on the target cells which mediate the therapeutic effect.

For example it has been known since the mid-1980's that interferons, in particular IFN-alpha, are able to increase apoptosis and decrease proliferation of certain cancer cells. IFN-alpha has been approved by the FDA for the treatment of several cancers including melanoma, renal cell carcinoma, B cell lymphoma, multiple myeloma, chronic myelogenous leukemia (CML) and hairy cell leukemia. A direct effect of IFN-alpha on the tumor cells is mediated by the IFN-alpha binding directly to the type I IFN receptor on those cells and stimulating apoptosis, terminal differentiation or reduced proliferation. A further indirect effect of IFN-alpha on non-cancer cells is to stimulate the immune system, which may produce an additional anti-cancer effect by causing the immune system to reject the tumor.

These biological activities are mediated by type I interferon receptors on the surface of the cancer cells which, when stimulated, initiate various signal transduction pathways leading to reduced proliferation and/or the induction of terminal differentiation or apoptosis. The type I interferon receptor is, however, also present on most non-cancerous cells. Activation of this receptor on non-cancerous cells by IFN-alpha causes the expression of numerous pro-inflammatory cytokines and chemokines, leading to toxicity and untoward effects. Such toxicity may cause severe flu-like symptoms, which prevents the dosing of IFN-alpha to a subject at levels that exert the maximum anti-proliferative and pro-apoptotic activity on the cancer cells.

When IFN-alpha2b is used to treat multiple myeloma, its utility resides, at least in part, in its binding to type I interferon receptors on the myeloma cells, which in turn triggers apoptosis and/or reduced proliferation and hence limits disease progression. Unfortunately, however, this IFN also binds healthy cells within the body, triggering a variety of other cellular responses, some of which are harmful.

A publication by Ozzello (Breast Cancer Research and Treatment 25:265-76, 1993) describes chemically conjugating human IFN-alpha to a tumor-targeting antibody, thereby localizing the direct inhibitory activity of IFN-alpha to the tumor as a way of reducing tumor growth rates, and demonstrated that such conjugates have anti-tumor activity in a xenograft model of a human cancer. The mechanism of the observed anti-cancer activity was attributed to a direct effect of IFN-alpha on the cancer cells, since the human IFN-alpha used in the experiments did not interact appreciably with the murine type I IFN receptor, which could have led to an indirect anti-cancer effect. Because of this lack of binding of the human IFN-alpha to the murine cells, the toxicity of the antibody-IFN-alpha conjugate relative to free INF-alpha was not assessed.

Antibodies and IFN-alpha may also be connected together in the form of a fusion protein. For example, WO 01/97844 describes a direct fusion of human IFN-alpha to the C-terminus of the heavy chain of an IgG specific for the tumor antigen CD20.

In general, IFN may be targeted to cancer cells. While this approach may result in an increase in activity of the IFN against cancer cells, it does not completely address the issue of undesired activity of the IFN on healthy cells. Fusing IFN-alpha to the C-terminus of the heavy chain of an IgG may prolong the half-life of the IFN alpha leading to undesirable adverse events. Accordingly, there exists a need to decrease off-target activity of ligand-based drugs, while retaining the "on-target" therapeutic effect of such ligands.

In the prior art, WO 2011/154453 discloses human anti CD38 antibodies suitable for the treatment, also used in combination with Interferon alpha 2b, WO 2012/092612 discloses monoclonal anti CD38 antibodies, exhibiting an affinity in the nanomolar range and mediating CDC and ADCC, US 2002/164788 discloses a humanised antibody specific for human CD38, WO 2007/042309 discloses murine, chimeric and fully human antibodies against CD38 and WO 2005/103083 discloses human antagonistic antibodies to CD38 that mediate ADCC and CDC and reduce the growth of human myeloma xenografts in a murine model.

### SUMMARY OF THE DISCLOSURE

The first aspect of the present invention provides an antibody that specifically binds to CD38, the antibody comprising a heavy chain variable region that comprises the amino acid sequence of SEQ ID NO: 156 and a light chain variable region that comprises the amino acid sequence of SEQ ID NO: 185.

The disclosure features new anti-CD38 antibodies and constructs comprising an anti-CD38 antibody and attenuated IFN-alpha. The antibodies, which comprise one or a plurality of mutations in their heavy and/or light chain variable regions retain the ability to specifically bind to CD38, including CD38 expressed on the surface of cells. The antibodies may be fused, for example, to an attenuated form of interferon alpha to form an anti-CD38 antibody-attenuated interferon fusion construct.The antibody according of the first aspect may comprise a human IgG1 heavy chain constant region or a human IgG4 heavy chain constant region. The human IgG4 heavy chain constant region may comprise a proline at position 228 according to the EU numbering system. The human IgG4 heavy chain constant region may comprise a tyrosine at position 252, a threonine at position 254, and a glutamic acid at position 256 of the constant region according to the EU numbering system.

In a second aspect, the present invention provides an anti-CD38 antibody-interferon alpha-2b fusion construct, comprising: an antibody of the first aspect; and an interferon alpha-2b comprising an alanine to aspartic acid or alanine to glycine substitution at the position corresponding to position 168 of the amino acid sequence of SEQ ID NO. 7.

The interferon alpha-2b may be an N-terminally truncated interferon alpha-2b, optionally wherein the N-terminally truncated interferon alpha-2b does not have the twenty-three N-terminal amino acids. The interferon alpha-2b may comprise the amino acid sequence of SEQ ID NO: 649. The interferon alpha-2b may comprise the amino acid sequence of SEQ ID NO: 651, or the amino acid sequence of SEQ ID NO: 647 or SEQ ID NO: 650.

In a third aspect of the invention, there is provided a composition, comprising the antibody of the first aspect or the construct of the second aspect and a pharmaceutically acceptable carrier.

The invention also provides the antibody of the first aspect, the construct of the second aspect or the composition of the third aspect for use in medicine.

The invention also provides the antibody of the first aspect, the construct of the second aspect or the composition of the third aspect for use in the treatment of B-cell lymphoma, multiple myeloma, non-Hodgkin's lymphoma, chronic myelogenous leukemia, chronic lymphocytic leukemia or acute myelogenous leukemia.

Disclosed herein is an isolated antibody that binds specifically to CD38 which comprises a heavy chain variable region comprising the amino acid sequence of SEQ ID NO: 559 and a light chain variable region comprising the amino acid sequence of SEQ ID NO: 664. Also disclosed is n isolated antibody that binds specifically to CD38 comprising a heavy chain variable region comprising the amino acid sequence of SEQ ID NO: 665 and a light chain variable region comprising the amino acid sequence of SEQ ID NO: 666. Also disclosed is an isolated antibody that binds specifically to CD38 comprises a heavy chain variable region comprising the amino acid sequence of SEQ ID NO: 739 and a light chain variable region comprising the amino acid sequence of SEQ ID NO: 664. The heavy chain variable region amino acid sequence of SEQ ID NO: 559 excludes the amino acid sequence of SEQ ID NO: 13. The light chain variable region amino acid sequence of SEQ ID NO: 664 excludes the amino acid sequence of SEQ ID NO: 14. Also disclosed is an isolated antibody that binds specifically to CD38 comprises a heavy chain CDR1 comprising the amino acid sequence of, SEQ ID NO: 200, SEQ ID NO: 514 or SEQ ID NO: 697, a heavy chain CDR2 comprising the amino acid sequence of SEQ ID NO: 202, SEQ ID NO: 516, SEQ ID NO: 544, SEQ ID NO: 698 or SEQ ID NO: 737, and a heavy chain CDR3 comprising the amino acid sequence of SEQ ID NO: 204, SEQ ID NO: 222, SEQ ID NO: 518, SEQ ID NO: 534, SEQ ID NO: 535, SEQ ID NO: 536, SEQ ID NO: 699 or SEQ ID NO: 738, and may further comprise a light chain CDR1 comprising the amino acid sequence of SEQ ID NO: 233, SEQ ID NO: 319, SEQ ID NO: 583, SEQ ID NO: 590 or SEQ ID NO: 696, a light chain CDR2 comprising the amino acid sequence of SEQ ID NO: 235, SEQ ID NO: 307, SEQ ID NO: 311, SEQ ID NO: 585, SEQ ID NO: 591 or SEQ ID NO: 605, a light chain CDR3 comprising the amino acid sequence of SEQ ID NO: 237, SEQ ID NO: 321, SEQ ID NO: 324, SEQ ID NO: 587 or SEQ ID NO: 594.

The heavy chain variable region may comprise the amino acid sequence of SEQ ID NO: 34, SEQ ID NO: 18, SEQ ID NO: 665, SEQ ID NO: 165, SEQ ID NO: 166, SEQ ID NO: 167, SEQ ID NO: 179, SEQ ID NO: 180, SEQ ID NO: 197, SEQ ID NO: 152, SEQ ID NO: 720, SEQ ID NO: 721, SEQ ID NO: 722, SEQ ID NO: 723, SEQ ID NO: 739, SEQ ID NO: 740, SEQ ID NO: 741, SEQ ID NO: 742, SEQ ID NO: 728, SEQ ID NO: 730, SEQ ID NO: 731. The light chain variable region may comprise the amino acid sequence of SEQ ID NO: 65, SEQ ID NO: 68, SEQ ID NO: 86, SEQ ID NO: 88, SEQ ID NO: 92, SEQ ID NO: 93, SEQ ID NO: 660, SEQ ID NO: 661, SEQ ID NO: 662, SEQ ID NO: 663. SEQ ID NO: 161, SEQ ID NO: 184, SEQ ID NO: 188, SEQ ID NO: 198 or SEQ ID NO: 700, SEQ ID NO: 701, SEQ ID NO: 704, SEQ ID NO: 705, SEQ ID NO: 706, SEQ ID NO: 707, SEQ ID NO: 708, SEQ ID NO: 709, SEQ ID NO: 710, SEQ ID NO: 711.

The antibody preferably is capable of binding to CD38-positive cells. The antibody may bind to a CD38-positive cell with an EC50 value of less than about 100 nM. The antibody may bind to a CD38-positive cell with an EC₅₀ value of less than about 75 nM. The antibody may bind to a CD38-positive cell with an EC₅₀ value of less than about 50 nM. The antibody may bind to a CD38-positive cell with an EC₅₀ value of less than about 30 nM. The antibody may bind to a CD38-positive cell with an EC₅₀ value of less than about 25 nM. The antibody may bind to a CD38-positive cell with an EC₅₀ value of less than about 20 nM. The antibody may bind to a CD38-positive cell with an EC₅₀ value of less than about 15 nM. The antibody may bind to a CD38-positive cell with an EC₅₀ value of less than about 13 nM. The antibody may bind to a CD38-positive cell with an EC₅₀ value of less than about 10 nM.

The antibody may be a monoclonal antibody, and is preferably a fully human antibody. The antibody may comprise an FAb. The antibody may comprise a human IgG1 constant region or a human IgG4 constant region. The IgG1 or the IgG4 constant region may comprise a tyrosine at position 252, a threonine at position 254, and a glutamic acid at position 256 according to the EU numbering system. The IgG4 constant region may comprise a proline at position 228 according to the EU numbering system, and the proline at position 228 may be in addition to a tyrosine at position 252, a threonine at position 254, and a glutamic acid at position 256.

In some aspects, the antibody is fused to attenuated interferon alpha-2b. The interferon alpha-2b may comprise a substitution of the alanine at position 145 to glycine or aspartic acid, including an interferon alpha-2b having the amino acid sequence of SEQ ID NO: 649 or SEQ ID NO: 651. The attenuated interferon alpha-2b may be fused directly to the C-terminus of the IgG1 or IgG4 constant region, and the antibody may comprise the amino acid sequence of SEQ ID NO: 9, SEQ ID NO: 10, SEQ ID NO: 652, SEQ ID NO: 653, SEQ ID NO: 654, SEQ ID NO: 655, SEQ ID NO: 656, SEQ ID NO: 657, SEQ ID NO: 658, or SEQ ID NO: 694. The antibody, including the antibody fused to an attenuated interferon alpha-2b may be comprised in a composition comprising a pharmaceutically acceptable carrier.

Isolated polynucleotides encoding the antibody and the antibody fused to an attenuated interferon alpha-2b are disclosed herein. The polynucleotide may comprise the nucleic acid sequence of SEQ ID NO: 667, SEQ ID NO: 670, SEQ ID NO: 671, SEQ ID NO: 672, SEQ ID NO: 673, SEQ ID NO: 674, SEQ ID NO: 668, SEQ ID NO: 669, SEQ ID NO: 675, SEQ ID NO: 676, or SEQ ID NO: 677, SEQ ID NO: 678, SEQ ID NO: 679, SEQ ID NO: 680, SEQ ID NO: 681, SEQ ID NO: 682, SEQ ID NO: 683, SEQ ID NO: 684, SEQ ID NO: 685, SEQ ID NO: 686, SEQ ID NO: 687, SEQ ID NO: 688, SEQ ID NO: 689, SEQ ID NO: 690, SEQ ID NO: 691, SEQ ID NO: 692, SEQ ID NO: 693, SEQ ID NO: 695 SEQ ID NO: 702, SEQ ID NO: 703, SEQ ID NO: 712, SEQ ID NO: 713, SEQ ID NO: 714, SEQ ID NO: 715, SEQ ID NO: 716, SEQ ID NO: 717, SEQ ID NO: 718, SEQ ID NO: 719, SEQ ID NO: 724, SEQ ID NO: 725, SEQ ID NO: 726, SEQ ID NO: 727 SEQ ID NO: 732, SEQ ID NO: 733, SEQ ID NO: 734, SEQ ID NO: 735, SEQ ID NO: 743, SEQ ID NO: 744, SEQ ID NO: 745, SEQ ID NO: 746 . The polynucleotides may comprise a vector. The vector may be used, for example, to transform a cell. A transformed cell comprising such polynucleotides is also provided. The transformed cell may comprise a mammalian cell, a yeast cell, or an insect cell.

Stable cells that express the antibodies are alsodisclosed herein. Antibody-expressing cells may be mammalian cells. Preferred cells are Chinese Hamster Ovary (CHO) cells.

Kits comprising antibodies fused to attenuated interferon alpha-2b are disclosed herein. The kits comprise the anti-CD38-attenuated interferon alpha-2b fusion construct, and instructions for using the construct in a method for inhibiting the proliferation of a tumor cell expressing CD38 and a receptor for interferon alpha-2b on its surface, instructions for using the construct in a method for inducing apoptosis in a tumor cell expressing CD38 and a receptor for interferon alpha-2b on its surface, instructions for using the construct in a method for treating a tumor comprising cells expressing CD38 and a receptor for interferon alpha-2b on their surface in a subject in need thereof, and optionally, a pharmaceutically acceptable carrier. Kits comprising anti-CD38 antibodies are disclosed herein, and such kits comprise the anti-CD38 antibody and instructions for using the antibody in a method for detecting a CD38-positive tumor cell in a tissue sample isolated from a subject, the antibody may optionally be fused to an attenuated interferon alpha-2b protein.

The anti-CD38 antibody-attenuated interferon alpha-2b fusion constructs may be used as a threrapy in the treatment of a tumor comprising cells expressing CD38 and a receptor for interferon alpha-2b on their surface. Generally, a treatment method comprises administering to a subject having the tumor an anti-CD38 antibody-attenuated interferon alpha-2b fusion construct in an amount effective to treat the tumor. The construct may comprise any construct described or exemplified herein. The subject is preferably a mammal, more preferably a non-human primate, and most preferably a human being. The tumor may comprise a β-cell lymphoma, multiple myeloma, non-Hodgkin's lymphoma, chronic myelogenous leukemia, chronic lymphocytic leukemia or acute myelogenous leukemia.

The anti-CD38 antibodies, optionally fused to an attenuated interferon alpha-2b protein, may be used in a method for detecting CD38 or a CD38-positive tumor cell in a tissue sample isolated from a subject. Generally, the method comprises contacting an antibody that binds specifically to CD38 with a tissue sample isolated from a subject and detecting a complex of the antibody and CD38 or a CD38-positive cell in the tissue sample. The tissue sample may be known to have or be suspected of having CD38-positive tumor cells. The tissue may comprise blood or bone marrow. The CD38-positive tumor cell may be a CD38-positive β-cell lymphoma cell, multiple myeloma cell, non-Hodgkin's lymphoma cell, chronic myelogenous leukemia cell, chronic lymphocytic leukemia cell, or acute myelogenous leukemia cell. The subject is preferably a mammal, more preferably a non-human primate, and most preferably a human being. The method may include the step of isolating the tissue sample from the subject. The method may further comprise contacting the antibody with a tissue sample that does not include any CD38-positive cells, for example, to serve as a negative control.

### BRIEF DESCRIPTION OF THE DRAWINGS

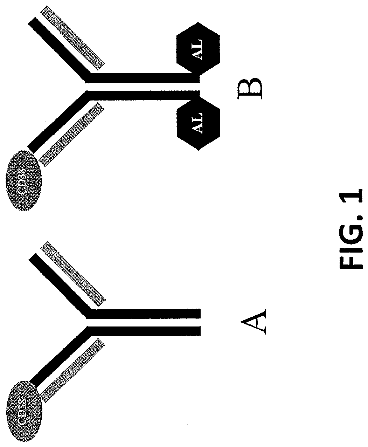

Figure 1 shows an example of an anti-CD38-attenuated interferon fusion construct.
Figures 2A and 2B show sequences of heavy chain variable regions of X02.1, related constructs, and the most homologous germline antibody sequence. CDRs defined by the Kabat numbering system are underlined.
Figures 3A and 3B show sequences of light chain variable regions of X02.1, related constructs, and the most homologous germline antibody sequence. CDRs defined by the Kabat numbering system are underlined.
Figures 4A-4D show the sequences of light chain variable regions of A02.1 and related constructs. CDRs defined by the Kabat numbering system are underlined
Figure 5 shows the consensus variable heavy chain sequence of A02.1 and related constructs. Boxed regions contain CDRs (as indicated) as defined by the Kabat numbering system and the enhanced Chothia numbering system. CDRs defined by the Kabat numbering system are shown in bold. CDRs defined by the enhanced Chothia numbering system are underlined.
Figure 6 shows the consensus variable light chain sequence of A02.1 and related constructs . Boxed regions contain CDRs (as indicated) as defined by the Kabat numbering system and the enhanced Chothia numbering system. CDRs defined by the Kabat numbering system are shown in bold. CDRs defined by the enhanced Chothia numbering system are underlined
Figures 7A-7C show sequences of heavy chain variable regions of humanized heavy chain variable regions. CDRs defined by the Kabat numbering system are underlined.
Figures 8A-8C show sequences of heavy chain variable regions of humanized light chain variable regions. CDRs defined by the Kabat numbering system are underlined.
Figures 9A and 9B show the variable heavy chain of A10.0 and related constructs. CDRs defined by the Kabat numbering system are underlined.
Figures 10A and 10B show the variable light chain of A10.0 and related constructs. CDRs defined by the Kabat numbering system are underlined.
Figure 11 shows the variable heavy chain consensus sequence of A10.0 and related constructs. Boxed regions contain CDRs (as indicated) as defined by the Kabat numbering system and the enhanced Chothia numbering system. CDRs defined by the Kabat numbering system are shown in bold. CDRs defined by the enhanced Chothia numbering system are underlined.
Figure 12 shows the variable light chain consensus sequence of A10.0 and related constructs. Boxed regions contain CDRs (as indicated) as defined by the Kabat numbering system and the enhanced Chothia numbering system. CDRs defined by the Kabat numbering system are shown in bold. CDRs defined by the enhanced Chothia numbering system are underlined.

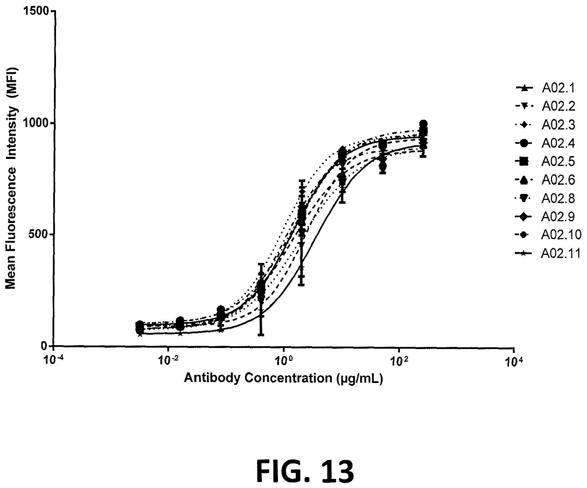

Figure 13 shows the binding activity of A02.1 variants to the CD38-expressing multiple myeloma cell line ARP-1 as measured by flow cytometry. The assay details are described in the Examples of this specification.

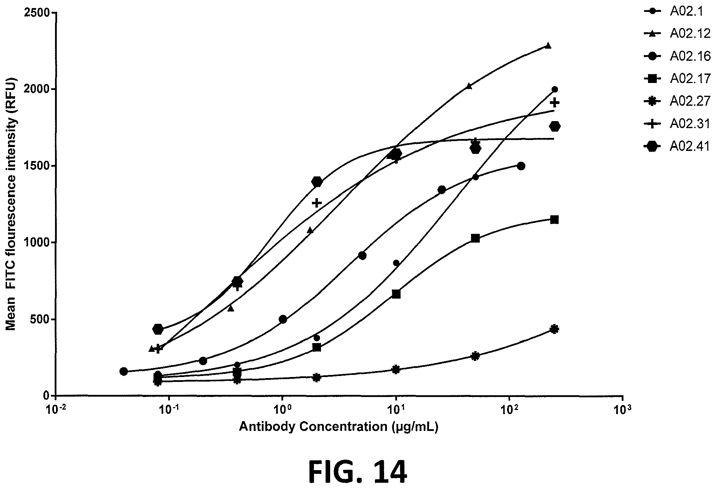

Figure 14 shows the binding activity of A02.1 variants to the CD38-expressing multiple myeloma cell line NCI-H929 as measured by flow cytometry. The assay details are described in the Examples of this specification.

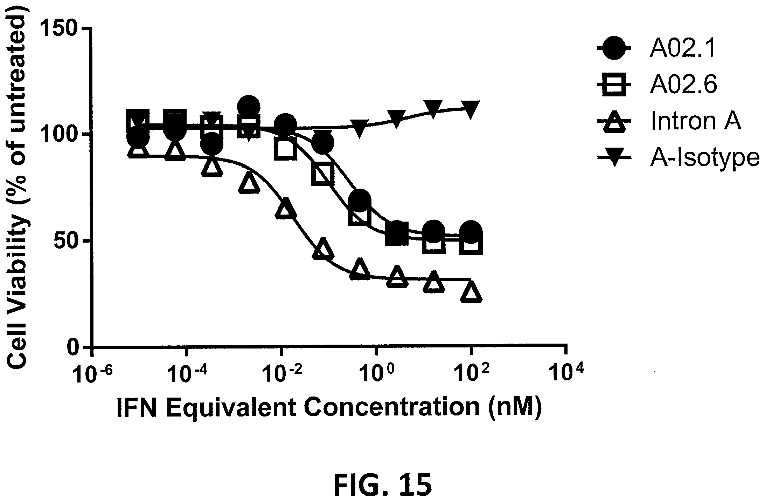

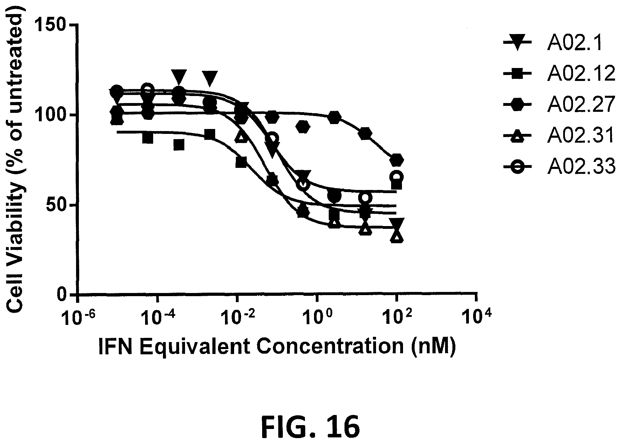

Figures 15 and 16 show the anti-proliferative activity of A02.1 variants on the multiple myeloma cell line ARP-1. A-isotype is an irrelevant specificity antibody fused with the attenuated interferon as a control. The assay details are described in the Examples (Cell proliferation assay).

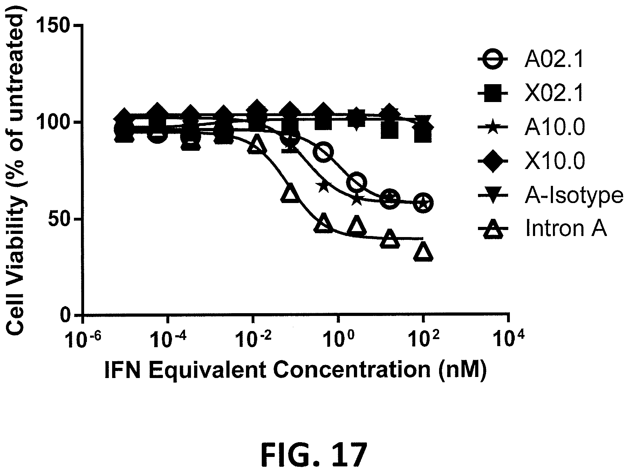

Figure 17 shows the anti-proliferative activity of IFN-alpha2b (Intron A) compared with A02.1 and A10.0 and their corresponding unfused antibodies X02.1 and X10.0 on the multiple myeloma cell line ARP-1. A-isotype is an irrelevant specificity antibody fused with the attenuated interferon as a control. The assay details are described in the Examples (Cell proliferation assay).
Figure 18 shows the relative fold change of Annexin V production in the CD38-expressing multiple myeloma cell line NCI-H929 when treated with A02.1 and A10.0 and their corresponding unfused antibodies X02.1 and X10.0 for 24 hours compared to an untreated control. A-isotype is an irrelevant specificity antibody fused with the attenuated interferon as a control. The assay details are described in the Examples (Annexin V assay).

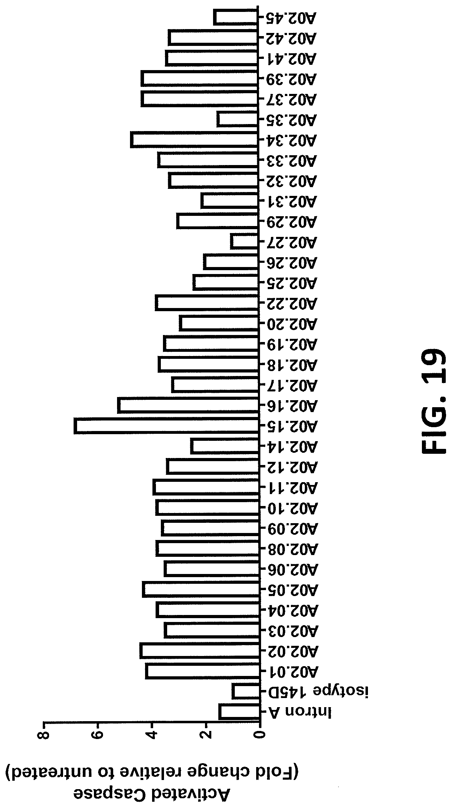

Figure 19 shows the relative fold change of caspase activation in the CD38-expressing multiple myeloma cell line H929 of IFN-alpha2b (Intron A) vs. A02.1 and related constructs in comparison to untreated cells. Isotype 145D is an irrelevant specificity antibody fused with the attenuated interferon as a control. The assay details are described in the Examples (Caspase assay).

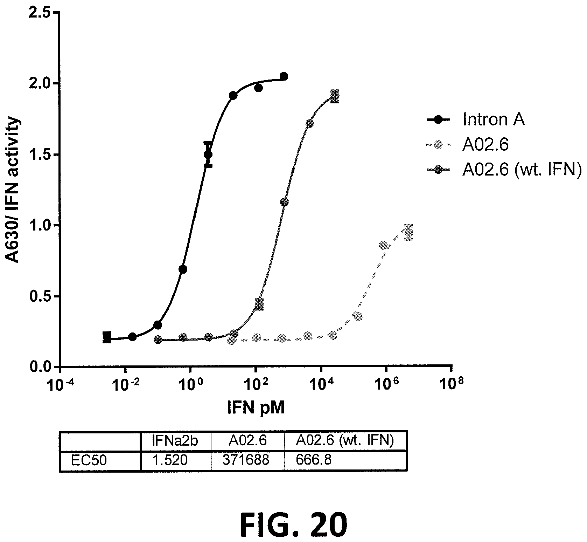

Figure 20 shows the off target activity of IFN-alpha2b (Intron A) versus A02.6 and A02.6 fused to wild-type IFN-alpha2b (A02.6 (wt. IFN)) on the CD38-negative cells. The assay details are described in the Examples (HEK-BLUE™).

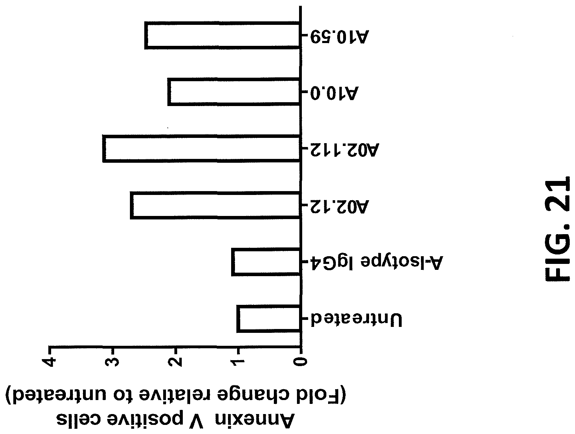

Figure 21 shows the relative fold change of Annexin V production in the CD38-expressing multiple myeloma cell line H929 between IgG1 and IgG4 subtypes of anti-CD38-attenuated IFN-alpha fusion protein constructs. A-isotype is a non-specific IgG4 antibody fused with the attenuated interferon as a control. The antibodies, A02.12 and A10.0 contain IgG4 constant regions fused to attenuated IFN-alpha while A02.112 and A10.59 contain IgG1 constant regions fused to attenuated IFN-alpha. The assay details are described in the Examples (Annexin V/7AAD assay).

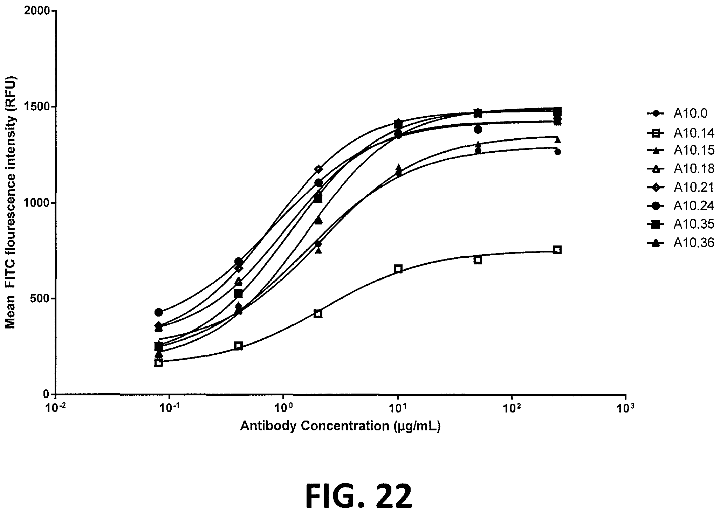

Figure 22 shows the binding activity of A10.0 variants to the CD38-expressing multiple myeloma cell line NCI-H929 as measured by flow cytometry. The assay details are described in the Examples of this specification.

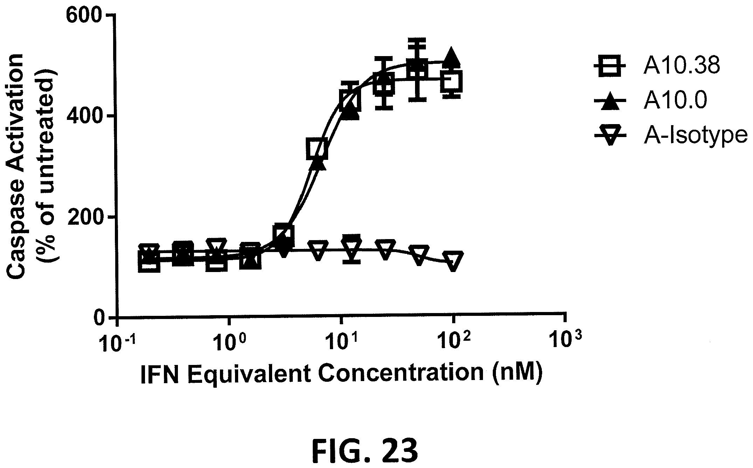

Figure 23 shows caspase activation in the CD38-expressing multiple myeloma cell line H929 of A10.0 and A10.38 compared to untreated cells. A-isotype is an irrelevant specificity antibody fused to the attenuated IFN as a control. The assay details are described in the Examples (Caspase assay).

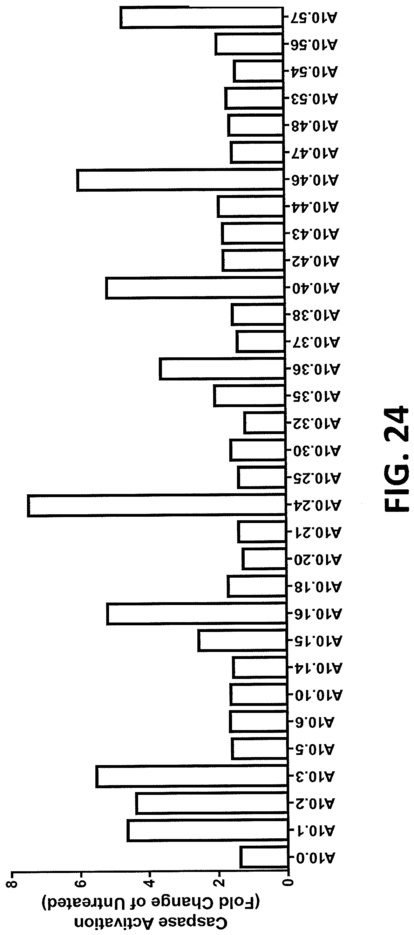

Figure 24 shows the relative fold change of caspase activation in the CD38 expressing multiple myeloma cell line H929 by A10.0 variants compared to untreated cells. The assay details are described in the Examples (Caspase assay).

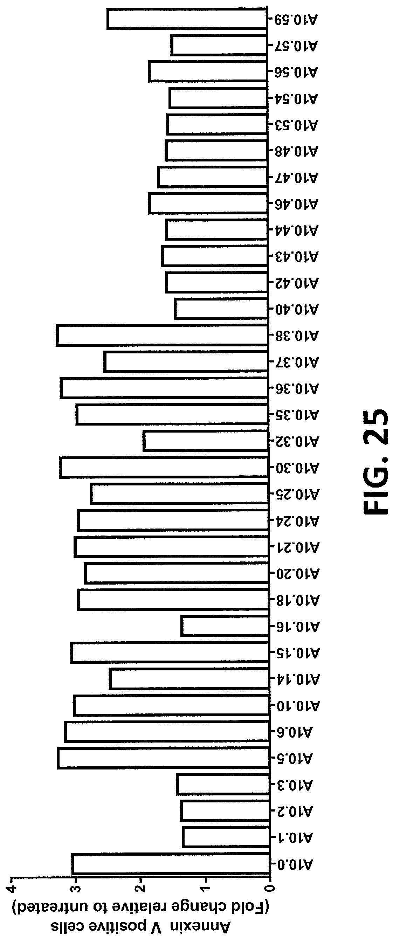

Figure 25 shows the relative fold change of production of Annexin V in the CD38-expressing multiple myeloma cell line H929 by A10.0 variants. The assay details are described in the Examples (Annexin V/7AAD assay).

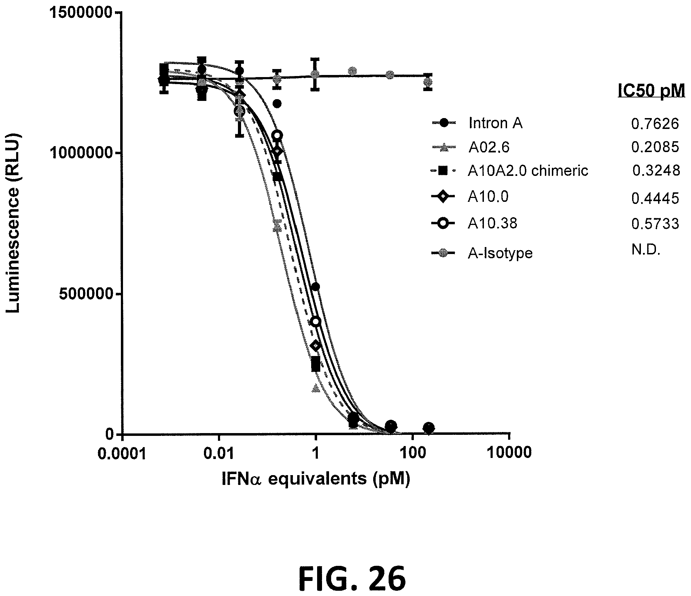

Figure 26 shows the anti-proliferative activity of IFN-alpha2b (Intron A) compared with A02.6, A10.0, A10.38 and parental A10A2.0 chimeric antibody constructs on the Burkitt's lymphoma cell line Daudi. A-isotype is an irrelevant specificity antibody fused to the attenuated IFN as a control. The assay details are described in the Examples (Cell proliferation assay).
Figure 27 shows the effects of humanized A10.0 versus the parental A10A2.0 chimeric antibody attenuated interferon construct on the growth of subcutaneous H929 myeloma tumors in SCID mice. The bar labeled "treatment phase" shows the duration of treatment with the compounds.
Figure 28 shows the non-antibody antigen targeted IFN activity of A10.0 variants fused to the same attenuated IFN-alpha2b protein. The assay details are described in the Examples ("Off-target assays"- iLite gene reporter assay).
Figure 29 shows the "Off-target" activity of IFN-alpha2b (Intron A) compared with A10.0 variants and the parental A10A2.0 chimeric antibody fused to wild-type IFN-alpha2b (A10A2.0 chimeric (wt.IFN)). The assay details are described in the Examples ("Off-target assays"- HEK-BLUE™).
Figure 30 shows variable heavy chain consensus sequences of X910/12-HC-L0-Interferon-alpha (A145D) IgG4 and related sequences. Boxed regions contain CDRs (as indicated) as defined by the Kabat numbering system and the enhanced Chothia numbering system. CDRs defined by the Kabat numbering system are shown in bold. CDRs defined by the enhanced Chothia numbering system are underlined.

### DETAILED DESCRIPTION OF THE DISCLOSURE

Various terms relating to aspects of disclosure are used throughout the specification and claims. Such terms are to be given their ordinary meaning in the art, unless otherwise indicated. Other specifically defined terms are to be construed in a manner consistent with the definition provided herein.

The terms subject and patient are used interchangeably and include any animal. Mammals are preferred, including companion and farm mammals, as well as rodents, including mice, rabbits, and rats, and other rodents. Non-human primates, such as Cynomolgus monkeys, are more preferred, and human beings are highly preferred.

A molecule such as an antibody has been "isolated" if it has been altered and/or removed from its natural environment by the hand of a human being.

As used herein, the singular forms "a," "an," and "the" include plural referents unless expressly stated otherwise.

An anti-CD38 antibody-attenuated interferon alpha-2b fusion construct includes, but is not limited to, any antibody described or exemplified herein that binds specifically to CD38 that is fused to an attenuated interferon alpha-2b protein, including an interferon alpha-2b of SEQ ID NO: 647, SEQ ID NO: 648, SEQ ID NO: 649, SEQ ID NO: 650, or SEQ ID NO: 651. In some aspects, fusing an unmutated interferon alpha-2b protein, such as SEQ ID NO: 7, to an anti-CD38 antibody attenuates the biologic activities of the interferon molecule. In this disclosure, attenuated interferon, attenuated interferon alpha-2b, IFN-alpha2b A145D, and IFN-alpha2b A145G are used interchangeably.

Specificity is not necessarily an absolute designation but may constitute a relative term signifying the degree of selectivity of an antibody IFN-alpha fusion protein construct for an antigen-positive cell compared to an antigen-negative cell. Specificity of an antibody IFN-alpha fusion protein construct for an antigen-positive cell is mediated by the variable regions of the antibody, and usually by the complementarity determining regions (CDRs) of the antibody. A construct may have 100-fold specificity for antigen-positive cells compared to antigen-negative cells.

Human CD38 comprises the amino acid sequence of SEQ ID NO: 1, and cynomolgus monkey CD38 comprises the amino acid sequence of SEQ ID NO: 2.

It has been further observed that interferon-alpha2b can be attenuated in terms of its biologic activity which is mediated through the interferon binding to an interferon receptor on a cell surface by introducing certain amino acid changes into the protein sequence. An attenuated interferon molecule can be fused to antibodies that specifically bind to CD38, such that the antibody may serve as a delivery vehicle for the attenuated interferon to CD38-positive cells with a resulting diminution of off target interferon activity caused by the attenuated interferon molecule. It has been further observed that fusing the attenuated interferon to the CD38 antibodies does not significantly affect the capacity of the antibody to specifically bind to CD38 on cells expressing CD38, including cells in the body of animals. It has been further observed that variants of the CD38 antibodies can be engineered and expressed such that the antibodies have reduced immunogenicity and enhanced stability and half life without a significant loss of specificity or affinity of the antibody to the CD38 antigen. These variant antibodies can be fused to an attenuated interferon.

Accordingly, antibodies that specifically bind to CD38 are featured. It has also been observed that such anti-CD38 antibodies may be employed as delivery vehicles for attenuated ligands such as interferon alpha. Without intending to be limited to any particular theory or mechanism of action, it is believed that the antibodies direct the interferon alpha to which they are attached to CD38-positive cells, where the interferon may interact with its receptor. It is believed that the antibody, as a delivery vehicle, compensates for the diminished capacity of the interferon molecule to bind to its receptor. In this sense, the attenuated interferon has reduced capacity to interact with its receptor on healthy cells, and particularly cells that do not express CD38. It is believed that by bringing the attenuated interferon into proximity with its receptor on CD38-positive cells, the antibodies may enhance the capacity of the attenuated interferon to bind to its relevant receptor and induce a therapeutic effect, while exhibiting a diminished capacity to induce undesirable effects on healthy cells that do not express CD38.

The antibodies may be polyclonal, but in some aspects, are not polyclonal. The antibodies preferably are monoclonal. The antibodies are preferably full length antibodies. Full length antibodies generally comprise a variable region heavy chain and a variable region light chain. The antibodies may comprise derivatives or fragments or portions of antibodies that retain the antigen-binding specificity, and also preferably retain most or all of the affinity, of the parent antibody molecule (*e.g.,* for CD38). For example, derivatives may comprise at least one variable region (either a heavy chain or light chain variable region). Other examples of suitable antibody derivatives and fragments include, without limitation, antibodies with polyepitopic specificity, bispecific antibodies, multi-specific antibodies, diabodies, single-chain molecules, as well as FAb, F(Ab')2, Fd, Fabc, and Fv molecules, single chain (Sc) antibodies, single chain Fv antibodies (scFv), individual antibody light chains, individual antibody heavy chains, fusions between antibody chains and other molecules, heavy chain monomers or dimers, light chain monomers or dimers, dimers consisting of one heavy and one light chain, and other multimers. Single chain Fv antibodies may be multivalent. All antibody isotypes may be used to produce antibody derivatives, fragments, and portions. Antibody derivatives, fragments, and/or portions may be recombinantly produced and expressed by any cell type, prokaryotic or eukaryotic.

In some embodiments an isolated antibody may refer to a monoclonal antibody to which IFN-alpha, or an attenuated IFN-alpha, has been fused to the C-terminus of the heavy chain IgG constant region. When the monoclonal antibody has a binding specificity to CD38 and the IFN-alpha is attenuated IFN-alpha 2b, the isolated antibody is also referred to as an Anti-CD38 attenuated IFN-alpha fusion protein, or an Anti-CD38 attenuated IFN-alpha fusion construct herein.

In a full-length antibody, each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FWR or FR). Each VH and VL is composed of three CDRs and four FWRs, arranged from amino-terminus to carboxy-terminus in the following order: FWR1, CDR1, FWR2, CDR2, FWR3, CDR3, FWR4. Typically, the antigen binding properties of an antibody are less likely to be disturbed by changes to FWR sequences than by changes to the CDR sequences. Immunoglobulin molecules can be of any type (*e.g*., IgG, IgE, IgM, IgD, IgA and IgY), class (*e.g.*, IgG1, IgG2, IgG3, IgG4, IgAI and IgA2) or subclass.

The antibodies may be derived from any species. For example, the antibodies may be mouse, rat, goat, horse, swine, bovine, camel, chicken, rabbit, donkey, llama, dromedary, shark, or human antibodies, as well as antibodies from any other animal species. For use in the treatment of humans, non-human derived antibodies may be structurally altered to be less antigenic upon administration to a human patient, including by chimerization or humanization or superhumanization.

The antibodies may be humanized antibodies. Humanized antibodies are those wherein the amino acids directly involved in antigen binding, *e.g.,* the complementarity determining regions (CDR), and in some cases the framework regions (FWR), or portions thereof, of the heavy and/or light chains are not of human origin, while the rest of the amino acids in the antibody are human or otherwise of human origin, *e.g.,* a human antibody scaffold. Humanized antibodies also include antibodies in which one or more residues of the human protein are modified by one or more amino acid substitutions and/or one or more FWR residues of the human protein are replaced by corresponding non-human residues. Humanized antibodies may also comprise residues which are found in neither the human antibody or in the non-human antibody. A humanized antibody may be a super-humanized antibody, *e.g.,* as described in U.S. Pat. No. 7,732,578. The antibodies may be humanized chimeric antibodies.

It is highly preferred if the antibodies are fully human. Fully human antibodies are those where the whole molecule is human or otherwise of human origin, or includes an amino acid sequence identical to a human form of the antibody. Fully human antibodies include those obtained from a human V gene library, for example, where human genes encoding variable regions of antibodies are recombinantly expressed. Fully human antibodies may be expressed in other organisms (*e.g.,* mice and xenomouse technology) or cells from other organisms transformed with genes encoding human antibodies. Fully human antibodies may nevertheless include amino acid residues not encoded by human sequences, *e.g.,* mutations introduced by random or site directed mutations.

The antibodies may be full length antibodies of any class, for example, IgG1, IgG2 or IgG4. The constant domains of such antibodies are preferably human. The variable regions of such antibodies may be of non-human origin, or preferably are human in origin or are humanized. Antibody fragments may also be used in place of the full length antibodies.

The antibodies may be minibodies. Minibodies comprise small versions of whole antibodies, which encode in a single chain the essential elements of a whole antibody. For example, the minibody may be comprised of the VH and VL domains of a native antibody fused to the hinge region and CH3 domain of an immunoglobulin molecule.

The antibody may comprise non-immunoglobulin derived protein frameworks. For example, reference may be made to (Ku & Schutz, Proc. Natl. Acad. Sci. USA 92: 6552-6556, 1995) which describes a four-helix bundle protein cytochrome b562 having two loops randomized to create CDRs, which have been selected for antigen binding.

Natural sequence variations may exist among heavy and light chains and the genes encoding them, and therefore, persons having ordinary skill in the art would expect to find some level of variation within the amino acid sequences, or the genes encoding them, of the antibodies described and exemplified herein. These variants preferably maintain the unique binding properties (*e.g.,* specificity and affinity) of the parent antibody. Such an expectation is due in part to the degeneracy of the genetic code, as well as to the known evolutionary success of conservative amino acid sequence variations, which do not appreciably alter the nature of the encoded protein. Accordingly, such variants and homologs are considered substantially the same as one another and are included within the scope of the disclosure. The antibodies thus include variants having single or multiple amino acid substitutions, deletions, additions, or replacements that retain the biological properties (*e.g.,* binding specificity and binding affinity) of the parent antibodies. The variants are preferably conservative, but may be non-conservative.

Amino acid positions assigned to CDRs and FWRs may be defined according to Kabat Sequences of Proteins of Immunological Interest, National Institutes of Health, Bethesda, Md., 1987 and 1991 (also referred to herein as the Kabat numbering system). In addition, the amino acid positions assigned to CDRs and FWRs may be defined according to the Enhanced Chothia Numbering Scheme (http://www.bioinfo.org.uk/mdex.html). The heavy chain constant region of an antibody can be defined by the EU numbering system (Edelman, GM et al. (1969)., Proc. Natl. Acad. USA, 63, 78-85).

According to the numbering system of Kabat, VH FWRs and CDRs may be positioned as follows: residues 1-30 (FWR1), 31-35 (CDR1), 36-49 (FWR2), 50-65 (CDR2), 66-94 (FWR3), 95-102 (CDR3) and 103-113 (FWR4), and VL FWRs and CDRs are positioned as follows: residues 1-23 (FWR1), 24-34 (CDR1), 35-49 (FWR2), 50-56 (CDR2), 57-88 (FWR3), 89-97 (CDR3) and 98-107 (FWR4). In some instances, variable regions may increase in length and according to the Kabat numbering system some amino acids may be designated by a number followed by a letter. This specification is not limited to FWRs and CDRs as defined by the Kabat numbering system, but includes all numbering systems, including the canonical numbering system or of Chothia et al. (1987) J. Mol. Biol. 196:901-17; Chothiaet al. (1989) Nature 342:877-83; and/or Al-Lazikani et al. (1997) J. Mol. Biol. 273:927-48; the numbering system of Honnegher et al. (2001) J. Mol. Biol., 309:657-70; or the IMGT system discussed in Giudicelli et al., (1997) Nucleic Acids Res. 25:206-11. The CDRs may be defined according to the Kabat numbering system.

For any of the heavy chain CDR2 subdomains described herein, according to the Kabat numbering system, the five C-terminal amino acids may not participate directly in antigen binding, and accordingly, it will be understood that any one or more of these five C-terminal amino acids may be substituted with another naturally-occurring amino acid without substantially adversely affecting antigen binding. For any of the light chain CDR1 subdomains described herein, according to the Kabat numbering system, the four N-terminal amino acids may not participate directly in antigen binding, and accordingly, it will be understood that any one or more of these four amino acids may be substituted with another naturally-occurring amino acid without substantially adversely affecting antigen binding. For example, as described by Padlan et al. (1995) FASEB J. 9:133-139, the five C terminal amino acids of heavy chain CDR2 and/or the four N-terminal amino acids of light chain CDR1 may not participate in antigen binding. Both the heavy chain CDR2 and the light chain CDR1 may not directly participate in antigen binding.

Chemical analogues of amino acids may be used in the antibodies described and/or exemplified herein. The use of chemical analogues of amino acids is useful, for example, for stabilizing the molecules such as if required to be administered to a subject. The analogues of the amino acids contemplated herein include, but are not limited to, modifications of side chains, incorporation of unnatural amino acids and/or their derivatives during peptide, polypeptide or protein synthesis and the use of crosslinkers and other methods which impose conformational constraints on the proteinaceous molecule or their analogues.

The antibodies may comprise post-translational modifications or moieties, which may impact antibody activity or stability. These modifications or moieties include, but are not limited to, methylated, acetylated, glycosylated, sulfated, phosphorylated, carboxylated, and amidated moieties and other moieties that are well known in the art. Moieties include any chemical group or combinations of groups commonly found on immunoglobulin molecules in nature or otherwise added to antibodies by recombinant expression systems, including prokaryotic and eukaryotic expression systems.

Examples of side chain modifications contemplated by the disclosure include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation via O-acylisourea formation followed by subsequent derivation, for example, to a corresponding amide.

Sulphydryl groups may be modified by methods such as carboxymethylation with iodoacetic acid or iodoacetamide; performic acid oxidation to cysteic acid; formation of mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Crosslinkers may be used, for example, to stabilize 3D conformations of the antibodies and constructs, using homo-bifunctional crosslinkers such as the bifunctional imido esters having (CH₂)n spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH).

The antibodies may be affinity matured, or may comprise amino acid changes that decrease immunogenicity, for example, by removing predicted MHC class II-binding motifs. The therapeutic utility of the antibodies described herein may be further enhanced by modulating their functional characteristics, such as antibody-dependent cell-mediated cytotoxicity (ADCC), complement-dependent cytotoxicity (CDC), serum half-life, biodistribution and binding to Fc receptors or the combination of any of these. This modulation can be achieved by protein-engineering, glyco-engineering or chemical methods. Depending on the therapeutic application required, it could be advantageous to either increase or decrease any of these activities. An example of glyco-engineering used the Potelligent® method as described in Shinkawa T. et al. (2003) J. Biol. Chem. 278: 3466-73.

The antibodies may include modifications that modulate its serum half-life and biodistribution, including modifications that modulate the antibody's interaction with the neonatal Fc receptor (FcRn), a receptor with a key role in protecting IgG from catabolism, and maintaining high serum antibody concentration. Serum half-life modulating modifications may occur in the Fc region of IgG1 or IgG4, including the triple substitution of M252Y/S254T/T256E (Numbering according to the EU numbering system (Edelman, G.M. et al. (1969) Proc. Natl. Acad. USA 63, 78-85)), (*e.g.,* SEQ ID NO: 656, SEQ ID NO: 657, SEQ ID NO: 658, SEQ ID NO: 694), as described in U.S. Pat. No. 7,083,784. Other substitutions may occur at positions 250 and 428, see *e.g.,* U.S. Pat. No 7,217,797, as well as at positions 307, 380 and 434, see, *e.g.,* WO 00/42072. Examples of constant domain amino acid substitutions which modulate binding to Fc receptors and subsequent function mediated by these receptors, including FcRn binding and serum half-life, are described in U.S. Publ. Nos. 2009/0142340, 2009/0068175, and 2009/0092599. Naked antibodies may have the heavy chain C-terminal lysine omitted or removed to reduce heterogeneity. The substitution of S228P (EU numbering) in the human IgG4 can stabilize antibody Fab-arm exchange in vivo (Labrin et al. (2009) Nature Biotechnology 27:8; 767-773).

The glycans linked to antibody molecules are known to influence interactions of antibody with Fc receptors and glycan receptors and thereby influence antibody activity, including serum half-life. Hence, certain glycoforms that modulate desired antibody activities can confer therapeutic advantage. Methods for generating engineered glycoforms include but are not limited to those described in U.S. Pat. Nos. 6,602,684, 7,326,681, and 7,388,081 and PCT Publ. No. WO 08/006554. Alternatively, the antibody sequences may be modified to remove relevant glycoform attachment sites.

The antibodies may be labeled or conjugated to any chemical or biomolecule moieties. Labeled antibodies may find use in therapeutic, diagnostic, or basic research applications. Such labels/conjugates can be detectable, such as fluorochromes, radiolabels, enzymes, fluorescent proteins, and biotin. The labels/conjugates may be chemotherapeutic agents, toxins, isotopes, and other agents used for treating conditions such as the killing of cancer cells. Chemotherapeutic agents may be any which is suitable for the purpose to which the antibody is being used.

The antibodies may be derivatized by known protecting/blocking groups to prevent proteolytic cleavage or enhance activity or stability.

The antibodies preferably have a binding affinity for an epitope on CD38 that includes a dissociation constant (Kd) of less than about 1 x 10⁻² M. In some embodiments, the Kd is less than about 1 x 10⁻³ M. In other embodiments, the Kd is less than about 1 x 10⁻⁴ M. In some embodiments, the Kd is less than about 1 x 10⁻⁵ M. In still other embodiments, the Kd is less than about 1 x 10⁻⁶ M. In other embodiments, the Kd is less than about 1 x 10⁻⁷ M. In other embodiments, the Kd is less than about 1 x 10⁻⁸ M. In other embodiments, the Kd is less than about 1 x 10⁻⁹ M. In other embodiments, the Kd is less than about 1 x 10⁻¹⁰ M. In still other embodiments, the Kd is less than about 1 x 10⁻¹¹ M. In some embodiments, the Kd is less than about 1 x 10⁻¹² M. In other embodiments, the Kd is less than about 1 x 10⁻¹³ M. In other embodiments, the Kd is less than about 1 x 10⁻¹⁴ M. In still other embodiments, the Kd is less than about 1 x 10⁻¹⁵ M. Affinity values refer to those obtained by standard methodologies, including surface plasmon resonance such as Biacore™ analyses or analysis using an Octet® Red 96 (Forte Bio) Dip-and-Read system.

The antibodies may comprise a single chain Fv molecule (scFv), Fab, or full IgG. Disclosed herein are any such antibodies that comprise a heavy chain having an amino acid sequence having at least about 85%, at least about 90%, at least about 95%, at least about 96%, at least about 97%, at least about 98%, at least about 99%, or at least about 100% sequence identity with the amino acid sequence of SEQ ID NO: 659 or SEQ ID NO: 665 or SEQ ID NO: 736, provided that a heavy chain comprising the amino acid sequence of SEQ ID NO: 659 or variant thereof excludes the amino acid sequence of SEQ ID NO: 13. It will be understood that antibodies comprising amino acid changes in their heavy chain retain the capability to specifically bind to CD38. The retained CD38 specific binding activity (including affinity) is preferably about the same as the binding activity (including affinity) of an antibody without any amino acid changes in the heavy chain, although the binding activity (including affinity) may be lesser or greater than an antibody without any amino acid changes in the heavy chain. The antibody may comprise a light chain having an amino acid sequence having at least about 85%, at least about 90%, at least about 95%, at least about 96%, at least about 97%, at least about 98%, at least about 99%, or at least about 100% sequence identity with the amino acid sequence of SEQ ID NO: 664 or SEQ ID NO: 666, provided that a light chain comprising the amino acid sequence of SEQ ID NO: 664 or variant thereof excludes the amino acid sequence of SEQ ID NO: 14. It will be understood that antibodies comprising amino acid changes in their light chain retain the capability to specifically bind to CD38. The retained CD38 specific binding activity (including affinity) is preferably about the same as the binding activity (including affinity) of an antibody without any amino acid changes in the light chain, although the binding activity (including affinity) may be lesser or greater than an antibody without any amino acid changes in the light chain.

The heavy chain FWR1 may comprise the amino acid sequence of SEQ ID NO: 199, SEQ ID NO: 206, SEQ ID NO: 214, SEQ ID NO: 215, SEQ ID NO: 217, SEQ ID NO: 219, SEQ ID NO: 389, SEQ ID NO: 396, SEQ ID NO: 400, SEQ ID NO: 404, SEQ ID NO: 408, SEQ ID NO: 412, SEQ ID NO: 416, SEQ ID NO: 420, SEQ ID NO: 424, SEQ ID NO: 428, SEQ ID NO: 432, SEQ ID NO: 466, SEQ ID NO: 470, SEQ ID NO: 472, SEQ ID NO: 474, SEQ ID NO: 476, SEQ ID NO: 478, SEQ ID NO: 480, SEQ ID NO: 482, SEQ ID NO: 486, SEQ ID NO: 488, SEQ ID NO: 513, SEQ ID NO: 537, SEQ ID NO: 542, SEQ ID NO: 547, SEQ ID NO: 552, SEQ ID NO: 557, SEQ ID NO: 562, SEQ ID NO: 567, SEQ ID NO: 572, SEQ ID NO: 577 or SEQ ID NO: 748, and the heavy chain FWR1 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 199, SEQ ID NO: 214, SEQ ID NO: 215, SEQ ID NO: 217, SEQ ID NO: 219, SEQ ID NO: 389, SEQ ID NO: 396, SEQ ID NO: 400, SEQ ID NO: 404, SEQ ID NO: 408, SEQ ID NO: 412, SEQ ID NO: 416, SEQ ID NO: 420, SEQ ID NO: 424, SEQ ID NO: 428, SEQ ID NO: 432, SEQ ID NO: 466, SEQ ID NO: 470, SEQ ID NO: 472, SEQ ID NO: 474, SEQ ID NO: 476, SEQ ID NO: 478, SEQ ID NO: 480, SEQ ID NO: 482, SEQ ID NO: 486, SEQ ID NO: 488, SEQ ID NO: 513, SEQ ID NO: 537, SEQ ID NO: 542, SEQ ID NO: 547, SEQ ID NO: 552, SEQ ID NO: 557, SEQ ID NO: 562, SEQ ID NO: 567, SEQ ID NO: 572, SEQ ID NO: 577 or SEQ ID NO: 748. The heavy chain FWR2 may comprise the amino acid sequence of SEQ ID NO: 201, SEQ ID NO: 211, SEQ ID NO: 229, SEQ ID NO: 391, SEQ ID NO: 397, SEQ ID NO: 401, SEQ ID NO: 405, SEQ ID NO: 409, SEQ ID NO: 413, SEQ ID NO: 417, SEQ ID NO: 421, SEQ ID NO: 425, SEQ ID NO: 429, SEQ ID NO: 433, SEQ ID NO: 515 , SEQ ID NO: 520, SEQ ID NO: 521, SEQ ID NO: 522, SEQ ID NO: 523, SEQ ID NO: 524, SEQ ID NO: 525, SEQ ID NO: 538, SEQ ID NO: 543, SEQ ID NO: 548, SEQ ID NO: 553, SEQ ID NO: 558, SEQ ID NO: 563, SEQ ID NO: 568, SEQ ID NO: 573, SEQ ID NO: 578, SEQ ID NO: 749 or SEQ ID NO: 750, and the heavy chain FWR2 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 201, SEQ ID NO: 211, SEQ ID NO: 229, SEQ ID NO: 391, SEQ ID NO: 397, SEQ ID NO: 401, SEQ ID NO: 405, SEQ ID NO: 409, SEQ ID NO: 413, SEQ ID NO: 417, SEQ ID NO: 421, SEQ ID NO: 425, SEQ ID NO: 429, SEQ ID NO: 433, SEQ ID NO: 515 , SEQ ID NO: 520, SEQ ID NO: 521, SEQ ID NO: 522, SEQ ID NO: 523, SEQ ID NO: 524, SEQ ID NO: 525, SEQ ID NO: 538, SEQ ID NO: 543, SEQ ID NO: 548, SEQ ID NO: 553, SEQ ID NO: 558, SEQ ID NO: 563, SEQ ID NO: 568, SEQ ID NO: 573, SEQ ID NO: 578, SEQ ID NO: 749 or SEQ ID NO: 750. The heavy chain FWR3 may comprise the amino acid sequence of SEQ ID NO: 203, SEQ ID NO: 210, SEQ ID NO: 212, SEQ ID NO: 213, SEQ ID NO: 216, SEQ ID NO: 218, SEQ ID NO: 221, SEQ ID NO: 226, SEQ ID NO: 227, SEQ ID NO: 230, SEQ ID NO: 393, SEQ ID NO: 399, SEQ ID NO: 403, SEQ ID NO: 407, SEQ ID NO: 411, SEQ ID NO: 415, SEQ ID NO: 419, SEQ ID NO: 423, SEQ ID NO: 427, SEQ ID NO: 431, SEQ ID NO: 435, SEQ ID NO: 468, SEQ ID NO: 517, SEQ ID NO: 530, SEQ ID NO: 531, SEQ ID NO: 532, SEQ ID NO: 533, SEQ ID NO: 540, SEQ ID NO: 545, SEQ ID NO: 550, SEQ ID NO: 555, SEQ ID NO: 560, SEQ ID NO: 565, SEQ ID NO: 570, SEQ ID NO: 575, SEQ ID NO: 580, SEQ ID NO: 751 or SEQ ID NO: 752 and the heavy chain FWR3 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 203, SEQ ID NO: 210, SEQ ID NO: 212, SEQ ID NO: 213, SEQ ID NO: 216, SEQ ID NO: 218, SEQ ID NO: 221, SEQ ID NO: 226, SEQ ID NO: 227, SEQ ID NO: 230, SEQ ID NO: 393, SEQ ID NO: 399, SEQ ID NO: 403, SEQ ID NO: 407, SEQ ID NO: 411, SEQ ID NO: 415, SEQ ID NO: 419, SEQ ID NO: 423, SEQ ID NO: 427, SEQ ID NO: 431, SEQ ID NO: 435, SEQ ID NO: 468, SEQ ID NO: 517, SEQ ID NO: 530, SEQ ID NO: 531, SEQ ID NO: 532, SEQ ID NO: 533, SEQ ID NO: 540, SEQ ID NO: 545, SEQ ID NO: 550, SEQ ID NO: 555, SEQ ID NO: 560, SEQ ID NO: 565, SEQ ID NO: 570, SEQ ID NO: 575, SEQ ID NO: 580 ,SEQ ID NO: 751 or SEQ ID NO: 752. The heavy chain FWR4 may comprise the amino acid sequence of SEQ ID NO: 205, SEQ ID NO: 395, SEQ ID NO: 519, SEQ ID NO: 541, SEQ ID NO: 546, SEQ ID NO: 551, SEQ ID NO: 556, SEQ ID NO: 561, SEQ ID NO: 566, SEQ ID NO: 571, SEQ ID NO: 576, SEQ ID NO: 581 or SEQ ID NO: 753, and the heavy chain FWR4 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 205, SEQ ID NO: 395, SEQ ID NO: 519, SEQ ID NO: 541, SEQ ID NO: 546, SEQ ID NO: 551, SEQ ID NO: 556, SEQ ID NO: 561, SEQ ID NO: 566, SEQ ID NO: 571, SEQ ID NO: 576, SEQ ID NO: 581 or SEQ ID NO: 753. It will be understood that antibodies comprising amino acid changes in the heavy chain framework region(s) (FWR1, FWR2, FWR3, and/or FWR4) retain the capability to specifically bind to CD38. The retained CD38 specific binding activity (including affinity) is preferably about the same as the binding activity (including affinity) of an antibody without any amino acid changes in any heavy chain framework region(s), although the binding activity (including affinity) may be lesser or greater than an antibody without any amino acid changes in any heavy chain framework region(s).

The heavy chain CDR1 may comprise the amino acid sequence of SEQ ID NO: 200, SEQ ID NO: 224, SEQ ID NO: 390, SEQ ID NO: 514, SEQ ID NO: 526, SEQ ID NO: 527, SEQ ID NO: 528, SEQ ID NO: 529, or SEQ ID NO: 697 and the heavy chain CDR1 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 200, SEQ ID NO: 224, SEQ ID NO: 390, SEQ ID NO: 514, SEQ ID NO: 526, SEQ ID NO: 527, SEQ ID NO: 528, SEQ ID NO: 529, or SEQ ID NO: 697. The heavy chain CDR2 may comprise the amino acid sequence of SEQ ID NO: 202, SEQ ID NO: 392, SEQ ID NO: 398, SEQ ID NO: 402, SEQ ID NO: 406, SEQ ID NO: 410, SEQ ID NO: 414, SEQ ID NO: 418, SEQ ID NO: 422, SEQ ID NO: 426, SEQ ID NO: 430, SEQ ID NO: 434, SEQ ID NO: 467, SEQ ID NO: 471, SEQ ID NO: 473, SEQ ID NO: 475, SEQ ID NO: 477, SEQ ID NO: 479, SEQ ID NO: 481, SEQ ID NO: 483, SEQ ID NO: 485, SEQ ID NO: 487, SEQ ID NO: 489, SEQ ID NO: 516, SEQ ID NO: 539, SEQ ID NO: 544, SEQ ID NO: 549, SEQ ID NO: 554, SEQ ID NO: 559, SEQ ID NO: 564, SEQ ID NO: 569, SEQ ID NO: 574, SEQ ID NO: 579, SEQ ID NO: 698 or SEQ ID NO: 737 and the heavy chain CDR2 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 202, SEQ ID NO: 392, SEQ ID NO: 398, SEQ ID NO: 402, SEQ ID NO: 406, SEQ ID NO: 410, SEQ ID NO: 414, SEQ ID NO: 418, SEQ ID NO: 422, SEQ ID NO: 426, SEQ ID NO: 430, SEQ ID NO: 434, SEQ ID NO: 467, SEQ ID NO: 471, SEQ ID NO: 473, SEQ ID NO: 475, SEQ ID NO: 477, SEQ ID NO: 479, SEQ ID NO: 481, SEQ ID NO: 483, SEQ ID NO: 485, SEQ ID NO: 487, SEQ ID NO: 489, SEQ ID NO: 516, SEQ ID NO: 539, SEQ ID NO: 544, SEQ ID NO: 549, SEQ ID NO: 554, SEQ ID NO: 559, SEQ ID NO: 564, SEQ ID NO: 569, SEQ ID NO: 574, SEQ ID NO: 579, SEQ ID NO: 698 or SEQ ID NO: 737. The heavy chain CDR3 may comprise the amino acid sequence of SEQ ID NO: 204, SEQ ID NO: 220, SEQ ID NO: 222, SEQ ID NO: 223, SEQ ID NO: 228, SEQ ID NO: 231, SEQ ID NO: 394, SEQ ID NO: 469, SEQ ID NO: 518, SEQ ID NO: 534, SEQ ID NO: 535, SEQ ID NO: 536, SEQ ID NO: 699 or SEQ ID NO: 738 and the heavy chain CDR3 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 204, SEQ ID NO: 220, SEQ ID NO: 222, SEQ ID NO: 223, SEQ ID NO: 228, SEQ ID NO: 231, SEQ ID NO: 394, SEQ ID NO: 469, SEQ ID NO: 518, SEQ ID NO: 534, SEQ ID NO: 535, SEQ ID NO: 536, SEQ ID NO: 699 or SEQ ID NO: 738. It will be understood that antibodies comprising amino acid changes in the heavy chain complementarity determining region(s) (CDR1, CDR2, and/or CDR3) retain the capability to specifically bind to CD38. The retained CD38 specific binding activity (including affinity) is preferably about the same as the binding activity (including affinity) of an antibody without any amino acid changes in any heavy chain complementarity determining region(s), although the binding activity (including affinity) may be lesser or greater than an antibody without any amino acid changes in any heavy chain complementarity determining region(s).

The light chain FWR1 may comprise the amino acid sequence of SEQ ID NO: 232, SEQ ID NO: 247, SEQ ID NO: 259, SEQ ID NO: 260, SEQ ID NO: 261, SEQ ID NO: 436, SEQ ID NO: 443, SEQ ID NO: 447, SEQ ID NO: 451, SEQ ID NO: 455, SEQ ID NO: 459, SEQ ID NO: 463, SEQ ID NO: 490, SEQ ID NO: 497, SEQ ID NO: 501, SEQ ID NO: 509, SEQ ID NO: 582, SEQ ID NO: 607, SEQ ID NO: 614, SEQ ID NO: 618, SEQ ID NO: 622, SEQ ID NO: 626, SEQ ID NO: 630, SEQ ID NO: 634 or SEQ ID NO: 638 and the light chain FWR1 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 232, SEQ ID NO: 247, SEQ ID NO: 259, SEQ ID NO: 260, SEQ ID NO: 261, SEQ ID NO: 436, SEQ ID NO: 443, SEQ ID NO: 447, SEQ ID NO: 451, SEQ ID NO: 455, SEQ ID NO: 459, SEQ ID NO: 463, SEQ ID NO: 490, SEQ ID NO: 497, SEQ ID NO: 501, SEQ ID NO: 509, SEQ ID NO: 582, SEQ ID NO: 607, SEQ ID NO: 614, SEQ ID NO: 618, SEQ ID NO: 622, SEQ ID NO: 626, SEQ ID NO: 630, SEQ ID NO: 634 or SEQ ID NO: 638. The light chain FWR2 may comprise the amino acid sequence of SEQ ID NO: 234, SEQ ID NO: 246, SEQ ID NO: 248, SEQ ID NO: 281, SEQ ID NO: 283, SEQ ID NO: 285, SEQ ID NO: 287, SEQ ID NO: 289, SEQ ID NO: 291, SEQ ID NO: 293, SEQ ID NO: 295, SEQ ID NO: 297, SEQ ID NO: 438, SEQ ID NO: 444, SEQ ID NO: 448, SEQ ID NO: 452, SEQ ID NO: 456, SEQ ID NO: 460, SEQ ID NO: 464, SEQ ID NO: 492, SEQ ID NO: 498, SEQ ID NO: 502, SEQ ID NO: 506, SEQ ID NO: 510, SEQ ID NO: 584, SEQ ID NO: 592, SEQ ID NO: 593, SEQ ID NO: 609, SEQ ID NO: 615, SEQ ID NO: 619, SEQ ID NO: 623, SEQ ID NO: 627, SEQ ID NO: 631, SEQ ID NO: 635 or SEQ ID NO: 639 and the light chain FWR2 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 234, SEQ ID NO: 246, SEQ ID NO: 248, SEQ ID NO: 281, SEQ ID NO: 283, SEQ ID NO: 285, SEQ ID NO: 287, SEQ ID NO: 289, SEQ ID NO: 291, SEQ ID NO: 293, SEQ ID NO: 295, SEQ ID NO: 297, SEQ ID NO: 438, SEQ ID NO: 444, SEQ ID NO: 448, SEQ ID NO: 452, SEQ ID NO: 456, SEQ ID NO: 460, SEQ ID NO: 464, SEQ ID NO: 492, SEQ ID NO: 498, SEQ ID NO: 502, SEQ ID NO: 506, SEQ ID NO: 510, SEQ ID NO: 584, SEQ ID NO: 592, SEQ ID NO: 593, SEQ ID NO: 609, SEQ ID NO: 615, SEQ ID NO: 619, SEQ ID NO: 623, SEQ ID NO: 627, SEQ ID NO: 631, SEQ ID NO: 635 or SEQ ID NO: 639. The light chain FWR3 may comprise the amino acid sequence of SEQ ID NO: 236, SEQ ID NO: 245, SEQ ID NO: 265, SEQ ID NO: 266, SEQ ID NO: 267, SEQ ID NO: 271, SEQ ID NO: 272, SEQ ID NO: 274, SEQ ID NO: 276, SEQ ID NO: 277, SEQ ID NO: 278, SEQ ID NO: 279, SEQ ID NO: 280, SEQ ID NO: 282, SEQ ID NO: 284, SEQ ID NO: 286, SEQ ID NO: 288, SEQ ID NO: 290, SEQ ID NO: 292, SEQ ID NO: 294, SEQ ID NO: 296, SEQ ID NO: 298, SEQ ID NO: 300, SEQ ID NO: 302, SEQ ID NO: 304, SEQ ID NO: 306, SEQ ID NO: 308, SEQ ID NO: 310, SEQ ID NO: 312, SEQ ID NO: 314, SEQ ID NO: 316, SEQ ID NO: 318, SEQ ID NO: 320, SEQ ID NO: 323, SEQ ID NO: 327, SEQ ID NO: 331, SEQ ID NO: 335, SEQ ID NO: 339, SEQ ID NO: 343, SEQ ID NO: 347, SEQ ID NO: 351, SEQ ID NO: 355, SEQ ID NO: 359, SEQ ID NO: 363, SEQ ID NO: 367, SEQ ID NO: 371, SEQ ID NO: 375, SEQ ID NO: 379, SEQ ID NO: 383, SEQ ID NO: 387, SEQ ID NO: 440, SEQ ID NO: 445, SEQ ID NO: 449, SEQ ID NO: 453, SEQ ID NO: 457, SEQ ID NO: 461, SEQ ID NO: 465, SEQ ID NO: 494, SEQ ID NO: 499, SEQ ID NO: 503, SEQ ID NO: 507, SEQ ID NO: 511, SEQ ID NO: 586, SEQ ID NO: 611, SEQ ID NO: 616, SEQ ID NO: 620, SEQ ID NO: 624, SEQ ID NO: 628, SEQ ID NO: 632, SEQ ID NO: 636 or SEQ ID NO: 640, and the light chain FWR3 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 236, SEQ ID NO: 245, SEQ ID NO: 265, SEQ ID NO: 266, SEQ ID NO: 267, SEQ ID NO: 271, SEQ ID NO: 272, SEQ ID NO: 274, SEQ ID NO: 276, SEQ ID NO: 277, SEQ ID NO: 278, SEQ ID NO: 279, SEQ ID NO: 280, SEQ ID NO: 282, SEQ ID NO: 284, SEQ ID NO: 286, SEQ ID NO: 288, SEQ ID NO: 290, SEQ ID NO: 292, SEQ ID NO: 294, SEQ ID NO: 296, SEQ ID NO: 298, SEQ ID NO: 300, SEQ ID NO: 302, SEQ ID NO: 304, SEQ ID NO: 306, SEQ ID NO: 308, SEQ ID NO: 310, SEQ ID NO: 312, SEQ ID NO: 314, SEQ ID NO: 316, SEQ ID NO: 318, SEQ ID NO: 320, SEQ ID NO: 323, SEQ ID NO: 327, SEQ ID NO: 331, SEQ ID NO: 335, SEQ ID NO: 339, SEQ ID NO: 343, SEQ ID NO: 347, SEQ ID NO: 351, SEQ ID NO: 355, SEQ ID NO: 359, SEQ ID NO: 363, SEQ ID NO: 367, SEQ ID NO: 371, SEQ ID NO: 375, SEQ ID NO: 379, SEQ ID NO: 383, SEQ ID NO: 387, SEQ ID NO: 440, SEQ ID NO: 445, SEQ ID NO: 449, SEQ ID NO: 453, SEQ ID NO: 457, SEQ ID NO: 461, SEQ ID NO: 465, SEQ ID NO: 494, SEQ ID NO: 499, SEQ ID NO: 503, SEQ ID NO: 507, SEQ ID NO: 511, SEQ ID NO: 586, SEQ ID NO: 611, SEQ ID NO: 616, SEQ ID NO: 620, SEQ ID NO: 624, SEQ ID NO: 628, SEQ ID NO: 632, SEQ ID NO: 636 or SEQ ID NO: 640. The light chain FWR4 may comprise the amino acid sequence of SEQ ID NO: 238, SEQ ID NO: 442, SEQ ID NO: 446, SEQ ID NO: 450, SEQ ID NO: 454, SEQ ID NO: 458, SEQ ID NO: 462, SEQ ID NO: 496, SEQ ID NO: 500, SEQ ID NO: 504, SEQ ID NO: 508, SEQ ID NO: 512, SEQ ID NO: 588, SEQ ID NO: 613, SEQ ID NO: 617, SEQ ID NO: 621, SEQ ID NO: 625, SEQ ID NO: 629, SEQ ID NO: 633, SEQ ID NO: 637 or SEQ ID NO: 641 and the light chain FWR4 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 238, SEQ ID NO: 442, SEQ ID NO: 446, SEQ ID NO: 450, SEQ ID NO: 454, SEQ ID NO: 458, SEQ ID NO: 462, SEQ ID NO: 496, SEQ ID NO: 500, SEQ ID NO: 504, SEQ ID NO: 508, SEQ ID NO: 512, SEQ ID NO: 588, SEQ ID NO: 613, SEQ ID NO: 617, SEQ ID NO: 621, SEQ ID NO: 625, SEQ ID NO: 629, SEQ ID NO: 633, SEQ ID NO: 637 or SEQ ID NO: 641. It will be understood that antibodies comprising amino acid changes in the light chain framework region(s) (FWR1, FWR2, FWR3, and/or FWR4) retain the capability to specifically bind to CD38. The retained CD38 specific binding activity (including affinity) is preferably about the same as the binding activity (including affinity) of an antibody without any amino acid changes in any light chain framework region(s), although the binding activity (including affinity) may be lesser or greater than an antibody without any amino acid changes in any light chain framework region(s).

The light chain CDR1 may comprise the amino acid sequence of SEQ ID NO: 233, SEQ ID NO: 250, SEQ ID NO: 525, SEQ ID NO: 255, SEQ ID NO: 262, SEQ ID NO: 263, SEQ ID NO: 319, SEQ ID NO: 322, SEQ ID NO: 325, SEQ ID NO: 329, SEQ ID NO: 333, SEQ ID NO: 337, SEQ ID NO: 341, SEQ ID NO: 345, SEQ ID NO: 349, SEQ ID NO: 353, SEQ ID NO: 357, SEQ ID NO: 361, SEQ ID NO: 365, SEQ ID NO: 369, SEQ ID NO: 373, SEQ ID NO: 377, SEQ ID NO: 381, SEQ ID NO: 385, SEQ ID NO: 437, SEQ ID NO: 491, SEQ ID NO: 583, SEQ ID NO: 589, SEQ ID NO: 590, SEQ ID NO: 608, or SEQ ID NO: 696, and the light chain CDR1 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 233, SEQ ID NO: 250, SEQ ID NO: 525, SEQ ID NO: 255, SEQ ID NO: 262, SEQ ID NO: 263, SEQ ID NO: 319, SEQ ID NO: 322, SEQ ID NO: 325, SEQ ID NO: 329, SEQ ID NO: 333, SEQ ID NO: 337, SEQ ID NO: 341, SEQ ID NO: 345, SEQ ID NO: 349, SEQ ID NO: 353, SEQ ID NO: 357, SEQ ID NO: 361, SEQ ID NO: 365, SEQ ID NO: 369, SEQ ID NO: 373, SEQ ID NO: 377, SEQ ID NO: 381, SEQ ID NO: 385, SEQ ID NO: 437, SEQ ID NO: 491, SEQ ID NO: 583, SEQ ID NO: 589, SEQ ID NO: 590, SEQ ID NO: 608, or SEQ ID NO: 696. The light chain CDR2 may comprise the amino acid sequence of SEQ ID NO: 235, SEQ ID NO: 249, SEQ ID NO: 253, SEQ ID NO: 264, SEQ ID NO: 299, SEQ ID NO: 301, SEQ ID NO: 303, SEQ ID NO: 305, SEQ ID NO: 307, SEQ ID NO: 309, SEQ ID NO: 311, SEQ ID NO: 313, SEQ ID NO: 315, SEQ ID NO: 317, SEQ ID NO: 326, SEQ ID NO: 330, SEQ ID NO: 334, SEQ ID NO: 338, SEQ ID NO: 342, SEQ ID NO: 346, SEQ ID NO: 350, SEQ ID NO: 354, SEQ ID NO: 358, SEQ ID NO: 362, SEQ ID NO: 366, SEQ ID NO: 370, SEQ ID NO: 374, SEQ ID NO: 378, SEQ ID NO: 382, SEQ ID NO: 386, SEQ ID NO: 439, SEQ ID NO: 493, SEQ ID NO: 585, SEQ ID NO: 591, SEQ ID NO: 605, SEQ ID NO: 610 or SEQ ID NO: 747, and the light chain CDR2 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 235, SEQ ID NO: 249, SEQ ID NO: 253, SEQ ID NO: 264, SEQ ID NO: 299, SEQ ID NO: 301, SEQ ID NO: 303, SEQ ID NO: 305, SEQ ID NO: 307, SEQ ID NO: 309, SEQ ID NO: 311, SEQ ID NO: 313, SEQ ID NO: 315, SEQ ID NO: 317, SEQ ID NO: 326, SEQ ID NO: 330, SEQ ID NO: 334, SEQ ID NO: 338, SEQ ID NO: 342, SEQ ID NO: 346, SEQ ID NO: 350, SEQ ID NO: 354, SEQ ID NO: 358, SEQ ID NO: 362, SEQ ID NO: 366, SEQ ID NO: 370, SEQ ID NO: 374, SEQ ID NO: 378, SEQ ID NO: 382, SEQ ID NO: 386, SEQ ID NO: 439, SEQ ID NO: 493, SEQ ID NO: 585, SEQ ID NO: 591, SEQ ID NO: 605, SEQ ID NO: 610 or SEQ ID NO: 747. The light chain CDR3 may comprise the amino acid sequence of SEQ ID NO: 237, SEQ ID NO: 244, SEQ ID NO: 251, SEQ ID NO: 254, SEQ ID NO: 256, SEQ ID NO: 257, SEQ ID NO: 258, SEQ ID NO: 268, SEQ ID NO: 269, SEQ ID NO: 270, SEQ ID NO: 273, SEQ ID NO: 275, SEQ ID NO: 321, SEQ ID NO: 324, SEQ ID NO: 328, SEQ ID NO: 332, SEQ ID NO: 336, SEQ ID NO: 340, SEQ ID NO: 344, SEQ ID NO: 348, SEQ ID NO: 352, SEQ ID NO: 356, SEQ ID NO: 360, SEQ ID NO: 364, SEQ ID NO: 368, SEQ ID NO: 372, SEQ ID NO: 376, SEQ ID NO: 380, SEQ ID NO: 384, SEQ ID NO: 388, SEQ ID NO: 441, SEQ ID NO: 495, SEQ ID NO: 587, SEQ ID NO: 594, SEQ ID NO: 595, SEQ ID NO: 596, SEQ ID NO: 597, SEQ ID NO: 598, SEQ ID NO: 599, SEQ ID NO: 600, SEQ ID NO: 601, SEQ ID NO: 602, SEQ ID NO: 603, SEQ ID NO: 604, SEQ ID NO: 606 or SEQ ID NO: 612, and the light chain CDR3 may comprise an amino acid sequence having at least about 85%, at least about 90%, at least about 92%, at least about 93%, at least about 94%, at least about 95%, at least about 96%, at least about 97%, at least about 98%, or at least about 99% sequence identity with the amino acid sequence of SEQ ID NO: 237, SEQ ID NO: 244, SEQ ID NO: 251, SEQ ID NO: 254, SEQ ID NO: 256, SEQ ID NO: 257, SEQ ID NO: 258, SEQ ID NO: 268, SEQ ID NO: 269, SEQ ID NO: 270, SEQ ID NO: 273, SEQ ID NO: 275, SEQ ID NO: 321, SEQ ID NO: 324, SEQ ID NO: 328, SEQ ID NO: 332, SEQ ID NO: 336, SEQ ID NO: 340, SEQ ID NO: 344, SEQ ID NO: 348, SEQ ID NO: 352, SEQ ID NO: 356, SEQ ID NO: 360, SEQ ID NO: 364, SEQ ID NO: 368, SEQ ID NO: 372, SEQ ID NO: 376, SEQ ID NO: 380, SEQ ID NO: 384, SEQ ID NO: 388, SEQ ID NO: 441, SEQ ID NO: 495, SEQ ID NO: 587, SEQ ID NO: 594, SEQ ID NO: 595, SEQ ID NO: 596, SEQ ID NO: 597, SEQ ID NO: 598, SEQ ID NO: 599, SEQ ID NO: 600, SEQ ID NO: 601, SEQ ID NO: 602, SEQ ID NO: 603, SEQ ID NO: 604, SEQ ID NO: 606 or SEQ ID NO: 612. It will be understood that antibodies comprising amino acid changes in the light chain complementarity determining region(s) (CDR1, CDR2, and/or CDR3) retain the capability to specifically bind to CD38. The retained CD38 specific binding activity (including affinity) is preferably about the same as the binding activity (including affinity) of an antibody without any amino acid changes in any light chain complementarity determining region(s), although the binding activity (including affinity) may be lesser or greater than an antibody without any amino acid changes in any light chain complementarity determining region(s).

The antibody may comprise particular heavy and light chain pairs. The heavy chains having the amino acid sequences of SEQ ID NO: 659 may be paired with any light chains having the amino acid sequences of SEQ ID NO: 664, or the heavy chains having the amino acid sequences of SEQ ID NO: 665 may be paired with any light chains having the amino acid sequences of SEQ ID NO: 666, or the heavy chain having the amino acid sequences of SEQ ID NO: 736 may be paired with any light chains having the amino acid sequences of SEQ ID NO: 664.

Variable heavy and variable light chain pairs may comprise pairs from the following table:

| **Antibody Name** | **Variable Heavy SEQ ID NO: (amino acid)** | **Variable Light SEQ ID NO: (amino acid)** |
|---|---|---|
| A02.10 | 19 | 14 |
| A02.11 | 20 | 14 |
| A02.112 | 34 | 65 |
| A02.12 | 34 | 65 |
| A02.13 | 35 | 65 |
| A02.16 | 34 | 92 |
| A02.17 | 34 | 93 |
| A02.18 | 34 | 73 |
| A02.19 | 34 | 74 |
| A02.2 | 13 | 65 |
| A02.20 | 34 | 75 |
| A02.21 | 34 | 76 |
| A02.22 | 34 | 77 |
| A02.23 | 34 | 78 |
| A02.24 | 34 | 79 |
| A02.25 | 34 | 80 |
| A02.26 | 34 | 81 |
| A02.27 | 34 | 82 |
| A02.28 | 34 | 83 |
| A02.29 | 34 | 84 |
| A02.3 | 17 | 65 |
| A02.30 | 34 | 85 |
| A02.31 | 34 | 86 |
| A02.32 | 34 | 87 |
| A02.33 | 34 | 88 |
| A02.34 | 34 | 89 |
| A02.35 | 34 | 90 |
| A02.36 | 34 | 91 |
| A02.37 | 34 | 66 |
| A02.38 | 34 | 113 |
| A02.39 | 34 | 112 |
| A02.4 | 18 | 65 |
| A02.40 | 111 | 65 |
| A02.41 | 110 | 65 |
| A02.43 | 110 | 113 |
| A02.44 | 111 | 112 |
| A02.46 | 34 | 67 |
| A02.47 | 34 | 68 |
| A02.48 | 34 | 69 |
| A02.49 | 34 | 70 |
| A02.5 | 19 | 65 |
| A02.50 | 34 | 71 |
| A02.51 | 34 | 72 |
| A02.52 | 34 | 94 |
| A02.53 | 34 | 95 |
| A02.54 | 34 | 96 |
| A02.55 | 34 | 97 |
| A02.56 | 34 | 98 |
| A02.57 | 34 | 99 |
| A02.58 | 34 | 100 |
| A02.59 | 34 | 101 |
| A02.6 | 20 | 65 |
| A02.60 | 34 | 102 |
| A02.61 | 34 | 103 |
| A02.62 | 34 | 104 |
| A02.63 | 34 | 105 |
| A02.64 | 34 | 106 |
| A02.65 | 34 | 107 |
| A02.66 | 34 | 108 |
| A02.67 | 34 | 109 |
| A02.8 | 17 | 14 |
| A02.9 | 18 | 14 |
| A10.1 | 165 | 161 |
| A10.10 | 174 | 161 |
| A10.11 | 175 | 161 |
| A10.12 | 176 | 161 |
| A10.13 | 177 | 161 |
| A10.14 | 178 | 161 |
| A10.15 | 179 | 161 |
| A10.16 | 180 | 161 |
| A10.17 | 156 | 181 |
| A10.18 | 156 | 182 |
| A10.19 | 156 | 183 |
| A10.2 | 166 | 161 |
| A10.20 | 156 | 184 |
| A10.21 | 156 | 185 |
| A10.22 | 156 | 186 |
| A10.23 | 156 | 187 |
| A10.24 | 156 | 188 |
| A10.25 | 156 | 189 |
| A10.26 | 156 | 190 |
| A10.27 | 156 | 191 |
| A10.28 | 156 | 192 |
| A10.29 | 156 | 193 |
| A10.3 | 167 | 161 |
| A10.30 | 156 | 194 |
| A10.31 | 156 | 195 |
| A10.32 | 156 | 196 |
| A10.35 | 197 | 161 |
| A10.36 | 156 | 198 |
| A10.38 | 152 | 161 |
| A10.39 | 152 | 181 |
| A10.4 | 168 | 161 |
| A10.40 | 152 | 182 |
| A10.41 | 152 | 183 |
| A10.42 | 152 | 184 |
| A10.43 | 152 | 185 |
| A10.44 | 152 | 186 |
| A10.45 | 152 | 187 |
| A10.46 | 152 | 188 |
| A10.47 | 152 | 189 |
| A10.48 | 152 | 190 |
| A10.49 | 152 | 191 |
| A10.5 | 169 | 161 |
| A10.50 | 152 | 192 |
| A10.51 | 152 | 193 |
| A10.52 | 152 | 194 |
| A10.53 | 152 | 195 |
| A10.54 | 152 | 196 |
| A10.57 | 152 | 198 |
| A10.59 | 156 | 161 |
| A10.6 | 170 | 161 |
| A10.7 | 171 | 161 |
| A10.8 | 172 | 161 |
| A10.9 | 173 | 161 |
| A10A2.0 (chimeric) | 148 | 157 |
| A10A2.1 | 149 | 158 |
| A10A2.10 | 150 | 160 |
| A10A2.11 | 150 | 161 |
| A10A2.12 | 150 | 162 |
| A10A2.13 | 150 | 163 |
| A10A2.14 | 150 | 164 |
| A10A2.15 | 151 | 158 |
| A10A2.16 | 151 | 159 |
| A10A2.17 | 151 | 160 |
| A10A2.18 | 151 | 161 |
| A10A2.19 | 151 | 162 |
| A10A2.2 | 149 | 159 |
| A10A2.20 | 151 | 163 |
| A10A2.21 | 151 | 164 |
| A10A2.22 | 152 | 158 |
| A10A2.23 | 152 | 159 |
| A10A2.24 | 152 | 160 |
| A10A2.25 | 152 | 161 |
| A10A2.26 | 152 | 162 |
| A10A2.27 | 152 | 163 |
| A10A2.28 | 152 | 164 |
| A10A2.29 | 153 | 158 |
| A10A2.3 | 149 | 160 |
| A10A2.30 | 153 | 159 |
| A10A2.31 | 153 | 160 |
| A10A2.32 | 153 | 161 |
| A10A2.33 | 153 | 162 |
| A10A2.34 | 153 | 163 |
| A10A2.35 | 153 | 164 |
| A10A2.36 | 154 | 158 |
| A10A2.37 | 154 | 159 |
| A10A2.38 | 154 | 160 |
| A10A2.39 | 154 | 161 |
| A10A2.4 | 149 | 161 |
| A10A2.40 | 154 | 162 |
| A10A2.41 | 154 | 163 |
| A10A2.42 | 154 | 164 |
| A10A2.43 | 154 | 158 |
| A10A2.44 | 155 | 159 |
| A10A2.45 | 155 | 160 |
| A10A2.46 | 155 | 161 |
| A10A2.47 | 155 | 162 |
| A10A2.48 | 155 | 163 |
| A10A2.49 | 155 | 164 |
| A10A2.5 | 149 | 162 |
| A10A2.50 | 156 | 158 |
| A10A2.51 | 156 | 159 |
| A10A2.52 | 156 | 160 |
| A10A2.53 | 156 | 161 |
| A10A2.54 | 156 | 162 |
| A10A2.55 | 156 | 163 |
| A10A2.56 | 156 | 164 |
| A10A2.6 | 149 | 163 |
| A10A2.7 | 149 | 164 |
| A10A2.8 | 150 | 158 |
| A10A2.9 | 150 | 159 |
| A5D1.0 (chimeric) | 114 | 125 |
| A5D1.1 | 115 | 126 |
| A5D1.10 | 116 | 129 |
| A5D1.11 | 116 | 130 |
| A5D1.12 | 116 | 131 |
| A5D1.13 | 117 | 126 |
| A5D1.14 | 117 | 127 |
| A5D1.15 | 117 | 128 |
| A5D1.16 | 117 | 129 |
| A5D1.17 | 117 | 130 |
| A5D1.18 | 117 | 131 |
| A5D1.19 | 118 | 126 |
| A5D1.2 | 115 | 127 |
| A5D1.20 | 118 | 127 |
| A5D1.21 | 118 | 128 |
| A5D1.22 | 118 | 129 |
| A5D1.23 | 118 | 130 |
| A5D1.24 | 118 | 131 |
| A5D1.25 | 119 | 126 |
| A5D1.26 | 119 | 127 |
| A5D1.27 | 119 | 128 |
| A5D1.28 | 119 | 129 |
| A5D1.29 | 119 | 130 |
| A5D1.3 | 115 | 128 |
| A5D1.30 | 119 | 131 |
| A5D1.31 | 120 | 126 |
| A5D1.32 | 120 | 127 |
| A5D1.33 | 120 | 128 |
| A5D1.34 | 120 | 129 |
| A5D1.35 | 120 | 130 |
| A5D1.36 | 120 | 131 |
| A5D1.37 | 121 | 126 |
| A5D1.38 | 121 | 127 |
| A5D1.39 | 121 | 128 |
| A5D1.4 | 115 | 129 |
| A5D1.40 | 121 | 129 |
| A5D1.41 | 121 | 130 |
| A5D1.42 | 121 | 131 |
| A5D1.43 | 122 | 126 |
| A5D1.44 | 122 | 127 |
| A5D1.45 | 122 | 128 |
| A5D1.46 | 122 | 129 |
| A5D1.47 | 122 | 130 |
| A5D1.48 | 122 | 131 |
| A5D1.49 | 123 | 126 |
| A5D1.5 | 115 | 130 |
| A5D1.50 | 123 | 127 |
| A5D1.51 | 123 | 128 |
| A5D1.52 | 123 | 129 |
| A5D1.53 | 123 | 130 |
| A5D1.54 | 123 | 131 |
| A5D1.55 | 124 | 126 |
| A5D1.56 | 124 | 127 |
| A5D1.57 | 124 | 128 |
| A5D1.58 | 124 | 129 |
| A5D1.59 | 124 | 130 |
| A5D1.6 | 115 | 131 |
| A5D1.60 | 124 | 131 |
| A5D1.7 | 116 | 126 |
| A5D1.8 | 116 | 127 |
| A5D1.9 | 116 | 128 |
| A5E8.0 (chimeric) | 132 | 143 |
| A5E8.1 | 133 | 144 |
| A5E8.10 | 135 | 145 |
| A5E8.11 | 135 | 146 |
| A5E8.12 | 135 | 147 |
| A5E8.13 | 136 | 144 |
| A5E8.14 | 136 | 145 |
| A5E8.15 | 136 | 146 |
| A5E8.16 | 136 | 147 |
| A5E8.17 | 137 | 144 |
| A5E8.18 | 137 | 145 |
| A5E8.19 | 137 | 146 |
| A5E8.2 | 133 | 145 |
| A5E8.20 | 137 | 147 |
| A5E8.21 | 138 | 144 |
| A5E8.22 | 138 | 145 |
| A5E8.23 | 138 | 146 |
| A5E8.24 | 138 | 147 |
| A5E8.25 | 139 | 144 |
| A5E8.26 | 139 | 145 |
| A5E8.27 | 139 | 146 |
| A5E8.28 | 139 | 147 |
| A5E8.29 | 140 | 144 |
| A5E8.3 | 133 | 146 |
| A5E8.30 | 140 | 145 |
| A5E8.31 | 140 | 146 |
| A5E8.32 | 140 | 147 |
| A5E8.33 | 141 | 144 |
| A5E8.34 | 141 | 145 |
| A5E8.35 | 141 | 146 |
| A5E8.36 | 141 | 147 |
| A5E8.37 | 142 | 144 |
| A5E8.38 | 142 | 145 |
| A5E8.39 | 142 | 146 |
| A5E8.4 | 133 | 147 |
| A5E8.40 | 142 | 147 |
| A5E8.5 | 134 | 144 |
| A5E8.6 | 134 | 145 |
| A5E8.7 | 134 | 146 |
| A5E8.8 | 134 | 147 |
| A5E8.9 | 135 | 144 |
| X02.10 | 19 | 14 |
| X02.100 | 13 | 58 |
| X02.101 | 13 | 59 |
| X02.102 | 13 | 60 |
| X02.103 | 13 | 61 |
| X02.104 | 13 | 62 |
| X02.105 | 13 | 63 |
| X02.106 | 13 | 64 |
| X02.107 | 13 | 65 |
| X02.108 | 32 | 14 |
| X02.11 | 20 | 14 |
| X02.110 | 33 | 14 |
| X02.114 | 13 | 660 |
| X02.115 | 13 | 661 |
| X02.116 | 13 | 662 |
| X02.117 | 13 | 663 |
| X02.118 | 34 | 700 |
| X02.119 | 34 | 701 |
| X02.120 | 728 | 700 |
| X02.121 | 729 | 700 |
| X02.122 | 730 | 700 |
| X02.123 | 731 | 700 |
| X02.124 | 728 | 701 |
| X02.125 | 729 | 701 |
| X02.126 | 730 | 701 |
| X02.127 | 731 | 701 |
| X02.68 | 21 | 14 |
| X02.69 | 22 | 14 |
| X02.70 | 23 | 14 |
| X02.71 | 24 | 14 |
| X02.72 | 25 | 14 |
| X02.73 | 26 | 14 |
| X02.74 | 27 | 14 |
| X02.75 | 28 | 14 |
| X02.76 | 29 | 14 |
| X02.77 | 30 | 14 |
| X02.78 | 31 | 14 |
| X02.8 | 17 | 14 |
| X02.80 | 13 | 38 |
| X02.81 | 13 | 39 |
| X02.82 | 13 | 40 |
| X02.83 | 13 | 41 |
| X02.84 | 13 | 42 |
| X02.85 | 13 | 43 |
| X02.86 | 13 | 44 |
| X02.87 | 13 | 45 |
| X02.88 | 13 | 46 |
| X02.89 | 13 | 47 |
| X02.9 | 18 | 14 |
| X02.90 | 13 | 48 |
| X02.91 | 13 | 49 |
| X02.92 | 13 | 50 |
| X02.93 | 13 | 51 |
| X02.94 | 13 | 52 |
| X02.95 | 13 | 53 |
| X02.96 | 13 | 54 |
| X02.97 | 13 | 55 |
| X02.98 | 13 | 56 |
| X02.99 | 13 | 57 |
| X10.100 | 720 | 706 |
| X10.101 | 721 | 706 |
| X10.102 | 722 | 706 |
| X10.103 | 723 | 706 |
| X10.104 | 739 | 706 |
| X10.105 | 740 | 706 |
| X10.106 | 741 | 706 |
| X10.107 | 742 | 706 |
| X10.108 | 720 | 707 |
| X10.109 | 721 | 707 |
| X10.110 | 722 | 707 |
| X10.111 | 723 | 707 |
| X10.112 | 739 | 707 |
| X10.113 | 740 | 707 |
| X10.114 | 741 | 707 |
| X10.115 | 742 | 707 |
| X10.116 | 720 | 708 |
| X10.117 | 721 | 708 |
| X10.118 | 722 | 708 |
| X10.119 | 723 | 708 |
| X10.120 | 739 | 708 |
| X10.121 | 740 | 708 |
| X10.122 | 741 | 708 |
| X10.123 | 742 | 708 |
| X10.124 | 720 | 709 |
| X10.125 | 721 | 709 |
| X10.126 | 722 | 709 |
| X10.127 | 723 | 709 |
| X10.128 | 739 | 709 |
| X10.129 | 740 | 709 |
| X10.130 | 741 | 709 |
| X10.131 | 742 | 709 |
| X10.132 | 720 | 710 |
| X10.133 | 721 | 710 |
| X10.134 | 722 | 710 |
| X10.135 | 723 | 710 |
| X10.136 | 739 | 710 |
| X10.137 | 740 | 710 |
| X10.138 | 741 | 710 |
| X10.139 | 742 | 710 |
| X10.140 | 720 | 711 |
| X10.141 | 721 | 711 |
| X10.142 | 722 | 711 |
| X10.143 | 723 | 711 |
| X10.144 | 739 | 711 |
| X10.145 | 740 | 711 |
| X10.146 | 741 | 711 |
| X10.147 | 742 | 711 |
| X10.60 | 156 | 704 |
| X10.61 | 156 | 705 |
| X10.62 | 156 | 706 |
| X10.63 | 156 | 707 |
| X10.64 | 156 | 708 |
| X10.65 | 156 | 709 |
| X10.66 | 156 | 710 |
| X10.67 | 156 | 711 |
| X10.68 | 720 | 161 |
| X10.69 | 721 | 161 |
| X10.70 | 722 | 161 |
| X10.71 | 723 | 161 |
| X10.72 | 739 | 161 |
| X10.73 | 740 | 161 |
| X10.74 | 741 | 161 |
| X10.75 | 742 | 161 |
| X10.76 | 152 | 704 |
| X10.77 | 152 | 705 |
| X10.78 | 152 | 706 |
| X10.79 | 152 | 707 |
| X10.80 | 152 | 708 |
| X10.81 | 152 | 709 |
| X10.82 | 152 | 710 |
| X10.83 | 152 | 711 |
| X10.84 | 720 | 704 |
| X10.85 | 721 | 704 |
| X10.86 | 722 | 704 |
| X10.87 | 723 | 704 |
| X10.88 | 739 | 704 |
| X10.89 | 740 | 704 |
| X10.90 | 741 | 704 |
| X10.91 | 742 | 704 |
| X10.92 | 720 | 705 |
| X10.93 | 721 | 705 |
| X10.94 | 722 | 705 |
| X10.95 | 723 | 705 |
| X10.96 | 739 | 705 |
| X10.97 | 740 | 705 |
| X10.98 | 741 | 705 |
| X10.99 | 742 | 705 |
| X910/12-HC-L0-IFN-alpha (A145D) IgG4 | 110 | 112 |
| X913/15-HC-L0-IFN-alpha (A145D) IgG4 | 111 | 113 |

The antibodies may be fused to attenuated ligands, for example, to form antibody-attenuated ligand constructs, which show an elevated antigen-specificity index with respect to activating signaling pathways due to the action of the attenuated ligand *on* a cell surface receptor. These constructs are based on the observation that, in the context of an antibody-ligand construct, the ligand portion can be mutated in *such* a way that the ligand activity on antigen-negative cells is dramatically attenuated, while the ligand activity on antigen-positive cells is only modestly, if at all, attenuated. Such constructs display one, two, three, four or five orders of magnitude greater potency on antigen-positive cells compared to antigen negative cells than does the free ligand. In some aspects, the antibody-attenuated ligand construct retains at least 1%, at least 10%, at least 20%, at least 30%, at least 40% or at least 50% of the potency on antigen-positive cells as the non-attenuated free *(i.e.,* not attached to an antibody) ligand. In some aspects, the antibody-attenuated ligand construct retains at least 30%, at least 50%, at least 75% or at least 90% of the maximal activity of the non-attenuated free (i.e. not attached to an antibody) ligand. Maximal activity includes the amount of signaling activity (or downstream effect thereof) at the high, plateau portion of a dose-response curve, where further increases in the *agent* does not further increase the amount of response.

In some aspects, the antibody fusion to and inclusion of an attenuating mutation(s) in the interferon ligand increases the antigen-specificity index (ASI) by greater than 10-fold, preferably greater than 50-fold, preferably greater than 100-fold, preferably greater than 1000-fold, or preferably greater than 10,000 fold, relative to an antibody without a fusion. The ASI comprises the fold-increased potency in signaling activity of the antibody-IFN ligand construct relative to the free non-mutated polypeptide ligand on target antigen-positive cells, multiplied by the fold decreased potency in signaling activity relative to the free non-mutated polypeptide ligand on target antigen-negative cells. Potency may be quantitatively represented by the EC₅₀ value, which is the mathematical midpoint of a dose-response curve, in which the dose refers to the concentration of ligand or antibody-ligand construct in an assay, and response refers to the quantitative response of the cells to the signaling activity of the ligand at a particular dose. Thus, for example, when a first compound is shown to possess an EC₅₀ (expressed for example in Molar units) that is 10-fold lower than a second compound's EC₅₀ on the same cells, typically when measured by the same method, the first compound is said to have a 10-fold higher potency. Conversely, when a first compound is shown to possess an EC₅₀ that is 10-fold higher than a second compound's EC₅₀ on the same cells, typically when measured by the same method, the first compound is said to have a 10-fold lower potency.

The antibodies are preferably capable of binding to CD38-positive cells. The antibody may bind to a CD38-positive cell with an EC₅₀ value of less than about 100 nM. The antibody may bind to a CD38-positive cell with an EC₅₀ value of less than about 75 nM. The antibody may bind to a CD38-positive cell with an EC₅₀ value of less than about 50 nM. The antibody may bind to a CD38-positive cell with an EC₅₀ value of less than about 30 nM. The antibody may bind to a CD38-positive cell with an EC₅₀ value of less than about 25 nM. The antibody may bind to a CD38-positive cell with an EC₅₀ value of less than about 20 nM. The antibody may bind to a CD38-positive cell with an EC₅₀ value of less than about 18 nM. The antibody may bind to a CD38-positive cell with an EC₅₀ value of less than about 15 nM. The antibody may bind to a CD38-positive cell with an EC₅₀ value of less than about 13 nM. The antibody may bind to a CD38-positive cell with an EC₅₀ value of less than about 10 nM.

The interferon joined to the antibody preferably comprises alterations in its amino acid sequence, including point mutations and/or deletions that render the interferon less active in stimulating its respective receptors on cells that lack cell surface expression of the CD38 antigen to which the antibody binds. A highly preferred variant of interferon alpha comprises an amino acid change at position 168 of the interferon alpha 2b molecule of SEQ ID NO: 7. For example, the amino acid at position 168, which is an alanine in the parent IFN-alpha2b molecule, is preferably changed to a glycine (Gly/G) (SEQ ID NO: 650) or aspartic acid (Asp/D) (SEQ ID NO: 647). In some aspects, the IFN-alpha2b is truncated at its N-terminus when the IFN-alpha2b is fused to an IgG heavy chain constant domain such as the human IgG1 or human IgG4 heavy chain constant domain. The truncated IFN-alpha2b does not have the twenty three N-terminal amino acids of SEQ ID NO: 7 (Met 1 through Gly 23 are deleted), and the truncated IFN-alpha2b comprises the amino acid sequence of SEQ ID NO: 648. The truncated IFN-alpha2b may also comprise the amino acid change at what was formerly position 168, but which becomes position 145 in the truncated protein (e.g., alanine 168 becomes alanine 145). In the truncated IFN-alpha2b, the alanine is preferably changed to a glycine (Gly/G) (SEQ ID NO: 651) or aspartic acid (Asp/D) (SEQ ID NO: 649). Interferon with A145D alteration (SEQ ID NO: 647 or SEQ ID NO: 649) is particularly preferred as the attenuated ligand fused to the antibodies of the disclosure. Any of these point-mutated, attenuated versions of IFN-alpha may be joined to any antibody described herein, for example, as an antibody-attenuated interferon construct.

The linkage between the antibody and the interferon preferably comprises a fusion, for example, a peptide bond between the N- or the C-terminus of the interferon and the Nor C-terminus of the heavy or the light chain of the antibody. In highly preferred aspects, no linker is present between the antibody and the interferon, and the antibody and interferon are thus directly fused. It is believed that direct fusion, without an intervening linker peptide, provides at least a measurable degree of attenuation of the interferon protein, and it is also believed that this attenuation is additive with the attenuation of the interferon protein that stems from the mutations introduced into the interferon protein, including those described or exemplified herein.

Polynucleotide sequences that encode antibodies and their subdomains (e.g., FWRs and CDRs) are featured in the disclosure. Polynucleotides include, but are not limited to, RNA, DNA, cDNA, hybrids of RNA and DNA, and single, double, or triple stranded strands of RNA, DNA, or hybrids thereof.

The polynucleotides may encode the heavy chain of an antibody that specifically binds to an epitope on CD38. The polynucleotide may encode a heavy chain comprising the amino acid sequence of any of SEQ ID NO: 667, SEQ ID NO: 668, SEQ ID NO: 679, SEQ ID NO: 680, SEQ ID NO: 681, SEQ ID NO: 682, SEQ ID NO: 683, SEQ ID NO: 684, SEQ ID NO:, SEQ ID NO: 685, SEQ ID NO: 686, SEQ ID NO: 695, SEQ ID NO: 724, SEQ ID NO: 725, SEQ ID NO: 726, SEQ ID NO: 727, SEQ ID NO: 732, SEQ ID NO: 733, SEQ ID NO: 734, SEQ ID NO: 735, SEQ ID NO: 743, SEQ ID NO: 744, SEQ ID NO: 745 or SEQ ID NO: 746. The polynucleotide may encode a light chain comprising the amino acid sequence of any of SEQ ID NO: 669, SEQ ID NO: 670, SEQ ID NO: 671, SEQ ID NO: 672, SEQ ID NO: 673, SEQ ID NO: 674, SEQ ID NO: 675, SEQ ID NO: 676, SEQ ID NO: 677, SEQ ID NO: 678, SEQ ID NO: 688, SEQ ID NO: 689, SEQ ID NO: 690, SEQ ID NO: 691, SEQ ID NO: 692, SEQ ID NO: 693, SEQ ID NO: 702, SEQ ID NO: 703, SEQ ID NO: 712, SEQ ID NO: 713, SEQ ID NO: 714, SEQ ID NO: 715, SEQ ID NO: 716, SEQ ID NO: 717, SEQ ID NO: 718 or SEQ ID NO: 719. The polynucleotide may comprise the nucleic acid sequence of any of SEQ ID NO: 667, SEQ ID NO: 668, SEQ ID NO: 679, SEQ ID NO: 680, SEQ ID NO: 681, SEQ ID NO: 682, SEQ ID NO: 683, SEQ ID NO: 684, SEQ ID NO:, SEQ ID NO: 685, SEQ ID NO: 686, SEQ ID NO: 695, SEQ ID NO: 724, SEQ ID NO: 725, SEQ ID NO: 726, SEQ ID NO: 727, SEQ ID NO: 732, SEQ ID NO: 733, SEQ ID NO: 734, SEQ ID NO: 735, SEQ ID NO: 743, SEQ ID NO: 744, SEQ ID NO: 745, SEQ ID NO: 746, SEQ ID NO: 669, SEQ ID NO: 670, SEQ ID NO: 671, SEQ ID NO: 672, SEQ ID NO: 673, SEQ ID NO: 674, SEQ ID NO: 675, SEQ ID NO: 676, SEQ ID NO: 677, SEQ ID NO: 678, SEQ ID NO: 688, SEQ ID NO: 689, SEQ ID NO: 690, SEQ ID NO: 691, SEQ ID NO: 692, SEQ ID NO: 693, SEQ ID NO: 702, SEQ ID NO: 703, SEQ ID NO: 712, SEQ ID NO: 713, SEQ ID NO: 714, SEQ ID NO: 715, SEQ ID NO: 716, SEQ ID NO: 717, SEQ ID NO: 718 or SEQ ID NO: 719. The polynucleotide may comprise a nucleic acid sequence having at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% sequence identity with any of SEQ ID NO: 667, SEQ ID NO: 668, SEQ ID NO: 679, SEQ ID NO: 680, SEQ ID NO: 681, SEQ ID NO: 682, SEQ ID NO: 683, SEQ ID NO: 684, SEQ ID NO:, SEQ ID NO: 685, SEQ ID NO: 686, SEQ ID NO: 695, SEQ ID NO: 724, SEQ ID NO: 725, SEQ ID NO: 726, SEQ ID NO: 727, SEQ ID NO: 732, SEQ ID NO: 733, SEQ ID NO: 734, SEQ ID NO: 735, SEQ ID NO: 743, SEQ ID NO: 744, SEQ ID NO: 745, SEQ ID NO: 746, SEQ ID NO: 669, SEQ ID NO: 670, SEQ ID NO: 671, SEQ ID NO: 672, SEQ ID NO: 673, SEQ ID NO: 674, SEQ ID NO: 675, SEQ ID NO: 676, SEQ ID NO: 677, SEQ ID NO: 678, SEQ ID NO: 688, SEQ ID NO: 689, SEQ ID NO: 690, SEQ ID NO: 691, SEQ ID NO: 692, SEQ ID NO: 693, SEQ ID NO: 702, SEQ ID NO: 703, SEQ ID NO: 712, SEQ ID NO: 713, SEQ ID NO: 714, SEQ ID NO: 715, SEQ ID NO: 716, SEQ ID NO: 717, SEQ ID NO: 718 or SEQ ID NO: 719, and such variants preferably encode the same amino acids encoded by the polynucleotide sequence of SEQ ID NO: 667, SEQ ID NO: 668, SEQ ID NO: 679, SEQ ID NO: 680, SEQ ID NO: 681, SEQ ID NO: 682, SEQ ID NO: 683, SEQ ID NO: 684, SEQ ID NO:, SEQ ID NO: 685, SEQ ID NO: 686, SEQ ID NO: 695, SEQ ID NO: 724, SEQ ID NO: 725, SEQ ID NO: 726, SEQ ID NO: 727, SEQ ID NO: 732, SEQ ID NO: 733, SEQ ID NO: 734, SEQ ID NO: 735, SEQ ID NO: 743, SEQ ID NO: 744, SEQ ID NO: 745, SEQ ID NO: 746, SEQ ID NO: 669, SEQ ID NO: 670, SEQ ID NO: 671, SEQ ID NO: 672, SEQ ID NO: 673, SEQ ID NO: 674, SEQ ID NO: 675, SEQ ID NO: 676, SEQ ID NO: 677, SEQ ID NO: 678, SEQ ID NO: 688, SEQ ID NO: 689, SEQ ID NO: 690, SEQ ID NO: 691, SEQ ID NO: 692, SEQ ID NO: 693, SEQ ID NO: 702, SEQ ID NO: 703, SEQ ID NO: 712, SEQ ID NO: 713, SEQ ID NO: 714, SEQ ID NO: 715, SEQ ID NO: 716, SEQ ID NO: 717, SEQ ID NO: 718 or SEQ ID NO: 719. Preferably, the antibodies encoded by the polynucleotide variants will specifically bind to CD38 with an affinity about equal to the affinity of the antibody encoded by the parent (non-variant) polynucleotide sequence. Affinity may be measured, for example, according to any technique described or exemplified herein, including techniques described in the Examples. Complements of the polynucleotide sequences and the variant polynucleotide sequences are also within the scope of the disclosure.

Disclosed herein are vectors comprising the polynucleotides of the disclosure. The vectors may be expression vectors. Recombinant expression vectors containing a sequence encoding a polypeptide of interest are thus provided. The expression vector may contain one or more additional sequences, such as but not limited to regulatory sequences, a selection marker, a purification tag, or a polyadenylation signal. Such regulatory elements may include a transcriptional promoter, enhancers, mRNA ribosomal binding sites, or sequences that control the termination of transcription and translation.

Expression vectors, especially mammalian expression vectors, may include one or more nontranscribed elements, such as an origin of replication, a suitable promoter and enhancer linked to the gene to be expressed, other 5' or 3' flanking nontranscribed sequences, 5' or 3' nontranslated sequences (such as necessary ribosome binding sites), a polyadenylation site, splice donor and acceptor sites, or transcriptional termination sequences. An origin of replication that confers the ability to replicate in a specific host may also be incorporated.

The vectors may be used to transform any of a wide array of host cells well known to those of skill in the art, and preferably host cells capable of expressing antibodies. Vectors include without limitation, plasmids, phagemids, cosmids, baculoviruses, bacmids, bacterial artificial chromosomes (BACs), yeast artificial chromosomes (YACs), and baculovirus, as well as other bacterial, eukaryotic, yeast, and viral vectors. Suitable host cells include without limitation CHO cells, HEK293 cells, or any eukaryotic stable cell line known or produced, and also include bacteria, yeast, and insect cells.

The antibodies may also be produced by hybridoma cells; methods to produce hybridomas being well known and established in the art.

It has been observed in accordance with the disclosure that when interferon alpha ligand, having one or more mutations that substantially decrease the affinity of the ligand for an interferon receptor, is linked to an anti-CD38 antibody that targets the mutated interferon alpha ligand to target cells which display the antibody's corresponding antigen, the ligand's activity on target antigen-positive cells is maintained while the ligand's activity on non-target antigen-negative cells is substantially reduced. The net result is a ligand signaling molecule that has a much greater potency in activation of its receptors on antigen-positive target cells compared to antigen-negative non-target cells, which provides a means for reducing toxicity arising from off-target ligand activity.

A polypeptide construct may comprise an IFN-alpha variant linked to an anti-CD38 antibody or antigen binding portion thereof. Such a polypeptide will be capable of exerting with high potency the IFN's anti-proliferative activity on CD38-positive tumor cells while exerting a much lower potency on CD38-negative, non-tumor cells within the body.

The disclosure also provides compositions comprising the antibodies and antibody-attenuated interferon constructs of the disclosure. These compositions can further comprise at least one of any suitable auxiliary, such as, but not limited to one or more, diluents, binders, stabilizers, buffers, salts, lipophilic solvents, preservatives, adjuvants, or other suitable carrier and/or excipient. Pharmaceutically acceptable auxiliaries are preferred. The compositions may comprise any of the antibodies and antibody-attenuated interferon constructs described and/or exemplified herein and an acceptable carrier such as a pharmaceutically acceptable carrier. Suitable carriers include any media that does not interfere with the biological activity of the antibody and/or the interferon and preferably is not toxic to a host to which it is administered. The carrier may be an aqueous solution, such as water, saline, or alcohol, or a physiologically compatible buffer, such as Hanks's solution, Ringer's solution, or physiological saline buffer. The carrier may contain formulatory agents, such as suspending, stabilizing and/or dispersing agents

Pharmaceutical excipients and additives useful in the composition include but are not limited to proteins, peptides, amino acids, lipids, and carbohydrates (e.g., sugars, including monosaccharides, di-, tri-, tetra-, and oligosaccharides; derivatized sugars such as alditols, aldonic acids, esterified sugars and other known sugars; and polysaccharides or sugar polymers), which can be present singly or in combination, comprising alone or in combination any suitable weight or volume. Exemplary protein excipients include serum albumin, such as human serum albumin (HSA), recombinant human albumin (rHA), gelatin, casein, and other known proteins. Representative amino acids which can also function in a buffering capacity include alanine, glycine, arginine, betaine, histidine, glutamic acid, aspartic acid, cysteine, lysine, leucine, isoleucine, valine, methionine, phenylalanine, and aspartame. One preferred amino acid is histidine. A second preferred amino acid is arginine.

Carbohydrate excipients suitable for use in the composition include, for example, monosaccharides, such as fructose, maltose, galactose, glucose, D-mannose, and sorbose; disaccharides, such as lactose, sucrose, trehalose, and cellobiose; polysaccharides, such as raffinose, melezitose, maltodextrins, dextrans, and starches; and alditols, such as mannitol, xylitol, maltitol, lactitol, xylitol sorbitol (glucitol), and myoinositol. Preferred carbohydrate excipients for use in the disclosure are mannitol, trehalose, and raffinose.

Antibody compositions can also include a buffer or a pH adjusting agent; typically, the buffer is a salt prepared from an organic acid or base. Representative buffers include organic acid salts, such as salts of citric acid, ascorbic acid, gluconic acid, carbonic acid, tartaric acid, succinic acid, acetic acid, or phthalic acid; Tris, tromethamine hydrochloride, or phosphate buffers. Preferred buffers for use in the present compositions are organic acid salts, such as citrate.

Additionally, the compositions of the disclosure can include polymeric excipients/additives, such as polyvinylpyrrolidones, ficolls (a polymeric sugar), dextrates (e.g., cyclodextrins, such as 2-hydroxypropyl-β-cyclodextrin), polyethylene glycols, antimicrobial agents, antioxidants, antistatic agents, surfactants (*e.g*., polysorbates such as "TWEEN® 20" and "TWEEN® 80"), lipids (*e.g.,* phospholipids, fatty acids), steroids (*e.g.,* cholesterol), and chelating agents (*e.g*., EDTA).

The compositions may also be formulated in sustained release vehicles or depot preparations. For example, the compositions may be formulated with suitable polymeric or hydrophobic materials (for example, as an emulsion in an acceptable oil) or ion exchange resins, or as sparingly soluble derivatives, for example, as a sparingly soluble salt. Liposomes and emulsions are well-known examples of delivery vehicles suitable for use as carriers for hydrophobic drugs.

The compositions may be formulated for administration to a subject in any suitable dosage form. The compositions may be formulated for oral, buccal, nasal, transdermal, parenteral, injectable, intravenous, subcutaneous, intramuscular, rectal, or vaginal administrations. The compositions may be formulated in a suitable controlled-release vehicle, with an adjuvant, or as a depot formulation.

Preparations for parenteral administration include sterile solutions ready for injection, sterile dry soluble products ready to be combined with a solvent just prior to use, including hypodermic tablets, sterile suspensions ready for injection, sterile dry insoluble products ready to be combined with a vehicle just prior to use and sterile emulsions.

An anti-CD38-attenuated interferon alpha-2b fusion construct may be used, for example, to inhibit, reduce, decrease, block, or prevent proliferation of a cell that expressed CD38 on its surface. Methods for inhibiting or reducing proliferation of a cell that expresses CD38 on its surface generally comprise contacting a cell expressing CD38 with an anti-CD38-attenuated interferon alpha-2b fusion construct in an amount effective to inhibit or reduce proliferation of the cell. The antibody that specifically binds to CD38 may be any antibody described or exemplified herein. The attenuated interferon alpha 2b may comprise IFN-alpha2b A145D or IFN-alpha2b A145G. The cell may be a lymphocyte, an autoimmune lymphocyte, or a tumor cell such as a leukemia cell, a multiple myeloma cell, or a lymphoma cell. The anti-CD38-attenuated interferon alpha-2b fusion construct may be comprised in a composition, for example, with a pharmaceutically acceptable carrier and optionally one or more auxiliaries or excipients, including any such carrier, auxiliary, or excipient described or exemplified herein. The methods may be carried out *in vitro, ex vivo, in vivo,* or *in situ.*

An anti-CD38-attenuated interferon alpha-2b fusion construct may also be used, for example, to induce, facilitate, or enhance apoptosis of a cell that expressed CD38 on its surface. Methods for inducing apoptosis in a cell that expresses CD38 on its surface generally comprise contacting a cell expressing CD38 with an anti-CD38-attenuated interferon alpha-2b fusion construct in an amount effective to induce apoptosis in the cell. The antibody that specifically binds to CD38 may be any antibody described or exemplified herein. The attenuated interferon alpha 2b may comprise IFN-alpha2b A145D or IFN-alpha2b A145G. The cell may be a lymphocyte, an autoimmune lymphocyte, or a tumor cell such as a leukemia cell, a multiple myeloma cell, or a lymphoma cell. The anti-CD38-attenuated interferon alpha-2b fusion construct may be comprised in a composition, for example, with a pharmaceutically acceptable carrier and optionally one or more auxiliaries or excipients, including any such carrier, auxiliary, or excipient described or exemplified herein. The methods may be carried out *in vitro, ex vivo, in vivo,* or *in situ.*

An anti-CD38-attenuated interferon alpha-2b fusion construct may also be used to treat a subject having a tumor that comprises and/or is mediated, at least in part, by cells that express CD38 on their surface. Methods for treating a tumor comprising cells expressing CD38 on their surface generally comprise administering to a subject in need thereof an anti-CD38-attenuated interferon alpha-2b fusion construct in an amount effective to treat the tumor in the subject. Effective treatment may include, for example, inhibiting or reducing proliferation of CD38-positive cells in the tumor and/or inducing apoptosis of CD38-positive cells in the tumor. The antibody that specifically binds to CD38 may be any antibody described or exemplified herein. The attenuated interferon alpha 2b may comprise IFN-alpha2b A145D or IFN-alpha2b A145G. The anti-CD38-attenuated interferon alpha-2b fusion construct may be comprised in a composition, for example, with a pharmaceutically acceptable carrier and optionally one or more auxiliaries or excipients, including any such carrier, auxiliary, or excipient described or exemplified herein.

The anti-CD38-attenuated interferon alpha-2b fusion constructs or composition comprising such constructs may be administered to the tumor by administering the constructs of composition to the blood. The anti-CD38-attenuated interferon alpha-2b fusion constructs or composition comprising such constructs may be administered such that the construct diffuses via blood flow to and/or into the tumor cells. The construct may be internalized by a tumor cell.

Use of an anti-CD38 antibody or anti-CD38 antibody-attenuated interferon alpha-2b fusion construct in the treatment of tumors is provided. Methods for treating tumors with an anti-CD38 antibody or anti-CD38 antibody-attenuated interferon alpha-2b fusion construct are disclosed herein. Any anti-CD38 antibody or anti-CD38 antibody-attenuated interferon alpha-2b fusion construct described or exemplified herein may be used. Tumors that may be treated include, but are not limited to AIDS related cancers, acoustic neuroma, acute lymphocytic leukemia, acute myeloid leukemia, adenocystic carcinoma, adrenocortical cancer, agnogenic myeloid metaplasia, alopecia, alveolar soft-part sarcoma, anal cancer, angiosarcoma, aplastic anemia, astrocytoma, ataxia-telangiectasia, basal cell carcinoma (skin), bladder cancer, bone cancers, bowel cancer, brain stem glioma, brain and CNS tumors, breast cancer, CNS tumors, carcinoid tumors, cervical cancer, childhood brain tumors, childhood cancer, childhood leukemia, childhood soft tissue sarcoma, chondrosarcoma, choriocarcinoma, chronic lymphocytic leukemia, chronic myeloid leukemia, colorectal cancers, cutaneous T-Cell lymphoma, dermatofibrosarcoma-protuberans, desmoplastic-small-round-cell-tumor, ductal carcinoma, endocrine cancers, endometrial cancer, ependymoma, esophageal cancer, Ewing's sarcoma, extra-hepatic bile duct cancer, eye cancer, eye: melanoma, retinoblastoma, fallopian tube cancer, fanconi anemia, fibrosarcoma, gall bladder cancer, gastric cancer, gastrointestinal cancers, gastrointestinal-carcinoid-tumor, genitourinary cancers, germ cell tumors, gestational-trophoblastic-disease, glioma, gynecological cancers, hematological malignancies, hairy cell leukemia, head and neck cancer, hepatocellular cancer, hereditary breast cancer, histiocytosis, Hodgkin's disease, human papillomavirus, hydatidiform mole, hypercalcemia, hypopharynx cancer, intraocular melanoma, islet cell cancer, Kaposi's sarcoma, kidney cancer, Langerhan's-cell-histiocytosis, laryngeal cancer, leiomyosarcoma, leukemia, Li-Fraumeni syndrome, lip cancer, liposarcoma, liver cancer, lung cancer, lymphedema, lymphoma, Hodgkin's lymphoma, non-Hodgkin's lymphoma, male breast cancer, malignant-rhabdoid-tumor-of-kidney, medulloblastoma, melanoma, merkel cell cancer, mesothelioma, metastatic cancer, mouth cancer, multiple endocrine neoplasia, mycosis fungoides, myelodysplastic syndromes, multiple myeloma, myeloproliferative disorders, nasal cancer, nasopharyngeal cancer, nephroblastoma, neuroblastoma, neurofibromatosis, nijmegen breakage syndrome, non-melanoma skin cancer, non-small-cell-lung-cancer-(NSCLC), ocular cancers, esophageal cancer, oral cavity cancer, oropharynx cancer, osteosarcoma, ostomy ovarian cancer, pancreas cancer, paranasal cancer, parathyroid cancer, parotid gland cancer, penile cancer, peripheral-neuroectodermal-tumors, pituitary cancer, polycythemia vera, prostate cancer, rare-cancers-and-associated-disorders, renal cell carcinoma, retinoblastoma, rhabdomyosarcoma, Rothmund-Thomson syndrome, salivary gland cancer, sarcoma, schwannoma, Sezary syndrome, skin cancer, small cell lung cancer (SCLC), small intestine cancer, soft tissue sarcoma, spinal cord tumors, squamous-cell-carcinoma-(skin), stomach cancer, synovial sarcoma, testicular cancer, thymus cancer, thyroid cancer, transitional-cell-cancer-(bladder), transitional-cell-cancer-(renal-pelvis-/-ureter), trophoblastic cancer, urethral cancer, urinary system cancer, uroplakins, uterine sarcoma, uterus cancer, vaginal cancer, vulva cancer, Waldenstrom's-macroglobulinemia and Wilms' tumor. In an embodiment the tumor is selected from a group of multiple myeloma or non-Hodgkin's lymphoma.

The methods may be used for treatment of multiple myeloma, leukemia, or lymphoma in a subject in need thereof. Such methods may further comprise treating the subject with a retinoid, such as all-trans retinoic acid. When the cell surface associated antigen is CD38, the tumor or cancer may be selected from multiple myeloma, non-Hodgkin's lymphoma, chronic myelogenous leukemia, chronic lymphocytic leukemia or acute myelogenous leukemia.

An anti-CD38-attenuated interferon alpha-2b fusion construct may be combined with other drugs and/or used in addition to other cancer treatment regimens or modalities such as radiation therapy or surgery. When anti-CD38-attenuated interferon alpha-2b fusion constructs are used in combination with known therapeutic agents the combination may be administered either in sequence (either continuously or broken up by periods of no treatment) or concurrently or as a mixture. In the case of cancer, there are numerous known anticancer agents that may be used in this context. Treatment in combination is also contemplated to encompass the treatment with either the anti-CD38-attenuated interferon alpha-2b fusion construct followed by a known treatment, or treatment with a known agent followed by treatment with the anti-CD38-attenuated interferon alpha-2b fusion construct, for example, as maintenance therapy. For example, in the treatment of cancer it is contemplated that the anti-CD38-attenuated interferon alpha-2b fusion construct may be administered in combination with an alkylating agent (such as mechlorethamine, cyclophosphamide, chlorambucil, ifosfamidecysplatin, or platinum-containing alkylating-like agents such as cisplatin, carboplatin and oxaliplatin), an antimetabolite (such as a purine or pyrimidine analogue or an antifolate agent, such as azathioprine and mercaptopurine), an anthracycline (such as Daunorubicin, Doxorubicin, Epirubicinldarubicin, Valrubicin, Mitoxantrone, or anthracycline analog), a plant alkaloid (such as a vinca alkaloid or a taxane, such as Vincristine, Vinblastine, Vinorelbine, Vindesine, paclitaxel or Dosetaxel), a topoisomerase inhibitor (such as a type I or type II topoisomerase inhibitor), a Podophyllotoxin (such as etoposide or teniposide), or a tyrosine kinase inhibitor (such as imatinibmesylate, Nilotinib, or Dasatinib).

In the case of the treatment of multiple myeloma, an anti-CD38-attenuated interferon alpha-2b fusion construct may be administered in combination with other suitable therapies, such as treatment of the subject with the administration of steroids such as dexamethasone, proteasome inhibitors (such as bortezomib or carfilzomib), immunomodulatory drugs (such as thalidomide, lenalidomide or pomalidomide), or induction chemotherapy followed by autologous hematopoietic stem cell transplantation, with or without other chemotherapeutic agents such as Melphalan hydrochloride or the chemotherapeutic agents listed above.

In the case of the treatment of Hodgkin's lymphoma, an anti-CD38-attenuated interferon alpha-2b fusion construct may be administered in combination with current therapeutic approaches, such as ABVD (Adriamycin (doxorubicin), bleomycin, vinblastine, and dacarbazine), or Stanford V (doxorubicin, bleomycin, vinblastine, vincristine, mechlorethamine, etoposide, prednisone), or BEACOPP (doxorubicin, bleomycin, vincristine, cyclophosphamide, procarbazine, etoposide, prednisone).

In the case of non-Hodgkin's lymphoma or other lymphomas, an anti-CD38-attenuated interferon alpha-2b fusion construct may be administered in combination current therapeutic approaches. Examples of drugs approved for non-Hodgkin lymphoma include Abitrexate (Methotrexate), Adriamycin PFS (Doxorubicin Hydrochloride), Adriamycin RDF (Doxorubicin Hydrochloride), Ambochlorin (Chlorambucil), Amboclorin (Chlorambucil), Arranon (Nelarabine), Bendamustine Hydrochloride, Bexxar (Tositumomab and Iodine I 131 Tositumomab), Blenoxane (Bleomycin), Bleomycin, Bortezomib, Chlorambucil, Clafen (Cyclophosphamide), Cyclophosphamide, Cytoxan (Cyclophosphamide), DenileukinDiftitox, DepoCyt (Liposomal Cytarabine), Doxorubicin Hydrochloride, DTIC-Dome (Dacarbazine), Folex (Methotrexate), Folex PFS (Methotrexate), Folotyn (Pralatrexate), Ibritumomab Tiuxetan, Istodax (Romidepsin), Leukeran (Chlorambucil), Linfolizin (Chlorambucil), Liposomal Cytarabine, Matulane (Procarbazine Hydrochloride), Methotrexate, Methotrexate LPF (Methotrexate), Mexate (Methotrexate), Mexate-AQ (Methotrexate), Mozobil (Plerixafor), Nelarabine, Neosar (Cyclophosphamide), Ontak (DenileukinDiftitox), Plerixafor, Pralatrexate, Rituxan (Rituximab), Rituximab, Romidepsin, Tositumomab and Iodine I 131 Tositumomab, Treanda (Bendamustine Hydrochloride), Velban (Vinblastine Sulfate), Velcade (Bortezomib), and Velsar (Vinblastine Sulfate), Vinblastine Sulfate, Vincasar PFS (Vincristine Sulfate), Vincristine Sulfate, Vorinostat, Zevalin (IbritumomabTiuxetan), Zolinza (Vorinostat). Examples of drug combinations used in treating non-Hodgkin lymphoma include CHOP (C = Cyclophosphamide, H = Doxorubicin Hydrochloride (Hydroxydaunomycin), O = Vincristine Sulfate (Oncovin), P = Prednisone); COPP (C = Cyclophosphamide, O = Vincristine Sulfate (Oncovin), P = Procarbazine Hydrochloride, P = Prednisone); CVP (C = Cyclophosphamide, V = Vincristine Sulfate, P = Prednisone); EPOCH (E = Etoposide, P = Prednisone, O = Vincristine Sulfate (Oncovin), C = Cyclophosphamide, H = Doxorubicin Hydrochloride (Hydroxydaunomycin)); ICE (I = Ifosfamide, C = Carboplatin, E = Etoposide) and R-CHOP (R = Rituximab, C = Cyclophosphamide, H = Doxorubicin Hydrochloride (Hydroxydaunomycin), O = Vincristine Sulfate (Oncovin), P = Prednisone.

An anti-CD38 antibody, or an anti-CD38-attenuated interferon alpha-2b fusion construct may be used to detect CD38-positive cells, including CD38-positive tumor cells. They may be used in methods for detecting a CD38-positive tumor cell in a tissue sample isolated from a subject, which methods may generally comprise contacting an anti-CD38 antibody, or an anti-CD38-attenuated interferon alpha-2b fusion construct, with a tissue sample isolated from a subject and detecting a complex of the antibody or construct and a CD38-positive cell in the tissue sample. The tissue sample preferably is blood. The cell may be a CD38-positive B-cell lymphoma cell, multiple myeloma cell, non-Hodgkin's lymphoma cell, chronic myelogenous leukemia cell, chronic lymphocytic leukemia cell, or acute myelogenous leukemia cell. The method may further comprise isolating the tissue sample from the subject.

The disclosure also features kits comprising any of the antibodies and anti-CD38-attenuated interferon alpha-2b fusion constructs described and exemplified herein. The kits may be used to supply antibodies and other agents for use in diagnostic, basic research, or therapeutic methods, among others.

A kit may comprise an anti-CD38-attenuated interferon alpha-2b fusion construct, the construct optionally comprised in a composition comprising a pharmaceutically acceptable carrier, and instructions for using the kit in one or more of a method for inhibiting or reducing proliferation of a tumor cell expressing CD38 on its surface, a method for inducing apoptosis in a tumor cell expressing CD38 on its surface, and/or a method for treating a tumor that comprises and/or is mediated by cells expressing CD38 on their surface. Such methods may be any method described or exemplified herein. The kits may comprise a pharmaceutically acceptable carrier. The kits may comprise one or more pharmaceutically acceptable auxiliaries and/or one or more pharmaceutically acceptable excipients. In the kits, the anti-CD38 antibody may be any antibody described or exemplified herein, and the attenuated interferon alpha-2b may comprise any attenuated interferon alpha-2b described or exemplified herein. The constructs may be comprised in sterile solutions ready for injection or intravenous administration, or may comprise a sterile, lyophilized form ready to be combined with a carrier just prior to use.

A kit may comprise an anti-CD38 antibody and instructions for using the kit in a method for detecting CD38-positive cells in a sample, including a tissue sample isolated from a subject. The anti-CD38 antibody may be any antibody described or exemplified herein. The antibody may optionally be fused to an attenuated interferon alpha-2b protein.

The following examples are provided to describe the disclosure in greater detail. They are intended to illustrate, not to limit, the disclosure.

### Reference Example 1

### Optimization of X355/02-HC-L0-IFN-alpha (A145D) IgG4

Other anti-CD38-attenuated IFN fusion proteins are described in PCT Application No. PCT/AU2012/001323. These include the antibody construct designated in the PCT application as X355/02-HC-L0-IFN-alpha (A145D) IgG4. In this specification, X355/02-HC-L0-IFN-alpha (A145D) IgG4 has been renamed as A02.1. The heavy chain sequence of the antibody comprises the amino acid sequence of SEQ ID NO: 11, and the light chain sequence comprises the amino acid sequence of SEQ ID NO: 12. The variable light chain of A02.1 (SEQ ID NO: 14) was co-expressed with its variable heavy chain A02.1 (SEQ ID NO: 13) formatted on a human IgG4 constant region containing the substitution S228P (EU Numbering) (SEQ ID NO: 3). This antibody is referred to herein as X02.1. A02.1 includes a fusion to IFN-alpha2b whilst X02.1 does not, despite both antibodies sharing identical heavy chain and light chains sequences.

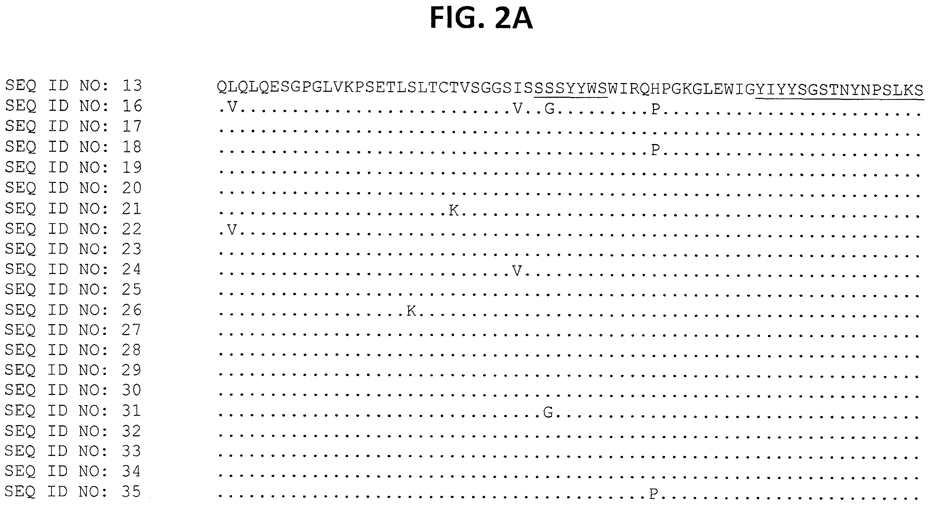

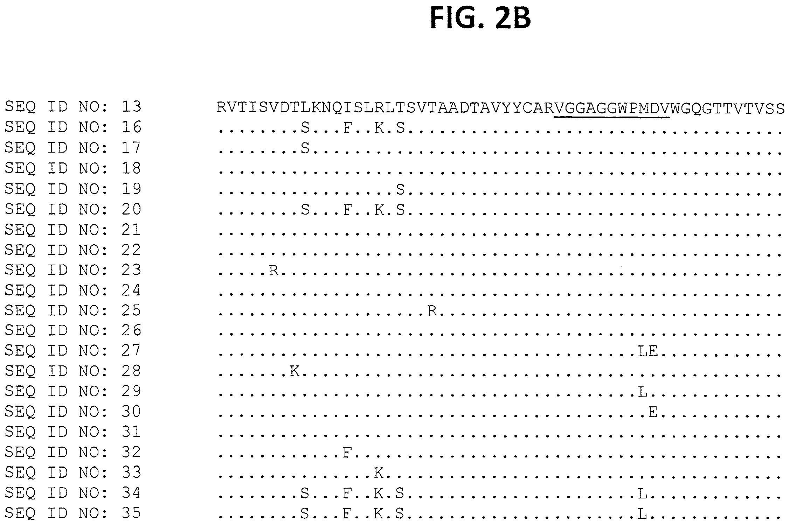

A BLAST search (Altschul SF (1997) Nucleic Acids Res. 25:3389-3402) against a database of human germline immunoglobulin genes was performed using the amino acid sequence of the variable heavy chain of X02.1. The closest human germline variable heavy chain gene was IGHV4-61^{∗}01 (SEQ ID NO: 16). An alignment of the X02.1 VH and IGHV4-61^{∗}01 is shown in Figure 2. The X02.1 variable heavy region differs by eight amino acids from its closest germline amino acid sequence. In order to reduce the immunogenicity of the X02.1 heavy chain variable region, germline amino acid residue substitutions could be produced at residues where it differs from the germline sequence and the resulting antibody variants tested for anti-CD38 binding activity.

Several heavy chain variants of the X02.1 parental sequence are detailed in Figure 2. These heavy chain variable regions were formatted onto the IgG4 S228P constant region, and co-expressed with the A02.1 light chain. Tables 1a and 1b detail the sequences of the variants tested along with their ability to bind human CD38 as assessed using flow cytometry and surface plasmon resonance (SPR). Briefly, antibody chains were transiently co-expressed in CHO cells and purified via Protein A chromatography as described in Example 5. Flow binding assays as described in Example 5 were used to assess the variants. The EC₅₀ of the dose response curve obtained for each antibody is also given in Tables 1a and 1b.

**Table 1a**

| **Antibody Designation** | **Variable Heavy Chain Amino Acid Substitution (Relative to X02.1)** | **Variable Heavy SEQ ID NO:** | **Variable Light SEQ ID NO:** | **CD38 binding by SPR** | **ARP-1 flow binding assay (EC₅₀ in µg/mL)** |
|---|---|---|---|---|---|
| X02.8 | L74S | 17 | 14 | 2.30 x10⁻⁸ | 18.3 |
| X02.9 | H40P | 18 | 14 | 2.63 x10⁻⁸ | N/T |
| X02.10 | T(82A)S | 19 | 14 | 2.07 x10⁻⁸ | N/T |
| X02.11 | L74S, I78F R81K, T(82A)S | 20 | 14 | 2.39 x10⁻⁸ | 18.1 |
| X02.108 | I78F | 32 | 14 | 2.63 x10⁻⁸ | N/T |
| X02.110 | R81K | 33 | 14 | 2.07 x10⁻⁸ | N/T |

| | | | | | |
|---|---|---|---|---|---|
| N/T- Protein was not able to be purified and was not tested. | | | | | |

**Table 1b**

| **Antibody Designation** | **Variable Heavy Chain Amino Acid Substitution (Relative to X02.1)** | **Variable Heavy SEQ ID NO:** | **Variable Light SEQ ID NO: [Verify]** | **CD38 binding by SPR** | **ARP-1 flow binding assay (EC₅₀ in µg/mL)** |
|---|---|---|---|---|---|
| X02.69 | Q2V | 22 | 14 | 3.68 x10⁻¹¹ | 16.8 |
| X02.71 | I29V | 24 | 14 | 1.29 x10⁻¹⁰ | 3.5 |
| X02.78 | S32G | 31 | 14 | 2.04 x10⁻¹¹ | N/T |

| | | | | | |
|---|---|---|---|---|---|
| N/T - Protein not purified or tested. | | | | | |

SPR binding of the variants detailed in Table 1a was evaluated separately to those of Table 1b. The K_{D} (M) of the parental antibody X02.1 ranged from 2.7 x 10⁻⁸ to 3.78 x 10⁻¹⁰ in the SPR binding experiments. Flow cytometry binding experiments showed antibodies X02.8, X02.11, X02.69 and X02.71 bound strongly to the CD38 positive cell line ARP-1.

Antibodies with the above amino acid substitutions were subsequently explored in the context of a fusion protein through conjugation to attenuated IFN-alpha2b (termed A02 when linked to IFN, with the number following the decimal representing the same variant having the X02 designation). These heavy chain variable regions were formatted onto an IgG4 constant region comprising the substitution S228P fused to A145D attenuated IFN-alpha2b and co-expressed in CHO or HEK cells with the A02.1 light chain as described in Example 5. Proteins that were successfully purified from cell supernatant were then tested in a flow binding assay to the cell line ARP-1. The EC₅₀ value of the dose response curve for each antibody is given in Table 2. All antibody-attenuated IFN fusion constructs tested bound to the CD38 positive cell line ARP-1. It was observed that heavy chain variant X02.9 (not fused to IFN) could not easily be purified whereas an identical variant fused to IFN (A02.9) was purified. In some cases, attenuated IFN fusion proteins could be expressed and purified, when the equivalent monoclonal antibody appeared more difficult to be expressed and/or purify.

**Table 2**

| **Anti-CD38-attenuated IFN fusion protein** | **Variable Heavy Chain Amino Acid Substitution (Relative to A02.1)** | **Variable Heavy SEQ ID NO:** | **Variable Light SEQ ID NO:** | **Protein A capture by SPR (RU)*** | **CD38 binding by SPR (RU) at 350 sec*** | **ARP-1 flow binding assay (EC₅₀ in µg/mL)** |
|---|---|---|---|---|---|---|
| A02.8 | L74S | 17 | 14 | 4697 | 833 | 1.9 |
| A02.9 | H40P | 18 | 14 | 4718 | 841 | 1.0 |
| A02.10 | T(82A)S | 19 | 14 | 4647 | 804 | 1.5 |
| A02.11 | L74S, I78F, R81K, T(82A)S | 20 | 14 | 4483 | 827 | 3.5 |

| | | | | | | |
|---|---|---|---|---|---|---|
| *The amount of Anti-CD38 attenuated IFN fusion protein in the cell culture supernatant is indicated by the Protein A capture by SPR. The CD38 binding by SPR refers to the amount of CD38 that remains bound to the surface after 350 seconds of the dissociation phase. | | | | | | |

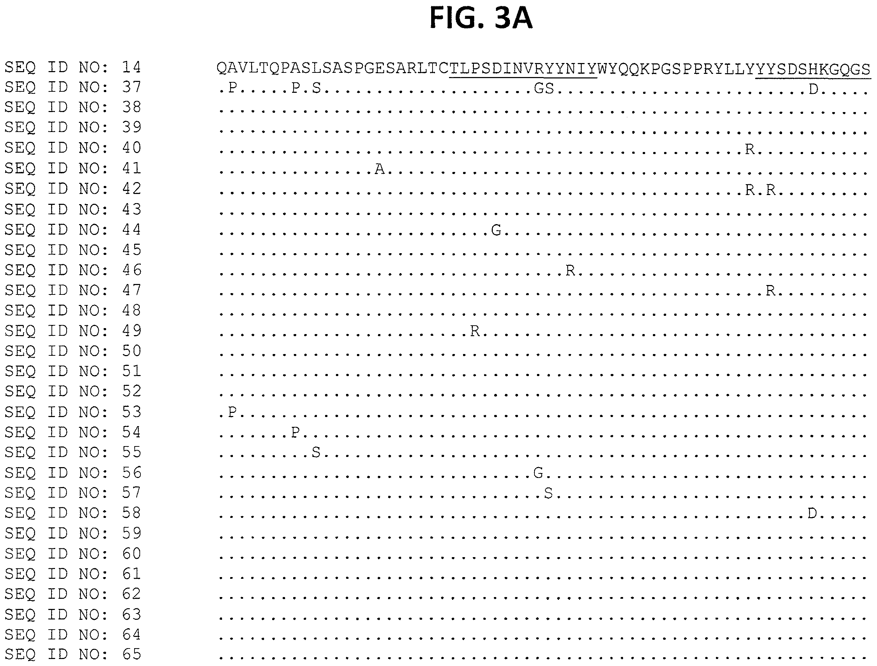

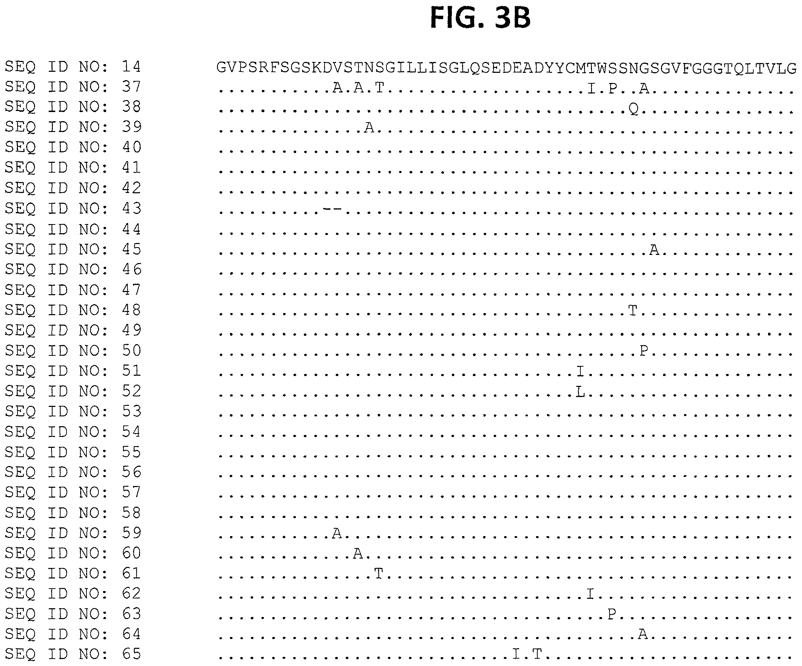

BLAST searches using the amino acid sequence of the A02.1 variable light chain were performed against the database of human germline immunoglobulin genes. The closest human germline variable light chain gene was IGLV5-37^{∗}01. An amino acid sequence alignment of A02.1VL and IGLV5-37^{∗}01 is given in Figure 3. This alignment illustrates a 12 amino acid difference between these sequences.

Several amino acid substitutions were made in the X02.1 variable light chain. These substitutions are shown in Figure 3. Co-expression of these light chain variable regions with the X02.1 variable heavy chain formatted onto an IgG4 constant region containing the substitution S228P was performed in CHO cells as described in Example 5.

Antibodies purified from CHO cell supernatants were subsequently tested in flow cytometry-based binding assays to the CD38 positive cell line ARP-1. Table 3 details EC₅₀ values of the dose response curve obtained for each antibody.

**Table 3**

| **Antibody Designation** | **Variable Light Chain Amino Acid Substitution (Relative to A02.1)** | **Variable Heavy SEQ ID NO:** | **Variable Light SEQ ID NO:** | **CD38 binding by SPR (KD)** | **ARP-1 flow binding assay (EC₅₀ in µg/mL)** | **Protein A HPLC mg/L** |
|---|---|---|---|---|---|---|
| X02.95 | A2P | 13 | 53 | 4.40 x10⁻¹² | N/T | 11.1 |
| X02.96 | A8P | 13 | 54 | 2.50 x10⁻¹⁰ | 30.0 | 19.9 |
| X02.97 | L11S | 13 | 55 | 7.51 x10⁻¹² | Low Binding | 15.0 |
| X02.98 | R29S | 13 | 56 | 5.84 x10⁻¹² | N/T | 8.7 |
| X02.99 | Y30S | 13 | 57 | 3.21 x10⁻¹² | 4.4 | 11.5 |
| X02.100 | H(54A)D | 13 | 58 | 1.60 x10⁻⁷ | Low Binding | 18.9 |
| X02.101 | V(66B)A | 13 | 59 | 2.89 x10⁻¹¹ | 13.7 | 21.0 |
| X02.102 | T68A | 13 | 60 | 1.20 x10⁻¹⁰ | 20.4 | 18.0 |
| X02.103 | S70T | 13 | 61 | 1.28 x10⁻⁹ | 9.4 | 27.9 |
| X02.104 | T90I | 13 | 62 | 1.0 x10⁻⁸ | 9.7 | 23.1 |
| X02.105 | S92P | 13 | 63 | 3.31 x10⁻⁸ | 12.7* | 19.3 |
| X02.106 | G95A | 13 | 64 | 4.47 x 10⁻⁸ | N/T | 11.6 |

| | | | | | | |
|---|---|---|---|---|---|---|
| N/T - Protein not purified or not tested. Low Binding - Minimal binding observed, not sufficient for an EC₅₀ value. *Antibody was tested in a flow binding assay against H929 cell line. Reported value is the EC₅₀ in µg/mL. | | | | | | |

Antibodies X02.96, X02.99, X02.101, X02.102, X02.103 and X02.104 bound strongly to the CD38 positive ARP-1 cell line. X02.105 was able to bind strongly to the CD38 positive, H929 cell line.

Amino acid sequence analysis of the variable heavy chain sequence of X02.1 and A02.1 identified amino acids that could potentially undergo oxidation or isomerization. These include a potential isomerization site at D101 and a potential oxidation site at M(100C). To remove the potential isomerization and oxidation sites, amino acid substitutions were made as follows: D(101)E (SEQ ID NO: 30), M(100C)L (SEQ ID NO: 29) and the combination of both D(101)E and M(100C)L (SEQ ID NO: 27) (Figure 2). Antibodies were made with combinations of these amino acid substitutions in the variable heavy chain as shown in Table 4. Antibody heavy chain variable regions were formatted with an IgG4 constant region containing the substitution S228P and co-expressed with the A02.1 light chain in CHO cells. Antibodies were then purified by Protein A chromatography and screened for binding to ARP-1 cells by flow cytometry. The binding data obtained is shown in Table 4.

**Table 4**

| **Antibody Designation** | **Variable Heavy Chain Amino Acid Substitution (Relative to X02.1)** | **Variable Heavy SEQ ID NO:** | **Variable Light SEQ ID NO:** | **CD38 binding by SPR (KD)** | **ARP-1 flow binding assay (EC₅₀ in µg/mL)** | **Protein A HPLC mg/L** |
|---|---|---|---|---|---|---|
| X02.76 | M(100C)L | 29 | 14 | 1.58 x 10⁻¹³ | 4.1 | 24.8 |
| X02.77 | D101E | 30 | 14 | 9.11 x 10⁻¹² | 3.7 | 15.6 |
| X02.74 | M(100C)L, D101E | 27 | 14 | 6.85 x 10⁻¹¹ | N/T | 21.8 |

| | | | | | | |
|---|---|---|---|---|---|---|
| N/T - Protein was not purified or not tested. | | | | | | |

Antibodies X02.76 and X02.77 maintained their strong binding to the ARP-1 cell line indicating that the amino acids substitutions to remove the potential oxidation and isomerization sites in the X02.1 and A02.1 heavy chain had little impact on their CD38 binding activity. Combining these substitutions to form antibody X02.74 resulted in an antibody that did not purify using the protocol in Example 5.

Amino acid analysis of the variable light chain sequence of X02.1 and A02.1 identified amino acids that could potentially undergo oxidation or deamidation. These included a potential deamidation site at N69 and potential oxidation site at M89. Additionally a putative N-linked glycosylation site was predicted to exist within CDR3 of the light chain at position N94. The presence of N-linked glycans can cause heterogeneity in therapeutic proteins, complicating development. To remove these potential issues the following point variants were synthesized: N69A (SEQ ID NO: 39), M89L (SEQ ID NO: 52) and M89I (SEQ ID NO: 51), N94T (SEQ ID NO: 48), N94Q (SEQ ID NO: 38), G95P (SEQ ID NO: 50) and S96A (SEQ ID NO: 45) (see Figure 3). Antibodies were generated by co-expression of the heavy- and light chains in CHO cells as detailed in Table 5. Antibodies were purified by Protein A chromatography and screened for binding to ARP-1 cells by flow cytometry. The binding data obtained is presented in Table 5.

**Table 5**

| **Antibody Designation** | **Variable Light Chain Amino Acid Substitution (Relative to A02.1)** | **Variable Heavy SEQ ID NO:** | **Variable Light SEQ ID NO:** | **CD38 binding by SPR** | **ARP-1 flow binding assay (EC₅₀ in µg/mL)** | **Protein A HPLC mg/L** |
|---|---|---|---|---|---|---|
| X02.81 | N69A | 13 | 39 | 2.67 x10⁻¹⁰ | N/T | 12.6 |
| X02.93 | M89I | 13 | 51 | 2.56 x10⁻¹¹ | 18.7 | 20.0 |
| X02.94 | M89L | 13 | 52 | 3.48 x10⁻¹² | 8.7 | 18.9 |
| X02.90 | N94T | 13 | 48 | 5.52 x10⁻¹⁰ | 26.2 | 23.0 |
| X02.80 | N94Q | 13 | 38 | 1.44 x10⁻⁹ | 13.2 | 30.3 |
| X02.92 | G95P | 13 | 50 | Low Binding | Low Binding | 18.1 |
| X02.87 | S96A | 13 | 45 | 1.99 x10⁻⁹ | 37.5 | 18.9 |

| | | | | | | |
|---|---|---|---|---|---|---|
| N/T - Protein was not purified or tested. Low Binding - Minimal binding observed, not sufficient for an EC₅₀ or KD value. | | | | | | |

X02.94 bound the CD38 positive cell line ARP-1 indicating that the substitution M89L had little impact on CD38 binding activity. The substitution N94Q in antibody X02.80 removed the potential N-linked glycosylation motif with minimal impact on CD38 binding activity as measure by flow cytometry (Table 5). Other substitutions that remove this glycosylation motif either resulted in antibodies that could not easily be purified or antibodies that exhibited attenuated binding to the CD38 positive cell line ARP-1. The potential deamidation site at position 69 was removed through substitution to alanine , though this antibody (X02.81) was not easily purified.

Other antibodies tested that comprised X02.1 variable heavy chain variants are listed in Table 6. These heavy chain variable regions were formatted on an IgG4 constant region containing an S228P substitution. These heavy chains were co-expressed with the A02.1 light chain in CHO cells. The antibodies were expressed and the resulting antibodies tested in flow cytometry-based assays for binding to the CD38-positive cell ARP-1. All variable heavy chain substitutions with the exception of T23K (SEQ ID NO: 21; X02.68) had minimal impact on binding to the CD38 positive cell line ARP-1 in flow cytometry-based assays.

**Table 6**

| **Antibody Designation** | **Variable Heavy Chain Amino Acid Substitution (Relative to X02.1)** | **Variable Heavy SEQ ID NO:** | **Variable Light SEQ ID NO:** | **CD38 binding by SPR** | **ARP-1 flow binding assay (EC₅₀ in µg/mL)** | **Protein A HPLC mg/L** |
|---|---|---|---|---|---|---|
| X02.73 | S19K | 26 | 14 | 3.85 x10⁻¹⁰ | 4.3 | 20.6 |
| X02.68 | T23K | 21 | 14 | 1.06 x10⁻¹¹ | N/T | 17.0 |
| X02.70 | V71R | 23 | 14 | 3.54 x10⁻⁹ | 7.2 | 32.8 |
| X02.75 | T73K | 28 | 14 | 6.38 x10⁻¹⁰ | 4.9 | 17.8 |
| X02.72 | T83R | 25 | 14 | 1.63 x10⁻⁹ | 3.6 | 30.6 |

| | | | | | | |
|---|---|---|---|---|---|---|
| N/T - Protein not purified or tested. | | | | | | |

Antibodies comprising other light chain variable region substitutions in the X02.1 sequence were also produced. These variant light chains were combined with the X02.1 heavy chain formatted onto an IgG4 constant region containing the substitution S228P and expressed in CHO cells as described in Example 5. A summary of the heavy- and light chains used to produce these antibody variants is given in Table 7. Antibodies X02.83, X02.85, X02.91, X02.82 bound strongly to the CD38 positive cell line ARP-1.

**Table 7**

| **Antibody Designation** | **Variable Light Chain Amino Acid Substitution (Relative to A02.1)** | **Variable Heavy SEQ ID NO:** | **Variable Light SEQ ID NO:** | **CD38 binding via SPR** | **ARP-1 flow binding assay (EC₅₀ in µg/mL)** | **Protein A HPLC mg/L** |
|---|---|---|---|---|---|---|
| X02.83 | E17A | 13 | 41 | 2.69 x10⁻⁹ | 8.2 | 32.8 |
| X02.86 | D(27A)G | 13 | 44 | 4.28 x10⁻⁹ | 120.4 | 30.0 |
| X02.85 | ΔD(L66A)*** | 13 | 43 | 2.70 x10⁻¹⁰ | 6.3 | 17.6 |
| | ΔV(L66B)*** | | | | | |
| X02.107 | E83I, D85T | 13 | 65 | 2.47 x10^{-8##} | N/T | 10.0* |
| X02.91 | P26R | 13 | 49 | 7.07 x10⁻¹⁰ | 17.6 | 21.1 |
| X02.88 | N32R | 13 | 46 | Low binding | Low Binding | 33.5 |
| X02.82 | Y49R | 13 | 40 | N/T | 3.8 | 27.9 |
| X02.89 | Y51R | 13 | 47 | Low binding | No Binding | 25.7 |
| X02.84 | Y49R, Y51R | 13 | 42 | Low binding | No Binding | 35.0 |

| | | | | | | |
|---|---|---|---|---|---|---|
| N/T - Protein was not purified or tested. Low Binding - Minimal binding observed, not sufficient for an EC₅₀ value. *** Δ indicates that this amino acid present in A02.1 light chain was removed from this sequence. *estimated protein value based on protein A capture level by SPR. ##The SPR binding for X02.107 was evaluated in a separate experiment in which the KD of the parental antibody X02.1 was 2.7 x 10⁻⁸. The KD of the parental antibody X02.1 is 3.78 x10⁻¹⁰ in the SPR binding experiment for all other antibodies tested. | | | | | | |

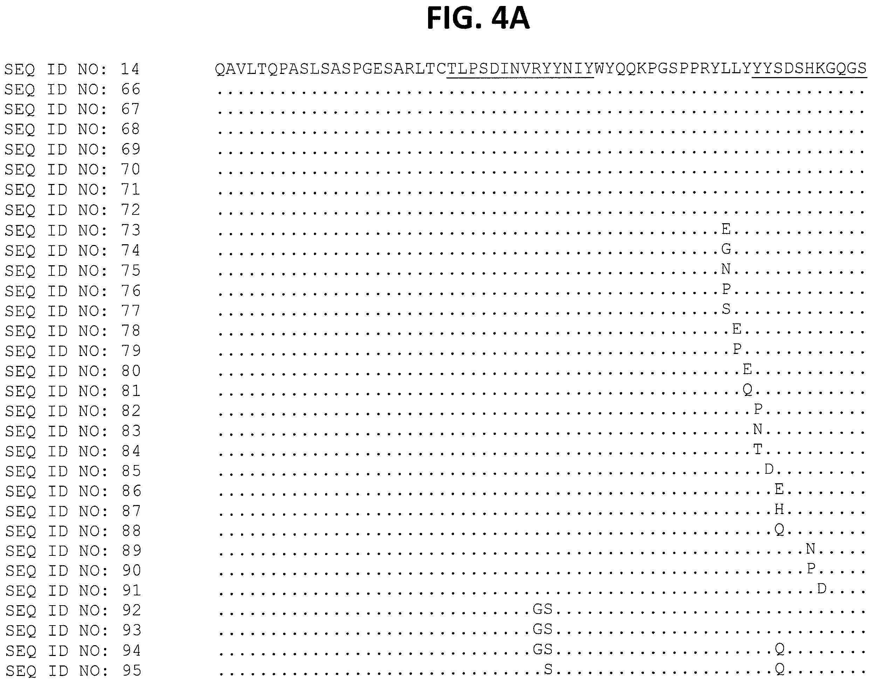

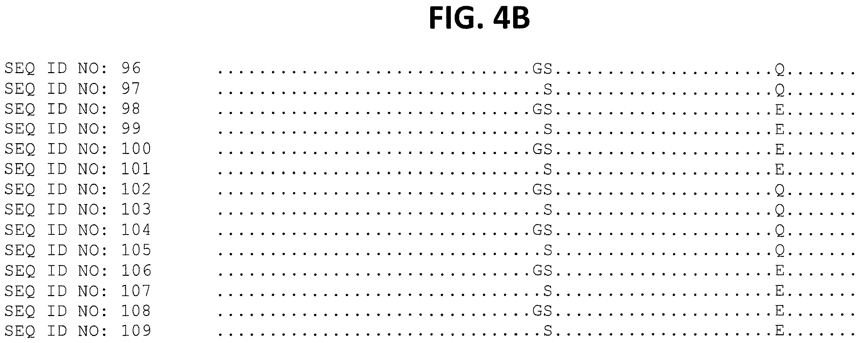

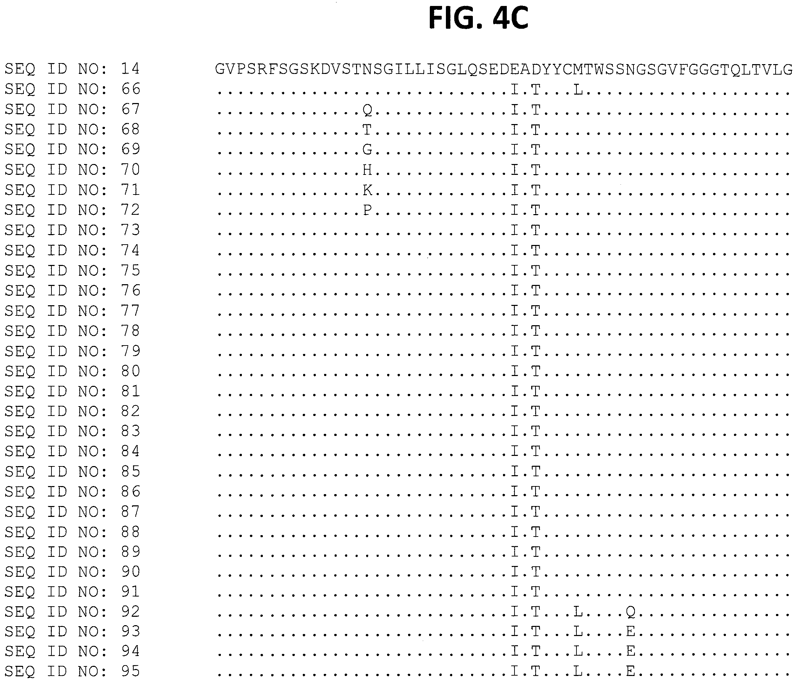

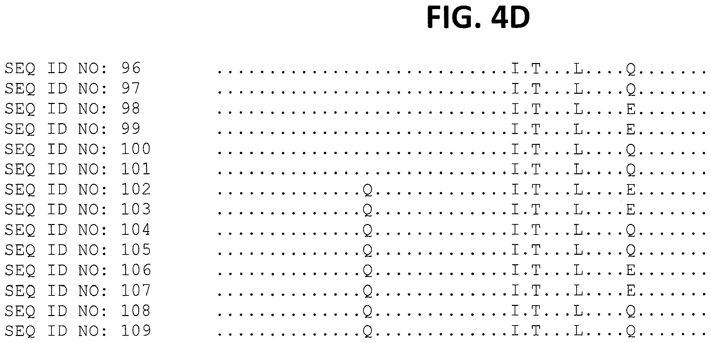

Substitutions causing little impact on CD38 binding activity and the purification of X02 variant antibodies were subsequently produced as armed antibodies through fusion to A145D attenuated IFN-alpha2b. X02.1 light chain substitutions were combined and the resulting variants co-expressed with point- and combinatorial variants of the X02.1 heavy chain in HEK293E cells, as listed in Table 8. These antibodies were primarily focused on removing the potential X02.1 light chain deamidation site, an oxidation site from CDR3 of the X02.1 heavy chain and a putative strong MHC Class II binding peptide from framework region 3 of the X02.1 heavy chain predicted via *in silico* analyses (Epibase, Lonza, UK), Figure 4.

**Table 8**

| **Anti-CD38-attenuated IFN fusion protein** | **Variable Heavy Chain Amino Acid Substitution (Relative to A02.1)** | **Variable Heavy SEQ ID NO:** | **Variable Light Chain Amino Acid Substitution (Relative to A02.1)** | **Variable Light SEQ ID NO:** |
|---|---|---|---|---|
| A02.2 | None | 13 | E83I, D85T | 65 |
| A02.3 | L74S | 17 | E83I, D85T | 65 |
| A02.4 | H40P | 18 | E83I, D85T | 65 |
| A02.5 | T(82A)S | 19 | E83I, D85T | 65 |
| A02.6 | L74S, I78F, R81K, T(82A)S | 20 | E83I, D85T | 65 |
| A02.12 | L74S, I78F, R81K, T(82A)S, M(100C)L | 34 | E83I, D85T | 65 |
| A02.13 | H40P, L74S, I78F, R81K, T(82A)S | 35 | E83I, D85T | 65 |
| A02.37 | L74S, I78F, R81K, T(82A)S, M(100C)L | 34 | E83I, D85T, M89L | 66 |
| A02.46 | L74S, I78F, R81K, T(82A)S, M(100C)L | 34 | E83I, D85T, N69Q | 67 |
| A02.47 | L74S, I78F, R81K, T(82A)S, M(100C)L | 34 | E83I, D85T, N69T | 68 |
| A02.48 | L74S, I78F, R81K, T(82A)S, M(100C)L | 34 | E83I, D85T, N69G | 69 |
| A02.49 | L74S, I78F, R81K, T(82A)S, M(100C)L | 34 | E83I, D85T, N69H | 70 |
| A02.50 | L74S, I78F, R81K, T(82A)S, M(100C)L | 34 | E83I, D85T, N69K | 71 |
| A02.51 | L74S, I78F, R81K, T(82A)S, M(100C)L | 34 | E83I, D85T, N69P | 72 |

The antibodies listed in Table 8 were analyzed for protein expression and binding to CD38 via surface plasmon resonance (SPR). Potency assays were also performed using cell culture supernatant taken from transfected cells to assess the relative activity of each of these anti-CD38-attenuated IFN fusion proteins as outlined in Example 5. The data obtained is given in Table 9.

**Table 9**

| **Anti-CD38-attenuated IFN fusion protein** | **Protein A HPLC (mg/L)** | **CD38 binding by SPR (RU) at 350 sec*** | **Annexin V Assay (Fold change relative to untreated cells)** | **Caspase Assay (Fold change relative to untreated cells)** | **Cell Proliferation Assay IC₅₀ (pM)** |
|---|---|---|---|---|---|
| A02.2 | 30.2 | 756 | N/T | N/T | N/T |
| A02.3 | 21.6 | 835 | N/T | N/T | N/T |
| A02.4 | 27.3 | 809 | N/T | N/T | N/T |
| A02.5 | 22.0 | 788 | N/T | N/T | N/T |
| A02.6 | 33.7 | 895 | N/T | N/T | N/T |
| A02.12 | 25.3 | N/A | 2.7 | 7.0 | 236 |
| A02.46 | 3.1 | 914 | 2.2 | 5.8 | 1190 |
| A02.47 | 26.5 | 1455 | 3.10 | 4.44 | N/T |
| A02.48 | 3.4 | 921 | 2.0 | 5.6 | 562 |
| A02.49 | 3.0 | 946 | 2.1 | 5.5 | 875 |
| A02.50 | 3.1 | 809 | 1.9 | 6.1 | 1681 |
| A02.51 | 1.8 | 368 | 2.0 | 5.8 | 3741 |

| | | | | | |
|---|---|---|---|---|---|
| The CD38 binding by SPR refers to the amount of CD38 that remains bound to the surface after 350 seconds of the dissociation phase. Annexin V Assay refers to cells positively stained by Annexin V-FITC after 24 h treatment with antibody constructs at 20 nM. Caspase Assay refers to caspase activation of cells after 24 h treatment with antibody constructs at 20 nM. N/A - Not Available; N/T - Not Tested. | | | | | |

Of the proteins tested A02.12 expressed well and demonstrated potency in the Annexin V, Caspase and cell proliferation assays. Substitution of N69T in antibody A02.47 did not affect expression levels or potency in Annexin V or Caspase Assays suggesting that removal of this deamidation site is possible. Substitution N69T could be incorporated into other constructs herein to remove this putative deamidation site with minimal losses in the functional activity of the resulting antibody.

### Reference Example 2

### In silico immunogenicity analysis of the A02.1 light chain amino acid sequence

Putative immunogenic epitopes were identified in the light chain variable region amino acid sequence of A02.1 using the Epibase analysis software (Lonza, UK). To remove putative immunogenic epitopes, substitutions were introduced into the A02.1 variable light chain (Figure 4). Light chains with lower predicted immunogenicity were co-expressed in HEK293E cells with the A02.12 heavy chain variable region (SEQ ID NO: 34) formatted onto an IgG4 constant region containing the substitution S228P fused to A145D-attenuated IFN. The antibody variants produced are detailed in Table 10.

**Table 10**

| **Anti-CD38-attenuated IFN fusion protein** | **Variable Heavy Chain Amino Acid Substitution (Relative to A02.1)** | **Variable Heavy SEQ ID NO:** | **Variable Light Chain Amino Acid Substitution (Relative to A02.1)** | **Variable Light SEQ ID NO:** |
|---|---|---|---|---|
| A02.18 | L74S, I78F, R81K, T(82A)S, M(100C)L | 34 | E83I, D85T, L47E | 73 |
| A02.19 | L74S, I78F, R81K, T(82A)S, M(100C)L | 34 | E83I, D85T, L47G | 74 |
| A02.20 | L74S, I78F, R81K, T(82A)S, M(100C)L | 34 | E83I, D85T, L47N | 75 |
| A02.21 | L74S, I78F, R81K, T(82A)S, M(100C)L | 34 | E83I, D85T, L47P | 76 |
| A02.22 | L74S, I78F, R81K, T(82A)S, M(100C)L | 34 | E83I, D85T, L47S | 77 |
| A02.23 | L74S, I78F, R81K, T(82A)S, M(100C)L | 34 | E83I, D85T, L48E | 78 |
| A02.24 | L74S, I78F, R81K, T(82A)S, M(100C)L | 34 | E83I, D85T, L48P | 79 |
| A02.25 | L74S, I78F, R81K, T(82A)S, M(100C)L | 34 | E83I, D85T, Y49E | 80 |
| A02.26 | L74S, I78F, R81K, T(82A)S, M(100C)L | 34 | E83I, D85T, Y49Q | 81 |
| A02.27 | L74S, I78F, R81K, T(82A)S, M(100C)L | 34 | E83I, D85T, Y50P | 82 |
| A02.28 | L74S, I78F, R81K, T(82A)S, M(100C)L | 34 | E83I, D85T, Y50N | 83 |
| A02.29 | L74S, I78F, R81K, T(82A)S, M(100C)L | 34 | E83I, D85T, Y50T | 84 |
| A02.30 | L74S, I78F, R81K, T(82A)S, M(100C)L | 34 | E83I, D85T, Y51D | 85 |
| A02.31 | L74S, I78F, R81K, T(82A)S, M(100C)L | 34 | E83I, D85T, S52E | 86 |
| A02.32 | L74S, I78F, R81K, T(82A)S, M(100C)L | 34 | E83I, D85T, S52H | 87 |
| A02.33 | L74S, I78F, R81K, T(82A)S, M(100C)L | 34 | E83I, D85T, S52Q | 88 |
| A02.34 | L74S, I78F, R81K, T(82A)S, M(100C)L | 34 | E83I, D85T, H(54A)N | 89 |
| A02.35 | L74S, I78F, R81K, T(82A)S, M(100C)L | 34 | E83I, D85T, H(54A)P | 90 |
| A02.36 | L74S, I78F, R81K, T(82A)S, M(100C)L | 34 | E83I, D85T, K(54B)D | 91 |

The above antibodies were analyzed for protein expression, binding to CD38 via SPR, and potency using the cell culture supernatant screen as described in Example 5. The results of these assays are detailed in Table 11. These data indicate that substitution of some residues to lower the predicted immunogenicity of the antibody results in Anti-CD38-attenuated IFN fusion proteins that express and have functional potency in the Annexin V, Caspase and Cell Proliferation Assays.

**Table 11**

| **Anti-CD38-attenuated IFN fusion protein** | **Protein A HPLC (mg/L)** | **CD38 binding by SPR (RU) at 350 sec*** | **Annexin V Assay (Fold change relative to untreated cells)** | **Caspase Assay (Fold change relative to untreated cells)** | **Cell Proliferation Assay IC₅₀ (pM)** |
|---|---|---|---|---|---|
| A02.18 | 9.3 | 745 | N/T | N/T | N/T |
| A02.19 | 8.0 | 741 | N/T | N/T | N/T |
| A02.20 | 9.1 | N/T | 2.2 | 5.6 | 37 |
| A02.21 | 3.3 | DNB | N/T | N/T | N/T |
| A02.22 | 10.4 | 738 | N/T | N/T | N/T |
| A02.23 | 15.9 | 192 | 2.7 | 6.9 | N/T |
| A02.24 | 23.5 | 87 | 1.4 | 2.3 | N/T |
| A02.25 | 25.7 | 80 | 3.0 | 4.3 | 2477 |
| A02.26 | 35.0 | 383 | 3.2 | 6.0 | 66 |
| A02.27 | 12.3 | DNB | 1.3 | 2.5 | 29910 |
| A02.28 | 16.1 | 422 | 2.7 | 6.3 | N/T |
| A02.29 | 19.7 | 150 | 2.7 | 5.8 | 133 |
| A02.30 | 25.2 | 122 | 1.9 | 2.5 | N/T |
| A02.31 | 28.4 | 359 | 3.0 | 7.1 | 514 |
| A02.32 | 13.5 | 663 | 2.7 | 7.7 | 60 |
| A02.33 | 11.2 | 107 | N/T | N/T | N/T |
| A02.34 | 16.6 | 407 | 4.8 | 5.1 | 503 |
| A02.35 | 11.2 | 738 | 2.4 | 4.8 | 3050 |
| A02.36 | 16.7 | 192 | 2.8 | 8.0 | N/T |

| | | | | | |
|---|---|---|---|---|---|
| The CD38 binding by SPR refers to the amount of CD38 that remains bound to the surface after 350 seconds of the dissociation phase. Annexin V Assay refers to cells positively stained by Annexin V-FITC after 24 h treatment with antibody constructs at 20 nM. Caspase Assay refers to caspase activation of cells after 24 h treatment with antibody constructs at 20 nM. DNB - Did not bind; N/T - Not Tested. | | | | | |

### Reference Example 3

### Multiple amino acid substitutions yield optimized A02.1 variants

By combining substitutions that improve the immunogenicity, manufacturability or potency of the anti-CD38 antibodies described above into a single gene construct, highly optimized anti-CD38 antibodies and anti-CD38-attenuated IFN fusion proteins were obtained. Table 12 summarizes such combinatorial substitutions and details heavy- and light chain combinations co-expressed in HEK293E cells and subsequently tested.

**Table 12**

| **Anti-CD38-attenuated IFN fusion protein** | **Variable Heavy Chain Amino Acid Substitution (Relative to A02.1)** | **Variable Heavy SEQ ID NO:** | **Variable Light Chain Amino Acid Substitution (Relative to A02.1)** | **Variable Light SEQ ID NO:** |
|---|---|---|---|---|
| A02.16 | L74S, I78F, R81K, T(82A)S, M(100C)L | 34 | R29G, Y30S E83I, D85T, M89L, N94Q | 92 |
| A02.17 | L74S, I78F, R81K, T(82A)S, M(100C)L | 34 | R29G, Y30S E83I, D85T, M89L, N94E | 93 |
| A02.52 | L74S, I78F, R81K, T(82A)S, M(100C)L | 34 | R29G, Y30S, S52Q, E83I, D85T, M89L, N94E | 94 |
| A02.53 | L74S, I78F, R81K, T(82A)S, M(100C)L | 34 | Y30S, S52Q, E83I, D85T, M89L, N94E | 95 |
| A02.54 | L74S, I78F, R81K, T(82A)S, M(100C)L | 34 | R29G, Y30S, S52Q, E83I, D85T, M89L, N94Q | 96 |
| A02.55 | L74S, I78F, R81K, T(82A)S, M(100C)L | 34 | Y30S, S52Q, E83I, D85T, M89L, N94Q | 97 |
| A02.56 | L74S, I78F, R81K, T(82A)S, M(100C)L | 34 | R29G, Y30S, S52E, M89L, E83I, D85T, N94E | 98 |
| A02.57 | L74S, I78F, R81K, T(82A)S, M(100C)L | 34 | Y30S, S52E, E83I, D85T, M89L, N94E | 99 |
| A02.58 | L74S, I78F, R81K, T(82A)S, M(100C)L | 34 | R29G, Y30S, S52E, E83I, D85T, M89L, N94Q | 100 |
| A02.59 | L74S, I78F, R81K, T(82A)S, M(100C)L | 34 | Y30S, S52E, E83I, D85T, M89L, N94Q | 101 |
| A02.60 | L74S, I78F, R81K, T(82A)S, M(100C)L | 34 | R29G, Y30S, S52Q, N69Q, E83I, D85T, M89L, N94E | 102 |
| A02.61 | L74S, 178F, R81K, T(82A)S, M(100C)L | 34 | Y30S, S52Q, N69Q, E83I, D85T, M89L, N94E | 103 |
| A02.62 | L74S, I78F, R81K, T(82A)S, M(100C)L | 34 | R29G, Y30S, S52Q, N69Q, E83I, D85T, M89L, N94Q | 104 |
| A02.63 | L74S, I78F, R81K, T(82A)S, M(100C)L | 34 | Y30S, S52Q, N69Q, E83I, D85T, M89L, N94Q | 105 |
| A02.64 | L74S, I78F, R81K, T(82A)S, M(100C)L | 34 | R29G, Y30S, S52E, N69Q, E83I, D85T, M89L, N94E | 106 |
| A02.65 | L74S, I78F, R81K, T(82A)S, M(100C)L | 34 | Y30S, S52E, N69Q, E83I, D85T, M89L, N94E | 107 |
| A02.66 | L74S, I78F, R81K, T(82A)S, M(100C)L | 34 | R29G, Y30S, S52E, N69Q, E83I, D85T, M89L, N94Q | 108 |
| A02.67 | L74S, I78F, R81K, T(82A)S, M(100C)L | 34 | Y30S, S52E, N69Q, E83I, D85T, M89L, N94Q | 109 |

Each antibody described in Table 12 was analyzed for protein expression, binding to CD38 via SPR, and potency using cell culture supernatant. The resulting data is given in Table 13. These results demonstrate that combining substitutions predicted to be beneficial *in silico*gave rise to some Anti-CD38-attenuated IFN fusion proteins that expressed and had functional potency in the Annexin V, Caspase and Cell Proliferation Assays.

**Table 13**

| **Anti-CD38-attenuated IFN fusion protein** | **Protein A HPLC (mg/L)** | **CD38 binding by SPR (RU) at 350 sec*** | **Annexin V Assay (Fold change relative to untreated cells)** | **Caspase Assay (Fold change relative to untreated cells)** | **Cell Proliferation Assay IC₅₀ (pM)** |
|---|---|---|---|---|---|
| A02.14 | 8.1 | 1247 | 2.18 | 5.47 | 398 |
| A02.15 | 29.6 | 1409 | 3.92 | 5.62 | 491 |
| A02.16 | 5.7 | 1050 | 2.4 | 7.9 | 636 |
| A02.17 | 10.0 | 1103 | 3.7 | 5.7 | 467 |
| A02.52 | 2.8 | 416 | 2.0 | 5.4 | 2665 |
| A02.53 | 3.0 | 545 | 2.2 | 4.7 | 6338 |
| A02.54 | 1.8 | 250 | 1.9 | 5.9 | 15350 |
| A02.55 | 2.1 | 436 | 2.2 | 4.5 | 12740 |
| A02.56 | 2.0 | 178 | 1.7 | 4.3 | 13860 |
| A02.57 | 2.7 | 345 | 2.5 | 6.6 | 6363 |
| A02.58 | 2.3 | 273 | 1.9 | 4.9 | 9142 |
| A02.59 | 1.2 | 388 | 2.0 | 4.7 | 6176 |
| A02.60 | 1.3 | DNB | 1.5 | 3.3 | 185600 |
| A02.61 | 1.2 | DNB | 1.7 | 4.1 | 65160 |
| A02.62 | 1.3 | DNB | 1.8 | 5.0 | 55590 |
| A02.63 | 1.2 | DNB | 1.7 | 3.4 | 152100 |
| A02.64 | 1.1 | DNB | 1.8 | 3.2 | 89120 |
| A02.65 | 1.4 | DNB | 1.6 | 4.1 | 37240 |
| A02.66 | 1.3 | DNB | 1.6 | 4.0 | 57540 |
| A02.67 | 1.6 | DNB | 2.3 | 3.9 | 82760 |

| | | | | | |
|---|---|---|---|---|---|
| The CD38 binding by SPR refers to the amount of CD38 that remains bound to the surface after 350 seconds of the dissociation phase. Annexin V Assay refer to cells positively stained by Annexin V-FITC after 24 h treatment with antibody constructs at 20 nM. Caspase Assay refers to caspase activation of cells after 24 h treatment with antibody constructs at 20 nM DNB - Did not bind; N/T - Not Tested. | | | | | |

### Reference Example 4

### Pairing of different heavy and light chain Anti-CD38 antibodies

In order to determine if functional anti-CD38-attenuated IFN fusion proteins could be obtained, the heavy (SEQ ID NO: 110) and light (SEQ ID NO: 112) chains from the antibody X910/12-HC-L0- IFN-alpha (A145D) IgG4 described in PCT/AU2012/001323, and the heavy (SEQ ID NO: 111) and light (SEQ ID NO: 113) chains from the antibody X913/15-HC-L0- IFN-alpha (A145D) IgG4 described in PCT/AU2012/001323, were paired with each other in various combinations and with heavy and lights chains described in the foregoing examples. A summary of the heavy and light chain pairings is provided listed in Table 14.

**Table 14**

| **Anti-CD38-attenuated IFN fusion protein** | **Variable Heavy SEQ ID NO:** | **Variable Light SEQ ID NO:** |
|---|---|---|
| A02.38 | 34 | 113 |
| A02.39 | 34 | 112 |
| A02.40 | 111 | 65 |
| A02.41 | 110 | 65 |
| X913/15-HC-L0- IFN-alpha (A145D) IgG4 | 111 | 113 |
| A02.43 | 110 | 113 |
| A02.44 | 111 | 112 |
| X910/12-HC-L0- IFN-alpha (A145D) IgG4 | 110 | 112 |

Each antibody produced was analyzed for protein expression, binding to CD38 via SPR, and potency using the cell culture supernatant potency assays. The results of these assays are presented in Table 15a. These data show that pairing different heavy and light chains from distinct antibodies gave rise to some Anti-CD38-attenuated IFN fusion proteins that expressed and had functional potency in the Annexin V, Caspase and Cell Proliferation Assays.

**Table 15a**

| **Anti-CD38-attenuated IFN fusion protein** | **Protein A HPLC (mg/L)** | **CD38 binding by SPR (RU) at 350 sec*** | **Annexin V Assay (Fold change relative to untreated cells)** | **Caspase Assay (Fold change relative to untreated cells)** |
|---|---|---|---|---|
| A02.38 | 2.9 | DNB | 0.9 | 4.3 |
| A02.39 | 21.3 | 66 | 2.5 | 6.6 |
| A02.40 | 2.1 | DNB | 1.0 | 4.4 |
| A02.41 | 50.0 | 165 | 2.6 | 6.7 |
| X913/15-HC-L0-IFN-alpha (A145D) IgG4 | 8.2 | 96 | 2.2 | 5.4 |
| A02.43 | 2.5 | DNB | 0.9 | 4.1 |
| A02.44 | 2.2 | DNB | 1.0 | 4.5 |
| X910/12-HC-L0-IFN-alpha (A145D) IgG4 | 27.1 | 125 | 2.6 | 5.9 |

| | | | | |
|---|---|---|---|---|
| The CD38 binding by SPR refers to the amount of CD38 that remains bound to the surface after 350 seconds of the dissociation phase. Annexin V Assay refer to cells positively stained by Annexin V-FITC after 24 h treatment with antibody constructs at 20 nM. Caspase Assay refers to caspase activation of cells after 24 h treatment with antibody constructs at 20 nM. DNB - Did not bind. | | | | |

A selection of the above Anti-CD38-attenuated IFN fusion proteins were purified and analyzed for binding to CD38 positive cells in cell based assays. In addition potency assays were repeated to give a more accurate determination of the relative activity of each Anti-CD38-attenuated IFN fusion protein. The results of these assays are given in Table 15b.

**Table 15b**

| **Anti-CD38-attenuated IFN fusion protein** | **ARP-1 Flow binding (EC₅₀ in µg/mL)** | **Annexin V Assay(Fold change relative to untreated cells)** | **Caspase Assay (Fold change relative to untreated cells)** | **Cell Proliferation Assay IC50 (pM)** | **Figures** |
|---|---|---|---|---|---|
| A02.1 | 1.45 | 1.86 | 4.2 | 278.2 | 13, 14, 15, 16, 17, 18, 19 |
| A02.2 | 2.46 | 1.94 | 4.4 | 175.7 | 13, 19 |
| A02.3 | 0.96 | 1.86 | 3.5 | 254.9 | 13, 19 |
| A02.4 | 1.50 | 1.88 | 3.8 | 198.3 | 13, 19 |
| A02.5 | 1.45 | 1.88 | 4.3 | 146.3 | 13, 19 |
| A02.6 | 1.42 | 2.03 | 3.5 | 102.3 | 13, 15, 19, 20 |
| A02.8 | 1.93 | 1.91 | 3.8 | 125.5 | 13, 19 |
| A02.9 | 1.03 | 1.96 | 3.6 | 107.1 | 13, 19 |
| A02.10 | 1.48 | 1.95 | 3.8 | 125.6 | 13, 19 |
| A02.11 | 3.48 | 1.98 | 3.9 | 374 | 13, 19 |
| A02.12 | 3.40* | 1.40 | 3.4 | 23.66 | 14, 16, 19, 21 |
| A02.14 | 10.01* | 1.82 | 2.5 | 398.20 | 19 |
| A02.15 | 1.97* | 3.09 | 6.8 | 491.70 | 19 |
| A02.16 | 3.89* | 2.66 | 5.2 | 636.80 | 14, 19 |
| A02.17 | 9.32* | 2.23 | 3.2 | 467.1 | 14, 19 |
| A02.18 | 1.64 | 1.55 | 3.7 | 78.72 | 19 |
| A02.19 | 1.07 | 1.63 | 3.5 | 230.3 | 19 |
| A02.20 | 15.92* | 1.61 | 2.9 | 36 | 19 |
| A02.22 | 1.58 | 1.91 | 3.8 | 207 | 19 |
| A02.25 | 0.37* | 1.42 | 2.4 | 2477 | 19 |
| A02.26 | 37.99* | 1.56 | 2.0 | 66 | 19 |
| A02.27 | LB* | 1.06 | 1.0 | 29910 | 14, 16, 19 |
| A02.29 | 0.48* | 1.55 | 3.0 | 133 | 19 |
| A02.31 | 0.26* | 1.78 | 2.1 | 514 | 14, 16, 19 |
| A02.32 | LB* | 1.83 | 3.3 | 605 | 19 |
| A02.33 | 1.54 | 1.96 | 3.7 | 741 | 16, 19 |
| A02.34 | 0.89* | 3.06 | 4.7 | 503 | 19 |
| A02.35 | 3.44* | 1.71 | 1.5 | 3050 | 19 |
| A02.37 | LB* | 2.05 | 4.3 | 128 | 19 |
| A02.39 | LB* | 1.92 | 4.3 | 3714 | 19 |
| A02.41 | 0.78* | 2.01 | 3.4 | 554 | 14, 19 |
| A02.42 | LB* | 1.52 | 3.3 | 310 | 19 |
| A02.45 | 0.71* | 1.66 | 1.6 | 1697 | 19 |
| A02.47 | 16.35* | 1.53 | 4.69 | 144.3 | 19 |

| | | | | | |
|---|---|---|---|---|---|
| The flow binding refers to the concentration of antibody required to achieve 50% of maximal mean fluorescence intensity. Annexin V Assay refer to cells positively stained by Annexin V-FITC after 24 h treatment with antibody constructs at 20 nM. Caspase Assay refers to caspase activation of cells after 24 h treatment with antibody constructs at 20 nM. LB - low binding, not sufficient for an EC₅₀ value. *Antibody was tested in a flow binding assay against H929 cell line. Reported value is the EC₅₀ in µg/mL. | | | | | |

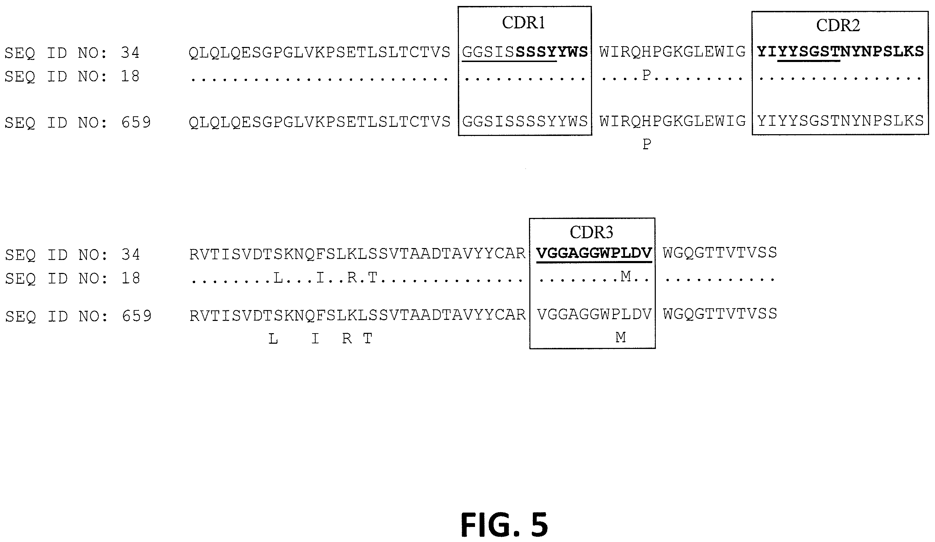

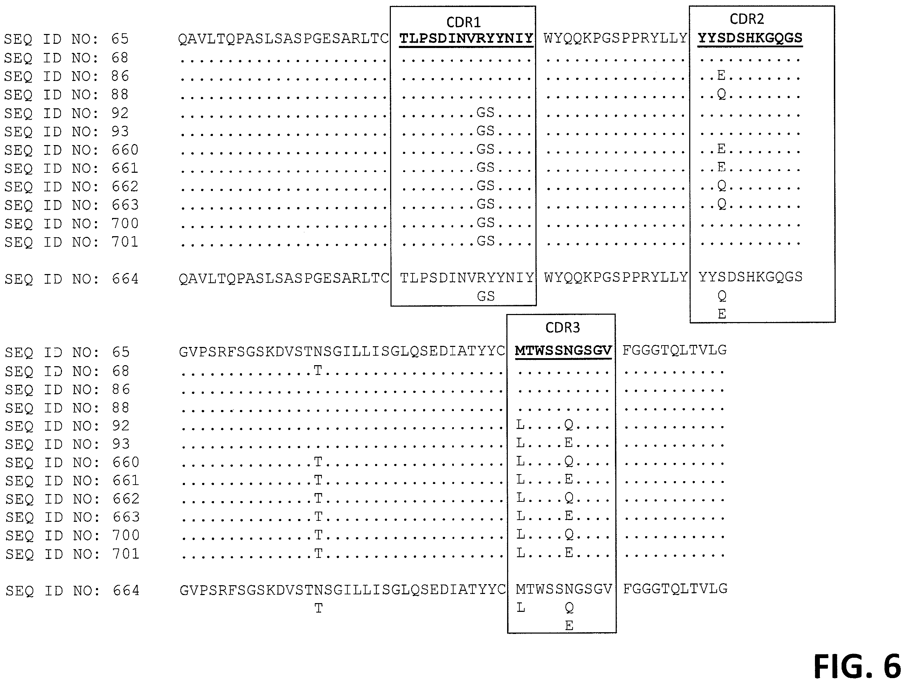

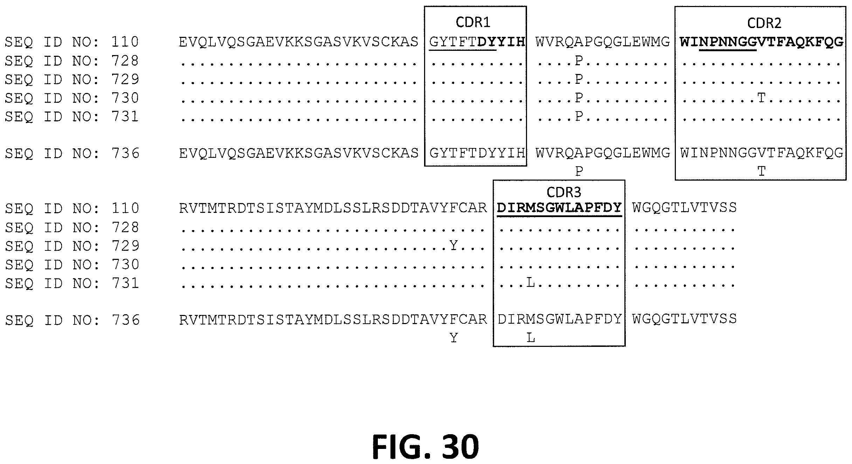

Figure 5 lists the consensus variable heavy chain and Figure 6 lists the consensus variable light chain of A02.1 related constructs with functional activity. It could be further envisioned that combinations of substitutions could be made such as those described for Anti-CD38 antibodies X02.114, X02.115, X02.116, X02.117, X02.118, X02.119 (Figure 6), X02.120, X02.121, X02.122, X02.123, X02.124, X02.125, X02.126 or X02.127 (Figure 30). Further the above Anti-CD38 antibodies could also be constructed as Anti-CD38-attenuated IFN fusion proteins and tested for functional activity as described herein.

### H929 multiple myeloma xenograft model

The *in vivo* potency of A02.1 has been tested previously in the NCI- H929 s.c. multiple myeloma model as described in Example 5. A02.1 was shown to have potent anti-tumor activity. The data is presented in PCT/AU2012/001323.

The H929 multiple myeloma xenograft model could be used to test the anti-tumor activity of any of the Anti-CD38-attenuated IFN fusion proteins described above.

### Attenuated IFN is required for potent apoptotic and caspase activation in tumor cell lines

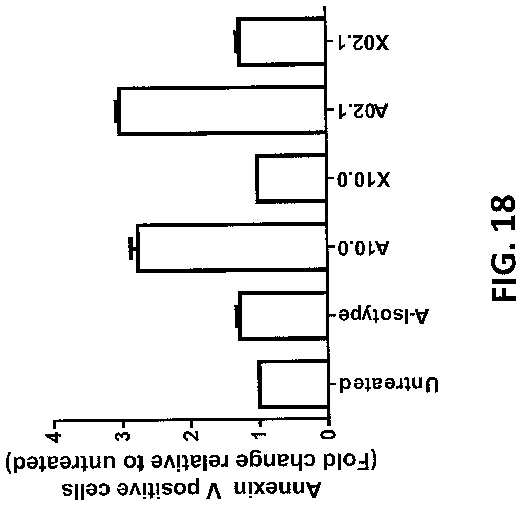

Using the Annexin V assay and the Caspase Assay it was demonstrated that the potent apoptotic activity and caspase activation is dependent on the Anti-CD38-attenuated IFN fusion proteins containing an attenuated IFN (Table 16a, Figure 18). In the Annexin V Assay the attenuated IFN containing proteins (A02.1 and A02.6) had 2-fold greater activity than the proteins not containing attenuated IFN (X02.1 and X02.6).

**Table 16a**

| **Anti-CD38-attenuated IFN fusion protein** | **Annexin V Assay (Fold change relative to untreated cells)** | **Caspase Assay (Fold change relative to untreated cells)** |
|---|---|---|
| A02.1 | 3.57 | 5.60 |
| X02.1 | 1.50 | 2.34 |
| A02.6 | 2.03 | 3.5 |
| X02.6 | 1.04 | 0.40 |

| | | |
|---|---|---|
| Annexin V Assay refer to cells positively stained by Annexin V-FITC after 24 h treatment with antibody constructs at 20 nM. Caspase Assay refers to caspase activation of cells after 24 h treatment with antibody constructs at 20 nM. | | |

### Example 1

### General Methods

Production of antibodies and antibody-fusion constructs in HEK-293E cells. DNA plasmids encoding protein constructs (antibodies and antibody-IFN-alpha2b related constructs) were prepared using HiSpeed Plasmid Maxi Kit (Qiagen, Valencia, CA) and then transfected into HEK293E cells (CNRC, Montreal, Canada), grown in F17 synthetic medium supplemented with 0.45% (w/v) D-(+)-Glucose (Sigma, Castle Hill, NSW), 25 µg/mL Geneticin (Invitrogen, Carlsbad, CA), and 1 x GlutaMAX (Invitrogen, Carlsbad, CA) using a commercially available transfection reagent and OptiMEM medium (Invitrogen, Carlsbad, CA). After allowing for expression for 6 days in an incubator supplied with 5% CO₂ and 120 rpm shaking, the culture media was isolated and subjected to affinity purification using Protein A Mab Select SuRe™ agarose beads (GE Healthcare, Pisataway, NJ). Purified protein constructs were buffer-exchanged into 0.2M arginine HCl, 25mM citric acid, 71.5mM sodium hydroxide at pH 6.0 using a PD Midi-Trap G-25 column (GE Healthcare, Piscataway, NJ) or a HiPrep 26/10 Desalting column (HiTrap Desalting HiPrep 26/10 Desalting). Purified protein constructs were then concentrated using 50 kDa Amicon Ultra centrifugal filter devices (Millipore, Billerica, MA), followed by protein concentration determination by reading absorbance at 280 nm.

### Production of antibodies and antibody-fusion constructs in CHO cells.

DNA plasmids encoding protein constructs (antibodies and antibody-IFN-alpha2b related constructs) were prepared using HiSpeed Plasmid Maxi Kit (Qiagen, Valencia, CA) and then transfected into CHO cells (Lonza) grown in Freestyle™ CHO Expression Medium (Invitrogen, Carlsbad, CA) using a commercially available transfection reagent and OptiPro SFM™ medium (Invitrogen, Carlsbad, CA). After allowing for expression for 6 days in an incubator supplied with 10% CO₂ and 120 rpm shaking, the culture media was isolated and subjected to affinity purification using Protein A Mab Select SuReagarose beads (GE Healthcare, Piscataway, NJ). Purified protein constructs were buffer-exchanged into 0.2M arginine.HCl, 25mM citric acid, 71.5mM sodium hydroxide at pH 6.0 using a PD Midi-Trap G-25 column (GE Healthcare, Piscataway, NJ) or a HiPrep 26/10 Desalting column (HiTrap Desalting HiPrep 26/10 Desalting). Purified protein constructs were then concentrated using 50 kDa Amicon Ultra centrifugal filter devices (Millipore, Billerica, MA), followed by protein concentration determination by reading absorbance at 280 nm.

Anti-CD38-attenuated IFN fusion proteins binding to CD38 as measured by Surface Plasmon Resonance (SPR). The capacity of anti-CD38 antibodies and anti-CD38-attenuated IFN fusion proteins to bind to human CD38 were measured using unpurified transfected cell supernatant prepared 7:1 with Non Specific Binding Reducer (GE Healthcare, Piscataway, NJ). Briefly, using a Biacore™ 3000 or a T200, Protein A was immobilized onto Flow Cell (FC) 1 (FC1) and FC2 (or alternatively FC3 and FC4) of a CM5 research grade sensor chip using amine coupling, giving approximately 1500 RU. FC2 (or FC4) was used as a reference throughout the experiments. The experiments were run at 37° C in HBS-P+ buffer (0.01 M HEPES, 0.15 M NaCl, 0.005% v/v Surfactant P20, pH 7.4). At a flow rate of 20 µl/min, both flow cells were regenerated with 10µL 50mM sodium hydroxide before 40 µL supernatant containing the protein was passed over FC1 (or FC3) only. 30µL of CD38 (10 µg/mL in running buffer) or 30 µL running buffer was injected over FC1 and FC2 with a 5 minute dissociation time. Both surfaces were regenerated twice with sodium hydroxide. Results were generated using the BIAevaluation software provided with the machine. Microsoft Excel was used for calculations. BIAevaluation software automatically subtracted the reference sensorgram giving a trace of FC2-1 (or FC4-3) for each sample. A double reference was performed for each antibody tested by subtracting the sensorgram with a CD38 injection from the sensorgram with a blank running buffer injection. The Protein A capture refers to the response units measured from a sensorgram at a fixed timepoint of 412.5s and this corresponds to the level of protein captured on the Protein A surface. CD38 binding is the response units measured at 507.5s and is an indication of the level of bound CD38 to the protein captured sensor. CD38 dissociation is the response units measured at 865.5s and is an indication of the level of CD38 bound to the protein captured surface after approximately 300s of dissociation phase. BIAevalution was used to fit the sensorgram using a Langmuir 1:1 equation in order to generate an equilibrium association constant (KD)

### Protein A HPLC.

Supernatants were analyzed by Protein A HPLC using a POROS A/20 2.1x30mm Id column (Applied Biosystems) connected to an Agilent 1100 chromatography system. The column was equilibrated with PBS pH7.4, and protein was eluted with PBS adjusted to pH 2.2. A standard curve was generated using known amounts of a monoclonal antibody in PBS. The chromatograms, at the wavelengths of 215 nm or 280 nm, were integrated using the manufacturer's software and the area under the curve (AUC) reported and interpolated against the generated standard curve to estimate concentration.

### Flow cytometry binding of antibodies and Anti-CD38-attenuated IFN fusion proteins to a human CD38 positive cell line, ARP-1 and H929.

The multiple myeloma cell line ARP-1 was a gift from Bart Barlogie MD, PhD, Director of the Myeloma Institute at the University of Arkansas Medical Center (Little Rock, AK). It is described in Hardin J. et al. (1994) Blood. 84:3063-70). The multiple myeloma cell line NCI-H929 (H929) was purchased from ATCC (CRL-9068, Gazdar, Blood 67: 1542-1549, 1986).

The ability of the antibodies or antibody-interferon constructs to bind the human CD38-positive myeloma cell lines ARP-1 or H929 in flow cytometry-based assays was tested. ARP-1 cells or H929 cells (5 x 10⁵, as judged by trypan blue exclusion) were incubated with each protein or with a human IgG4 monoclonal antibody with irrelevant specificity protein construct at various concentrations in 50 µL of FACS buffer (PBS plus 1% fetal calf serum, FCS, 0.2M HEPES, 0.5M EDTA) in 96 well plates for 60 minutes on ice in the dark. Cells were washed three times with FACS buffer before incubation for 30 minutes in 50 µL of FACS buffer containing goat anti-human IgG (Fc-specific, conjugated to fluorescein isothiocyanate, FITC; Sigma-Aldrich, St. Louis, MO). After washing three times with FACS buffer, cells were fixed with 50µL of PBS containing 4% formaldehyde v/v and incubated at 4° C in the dark for 16 hours. Incubated cells in suspension were diluted with an additional 150 µL of FACS buffer and analyzed for binding by flow cytometry on a FACS Canto II (BD Biosciences, San Diego, CA) using forward scatter, side scatter and fluorescence intensity in the FITC channel. The value reported is the mean fluorescence intensity (MFI).

### Target Assays

Daudi cell proliferation assay: This assay was used to quantify the anti-proliferative activity of IFNs and antibody-IFN fusion protein constructs on cells that display CD38. Daudi cells express CD38 as a cell surface associated antigen. The viability of cells was measured using the reagent CellTiter-Glo®, Cat #G7570, from Promega (Madison, Wisconsin). This is a luminescence-based assay that determines the viability of cells in culture based on quantitation of ATP. The signal strength is proportional to the number of viable cells in a microtiter plate well. The details of the assay are as follows: Daudi cells (obtained from ATCC, Manassas, VA) were cultured in a T75 flask (TPP, Trasadingen, Switzerland, cat# 90076) to a preferred density of between 0.5 x 10⁵ and 0.8 x 10⁵ viable cells/mL in RPMI 1640 (Mediatech, Inc., Manassas, VA, cat # 10-040- CV) with 10% Fetal Bovine Serum (FBS; Hyclone, Logan, UT cat# SH30070.03). Cells were harvested by centrifuging at 400 x g for five minutes, decanting the supernatant, and resuspending the cell pellet in RPMI 1640 + 10% FBS. Cells were then counted and the density was adjusted to 3.0 x 10⁵ cells/mL in RPMI 1640 + 10% FBS. Then, 50µL of cell suspension was aliquoted into each well of a 96 well round bottom tissue culture plate (hereafter, "experimental plate") (TPP, cat# 92067). On a separate, sterile 96 well plate (hereafter, "dilution plate"; Costar, Corning, NY cat# 3879), test articles were serially diluted in duplicate in RPMI 1640 + 10% FBS. Then, 50 µL/well was transferred from the dilution plate to the experimental plate. The experimental plate was then incubated for four days at 37° C with 5% CO₂. A mixture of the manufacturer-supplied assay buffer and assay substrate (hereafter, "CellTiter-Glo® reagent", mixed according to the manufacturer's instructions) was added to the experimental plate at 100 µL/well. The plate was shaken for two minutes.

Then, 100 µL/well was transferred from the experimental plate to a 96 well flat bottom white opaque plate (hereafter, "assay plate"; BD Biosciences, Franklin 5 Lakes, NJ cat# 35 3296). The content of the assay plate was then allowed to stabilize in the dark for 15 minutes at room temperature. The plate was read on a Victor 3V Multilabel Counter (Perkin Elmer, Waltham, MA, model# 1420-041) on the luminometry channel and the luminescence was measured. Results are presented as "relative luminescence units" (RLU).
Data was analyzed using Prism 5 (Graphpad, San Diego, CA) using non-linear regression and three parameter curve fit to determine the midpoint of the curve (EC50).

ARP-1 Cell proliferation assay: This assay was used to quantify the anti-proliferative activity of IFNs and antibody-IFN fusion protein constructs against CD38 antigen positive cells. ARP-1 cells express CD38 as cell surface associated antigens. The viability of cells was measured using the reagent CellTiter-Glo®, Cat #G7570, from Promega (Madison, Wisconsin). This is a luminescence-based assay that determines the viability of cells in culture by quantitation of ATP. The signal strength is proportional to the number of viable cells in a microtiter plate well.

The details of the assay are as follows: ARP-1 cells were cultured in a T175 flask (Costar, Corning, NY Lakes, NJ, cat# CLS431080) to a preferred density of between 2.0 x 10⁵ and 2.0 x 10⁶ viable cells/mL in RPMI 1640 (Life Technologies, Mulgrave, VIC, cat # 11875-093) with 10% Fetal Bovine Serum (FBS; AusGeneX, Molendinar, QLD, Australia cat# FBS500S). Cells were harvested by centrifuging at 400 x g for five minutes, decanting the supernatant, and resuspending the cell pellet in RPMI 1640 + 10% FBS. Cells were then counted and the density was adjusted to 2.0 x 10⁵ cells/mL in RPMI 1640 + 10% FBS. Then, 50 µl of the cell suspension was aliquoted into each well of a 96-well flat bottom white opaque plate (hereafter, "experimental plate"; Costar, Corning, NY Lakes, NJ, cat# CLS3917). On a separate, sterile 96-well plate (hereafter, "dilution plate"; Costar, Corning, NY cat# 3799), test articles were serially diluted in duplicate in RPMI 1640 + 10% FBS. Subsequently, 50 µL/well was transferred from the dilution plate to the experimental plate. The experimental plate was then incubated for four days at 37° C with 5% CO₂. Each experimental plate included the parental antibody IFN construct as the relative control.

A mixture of the manufacturer-supplied assay buffer and assay substrate (CellTiter-Glo® reagent, mixed according to the manufacturer's instructions) was added to the experimental plate at 100 µL/well. The plate was shaken for two minutes. The content of the assay plate was then allowed to stabilize in the dark for 15 minutes at room temperature. The plate was read on a FLUOstar Galaxy plate reader (BMG Labtech, Durham, NC) on the luminometry channel and the luminescence was measured. Data was analyzed using Prism 5 (Graphpad, San Diego, CA) using non-linear regression and three parameter curve fit to determine the midpoint of the curve (EC₅₀).

Annexin V assay: H929 cells were harvested by centrifuging at 400 x g for five minutes, decanting the supernatant, and resuspending the cell pellet in RPMI 1640 + 10% FBS. Cells were then counted and the density was adjusted to 1.0 x 10⁶ cells/mL in RPMI 1640 + 10% FBS. Then, 50 µL of the cell suspension was aliquoted into each well of 96-well round bottom clear plates (hereafter, "experimental plate;" Costar, Corning, NY cat# CL3799). On a separate, sterile 96-well plate (hereafter, "dilution plate"; Costar, Corning, NY cat# CL3799), test articles were diluted to 40 nM in quaduplicate in RPMI 1640 + 10% FBS. Subsequently, 50 µL/well was transferred from the dilution plate to the experimental plate. The experimental plate was then incubated for 24 hours at 37° C with 5% CO₂. The cells were then centrifuged at 400 x g for 5 min, supernatant decanted and resuspended in 100 µl of HEPES buffer containing Annexin V-FITC (1/200) and 7-AAD (1/50). The cells were then incubated for 15 min at room temperature and subsequently analyzed for Annexin V and 7-AAD staining by flow cytometry on a FACS Canto II (BD Biosciences, San Diego, CA) using forward scatter, side scatter, FITC and PerCP-Cy5.5 channels. Annexin V positive cells refer to cells positively stained by Annexin V-FITC after 24 h treatment with antibody constructs at 20 nM and is expressed as fold change relative to untreated cells.

Caspase assay: Activated caspases 2, 3, 6, 7, 8, 9, 10 were measured with the reagent Homogeneous Caspases Assay, fluorimetric Cat #12236869001, from Roche (West Sussex, UK) after treatment with test antibodies. The details of the assay follow.

ARP-1 cells, which express high levels of CD38, were cultured in a T175 flask (Costar, Corning, NY, cat# CLS431080) to a preferred density of between 2.0 x 10⁵ and 2.0 x 10⁶ viable cells/mL in RPMI 1640 (Life Technologies, Mulgrave, VIC, cat # 11875-093) with 10% FBS (AusGeneX, Molendinar, QLD, Australia cat# FBS500S). Cells were harvested by centrifuging at 400 x g for five minutes, decanting the supernatant, and resuspending the cell pellet in RPMI 1640 Phenol red-free (Life Technologies, Mulgrave, VIC, cat # 11835-030) + 10% FBS. Cells were then counted and the density was adjusted to 2.0 x 10⁵ cells/mL in RPMI 1640 Phenol red free + 10% FBS. Then, 50 µL of the cell suspension was aliquoted into each well of a 96-well flat bottom black-walled clear bottom plate (hereafter, "experimental plate"; Costar, Corning, NY cat# CLS3603). On a separate, sterile 96-well plate (hereafter, "dilution plate"; Costar, Corning, NY cat# 3799), test articles were diluted to 40 nM in quadruplicate in RPMI 1640 Phenol red free + 10% FBS. Subsequently, 50 µL/well was transferred from the dilution plate to the experimental plate. The experimental plate was then incubated for 24 hours at 37° C with 5% CO₂. The manufacturer-supplied assay buffer was added to the manufacturer-supplied substrate and mixed according to the manufacturer's instructions to create the "substrate solution." Then, 100 µL of the substrate solution was added to each well of the assay plate. The plate was shaken for 2 minutes. The plate was then incubated at room temperature for 15 minutes in the dark and finally read on FLUOstar Galaxy plate reader (BMG Labtech, Durham, NC) with an excitation filter 470-500 nm and emission filter 500-560 nm and the fluorescence measured and presented as fold change relative to untreated cells. Caspase Assay refers to caspase activation of cells after 24 h treatment with antibody constructs at 20 nM.

### Off-Target Assays

iLite gene reporter assay: The "off-target" iLite assay (PBL Interferon Source, Piscataway, NJ, Cat# 51100) was performed largely as described by the manufacturer, with the addition of a human IgG blocking step. The iLite cell line is described by the manufacturer as "a stable transfected cell line derived from a commercially available promonocytic human cell line characterized by the expression of MHC Class II antigens, in particular the human lymphocyte antigen (HLADR), on the cell surface." The cell line contains a stably transfected luciferase gene, the expression of which is driven by an interferon-response element (IRE), which allows for interferon activity to be quantified based on luminescence output. The manufacturer supplied iLite plate (hereafter, assay plate) and diluent were removed from the -80° C freezer and allowed to equilibrate to room temperature. Then, 50 µL of the diluent was added per well to the assay plate. The vial of manufacturer-supplied reporter cells was removed from the -80° C freezer and thawed in a 37° C water bath. Then, 25 µL aliquots of cells were dispensed into each well of the assay plate. Next, 12.5 µl of 8 mg/mL human IgG that was diluted into RPMI 1640 + 10% FBS (Sigma Chemicals, St. Louis, MO; cat# 14506) was added per well. The contents were mixed and incubated at 37° C for 15 minutes. On a separate "dilution plate," test articles were serially diluted in duplicate in RPMI 1640 + 10% FBS. Then, 12.5 µL of the test articles were transferred from the dilution plate to the assay plate. The assay plate was then incubated at 37° C with 5% CO₂ for 17 hours. The manufacturer-supplied assay buffer and substrate were removed from the -80° C freezer and allowed to equilibrate to room temperature for two hours. The manufacturer-supplied assay buffer was added to the manufacturer-supplied substrate vial and mixed well according to the manufacturer's instructions to create the "luminescence solution." Then, 100 µL of the luminescence solution was added to each well of the assay plate. The plate was shaken for 2 minutes. The plate was then incubated at room temperature for 5 minutes in the dark and finally read on a Victor 3V Multilabel Counter on a luminometry channel and the luminescence measured and presented as RLU. The data was analyzed with Graphpad Prism 5 as described for the "on-target (Daudi) assay." To test anti-CD38 antibody-IFN fusion protein constructs in the iLite assay, manufacturer-supplied diluent was supplemented with 0.25 mg/mL anti-CD38 antibody (same antibody clone being tested as an antibody-IFN fusion protein construct, to block any binding of the anti-CD38 antibody-IFN fusion protein constructs to the CD38 expressed on the iLite cells).

HEK-Blue™ Off-target assay: The assay was used to quantify the ability of antibody-IFN fusion constructs to bind interferon-alpha/β receptor (IFNAR) using the HEK-Blue™ IFN-alpha/β cell line (InvivoGen, San Diego, CA). The "off-target (HB-IFN) assay" was performed largely as described by the manufacturer of the HEK-Blue™ IFN-alpha/β cell line. HEK-Blue™ IFN-alpha/β Cells are specifically designed to monitor the activation of the JAK-STAT pathway, which is induced by type I IFNs. The cells were generated by introducing the human STAT2 and IRF9 genes into HEK293 cells to obtain a fully active type I IFN signalling pathway. The HEK-Blue™ IFN-alpha/β Cells stably express a reporter gene, secreted embryonic alkaline phosphatase (SEAP), under the control of the ISG54 promoter. ISG54 is a well-known ISG activated through an ISRE-dependent mechanism by type I IFNs. Upon IFN-alpha or IPNβ stimulation, HEK-Blue™ IFN-alpha/β cells activate the JAK-STAT pathway and then the expression of the SEAP reporter gene. SEAP is secreted into the media and can be quantitated using the colorimetric reagent QUANTI-Blue™. Briefly, HEK-Blue IFN-alpha/β cells (Invivogen, San Diego CA cat# hkb-ifnab) were thawed and cultured in DMEM media (Mediatech, Manassas VA, cat# 10-013-CV) + 10% FBS (Hyclone, Logan UT, cat# SH30070.03) that had been heat inactivated (HI FBS). When the cells reached 60-80% confluence, they were lifted with Cell Stripper (Mediatech, cat# 25-056-CI). Cells were washed twice in DMEM + HI FBS and counted. Cells were adjusted to 3.3 x 10⁵ viable cells/mL in DMEM + HI FBS and 150 µL was aliquoted per well into a flat bottom 96 well tissue culture plate (hereafter, the "experimental plate"). Then, 50µL of IFN-alpha2b or fusion protein construct, diluted into DMEM + HI FBS, was added per well. The plate was incubated at 37°C 5% CO₂ for 16-24 hours. QUANTI-Blue (Invivogen, cat# rep-qb1) was prepared according to the manufacturer's directions. QUANTI-Blue (150 µL) was aliquoted into each well of a flat bottom plate (hereafter, the "assay plate"). Then, 50 µL supernatant per well from the experimental plate was transferred to assay plate. Assay plate was then incubated at 37° C for 1-3 hours. Assay plate absorbance at 630nm was read on a model 1420-41 Victor 3V Multilabel Counter from Perkin-Elmer. Data was analyzed using Graph Pad Prism.

### H929 xenograft model

The effect of different doses of the A10.38 and A10.0 anti-CD38-attenuated IFN-alpha fusion protein constructs, were compared to the non-CD38-targeted fusion protein construct, on myeloma tumor growth. For these comparisons, the NCI- H929 s.c. multiple myeloma model was used.

The multiple myeloma cell line, NCI-H929 (ATCC CRL-9068, Gazdar, Blood 67: 1542-1549, 1986) is grown subcutaneously in immunocompromised (SCID) mice.

Eight to twelve week old CB.17 SCID mice were injected subcutaneously in the flank with 1 x 10⁷ NCI-H929 tumor cells in 50% Matrigel™. When average tumor size reached 170-350 mm³, mice were grouped into 4 cohorts of 7 mice each and treatment began at time zero (T0). All treatments were given by intraperitoneal injection, (i.p.) twice weekly for 3 weeks (indicated by bar under graph). All compounds were dosed at 100 µg/dose (approximately 4.5 mg/kg) except vehicle group. Tumor volume was measured twice weekly by caliper measurement. Endpoint was tumor volume of 2,000 mm³.

The effect of different doses of the A02. 6, A10.0 and A10.38 anti-CD38-attenuated IFN-alpha fusion protein constructs, were compared to vehicle, on myeloma tumor growth. For these comparisons, the NCI- H929 s.c. multiple myeloma model was used.

The multiple myeloma cell line, NCI-H929 (ATCC CRL-9068, Gazdar, Blood 67: 1542-1549, 1986) is grown subcutaneously in immunocompromised (SCID) mice.

Eight to twelve week old CB.17 SCID mice were injected subcutaneously in the flank with 1x10⁷ NCI-H929 tumor cells in 50% Matrigel. When average tumor size reaches 90mm³, mice will be grouped into 4 cohorts of 5 mice each and treatment begin at time zero (T0). All treatments will be given by intraperitoneal injection, (i.p.) twice weekly for 3 weeks (indicated by bar under graph). All compounds will be dosed at 100 µg/dose (approximately 4.5 mg/kg) except vehicle group. Tumor volume will be measured twice weekly by caliper measurement.

### Reference Example 5

### Anti-CD38-attenuated IFN fusion protein with alternative constant region

A02.12 comprises an anti-CD38-attenuated IFN fusion protein in which the constant region of the protein is HC-L0-IFN-alpha (A145D) IgG4 (SEQ ID NO: 9). The heavy chain variable region of this antibody was reformatted onto an IgG1 constant region fused to A145D attenuated IFN-alpha2b (SEQ ID NO: 10). Co-expression of this heavy chain with the light chain of X02.107 (SEQ ID NO: 65) in HEK293E cells yielded antibody A02.112. Comparison of antibodies A02.12 and A02.112 using flow cytometry-based CD38-binding assays and potency assays demonstrates that other antibody constant regions, such as human IgG1, may also be used resulting in antibody-attenuated IFN fusion proteins with potent biologic activity equivalent to those generated using a human IgG4 constant region (Table 16b).

**Table 16b**

| **Anti-CD38-attenuated IFN fusion protein** | **Flow binding (EC₅₀ in µg/mL)** | **Annexin V Assay (Fold change relative to untreated cells)** | **Caspase Assay (Fold change relative to untreated cells)** | **Cell Proliferation Assay IC₅₀ (pM)** | **Figures** |
|---|---|---|---|---|---|
| A02.12 | 3.40* | 1.40 | 2.70 | 23.66 | Fig 21 |
| A02.112 | <0.3* | 3.14 | 3.74 | N/T | Fig 21 |

| | | | | | |
|---|---|---|---|---|---|
| *Antibody was tested in a flow binding assay against H929 cell line. Reported value is the EC₅₀ in µg/mL.Annexin V Assay refer to cells positively stained by Annexin V-FITC after 24 h treatment with antibody constructs at 20 nM. Caspase Assay refers to caspase activation of cells after 24 h treatment with antibody constructs at 20 nM. N/T - Not Tested. | | | | | |

### Reference Example 6

### Humanization of R5D1, R5E8 and R10A2 variable regions

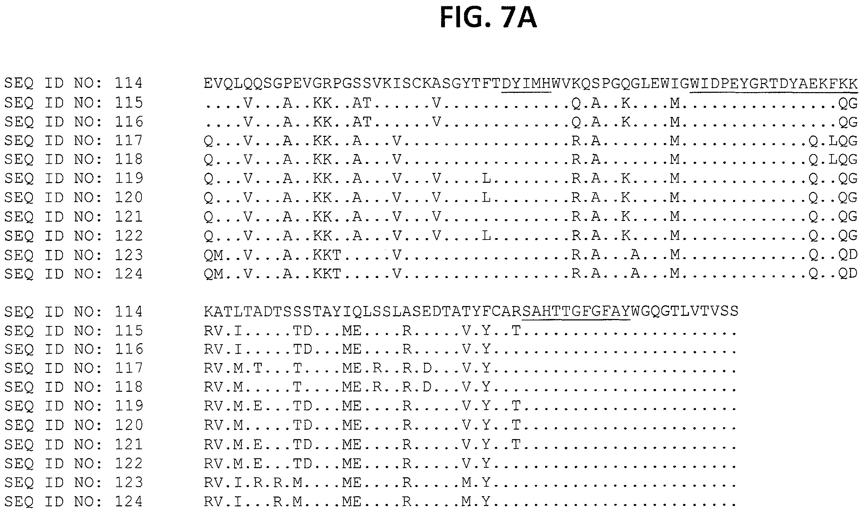

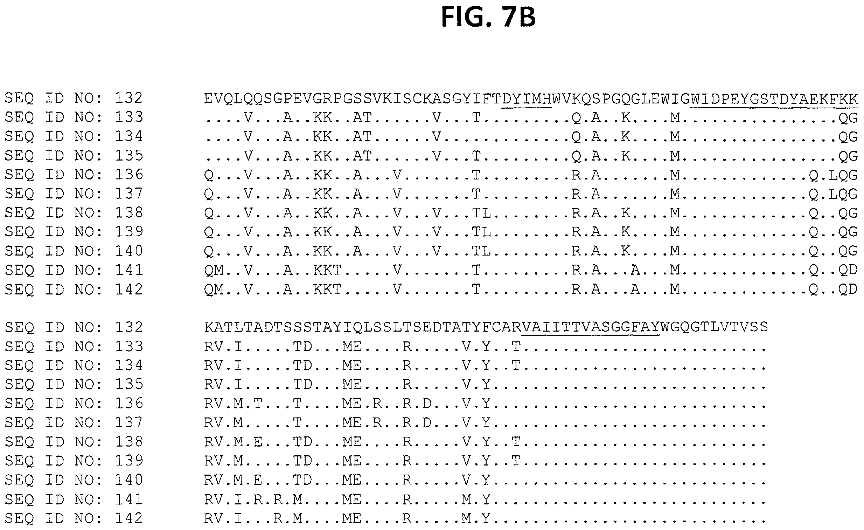

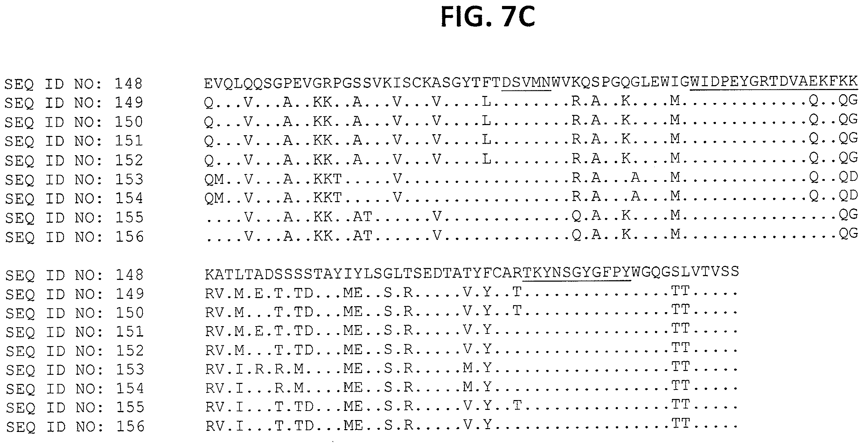

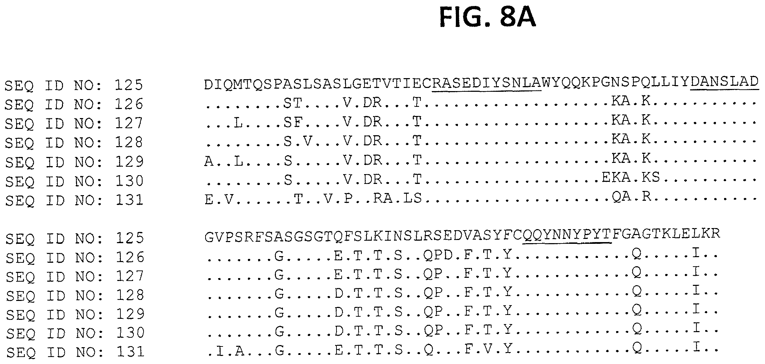

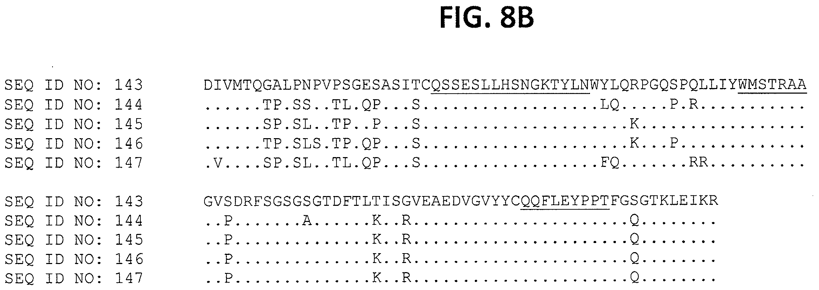

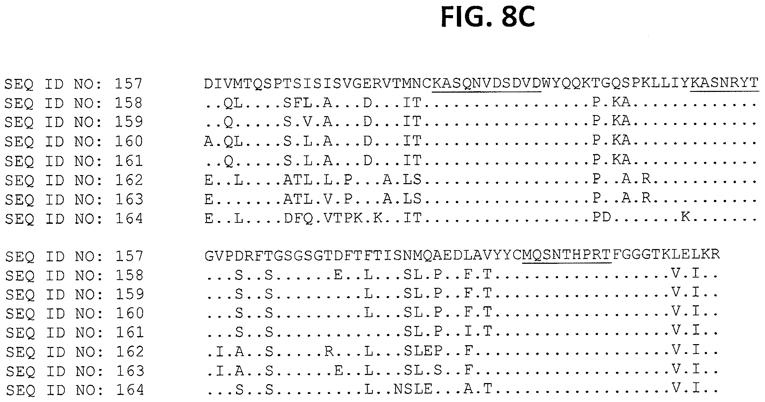

Rat-derived anti-CD38 antibodies R5D1, R5E8 and R10A2 are described in PCT/AU2012/001323 and were selected for humanization. The variable regions of these antibodies were superhumanized as described in U.S. Publ. No. 2003/0039649. Briefly, canonical structures were assigned to each rodent heavy and light chain through inspection of their respective amino acid sequences. R10A2 was assigned the canonical structure 2-1-1/1-2 (V_{L}/V_{H}), R5E8 was assigned the canonical structure 4-1-1/1-2, and R5D1 was assigned the canonical structure 2-1-1/1-2. Human germline sequences of the same canonical structure were used as acceptor frameworks for the grafting of donor CDRs. Variants of the resulting superhumanized antibody genes containing amino acid substitutions at positions within their sequences deemed likely to be important for maintenance of their binding activity were also designed. The different heavy chain superhumanized variable regions are shown in Figure 7. The different light chain superhumanized variable regions are shown in Figure 8.

Heavy chain variable region sequences were subcloned into vector pEE6.4 containing a human IgG4 constant region possessing the substitution S228P fused to A145D attenuated IFN-alpha2b (SEQ ID NO: 9). Light chain variable regions were subcloned into vector pEE12.4 containing a human kappa constant region (SEQ ID NO: 5). Antibodies were produced through co-expression of heavy chains in pEE6.4 and light chains in pEE12.4 in CHO cells as described previously. Table 17 summarises the heavy- and light chain pairings used to produce each superhumanized 5D1-based protein. Table 18 details the heavy- and light chain pairings for the superhumanized 5E8-based protein generated, whilst the heavy- and light chain pairings used to generate superhumanzied 10A2-based proteins are given in Table 19. One-shot equilibrium dissociation constant (K_{D}) ranking of the superhumanized antibodies was performed by BIAcore™ analysis of the resulting CHO transfection supernatants. The method was used to determine if the antibodies expressed (Protein A capture) and had a level of binding activity to human CD38.

**Table 17**

| **Anti-CD38-attenuated IFN fusion protein** | **Variable Heavy SEQ ID NO:** | **Variable Light SEQ ID NO:** | **K_{D}** _{(M)} | **Protein A capture (RU)** |
|---|---|---|---|---|
| A5D1.0 (chimeric) | 114 | 125 | 2.28 x 10⁻⁹ | N/A |
| A5D1.1 | 115 | 126 | 2.95 x 10⁻⁸ | 175 |
| A5D1.2 | 115 | 127 | 2.95 x 10⁻⁸ | 289 |
| A5D1.3 | 115 | 128 | 2.35 x 10⁻⁸ | 248 |
| A5D1.4 | 115 | 129 | 2.85 x 10⁻⁸ | 427 |
| A5D1.5 | 115 | 130 | 1.84 x 10⁻⁷ | 269 |
| A5D1.6 | 115 | 131 | 2.32 x 10⁻⁸ | 338 |
| A5D1.7 | 116 | 126 | 1.05 x 10⁻⁸ | 132 |
| A5D1.8 | 116 | 127 | 6.80 x 10⁻⁹ | 263 |
| A5D1.9 | 116 | 128 | 9.93 x 10⁻⁸ | 128 |
| A5D1.10 | 116 | 129 | 5.69 x 10⁻⁹ | 358 |
| A5D1.11 | 116 | 130 | 1.64 x 10⁻⁸ | 250 |
| A5D1.12 | 116 | 131 | 5.61 x 10⁻⁹ | 345 |
| A5D1.13 | 117 | 126 | 1.44 x 10⁻⁸ | 213 |
| A5D1.14 | 117 | 127 | 1.52 x 10⁻⁸ | 344 |
| A5D1.15 | 117 | 128 | 1.46 x 10⁻⁸ | 167 |
| A5D1.16 | 117 | 129 | 1.37 x 10⁻⁸ | 524 |
| A5D1.17 | 117 | 130 | 3.28 x 10⁻⁸ | 410 |
| A5D1.18 | 117 | 131 | 1.01 x 10⁻⁸ | 396 |
| A5D1.19 | 118 | 126 | 1.01 x 10⁻⁸ | 245 |
| A5D1.20 | 118 | 127 | 1.07 x 10⁻⁸ | 282 |
| A5D1.21 | 118 | 128 | 7.94 x 10⁻⁹ | 351 |
| A5D1.22 | 118 | 129 | 8.97 x 10⁻⁹ | 566 |
| A5D1.23 | 118 | 130 | 2.14 x 10⁻⁸ | 336 |
| A5D1.24 | 118 | 131 | 8.01 x 10⁻⁹ | 319 |
| A5D1.25 | 119 | 126 | DNB | 165 |
| A5D1.26 | 119 | 127 | DNB | 286 |
| A5D1.27 | 119 | 128 | DNB | 265 |
| A5D1.28 | 119 | 129 | DNB | 493 |
| A5D1.29 | 119 | 130 | DNB | 275 |
| A5D1.30 | 119 | 131 | DNB | 263 |
| A5D1.31 | 120 | 126 | 1.05 x 10⁻⁷ | 206 |
| A5D1.32 | 120 | 127 | 1.20 x 10⁻⁷ | 318 |
| A5D1.33 | 120 | 128 | 9.83 x 10⁻⁸ | 176 |
| A5D1.34 | 120 | 129 | 1.06 x 10⁻⁷ | 497 |
| A5D1.35 | 120 | 130 | 6.07 x 10⁻⁷ | 211 |
| A5D1.36 | 120 | 131 | 8.58 x 10⁻⁸ | 331 |
| A5D1.37 | 121 | 126 | 1.01 x 10⁻⁷ | 184 |
| A5D1.38 | 121 | 127 | 1.21 x 10⁻⁷ | 315 |
| A5D1.39 | 121 | 128 | 9.55 x 10⁻⁸ | 191 |
| A5D1.40 | 121 | 129 | 1.22 x 10⁻⁷ | 460 |
| A5D1.41 | 121 | 130 | 5.60 x 10⁻⁷ | 409 |
| A5D1.42 | 121 | 131 | 8.54 x 10⁻⁸ | 301 |
| A5D1.43 | 122 | 126 | 1.78 x 10⁻⁸ | 150 |
| A5D1.44 | 122 | 127 | 1.76 x 10-⁸ | 226 |
| A5D1.45 | 122 | 128 | 1.42 x 10⁻⁸ | 177 |
| A5D1.46 | 122 | 129 | 1.51 x 10⁻⁸ | 401 |
| A5D1.47 | 122 | 130 | 1.89 x 10⁻⁸ | 364 |
| A5D1.48 | 122 | 131 | 1.20 x 10⁻⁸ | 273 |
| A5D1.49 | 123 | 126 | 6.32 x 10⁻⁹ | 141 |
| A5D1.50 | 123 | 127 | 5.64 x 10⁻⁹ | 212 |
| A5D1.51 | 123 | 128 | 4.97 x 10⁻⁹ | 188 |
| A5D1.52 | 123 | 129 | 4.07 x 10⁻⁹ | 493 |
| A5D1.53 | 123 | 130 | 6.98 x 10⁻⁹ | 561 |
| A5D1.54 | 123 | 131 | 4.49 x 10⁻⁹ | 253 |
| A5D1.55 | 124 | 126 | 6.48 x 10⁻⁹ | 203 |
| A5D1.56 | 124 | 127 | 8.44 x 10⁻⁹ | 144 |
| A5D1.57 | 124 | 128 | 5.59 x 10⁻⁹ | 233 |
| A5D1.58 | 124 | 129 | 5.37 x 10⁻⁹ | 376 |
| A5D1.59 | 124 | 130 | 1.05 x 10⁻⁸ | 313 |
| A5D1.60 | 124 | 131 | 4.57 x 10⁻⁹ | 429 |

| | | | | |
|---|---|---|---|---|
| DNB - did not bind. N/A - Not available. | | | | |

**Table 18**

| **Anti-CD38-attenuated IFN fusion protein** | **Variable Heavy SEQ ID NO:** | **Variable Light SEQ ID NO:** | **K_{D}** _{(M)} | **Protein A capture (RU)** |
|---|---|---|---|---|
| A5E8.0 (chimeric) | 132 | 143 | 5.50 x 10⁻⁹ | N/A |
| A5E8.1 | 133 | 144 | 2.31 x 10⁻⁷ | 267 |
| A5E8.2 | 133 | 145 | 2.37 x 10⁻⁷ | 459 |
| A5E8.3 | 133 | 146 | 3.59 x 10⁻⁷ | 281 |
| A5E8.4 | 133 | 147 | DNB | 420 |
| A5E8.5 | 134 | 144 | 1.75 x 10⁻⁷ | 172 |
| A5E8.6 | 134 | 145 | 1.57 x 10⁻⁷ | 611 |
| A5E8.7 | 134 | 146 | 2.58 x 10⁻⁷ | 201 |
| A5E8.8 | 134 | 147 | 8.09 x 10⁻⁷ | 308 |
| A5E8.9 | 135 | 144 | 1.05 x 10⁻⁸ | 153 |
| A5E8.10 | 135 | 145 | 2.13 x 10⁻⁸ | 503 |
| A5E8.11 | 135 | 146 | 2.69 x 10⁻⁸ | 372 |
| A5E8.12 | 135 | 147 | DNB | 212 |
| A5E8.13 | 136 | 144 | 3.98 x 10⁻⁸ | 301 |
| A5E8.14 | 136 | 145 | 1.26 x 10⁻⁷ | 543 |
| A5E8.15 | 136 | 146 | 1.39 x 10⁻⁷ | 504 |
| A5E8.16 | 136 | 147 | DNB | 284 |
| A5E8.17 | 137 | 144 | 2.76 x 10⁻⁸ | 397 |
| A5E8.18 | 137 | 145 | 8.81 x 10⁻⁸ | 430 |
| A5E8.19 | 137 | 146 | 1.09 x 10⁻⁷ | 220 |
| A5E8.20 | 137 | 147 | DNB | 397 |
| A5E8.21 | 138 | 144 | DNB | 277 |
| A5E8.22 | 138 | 145 | DNB | 409 |
| A5E8.23 | 138 | 146 | DNB | 339 |
| A5E8.24 | 138 | 147 | DNB | 266 |
| A5E8.25 | 139 | 144 | DNB | 283 |
| A5E8.26 | 139 | 145 | DNB | 395 |
| A5E8.27 | 139 | 146 | DNB | 277 |
| A5E8.28 | 139 | 147 | DNB | 290 |
| A5E8.29 | 140 | 144 | 3.91 x 10⁻⁸ | 207 |
| A5E8.30 | 140 | 145 | 5.00 x 10⁻⁸ | 255 |
| A5E8.31 | 140 | 146 | 6.61 x 10⁻⁸ | 267 |
| A5E8.32 | 140 | 147 | DNB | 42 |
| A5E8.33 | 141 | 144 | 1.12 x 10⁻⁸ | 134 |
| A5E8.34 | 141 | 145 | 1.63 x 10⁻⁸ | 301 |
| A5E8.35 | 141 | 146 | 1.85 x 10⁻⁸ | 177 |
| A5E8.36 | 141 | 147 | DNB | 10 |
| A5E8.37 | 142 | 144 | 8.19 x 10⁻⁸ | 200 |
| A5E8.38 | 142 | 145 | 1.55 x 10⁻⁸ | 328 |
| A5E8.39 | 142 | 146 | 1.74 x 10⁻⁸ | 232 |
| A5E8.40 | 142 | 147 | DNB | 126 |

| | | | | |
|---|---|---|---|---|
| DNB - did not bind. N/A - not available. | | | | |

**Table 19**

| **Anti-CD38-attenuated IFN fusion protein** | **SEQ ID: Variable Heavy** | **SEQ ID: Variable Light** | **K_{D}** _{(M)} | **Protein A capture (RU)** |
|---|---|---|---|---|
| A10A2.0 (chimeric) | 148 | 157 | 5.98 x 10⁻¹⁰ | N/A |
| A10A2.1 | 149 | 158 | DNB | 728 |
| A10A2.2 | 149 | 159 | DNB | 689 |
| A10A2.3 | 149 | 160 | DNB | 850 |
| A10A2.4 | 149 | 161 | DNB | 996 |
| A10A2.5 | 149 | 162 | DNB | 761 |
| A10A2.6 | 149 | 163 | DNB | 890 |
| A10A2.7 | 149 | 164 | DNB | 725 |
| A10A2.8 | 150 | 158 | 7.18 x 10⁻¹ | 718 |
| A10A2.9 | 150 | 159 | 6.62 x 10⁻¹ | 627 |
| A10A2.10 | 150 | 160 | 9.13 x 10⁻⁷ | 850 |
| A10A2.11 | 150 | 161 | 2.37 x 10⁻⁷ | 956 |
| A10A2.12 | 150 | 162 | 1.18 x 10⁻⁶ | 864 |
| A10A2.13 | 150 | 163 | 6.80 x 10⁻⁷ | 765 |
| A10A2.14 | 150 | 164 | DNB | 645 |
| A10A2.15 | 151 | 158 | 1.15 x 10⁻⁷ | 488 |
| A10A2.16 | 151 | 159 | 8.11 x 10⁻⁸ | 759 |
| A10A2.17 | 151 | 160 | 1.84 x 10⁻⁷ | 684 |
| A10A2.18 | 151 | 161 | 3.39 x 10^{-8/} | 907 |
| A10A2.19 | 151 | 162 | 1.84 x 10⁻⁷ | 831 |
| A10A2.20 | 151 | 163 | 1.23 x 10⁻⁷ | 560 |
| A10A2.21 | 151 | 164 | DNB | 337 |
| A10A2.22 | 152 | 158 | 2.70 x 10⁻⁹ | 890 |
| A10A2.23 | 152 | 159 | 2.17 x 10⁻⁹ | 828 |
| A10A2.24 | 152 | 160 | 3.04 x 10⁻⁹ | 803 |
| A10A2.25 | 152 | 161 | 1.51 x 10⁻³ | 1054 |
| A10A2.26 | 152 | 162 | 3.51 x 10⁻⁹ | 741 |
| A10A2.27 | 152 | 163 | 2.42 x 10⁻⁹ | 603 |
| A10A2.28 | 152 | 164 | 3.69 x 10⁻⁸ | 384 |
| A10A2.29 | 153 | 158 | 2.77 x 10⁻⁸ | 93 |
| A10A2.30 | 153 | 159 | 2.15 x 10⁻⁸ | 86 |
| A10A2.31 | 153 | 160 | 5.82 x 10⁻⁸ | 33 |
| A10A2.32 | 153 | 161 | 8.49 x 10⁻⁹ | 169 |
| A10A2.33 | 153 | 162 | 5.66 x 10⁻⁸ | 62 |
| A10A2.34 | 153 | 163 | 3.88 x 10⁻⁸ | 56 |
| A10A2.35 | 153 | 164 | DNB | DNE |
| A10A2.36 | 154 | 158 | 8.38 x 10⁻⁹ | 221 |
| A10A2.37 | 154 | 159 | 1.39 x 10⁻⁹ | 858 |
| A10A2.38 | 154 | 160 | 1.08 x 10⁻⁸ | 178 |
| A10A2.39 | 154 | 161 | 3.80 x 10⁻⁹ | 357 |
| A10A2.40 | 154 | 162 | 1.34 x 10⁻⁸ | 217 |
| A10A2.41 | 154 | 163 | 8.73 x 10⁻⁹ | 202 |
| A10A2.42 | 154 | 164 | 2.09 x 10⁻¹ | 175 |
| A10A2.43 | 154 | 158 | 2.45 x 10⁻¹ | 621 |
| A10A2.44 | 155 | 159 | 6.23 x 10⁻⁹ | 220 |
| A10A2.45 | 155 | 160 | 2.84 x 10⁻⁷ | 881 |
| A10A2.46 | 155 | 161 | 1.39 x 10⁻⁷ | 1000 |
| A10A2.47 | 155 | 162 | 3.28 x 10⁻⁷ | 9 |
| A10A2.48 | 155 | 163 | 2.52 x 10⁻⁷ | 565 |
| A10A2.49 | 155 | 164 | DNB | 499 |
| A10A2.50 | 156 | 158 | 1.61 x 10⁻⁹ | 567 |
| A10A2.51 | 156 | 159 | 2.00 x 10⁻⁷ | 603 |
| A10A2.52 | 156 | 160 | 1.69 x 10⁻⁹ | 723 |
| A10A2.53 | 156 | 161 | 1.20 x 10⁻⁹ | 729 |
| A10A2.54 | 156 | 162 | 1.92 x 10⁻⁹ | 639 |
| A10A2.55 | 156 | 163 | 1.47 x 10⁻⁹ | 692 |
| A10A2.56 | 156 | 164 | 1.97 x 10⁻⁷ | 383 |

| | | | | |
|---|---|---|---|---|
| DNB - did not bind. DNE - did not express. | | | | |

For each family of humanized antibodies - 5D1, 5E8 and 10A2 - several humanized heavy and light chain combinations failed to either express protein, or to bind to human CD38. A considerable number of antibodies across all 3 families of humanized antibodies expressed and bound to human CD38 with equilibrium dissociation constants in the nanomolar (nM) range. A10A2.53 and A10A2.25, which share a common light chain were chosen for further optimization. A10A2.53 was renamed A10.0 and A10A2.25 was renamed A10.38.

### Example 2

### Improved variants of A10.0

The A10.0 antibody was optimized through alterations to the variable heavy and/or light chain sequences with the aim of yielding a positive effect on the biophysical and *in silico* immunogenicity of the antibody whilst causing minimal impact on the functional activity of the antibody.

### In-silico immunogenicity analysis of A10.0 heavy- and light chains

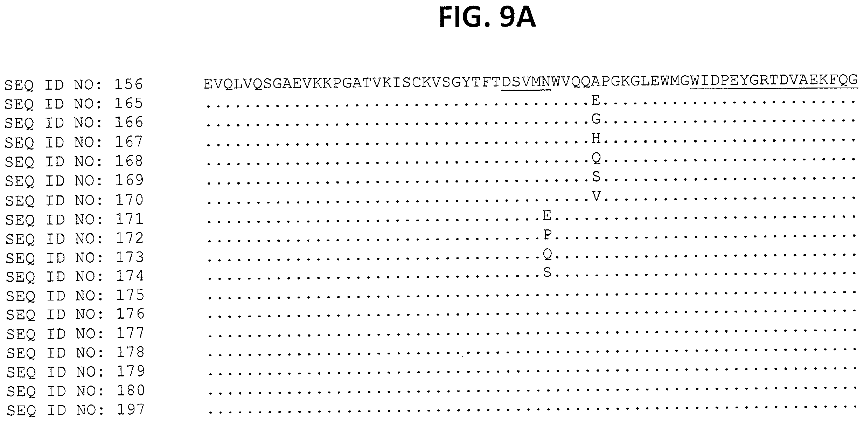

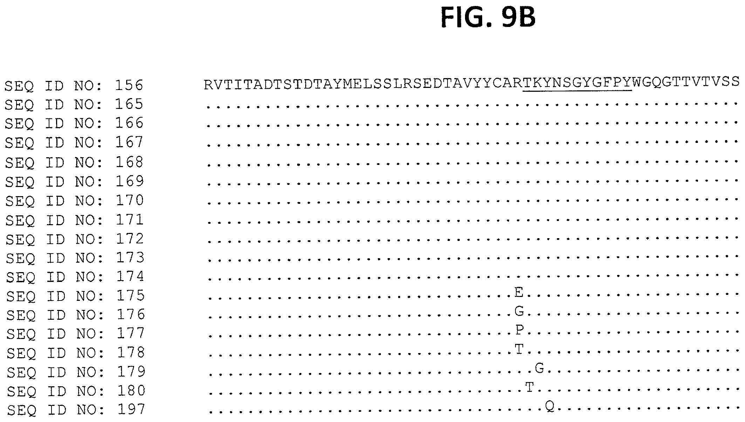

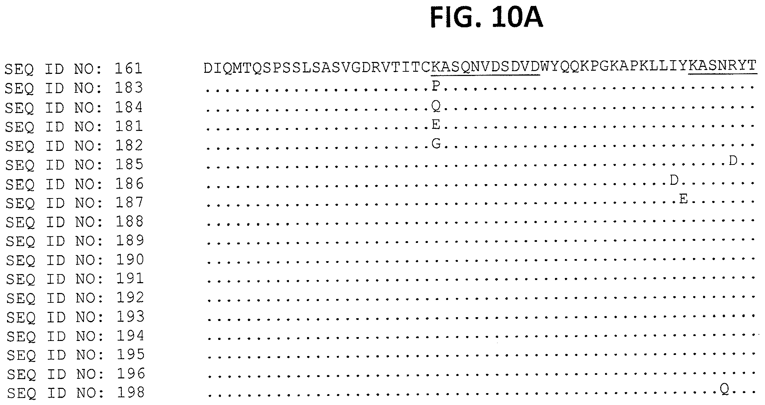

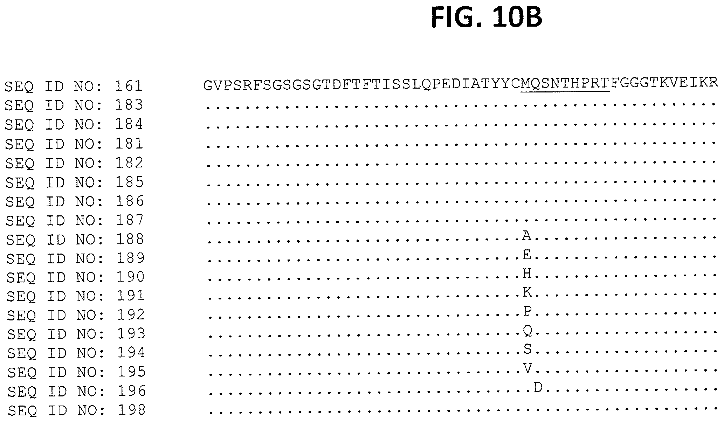

*In silico* immunogenicity analyses of the A10.0 heavy- and light chain variable regions were made using the Epibase software package. Several amino acid substitutions were introduced into the heavy- and light chain variable regions of A10.0 to remove potential immunogenic epitopes. An amino acid sequence alignment of the heavy chain variable region variants produced aligned with the humanised heavy chain (SEQ ID NO: 156) is shown in Figure 9. An amino acid sequence alignment of the light chain variable region variants aligned with the humanised light chain (SEQ ID NO: 161) is shown in Figure 10. Details of the heavy- and light chains variants co-expressed in HEK293E cells to produce proteins are summarised in Table 20.

**Table 20**

| **Anti-CD38-attenuated IFN fusion protein** | **VH Amino Acid Substitution (Relative to A10.0)** | **Variable Heavy SEQ ID NO:** | **VK Amino Acid Substitution (Relative to A10.0)** | **Variable Light SEQ ID NO:** |
|---|---|---|---|---|
| A10.1 | A40E | 165 | N/A | 161 |
| A10.2 | A40G | 166 | N/A | 161 |
| A10.3 | A40H | 167 | N/A | 161 |
| A10.4 | A40Q | 168 | N/A | 161 |
| A10.5 | A40S | 169 | N/A | 161 |
| A10.6 | A40V | 170 | N/A | 161 |
| A10.7 | N35E | 171 | N/A | 161 |
| A10.8 | N35P | 172 | N/A | 161 |
| A10.9 | N35Q | 173 | N/A | 161 |
| A10.10 | N35S | 174 | N/A | 161 |
| A10.11 | R94E | 175 | N/A | 161 |
| A10.12 | R94G | 176 | N/A | 161 |
| A10.13 | R94P | 177 | N/A | 161 |
| A10.14 | R94T | 178 | N/A | 161 |
| A10.15 | K96G | 179 | N/A | 161 |
| A10.16 | K96T | 180 | N/A | 161 |
| A10.17 | N/A | 156 | K24E | 181 |
| A10.18 | N/A | 156 | K24G | 182 |
| A10.19 | N/A | 156 | K24P | 183 |
| A10.20 | N/A | 156 | K24Q | 184 |
| A10.21 | N/A | 156 | R54D | 185 |
| A10.22 | N/A | 156 | 148D | 186 |
| A10.23 | N/A | 156 | Y49E | 187 |
| A10.24 | N/A | 156 | M89A | 188 |
| A10.25 | N/A | 156 | M89E | 189 |
| A10.26 | N/A | 156 | M89H | 190 |
| A10.27 | N/A | 156 | M89K | 191 |
| A10.28 | N/A | 156 | M89P | 192 |
| A10.29 | N/A | 156 | M89Q | 193 |
| A10.30 | N/A | 156 | M89S | 194 |
| A10.31 | N/A | 156 | M89V | 195 |
| A10.32 | N/A | 156 | Q90D | 196 |

Each antibody generated using the heavy- and light chain pairings outlined in Table 20 was assessed for protein expression level and binding to CD38 via SPR. Furthermore, potency assays were performed using cell culture supernatants to assess the relative functional activity of each of these anti-CD38 antibody-attenuated IFN fusion proteins, Table 21.

**Table 21**

| **Anti-CD38-attenuated IFN fusion protein** | **Protein A HPLC (mg/L)** | **CD38 binding by SPR (RU) at 350 sec*** | **Annexin V (Fold change relative to untreated cells) Assay** | **Caspase Assay (Fold change relative to untreated cells)** | **Cell Proliferation Assay (IC₅₀pM)** |
|---|---|---|---|---|---|
| A10.1 | 16.9 | 1824 | 1.66 | 3.41 | 4078 |
| A10.2 | 16.7 | 1821 | 1.66 | 5.19 | 7622 |
| A10.3 | 25.0 | 2166 | 1.63 | 5.46 | 2148 |
| A10.4 | 23.7 | 2169 | 1.63 | 5.78 | 4108 |
| A10.5 | 28.0 | 2240 | 1.64 | 5.80 | 3046 |
| A10.6 | 31.0 | 2097 | 1.57 | 5.76 | 2283 |
| A10.7 | 26.5 | DNB | 1.18 | 1.09 | No IC₅₀ |
| A10.8 | 2.4 | DNB | N/T | N/T | N/T |
| A10.9 | 18.3 | 176 | 1.48 | 2.07 | No IC₅₀ |
| A10.10 | 32.2 | 1072 | 1.57 | 4.97 | 18870 |
| A10.11 | 28.3 | 98 | 1.57 | 3.64 | No IC₅₀ |
| A10.12 | 30.7 | DNB | 1.22 | 1.99 | No IC₅₀ |
| A10.13 | 30.6 | 123 | 1.31 | 2.67 | No IC₅₀ |
| A10.14 | 30.5 | 247 | 1.19 | 5.11 | 68270 |
| A10.15 | 41.8 | 1254 | 1.52 | 5.44 | 5169 |
| A10.16 | 24.2 | 1210 | 1.70 | 4.57 | 5224 |
| A10.17 | 18.2 | 1686 | 1.79 | 6.11 | 3054 |
| A10.18 | 32.5 | 2457 | 1.89 | 6.16 | 2178 |
| A10.19 | 1.6 | DNB | 1.73 | 2.39 | No IC₅₀ |
| A10.20 | 12.2 | 1355 | 4.65 | 7.72 | 564 |
| A10.21 | 19.9 | 1837 | 1.84 | 5.56 | 5330 |
| A10.22 | 5.5 | 480 | N/T | N/T | N/T |
| A10.23 | 20.6 | 255 | 1.71 | 3.85 | 59720 |
| A10.24 | 34.6 | 1943 | 4.14 | 6.75 | 399 |
| A10.25 | 28.3 | 1778 | 1.87 | 6.09 | 4910 |
| A10.26 | 5.7 | 706 | N/T | N/T | N/T |
| A10.27 | 7.4 | 136 | N/T | N/T | N/T |
| A10.28 | 2.2 | 48 | N/T | N/T | N/T |
| A10.29 | 10.9 | 1443 | N/T | N/T | No IC₅₀ |
| A10.30 | 25.4 | 1865 | 1.98 | 6.21 | 1438 |
| A10.31 | 5.8 | 469 | N/T | N/T | N/T |
| A10.32 | 34.5 | 615 | 3.80 | 6.97 | 3628 |

| | | | | | |
|---|---|---|---|---|---|
| The CD38 binding by SPR refers to the amount of CD38 that remains bound to the surface after 350 seconds of the dissociation phase. Annexin V Assay refer to cells positively stained by Annexin V-FITC after 24 h treatment with antibody constructs at 20 nM. Caspase Assay refers to caspase activation of cells after 24 h treatment with antibody constructs at 20 nM. DNB - Did not bind; N/T- not tested; No IC₅₀ - potency not sufficient for an IC₅₀ value. | | | | | |

Analyses of the amino acid sequences of the variable heavy- and light chain sequences of A10.0 identified several potential deamidation sites and one potential oxidation site. Variable heavy chain substitution N98Q was prepared to remove a deamidation site from CDR3 of the heavy chain, SEQ ID NO: 197. A further variant of the A10.0 variable light chain containing the CDR2 substitution N53Q (SEQ ID NO: 198) was generated to remove this putative deamidation site. M89 within CDR3 of the light chain was also altered through amino acid substitutions at this position with the combined aims of removing this potential oxidation site and reducing the predicted immunogenicity of this region of the light chain. These substitutions are outlined in Table 22, along with the heavy and light chain pairings co-expressed to produce each anti-CD38-attenuated IFN fusion protein.

**Table 22**

| **Anti-CD38-attenuated IFN fusion protein** | **Amino Acid Substitution (Relative to A10.0)** | **Variable Heavy SEQ ID NO:** | **Variable Light SEQ ID NO:** |
|---|---|---|---|
| A10.35 | Heavy Chain N(98)Q | 197 | 161 |
| A10.36 | Light Chain N(53)Q | 156 | 198 |

Each antibody generated using the heavy- and light chain pairings outlined in Table 22 was assessed for protein expression level and binding to CD38 via SPR. Furthermore, potency assays were performed using cell culture supernatants to assess the relative functional activity of each of these anti-CD38 antibody-attenuated IFN fusion proteins, shown in Table 23.

**Table 23**

| **Anti-CD38-attenuated IFN fusion protein** | **Protein A HPLC (mg/L)** | **CD38 binding by SPR (RU) at 350 sec*** | **Annexin V assay (Fold change relative to untreated cells)Assay** | **Caspase Assay (Fold change relative to untreated cells)** | **Cell Proliferation Assay IC₅₀ (pM)** |
|---|---|---|---|---|---|
| A10.35 | 34.5 | 1889 | 1.83 | 6.16 | 6241 |
| A10.36 | 52.4 | 1895 | 3.95 | 5.90 | 534.9 |

| | | | | | |
|---|---|---|---|---|---|
| The CD38 binding by SPR refers to the amount of CD38 that remains bound to the surface after 350 seconds of the dissociation phase. Annexin V Assay refer to cells positively stained by Annexin V-FITC after 24 h treatment with antibody constructs at 20 nM. Caspase Assay refers to caspase activation of cells after 24 h treatment with antibody constructs at 20 nM. | | | | | |

### Example 3

### Generating improved variants of A10.38

A10.0 and A10.38 share a common light chain. The optimized light chain sequences of A10.0 were paired with the heavy chain of the A10.38 antibody with the aim of yielding a positive effect on the antibody's biophysical and *in silico* immunogenicity properties whilst having a minimal impact on functional activity. A summary of the changes and the pairings of heavy and light chains are described in Table 24.

**Table 24**

| **Anti-CD38-attenuated IFN fusion protein** | **SEQ ID: Variable Heavy** | **VK Amino Acid Substitution (Relative to A10.38)** | **SEQ ID: Variable Light** |
|---|---|---|---|
| A10.38 | 152 | N/A | 161 |
| A10.39 | 152 | K24E | 181 |
| A10.40 | 152 | K24G | 182 |
| A10.41 | 152 | K24P | 183 |
| A10.42 | 152 | K24Q | 184 |
| A10.43 | 152 | R54D | 185 |
| A10.44 | 152 | 148D | 186 |
| A10.45 | 152 | Y49E | 187 |
| A10.46 | 152 | M89A | 188 |
| A10.47 | 152 | M89E | 189 |
| A10.48 | 152 | M89H | 190 |
| A10.49 | 152 | M89K | 191 |
| A10.50 | 152 | M89P | 192 |
| A10.51 | 152 | M89Q | 193 |
| A10.52 | 152 | M89S | 194 |
| A10.53 | 152 | M89V | 195 |
| A10.54 | 152 | Q90D | 196 |
| A10.57 | 152 | N53Q | 198 |

Each of the above antibodies was assessed for protein expression level and binding to CD38 via SPR. Potency assays were performed using cell culture supernatants to assess the relative functional activity of each of these anti-CD38 antibody-attenuated IFN fusion proteins, Table 25.

**Table 25**

| **Anti-CD38-attenuated IFN fusion protein** | **Protein A HPLC (mg/L)** | **CD38 binding by SPR(RU) at 350 sec*** | **Annexin V Assay (Fold change relative to untreated cells)** | **Caspase Assay (Fold change relative to untreated cells)** |
|---|---|---|---|---|
| A10.38 | 119.7 | 1540 | 3.22 | 5.17 |
| A10.39 | 113.4 | 1444 | 3.39 | 4.79 |
| A10.40 | 117.8 | 1562 | 3.28 | 5.12 |
| A10.41 | 89.7 | 1459 | 3.27 | 5.12 |
| A10.42 | 111.7 | 1443 | 3.32 | 5.60 |
| A10.43 | 94.1 | 1426 | 3.21 | 6.15 |
| A10.44 | 51.9 | 969 | 3.08 | 5.66 |
| A10.45 | 111.7 | 333 | 2.76 | 5.01 |
| A10.46 | 120.0 | 1547 | 3.24 | 4.80 |
| A10.47 | 107.3 | 1337 | 3.45 | 4.25 |
| A10.48 | 45.5 | 865 | 3.06 | 5.48 |
| A10.49 | 55.8 | 213 | 3.46 | 7.63 |
| A10.50 | 11.3 | 172 | 2.96 | 5.61 |
| A10.51 | 51.6 | 1320 | 2.34 | 6.16 |
| A10.52 | 70.0 | 1512 | 3.21 | 5.62 |
| A10.53 | 40.0 | 536 | 3.46 | 4.68 |
| A10.54 | 61.3 | 583 | 3.10 | 6.20 |
| A10.57 | 67.1 | 1431 | 3.06 | 6.04 |

| | | | | |
|---|---|---|---|---|
| The CD38 binding by SPR refers to the amount of CD38 that remains bound to the surface after 350 seconds of the dissociation phase. Annexin V Assay refer to cells positively stained by Annexin V-FITC after 24 h treatment with antibody constructs at 20 nM. Caspase Assay refers to caspase activation of cells after 24 h treatment with antibody constructs at 20 nM. N/T - Not Tested. | | | | |

### Attenuated IFN is required for potent apoptotic and caspase activation in tumor cell lines

The relative potency of anti-CD38 antibodies A10.0 (attenuated IFN fusion) and X10.0 (no fusion) were compared using the Annexin V, Caspase and the Cell Proliferation Assays outlined in Example 5. The relative potency of A10.38 and X10.38 was also compared, Table 26.

**Table 26**

| **Anti-CD38-attenuated IFN fusion protein** | **Annexin V Assay (Fold change relative to untreated cells)** | **Caspase Assay (Fold change relative to untreated cells)** | **Cell Proliferation Assay IC₅₀ (pM)** | **Figures** |
|---|---|---|---|---|
| A10.0 | 2.10 | 4.23 | 2081 | Fig 18 |
| X10.0 | 1.27 | 1.70 | No IC50 | Fig 18 |
| A10.38 | 3.22 | 5.17 | 1118 | Fig 25 |
| X10.38 | 1.46 | 2.09 | No IC50 | Fig 25 |

| | | | | |
|---|---|---|---|---|
| Annexin V Assay refer to cells positively stained by Annexin V-FITC after 24 h treatment with antibody constructs at 20 nM. Caspase Assay refers to caspase activation of cells after 24 h treatment with antibody constructs at 20 nM. | | | | |

These data demonstrate the potent apoptotic activity exhibited by antibodies A10.0 and A10.38 relative to X10.0 and X10.38 respectively necessitates the presence of the attenuated IFN fusion. No anti-proliferative activity was observed with antibodies without an attenuated IFN.

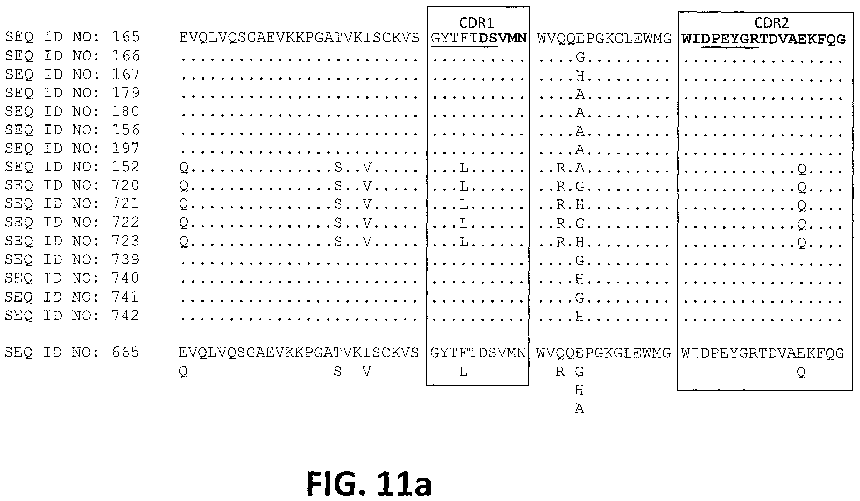

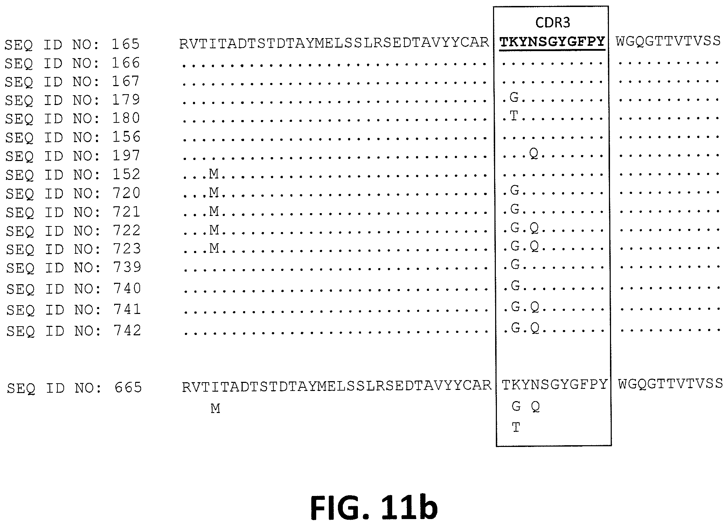

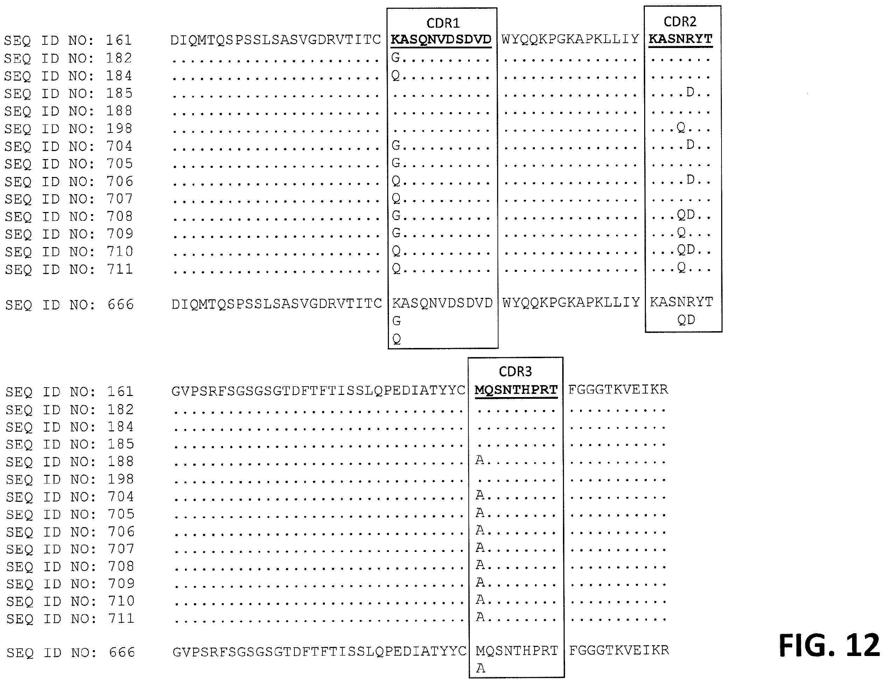

A consensus sequence alignment of heavy chain variable regions from proteins with functional activity is shown in Figure 11. A consensus sequence alignment of light chain variable regions from proteins with functional activity is shown in Figure 12. It could be further envisioned that combinations of substitutions could be made such as those described for Anti-CD38 antibodies X10.60, X10.61, X10.62, X10.63, X10.64, X10.65, X10.66, X10.67, X10.68, X10.69, X10.70, X10.71, X10.72, X10.73, X10.74, X10.75, X10.76, X10.77, X10.78, X10.79, X10.80, X10.81, X10.82, X10.83, X10.84, X10.85, X10.86, X10.87, X10.88, X10.89, X10.90, X10.91, X10.92, X10.93, X10.94, X10.95, X10.96, X10.97, X10.98, X10.99, X10.100, X10.101, X10.102, X10.103, X10.104, X10.105, X10.106, X10.107, X10.108, X10.109, X10.110, X10.111, X10.112, X10.113, X10.114, X10.115, X10.116, X10.117, X10.118, X10.119, X10.120, X10.121, X10.122, X10.123, X10.124, X10.125, X10.126, X10.127, X10.128, X10.129, X10.130, X10.131, X10.132, X10.133, X10.134, X10.135, X10.136, X10.137, X10.138, X10.139, X10.140, X10.141, X10.142, X10.143, X10.144, X10.145, X10.146, X10.147 (Figure 11, Figure 12). Further the above Anti-CD38 antibodies could also be constructed as Anti-CD38-attenuated IFN fusion proteins and tested for functional activity as described herein.

### H929 multiple myeloma xenograft model

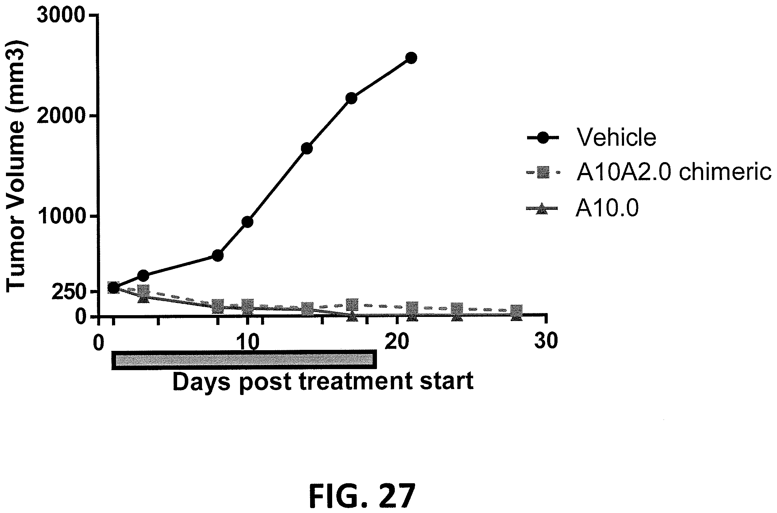

The *in vivo* potency of 10A2 variants A10.0 and A10A2.0 were evaluated in an NCI-H929 s.c. mouse multiple myeloma model, Figure 27. Both were shown to have potent antitumour activity in this model. Such a model could be used to test for anti-tumor activity of other protein constructs described within.

### Off-Target Activity for the 10A2 variants

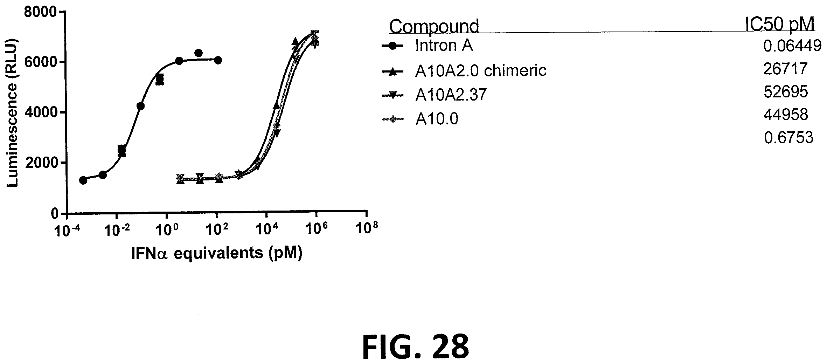

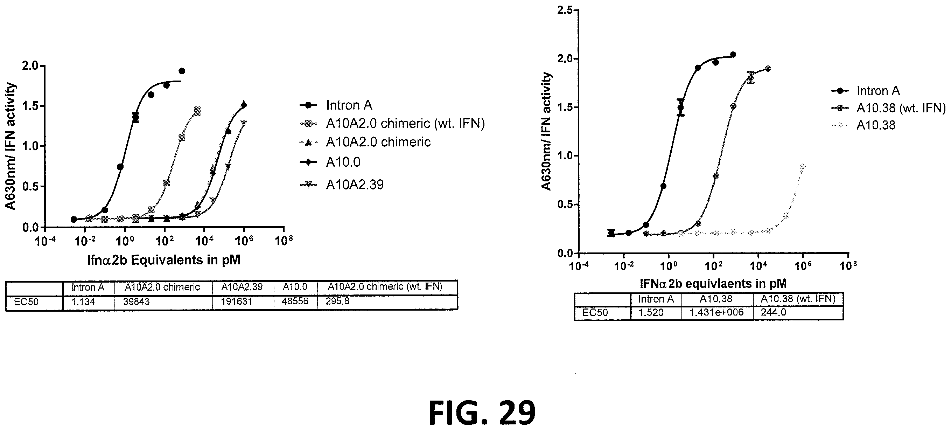

The off-target activity of the 10A2 variants A10.0, A10.38, A10A2.37 and A10A2.39 in comparison with the parental A10A2.0 chimeric antibody fused to wildtype and attenuated interferon 145D was assessed in either the iLite reporter gene assay and/or the HEK Blue assay and is shown in Figure 28 and Figure 29. The EC₅₀ values are provided in Figure 28 and Figure 29. The off-target activity confirms the attenuation of the interferon and the need for the antibody to be targeted to CD38 to restore function.

### Further in-vitro potency data for A10.0 and related constructs

A selection of the above Anti-CD38-attenuated IFN fusion proteins were purified and analysed for binding to CD38 positive cells in cell based assays. In addition potency assays were repeated to more accurately determine the relative activity of each of these Anti-CD38-attenuated IFN fusion proteins. The methods for these various assays are described in Example 1. The results of each of these assays are given in Table 27.

**Table 27**

| **Anti-CD38-attenuated IFN fusion protein** | **H929 Flow binding (EC₅₀ in µg/mL)** | **Annexin V Assay (Fold change relative to untreated cells)** | **Caspase Assay (Fold change relative to untreated cells)** | **Cell Proliferation Assay IC₅₀ (pM)** | **Figures** |
|---|---|---|---|---|---|
| A10.0 | 1.49 | 3.05 | 1.38 | 120.8 | 17,18,21,22,23, 24,25,26,27 28,29 |
| A10.1 | 1.03 | 1.34 | 4.63 | 63.6 | 24, 25 |
| A10.2 | 0.57 | 1.37 | 4.37 | 45.6 | 24, 25 |
| A10.3 | 0.55 | 1.43 | 5.52 | 65.8 | 24, 25 |
| A10.5 | 0.48 | 3.27 | 1.61 | 53.79 | 24, 25 |
| A10.6 | 0.35 | 3.16 | 1.66 | 98.85 | 24, 25 |
| A10.10 | 6.84 | 3.02 | 1.64 | 1967.00 | 24, 25 |
| A10.14 | 2.26 | 2.46 | 1.56 | 2207 | 22, 24, 25 |
| A10.15 | 2.06 | 3.06 | 2.55 | 174.4 | 22, 24, 25 |
| A10.16 | 1.17 | 1.35 | 5.18 | 49.5 | 24, 25 |
| A10.18 | 1.03 | 2.95 | 1.70 | 124.2 | 22, 24, 25 |
| A10.20 | 2.11 | 2.84 | 1.26 | 656 | 24, 25 |
| A10.21 | 0.78 | 3.0 | 1.39 | 147.3 | 22, 24, 25 |
| A10.24 | 0.81 | 2.95 | 7.47 | 87.99 | 22, 24, 25 |
| A10.25 | 1.37 | 2.75 | 1.38 | 27.69 | 24, 25 |
| A10.30 | 0.88 | 3.22 | 1.60 | 18.73 | 24, 25 |
| A10.32 | 51.69 | 1.93 | 1.19 | 381.4 | 24, 25 |
| A10.35 | 1.10 | 2.97 | 2.05 | 93.96 | 22, 24, 25 |
| A10.36 | 1.53 | 3.21 | 3.61 | 57.83 | 22, 24, 25 |
| A10.37 | 18.57 | 2.53 | 1.40 | 163.5 | 24, 25 |
| A10.38 | 1.13 | 3.27 | 1.53 | 36.79 | 23, 24, 25, 26, 29 |
| A10.40 | 0.99 | 1.44 | 5.16 | 3.02 | 24, 25 |
| A10.42 | 1.61 | 1.58 | 1.78 | 155.3 | 24, 25 |
| A10.43 | 1.20 | 1.64 | 1.79 | 120.9 | 24, 25 |
| A10.44 | 1.65 | 1.58 | 1.91 | 308.6 | 24, 25 |
| A10.46 | 0.73 | 1.84 | 5.99 | 2.63 | 24, 25 |
| A10.47 | 0.79 | 1.70 | 1.53 | 5.707 | 24, 25 |
| A10.48 | 1.72 | 1.58 | 1.60 | 22.33 | 24, 25 |
| A10.53 | 1.32 | 1.56 | 1.67 | 56.01 | 24, 25 |
| A10.54 | 5.90 | 1.52 | 1.43 | 2008 | 24, 25 |
| A10.56 | 2.43 | 1.84 | 1.95 | 141.7 | 24, 25 |
| A10.57 | 1.54 | 1.49 | 4.71 | 126.1 | 24, 25 |
| A10.59 | 0.89 | 2.48 | 2.38 | 45.75 | 21, 24, 25 |

| | | | | | |
|---|---|---|---|---|---|
| The flow binding was determined in H929 cell line. Annexin V Assay refer to cells positively stained by Annexin V-FITC after 24 h treatment with antibody constructs at 20 nM. Caspase Assay refers to caspase activation of cells after 24 h treatment with antibody constructs at 20 nM. | | | | | |

### Anti-CD38-attenuated IFN fusion protein with alternative constant region

A10.0 comprises an anti-CD38-attenuated IFN fusion protein in which the constant region of the protein is HC-L0-IFN-alpha (A145D) IgG4 (SEQ ID NO: 9). Using gene synthesis, the constant region of this protein was replaced with HC-L0-IFN-alpha (A145D) IgG1 (SEQ ID NO: 10), paired with A10.0 light chain (SEQ ID NO: 161) and given the designation A10.59. The protein was expressed and was found to be potent in functional assays (Table 28). While the majority of the proteins tested in the foregoing examples were constructed on the human IgG4 constant region, these data demonstrate that other antibody constant regions, such as human IgG1, may also be used, with the resultant antibody-attenuated IFN fusion construct having potent biologic activity equivalent to constructs that utilize a human IgG4 constant region.

**Table 28**

| Anti-CD38-attenuated IFN fusion protein | H929 Flow binding (EC₅₀ in µg/mL) | Annexin V Assay (Fold change relative to untreated cells) | Caspase Assay(Fold change relative to untreated cells) | Cell Proliferation Assay IC₅₀ (pM)* | Figures |
|---|---|---|---|---|---|
| A10.0 | 1.50 | 3.05 | 1.89 | 2081 | 21, 24, 25 |
| A10.59 | 0.89 | 2.48 | 2.38 | 328.6 | 21, 24, 25 |

| | | | | | |
|---|---|---|---|---|---|
| Annexin V Assay refer to cells positively stained by Annexin V-FITC after 24 h treatment with antibody constructs at 20 nM. Caspase Assay refers to caspase activation of cells after 24 h treatment with antibody constructs at 20 nM. N/T is Not Tested, *Data obtained from Cell Proliferation Assay assessed with cell culture supernatant. | | | | | |

Table 29 lists the pairing of variable heavy chain, variable light chain and constant region for each antibody described herein. Table 30 lists the sequences used in the disclosure AA refers to amino acid (sequence type) and DNA refers to polynucleotide (sequence type).

**Table 29**

| **Antibody Name** | **Variable Heavy SEQ ID NO: (amino acid)** | **Variable Light SEQ ID NO: (amino acid)** | **Heavy Chain Constant Region SEQ ID NO: (amino acid)** |
|---|---|---|---|
| A02.10 | 19 | 14 | 9 |
| A02.11 | 20 | 14 | 9 |
| A02.112 | 34 | 65 | 10 |
| A02.12 | 34 | 65 | 9 |
| A02.13 | 35 | 65 | 9 |
| A02.16 | 34 | 92 | 9 |
| A02.17 | 34 | 93 | 9 |
| A02.18 | 34 | 73 | 9 |
| A02.19 | 34 | 74 | 9 |
| A02.2 | 13 | 65 | 9 |
| A02.20 | 34 | 75 | 9 |
| A02.21 | 34 | 76 | 9 |
| A02.22 | 34 | 77 | 9 |
| A02.23 | 34 | 78 | 9 |
| A02.24 | 34 | 79 | 9 |
| A02.25 | 34 | 80 | 9 |
| A02.26 | 34 | 81 | 9 |
| A02.27 | 34 | 82 | 9 |
| A02.28 | 34 | 83 | 9 |
| A02.29 | 34 | 84 | 9 |
| A02.3 | 17 | 65 | 9 |
| A02.30 | 34 | 85 | 9 |
| A02.31 | 34 | 86 | 9 |
| A02.32 | 34 | 87 | 9 |
| A02.33 | 34 | 88 | 9 |
| A02.34 | 34 | 89 | 9 |
| A02.35 | 34 | 90 | 9 |
| A02.36 | 34 | 91 | 9 |
| A02.37 | 34 | 66 | 9 |
| A02.38 | 34 | 113 | 9 |
| A02.39 | 34 | 112 | 9 |
| A02.4 | 18 | 65 | 9 |
| A02.40 | 111 | 65 | 9 |
| A02.41 | 110 | 65 | 9 |
| A02.43 | 110 | 113 | 9 |
| A02.44 | 111 | 112 | 9 |
| A02.46 | 34 | 67 | 9 |
| A02.47 | 34 | 68 | 9 |
| A02.48 | 34 | 69 | 9 |
| A02.49 | 34 | 70 | 9 |
| A02.5 | 19 | 65 | 9 |
| A02.50 | 34 | 71 | 9 |
| A02.51 | 34 | 72 | 9 |
| A02.52 | 34 | 94 | 9 |
| A02.53 | 34 | 95 | 9 |
| A02.54 | 34 | 96 | 9 |
| A02.55 | 34 | 97 | 9 |
| A02.56 | 34 | 98 | 9 |
| A02.57 | 34 | 99 | 9 |
| A02.58 | 34 | 100 | 9 |
| A02.59 | 34 | 101 | 9 |
| A02.6 | 20 | 65 | 9 |
| A02.60 | 34 | 102 | 9 |
| A02.61 | 34 | 103 | 9 |
| A02.62 | 34 | 104 | 9 |
| A02.63 | 34 | 105 | 9 |
| A02.64 | 34 | 106 | 9 |
| A02.65 | 34 | 107 | 9 |
| A02.66 | 34 | 108 | 9 |
| A02.67 | 34 | 109 | 9 |
| A02.8 | 17 | 14 | 9 |
| A02.9 | 18 | 14 | 9 |
| A10.1 | 165 | 161 | 9 |
| A10.10 | 174 | 161 | 9 |
| A10.11 | 175 | 161 | 9 |
| A10.12 | 176 | 161 | 9 |
| A10.13 | 177 | 161 | 9 |
| A10.14 | 178 | 161 | 9 |
| A10.15 | 179 | 161 | 9 |
| A10.16 | 180 | 161 | 9 |
| A10.17 | 156 | 181 | 9 |
| A10.18 | 156 | 182 | 9 |
| A10.19 | 156 | 183 | 9 |
| A10.2 | 166 | 161 | 9 |
| A10.20 | 156 | 184 | 9 |
| A10.21 | 156 | 185 | 9 |
| A10.22 | 156 | 186 | 9 |
| A10.23 | 156 | 187 | 9 |
| A10.24 | 156 | 188 | 9 |
| A10.25 | 156 | 189 | 9 |
| A10.26 | 156 | 190 | 9 |
| A10.27 | 156 | 191 | 9 |
| A10.28 | 156 | 192 | 9 |
| A10.29 | 156 | 193 | 9 |
| A10.3 | 167 | 161 | 9 |
| A10.30 | 156 | 194 | 9 |
| A10.31 | 156 | 195 | 9 |
| A10.32 | 156 | 196 | 9 |
| A10.35 | 197 | 161 | 9 |
| A10.36 | 156 | 198 | 9 |
| A10.38 | 152 | 161 | 9 |
| A10.39 | 152 | 181 | 9 |
| A10.4 | 168 | 161 | 9 |
| A10.40 | 152 | 182 | 9 |
| A10.41 | 152 | 183 | 9 |
| A10.42 | 152 | 184 | 9 |
| A10.43 | 152 | 185 | 9 |
| A10.44 | 152 | 186 | 9 |
| A10.45 | 152 | 187 | 9 |
| A10.46 | 152 | 188 | 9 |
| A10.47 | 152 | 189 | 9 |
| A10.48 | 152 | 190 | 9 |
| A10.49 | 152 | 191 | 9 |
| A10.5 | 169 | 161 | 9 |
| A10.50 | 152 | 192 | 9 |
| A10.51 | 152 | 193 | 9 |
| A10.52 | 152 | 194 | 9 |
| A10.53 | 152 | 195 | 9 |
| A10.54 | 152 | 196 | 9 |
| A10.57 | 152 | 198 | 9 |
| A10.59 | 156 | 161 | 10 |
| A10.6 | 170 | 161 | 9 |
| A10.7 | 171 | 161 | 9 |
| A10.8 | 172 | 161 | 9 |
| A10.9 | 173 | 161 | 9 |
| A10A2.0 (chimeric) | 148 | 157 | 9 |
| A10A2.1 | 149 | 158 | 9 |
| A10A2.10 | 150 | 160 | 9 |
| A10A2.11 | 150 | 161 | 9 |
| A10A2.12 | 150 | 162 | 9 |
| A10A2.13 | 150 | 163 | 9 |
| A10A2.14 | 150 | 164 | 9 |
| A10A2.15 | 151 | 158 | 9 |
| A10A2.16 | 151 | 159 | 9 |
| A10A2.17 | 151 | 160 | 9 |
| A10A2.18 | 151 | 161 | 9 |
| A10A2.19 | 151 | 162 | 9 |
| A10A2.2 | 149 | 159 | 9 |
| A10A2.20 | 151 | 163 | 9 |
| A10A2.21 | 151 | 164 | 9 |
| A10A2.22 | 152 | 158 | 9 |
| A10A2.23 | 152 | 159 | 9 |
| A10A2.24 | 152 | 160 | 9 |
| A10A2.25 | 152 | 161 | 9 |
| A10A2.26 | 152 | 162 | 9 |
| A10A2.27 | 152 | 163 | 9 |
| A10A2.28 | 152 | 164 | 9 |
| A10A2.29 | 153 | 158 | 9 |
| A10A2.3 | 149 | 160 | 9 |
| A10A2.30 | 153 | 159 | 9 |
| A10A2.31 | 153 | 160 | 9 |
| A10A2.32 | 153 | 161 | 9 |
| A10A2.33 | 153 | 162 | 9 |
| A10A2.34 | 153 | 163 | 9 |
| A10A2.35 | 153 | 164 | 9 |
| A10A2.36 | 154 | 158 | 9 |
| A10A2.37 | 154 | 159 | 9 |
| A10A2.38 | 154 | 160 | 9 |
| A10A2.39 | 154 | 161 | 9 |
| A10A2.4 | 149 | 161 | 9 |
| A10A2.40 | 154 | 162 | 9 |
| A10A2.41 | 154 | 163 | 9 |
| A10A2.42 | 154 | 164 | 9 |
| A10A2.43 | 154 | 158 | 9 |
| A10A2.44 | 155 | 159 | 9 |
| A10A2.45 | 155 | 160 | 9 |
| A10A2.46 | 155 | 161 | 9 |
| A10A2.47 | 155 | 162 | 9 |
| A10A2.48 | 155 | 163 | 9 |
| A10A2.49 | 155 | 164 | 9 |
| A10A2.5 | 149 | 162 | 9 |
| A10A2.50 | 156 | 158 | 9 |
| A10A2.51 | 156 | 159 | 9 |
| A10A2.52 | 156 | 160 | 9 |
| A10A2.53 | 156 | 161 | 9 |
| A10A2.54 | 156 | 162 | 9 |
| A10A2.55 | 156 | 163 | 9 |
| A10A2.56 | 156 | 164 | 9 |
| A10A2.6 | 149 | 163 | 9 |
| A10A2.7 | 149 | 164 | 9 |
| A10A2.8 | 150 | 158 | 9 |
| A10A2.9 | 150 | 159 | 9 |
| A5D1.0 (chimeric) | 114 | 125 | 9 |
| A5D1.1 | 115 | 126 | 9 |
| A5D1.10 | 116 | 129 | 9 |
| A5D1.11 | 116 | 130 | 9 |
| A5D1.12 | 116 | 131 | 9 |
| A5D1.13 | 117 | 126 | 9 |
| A5D1.14 | 117 | 127 | 9 |
| A5D1.15 | 117 | 128 | 9 |
| A5D1.16 | 117 | 129 | 9 |
| A5D1.17 | 117 | 130 | 9 |
| A5D1.18 | 117 | 131 | 9 |
| A5D1.19 | 118 | 126 | 9 |
| A5D1.2 | 115 | 127 | 9 |
| A5D1.20 | 118 | 127 | 9 |
| A5D1.21 | 118 | 128 | 9 |
| A5D1.22 | 118 | 129 | 9 |
| A5D1.23 | 118 | 130 | 9 |
| A5D1.24 | 118 | 131 | 9 |
| A5D1.25 | 119 | 126 | 9 |
| A5D1.26 | 119 | 127 | 9 |
| A5D1.27 | 119 | 128 | 9 |
| A5D1.28 | 119 | 129 | 9 |
| A5D1.29 | 119 | 130 | 9 |
| A5D1.3 | 115 | 128 | 9 |
| A5D1.30 | 119 | 131 | 9 |
| A5D1.31 | 120 | 126 | 9 |
| A5D1.32 | 120 | 127 | 9 |
| A5D1.33 | 120 | 128 | 9 |
| A5D1.34 | 120 | 129 | 9 |
| A5D1.35 | 120 | 130 | 9 |
| A5D1.36 | 120 | 131 | 9 |
| A5D1.37 | 121 | 126 | 9 |
| A5D1.38 | 121 | 127 | 9 |
| A5D1.39 | 121 | 128 | 9 |
| A5D1.4 | 115 | 129 | 9 |
| A5D1.40 | 121 | 129 | 9 |
| A5D1.41 | 121 | 130 | 9 |
| A5D1.42 | 121 | 131 | 9 |
| A5D1.43 | 122 | 126 | 9 |
| A5D1.44 | 122 | 127 | 9 |
| A5D1.45 | 122 | 128 | 9 |
| A5D1.46 | 122 | 129 | 9 |
| A5D1.47 | 122 | 130 | 9 |
| A5D1.48 | 122 | 131 | 9 |
| A5D1.49 | 123 | 126 | 9 |
| A5D1.5 | 115 | 130 | 9 |
| A5D1.50 | 123 | 127 | 9 |
| A5D1.51 | 123 | 128 | 9 |
| A5D1.52 | 123 | 129 | 9 |
| A5D1.53 | 123 | 130 | 9 |
| A5D1.54 | 123 | 131 | 9 |
| A5D1.55 | 124 | 126 | 9 |
| A5D1.56 | 124 | 127 | 9 |
| A5D1.57 | 124 | 128 | 9 |
| A5D1.58 | 124 | 129 | 9 |
| A5D1.59 | 124 | 130 | 9 |
| A5D1.6 | 115 | 131 | 9 |
| A5D1.60 | 124 | 131 | 9 |
| A5D1.7 | 116 | 126 | 9 |
| A5D1.8 | 116 | 127 | 9 |
| A5D1.9 | 116 | 128 | 9 |
| A5E8.0 (chimeric) | 132 | 143 | 9 |
| A5E8.1 | 133 | 144 | 9 |
| A5E8.10 | 135 | 145 | 9 |
| A5E8.11 | 135 | 146 | 9 |
| A5E8.12 | 135 | 147 | 9 |
| A5E8.13 | 136 | 144 | 9 |
| A5E8.14 | 136 | 145 | 9 |
| A5E8.15 | 136 | 146 | 9 |
| A5E8.16 | 136 | 147 | 9 |
| A5E8.17 | 137 | 144 | 9 |
| A5E8.18 | 137 | 145 | 9 |
| A5E8.19 | 137 | 146 | 9 |
| A5E8.2 | 133 | 145 | 9 |
| A5E8.20 | 137 | 147 | 9 |
| A5E8.21 | 138 | 144 | 9 |
| A5E8.22 | 138 | 145 | 9 |
| A5E8.23 | 138 | 146 | 9 |
| A5E8.24 | 138 | 147 | 9 |
| A5E8.25 | 139 | 144 | 9 |
| A5E8.26 | 139 | 145 | 9 |
| A5E8.27 | 139 | 146 | 9 |
| A5E8.28 | 139 | 147 | 9 |
| A5E8.29 | 140 | 144 | 9 |
| A5E8.3 | 133 | 146 | 9 |
| A5E8.30 | 140 | 145 | 9 |
| A5E8.31 | 140 | 146 | 9 |
| A5E8.32 | 140 | 147 | 9 |
| A5E8.33 | 141 | 144 | 9 |
| A5E8.34 | 141 | 145 | 9 |
| A5E8.35 | 141 | 146 | 9 |
| A5E8.36 | 141 | 147 | 9 |
| A5E8.37 | 142 | 144 | 9 |
| A5E8.38 | 142 | 145 | 9 |
| A5E8.39 | 142 | 146 | 9 |
| A5E8.4 | 133 | 147 | 9 |
| A5E8.40 | 142 | 147 | 9 |
| A5E8.5 | 134 | 144 | 9 |
| A5E8.6 | 134 | 145 | 9 |
| A5E8.7 | 134 | 146 | 9 |
| A5E8.8 | 134 | 147 | 9 |
| A5E8.9 | 135 | 144 | 9 |
| X02.10 | 19 | 14 | 3 |
| X02.100 | 13 | 58 | 3 |
| X02.101 | 13 | 59 | 3 |
| X02.102 | 13 | 60 | 3 |
| X02.103 | 13 | 61 | 3 |
| X02.104 | 13 | 62 | 3 |
| X02.105 | 13 | 63 | 3 |
| X02.106 | 13 | 64 | 3 |
| X02.107 | 13 | 65 | 3 |
| X02.108 | 32 | 14 | 3 |
| X02.11 | 20 | 14 | 3 |
| X02.110 | 33 | 14 | 3 |
| X02.114 | 13 | 660 | 3 |
| X02.115 | 13 | 661 | 3 |
| X02.116 | 13 | 662 | 3 |
| X02.117 | 13 | 663 | 3 |
| X02.118 | 34 | 700 | 3 |
| X02.119 | 34 | 701 | 3 |
| X02.120 | 728 | 700 | 3 |
| X02.121 | 729 | 700 | 3 |
| X02.122 | 730 | 700 | 3 |
| X02.123 | 731 | 700 | 3 |
| X02.124 | 728 | 701 | 3 |
| X02.125 | 729 | 701 | 3 |
| X02.126 | 730 | 701 | 3 |
| X02.127 | 731 | 701 | 3 |
| X02.68 | 21 | 14 | 3 |
| X02.69 | 22 | 14 | 3 |
| X02.70 | 23 | 14 | 3 |
| X02.71 | 24 | 14 | 3 |
| X02.72 | 25 | 14 | 3 |
| X02.73 | 26 | 14 | 3 |
| X02.74 | 27 | 14 | 3 |
| X02.75 | 28 | 14 | 3 |
| X02.76 | 29 | 14 | 3 |
| X02.77 | 30 | 14 | 3 |
| X02.78 | 31 | 14 | 3 |
| X02.8 | 17 | 14 | 3 |
| X02.80 | 13 | 38 | 3 |
| X02.81 | 13 | 39 | 3 |
| X02.82 | 13 | 40 | 3 |
| X02.83 | 13 | 41 | 3 |
| X02.84 | 13 | 42 | 3 |
| X02.85 | 13 | 43 | 3 |
| X02.86 | 13 | 44 | 3 |
| X02.87 | 13 | 45 | 3 |
| X02.88 | 13 | 46 | 3 |
| X02.89 | 13 | 47 | 3 |
| X02.9 | 18 | 14 | 3 |
| X02.90 | 13 | 48 | 3 |
| X02.91 | 13 | 49 | 3 |
| X02.92 | 13 | 50 | 3 |
| X02.93 | 13 | 51 | 3 |
| X02.94 | 13 | 52 | 3 |
| X02.95 | 13 | 53 | 3 |
| X02.96 | 13 | 54 | 3 |
| X02.97 | 13 | 55 | 3 |
| X02.98 | 13 | 56 | 3 |
| X02.99 | 13 | 57 | 3 |
| X10.100 | 720 | 706 | 3 |
| X10.101 | 721 | 706 | 3 |
| X10.102 | 722 | 706 | 3 |
| X10.103 | 723 | 706 | 3 |
| X10.104 | 739 | 706 | 3 |
| X10.105 | 740 | 706 | 3 |
| X10.106 | 741 | 706 | 3 |
| X10.107 | 742 | 706 | 3 |
| X10.108 | 720 | 707 | 3 |
| X10.109 | 721 | 707 | 3 |
| X10.110 | 722 | 707 | 3 |
| X10.111 | 723 | 707 | 3 |
| X10.112 | 739 | 707 | 3 |
| X10.113 | 740 | 707 | 3 |
| X10.114 | 741 | 707 | 3 |
| X10.115 | 742 | 707 | 3 |
| X10.116 | 720 | 708 | 3 |
| X10.117 | 721 | 708 | 3 |
| X10.118 | 722 | 708 | 3 |
| X10.119 | 723 | 708 | 3 |
| X10.120 | 739 | 708 | 3 |
| X10.121 | 740 | 708 | 3 |
| X10.122 | 741 | 708 | 3 |
| X10.123 | 742 | 708 | 3 |
| X10.124 | 720 | 709 | 3 |
| X10.125 | 721 | 709 | 3 |
| X10.126 | 722 | 709 | 3 |
| X10.127 | 723 | 709 | 3 |
| X10.128 | 739 | 709 | 3 |
| X10.129 | 740 | 709 | 3 |
| X10.130 | 741 | 709 | 3 |
| X10.131 | 742 | 709 | 3 |
| X10.132 | 720 | 710 | 3 |
| X10.133 | 721 | 710 | 3 |
| X10.134 | 722 | 710 | 3 |
| X10.135 | 723 | 710 | 3 |
| X10.136 | 739 | 710 | 3 |
| X10.137 | 740 | 710 | 3 |
| X10.138 | 741 | 710 | 3 |
| X10.139 | 742 | 710 | 3 |
| X10.140 | 720 | 711 | 3 |
| X10.141 | 721 | 711 | 3 |
| X10.142 | 722 | 711 | 3 |
| X10.143 | 723 | 711 | 3 |
| X10.144 | 739 | 711 | 3 |
| X10.145 | 740 | 711 | 3 |
| X10.146 | 741 | 711 | 3 |
| X10.147 | 742 | 711 | 3 |
| X10.60 | 156 | 704 | 3 |
| X10.61 | 156 | 705 | 3 |
| X10.62 | 156 | 706 | 3 |
| X10.63 | 156 | 707 | 3 |
| X10.64 | 156 | 708 | 3 |
| X10.65 | 156 | 709 | 3 |
| X10.66 | 156 | 710 | 3 |
| X10.67 | 156 | 711 | 3 |
| X10.68 | 720 | 161 | 3 |
| X10.69 | 721 | 161 | 3 |
| X10.70 | 722 | 161 | 3 |
| X10.71 | 723 | 161 | 3 |
| X10.72 | 739 | 161 | 3 |
| X10.73 | 740 | 161 | 3 |
| X10.74 | 741 | 161 | 3 |
| X10.75 | 742 | 161 | 3 |
| X10.76 | 152 | 704 | 3 |
| X10.77 | 152 | 705 | 3 |
| X10.78 | 152 | 706 | 3 |
| X10.79 | 152 | 707 | 3 |
| X10.80 | 152 | 708 | 3 |
| X10.81 | 152 | 709 | 3 |
| X10.82 | 152 | 710 | 3 |
| X10.83 | 152 | 711 | 3 |
| X10.84 | 720 | 704 | 3 |
| X10.85 | 721 | 704 | 3 |
| X10.86 | 722 | 704 | 3 |
| X10.87 | 723 | 704 | 3 |
| X10.88 | 739 | 704 | 3 |
| X10.89 | 740 | 704 | 3 |
| X10.90 | 741 | 704 | 3 |
| X10.91 | 742 | 704 | 3 |
| X10.92 | 720 | 705 | 3 |
| X10.93 | 721 | 705 | 3 |
| X10.94 | 722 | 705 | 3 |
| X10.95 | 723 | 705 | 3 |
| X10.96 | 739 | 705 | 3 |
| X10.97 | 740 | 705 | 3 |
| X10.98 | 741 | 705 | 3 |
| X10.99 | 742 | 705 | 3 |
| X910/12-HC-L0-IFN-alpha (A145D) IgG4 | 110 | 112 | 9 |
| X913/15-HC-L0- IFN-alpha (A145D) IgG4 | 111 | 113 | 9 |

**Table 30**

| **SEQ ID NO:** | **Type** | **Description** |
|---|---|---|
| 1 | AA | Human CD38 |
| 2 | AA | Cynomolgus CD38 |
| 3 | AA | Human IgG4 constant heavy chain |
| 4 | AA | Human IgG1 constant heavy chain |
| 5 | AA | Human kappa constant region |
| 6 | AA | Human lambda constant region |
| 7 | AA | IFN-alpha2b |
| 8 | AA | Intron A |
| 9 | AA | HC-L0-IFN-alpha (A145D) IgG4 |
| 10 | AA | HC-L0-IFN-alpha (A145D) IgG1 |
| 11 | AA | A02.1 heavy chain |
| 12 | AA | A02.1 light chain |
| 13 | AA | A02.1 variable heavy chain |
| 14 | AA | A02.1 variable light chain |
| 15 | AA | X02.1VH variable heavy chain |
| 16 | AA | IGHV4-61*01 germline sequence |
| 17 | AA | X02.8VH variable heavy chain |
| 18 | AA | X02.9VH variable heavy chain |
| 19 | AA | X02.10VH variable heavy chain |
| 20 | AA | X02.11VH variable heavy chain |
| 21 | AA | X02.68VH variable heavy chain |
| 22 | AA | X02.69VH variable heavy chain |
| 23 | AA | X02.70VH variable heavy chain |
| 24 | AA | X02.71VH variable heavy chain |
| 25 | AA | X02.72VH variable heavy chain |
| 26 | AA | X02.73VH variable heavy chain |
| 27 | AA | X02.74VH variable heavy chain |
| 28 | AA | X02.75VH variable heavy chain |
| 29 | AA | X02.76VH variable heavy chain |
| 30 | AA | X02.77VH variable heavy chain |
| 31 | AA | X02.78VH variable heavy chain |
| 32 | AA | X02.108VH variable heavy chain |
| 33 | AA | X02.110VH variable heavy chain |
| 34 | AA | A02.12VH variable heavy chain |
| 35 | AA | A02.13VH variable heavy chain |
| 36 | AA | A02.1VL variable light chain |
| 37 | AA | IGLV5-37*01 germline sequence |
| 38 | AA | X02.80VL variable light chain |
| 39 | AA | X02.81VL variable light chain |
| 40 | AA | X02.82VL variable light chain |
| 41 | AA | X02.83VL variable light chain |
| 42 | AA | X02.84VL variable light chain |
| 43 | AA | X02.85VL variable light chain |
| 44 | AA | X02.86VL variable light chain |
| 45 | AA | X02.87VL variable light chain |
| 46 | AA | X02.88VL variable light chain |
| 47 | AA | X02.89VL variable light chain |
| 48 | AA | X02.90VL variable light chain |
| 49 | AA | X02.91VL variable light chain |
| 50 | AA | X02.92VL variable light chain |
| 51 | AA | X02.93VL variable light chain |
| 52 | AA | X02.94VL variable light chain |
| 53 | AA | X02.95VL variable light chain |
| 54 | AA | X02.96VL variable light chain |
| 55 | AA | X02.97VL variable light chain |
| 56 | AA | X02.98VL variable light chain |
| 57 | AA | X02.99VL variable light chain |
| 58 | AA | X02.100VL variable light chain |
| 59 | AA | X02.101VL variable light chain |
| 60 | AA | X02.102VL variable light chain |
| 61 | AA | X02.103VL variable light chain |
| 62 | AA | X02.104VL variable light chain |
| 63 | AA | X02.105VL variable light chain |
| 64 | AA | X02.106VL variable light chain |
| 65 | AA | X02.107VL variable light chain |
| 66 | AA | A02.37VL variable light chain |
| 67 | AA | A02.46VL variable light chain |
| 68 | AA | A02.47VL variable light chain |
| 69 | AA | A02.48VL variable light chain |
| 70 | AA | A02.49VL variable light chain |
| 71 | AA | A02.50VL variable light chain |
| 72 | AA | A02.51VL variable light chain |
| 73 | AA | A02.18VL variable light chain |
| 74 | AA | A02.19VL variable light chain |
| 75 | AA | A02.20VL variable light chain |
| 76 | AA | A02.21VL variable light chain |
| 77 | AA | A02.22VL variable light chain |
| 78 | AA | A02.23VL variable light chain |
| 79 | AA | A02.24VL variable light chain |
| 80 | AA | A02.25VL variable light chain |
| 81 | AA | A02.26VL variable light chain |
| 82 | AA | A02.27VL variable light chain |
| 83 | AA | A02.28VL variable light chain |
| 84 | AA | A02.29VL variable light chain |
| 85 | AA | A02.30VL variable light chain |
| 86 | AA | A02.31VL variable light chain |
| 87 | AA | A02.32VL variable light chain |
| 88 | AA | A02.33VL variable light chain |
| 89 | AA | A02.34VL variable light chain |
| 90 | AA | A02.35VL variable light chain |
| 91 | AA | A02.36VL variable light chain |
| 92 | AA | X02.16VL variable light chain |
| 93 | AA | X02.17VL variable light chain |
| 94 | AA | A02.52VL variable light chain |
| 95 | AA | A02.53VL variable light chain |
| 96 | AA | A02.54VL variable light chain |
| 97 | AA | A02.55VL variable light chain |
| 98 | AA | A02.56VL variable light chain |
| 99 | AA | A02.57VL variable light chain |
| 100 | AA | A02.58VL variable light chain |
| 101 | AA | A02.59VL variable light chain |
| 102 | AA | A02.60VL variable light chain |
| 103 | AA | A02.61VL variable light chain |
| 104 | AA | A02.62VL variable light chain |
| 105 | AA | A02.63VL variable light chain |
| 106 | AA | A02.64VL variable light chain |
| 107 | AA | A02.65VL variable light chain |
| 108 | AA | A02.66VL variable light chain |
| 109 | AA | A02.67VL variable light chain |
| 110 | AA | 910VH variable heavy chain |
| 111 | AA | 915 VH variable heavy chain |
| 112 | AA | 912VL variable light chain |
| 113 | AA | 913VL variable light chain |
| 114 | AA | Chimeric 5D1-E2-VH |
| 115 | AA | 5d1_1-f^{∗}01VH |
| 116 | AA | 5d1_1-f^{∗}01VH94R |
| 117 | AA | 5d1_1-18^{∗}01VH |
| 118 | AA | 5d1_1-18^{∗}01VH71A |
| 119 | AA | 5d1_1-24^{∗}01VH |
| 120 | AA | 5d1_1-24^{∗}01VH71A |
| 121 | AA | 5d1_1-24^{∗}01VH29F |
| 122 | AA | 5d1_1-24^{∗}01VH94R |
| 123 | AA | 5d1_1-45^{∗}01VH |
| 124 | AA | 5d1_1-45^{∗}01VH71A |
| 125 | AA | Chimeric 5D1VK |
| 126 | AA | 5d1_1-5^{∗}01VK |
| 127 | AA | 5d1_1-9^{∗}01VK |
| 128 | AA | 5d1_1-12^{∗}01VK |
| 129 | AA | 5d1_1D-13^{∗}01VK |
| 130 | AA | 5d1_1D-16^{∗}*01VK |
| 131 | AA | 5d1_3-15^{∗}01VK |
| 132 | AA | Chimeric 5E8 |
| 133 | AA | 5E8-1-f^{∗}01VH |
| 134 | AA | 5E8-1-f^{∗}01VH301 |
| 135 | AA | 5E8-1-f^{∗}01VH94R |
| 136 | AA | 5E8-1-18^{∗}01VH |
| 137 | AA | 5E8-1-18^{∗}01VH71A |
| 138 | AA | 5E8-1-24^{∗}01VH |
| 139 | AA | 5E8-1-24^{∗}01VH71A |
| 140 | AA | 5E8-1-24^{∗}01VH94R |
| 141 | AA | 5E8-1-45^{∗}01VH |
| 142 | AA | 5E8-1-45^{∗}01VH71A |
| 143 | AA | chimeric 5E8VK |
| 144 | AA | 5E8-2-24^{∗}01VK |
| 145 | AA | 5E8-2D-28^{∗}01VK |
| 146 | AA | 5E8-2D-29^{∗}01VK |
| 147 | AA | 5E8-2-30^{∗}01VK |
| 148 | AA | 10A2 chimeric VH |
| 149 | AA | 10A2_1-24^{∗}01VH |
| 150 | AA | 10A2_1-24^{∗}01VH71A |
| 151 | AA | 10A2_1-24^{∗}01VH94R |
| 152 | AA | 10A2_1-24^{∗}0171A94R |
| 153 | AA | 10A2_1-45^{∗}01VH |
| 154 | AA | 10A2_1-45^{∗}01VH71A |
| 155 | AA | 10A2_1-f^{∗}01VH |
| 156 | AA | 10A2_1-f^{∗}01VH94R |
| 157 | AA | 10A2 chimeric VK |
| 158 | AA | 10A2_1-9^{∗}01Vk |
| 159 | AA | 10A2_1-12^{∗}01Vk |
| 160 | AA | 10A2_1D-13^{∗}01Vk |
| 161 | AA | 10A2_1-33^{∗}01Vk |
| 162 | AA | 10A2_3-11^{∗}02Vk |
| 163 | AA | 10A2_3-15^{∗}01Vk |
| 164 | AA | 10A2_6-21^{∗}01Vk |
| 165 | AA | 10A2VH + A40E |
| 166 | AA | 10A2VH + A40G |
| 167 | AA | 10A2VH + A40H |
| 168 | AA | 10A2VH + A40Q |
| 169 | AA | 10A2VH + A40S |
| 170 | AA | 10A2VH + A40V |
| 171 | AA | 10A2VH + N35E |
| 172 | AA | 10A2VH + N35P |
| 173 | AA | 10A2VH + N35Q |
| 174 | AA | 10A2VH + N35S |
| 175 | AA | 10A2VH + R94E |
| 176 | AA | 10A2VH + R94G |
| 177 | AA | 10A2VH + R94P |
| 178 | AA | 10A2VH + R94T |
| 179 | AA | 10A2VH + K96G |
| 180 | AA | 10A2VH + K96T |
| 181 | AA | 10A2VK + K24E |
| 182 | AA | 10A2VK + K24G |
| 183 | AA | 10A2VK + K24P |
| 184 | AA | 10A2VK + K24Q |
| 185 | AA | 10A2VK + R54D |
| 186 | AA | 10A2VK + I48D |
| 187 | AA | 10A2VK + Y49E |
| 188 | AA | 10A2VK + M89A |
| 189 | AA | 10A2VK + M89E |
| 190 | AA | 10A2VK + M89H |
| 191 | AA | 10A2VK + M89K |
| 192 | AA | 10A2VK + M89P |
| 193 | AA | 10A2VK + M89Q |
| 194 | AA | 10A2VK + M89S |
| 195 | AA | 10A2VK + M89V |
| 196 | AA | 10A2VK + Q90D |
| 197 | AA | 10A2VH (AQ) + N98Q |
| 198 | AA | 10A2VK (AV) + N53Q |
| 199 | AA | X02.1VH FWR1 |
| 200 | AA | X02.1VH CDR1 |
| 201 | AA | X02.1VH FWR2 |
| 202 | AA | X02.1VH CDR2 |
| 203 | AA | X02.1VH FWR3 |
| 204 | AA | X02.1VH CDR3 |
| 205 | AA | X02.1VH FWR4 |
| 206 | AA | IGHV4-61*01 FWR1 |
| 207 | AA | IGHV4-61*01 CDR1 |
| 208 | AA | IGHV4-61*01 FWR2 |
| 209 | AA | IGHV4-61*01 FWR3 |
| 210 | AA | X02.8VH FWR3 |
| 211 | AA | X02.9VH FWR2 |
| 212 | AA | X02.10VH FWR3 |
| 213 | AA | X02.11VH FWR3 |
| 214 | AA | X02.68VH FWR1 |
| 215 | AA | X02.69VH FWR1 |
| 216 | AA | X02.70VH FWR3 |
| 217 | AA | X02.71VH FWR1 |
| 218 | AA | X02.72VH FWR3 |
| 219 | AA | X02.73VH FWR1 |
| 220 | AA | X02.74VH CDR3 |
| 221 | AA | X02.75VH FWR3 |
| 222 | AA | X02.76VH CDR3 |
| 223 | AA | X02.77VH CDR3 |
| 224 | AA | X02.78VH CDR1 |
| 225 | AA | X02.108 FWR3 |
| 226 | AA | X02.110VH FWR3 |
| 227 | AA | A02.12VH FWR3 |
| 228 | AA | A02.12VH CDR3 |
| 229 | AA | A02.13VH FWR2 |
| 230 | AA | A02.13VH FWR3 |
| 231 | AA | A02.13VH CDR3 |
| 232 | AA | A02.1VL FWR1 |
| 233 | AA | A02.1VL CDR1 |
| 234 | AA | A02.1VL FWR2 |
| 235 | AA | A02.1VL CDR2 |
| 236 | AA | A02.1VL FWR3 |
| 237 | AA | A02.1VL CDR3 |
| 238 | AA | A02.1VL FWR4 |
| 239 | AA | IGLV5-37*01 FWR1 |
| 240 | AA | IGLV5-37*01 CDR1 |
| 241 | AA | IGLV5-37*01 CDR2 |
| 242 | AA | IGLV5-37*01 FWR3 |
| 243 | AA | IGLV5-37*01 CDR3 |
| 244 | AA | X02.80VL CDR3 |
| 245 | AA | X02.81VL FWR3 |
| 246 | AA | X02.82VL FWR2 |
| 247 | AA | X02.83VL FWR1 |
| 248 | AA | X02.84VL FWR2 |
| 249 | AA | X02.84VL CDR2 |
| 250 | AA | X02.86VL CDR1 |
| 251 | AA | X02.87VL CDR3 |
| 252 | AA | X02.88VL CDR1 |
| 253 | AA | X02.89VL CDR2 |
| 254 | AA | X02.90VL CDR3 |
| 255 | AA | X02.91VL CDR1 |
| 256 | AA | X02.92VL CDR3 |
| 257 | AA | X02.93VL CDR3 |
| 258 | AA | X02.94VL CDR3 |
| 259 | AA | X02.95VL FWR1 |
| 260 | AA | X02.96VL FWR1 |
| 261 | AA | X02.97VL FWR1 |
| 262 | AA | X02.98VL CDR1 |
| 263 | AA | X02.99VL CDR1 |
| 264 | AA | X02.100VL CDR2 |
| 265 | AA | X02.101VL FWR3 |
| 266 | AA | X02.102VL FWR3 |
| 267 | AA | X02.103VL FWR3 |
| 268 | AA | X02.104VL CDR3 |
| 269 | AA | X02.105VL CDR3 |
| 270 | AA | X02.106VL CDR3 |
| 271 | AA | X02.107VL FWR3 |
| 272 | AA | A02.37VL FWR3 |
| 273 | AA | A02.37VL CDR3 |
| 274 | AA | A02.46VL FWR3 |
| 275 | AA | A02.46VL CDR3 |
| 276 | AA | A02.47VL FWR3 |
| 277 | AA | A02.48VL FWR3 |
| 278 | AA | A02.49VL FWR3 |
| 279 | AA | A02.50VL FWR3 |
| 280 | AA | A02.51VL FWR3 |
| 281 | AA | A02.18VL FWR2 |
| 282 | AA | A02.18VL FWR3 |
| 283 | AA | A02.19VL FWR2 |
| 284 | AA | A02.19VL FWR3 |
| 285 | AA | A02.20VL FWR2 |
| 286 | AA | A02.20VL FWR3 |
| 287 | AA | A02.21VL FWR2 |
| 288 | AA | A02.21VL FWR3 |
| 289 | AA | A02.22VL FWR2 |
| 290 | AA | A02.22VL FWR3 |
| 291 | AA | A02.23VL FWR2 |
| 292 | AA | A02.23VL FWR3 |
| 293 | AA | A02.24VL FWR2 |
| 294 | AA | A02.24VL FWR3 |
| 295 | AA | A02.25VL FWR2 |
| 296 | AA | A02.25VL FWR3 |
| 297 | AA | A02.26VL FWR2 |
| 298 | AA | A02.26VL FWR3 |
| 299 | AA | A02.27VL CDR2 |
| 300 | AA | A02.27VL FWR3 |
| 301 | AA | A02.28VL CDR2 |
| 302 | AA | A02.28VL FWR3 |
| 303 | AA | A02.29VL CDR2 |
| 304 | AA | A02.29VL FWR3 |
| 305 | AA | A02.30VL CDR2 |
| 306 | AA | A02.30VL FWR3 |
| 307 | AA | A02.31VL CDR2 |
| 308 | AA | A02.31VL FWR3 |
| 309 | AA | A02.32VL CDR2 |
| 310 | AA | A02.32VL FWR3 |
| 311 | AA | A02.33VL CDR2 |
| 312 | AA | A02.33VL FWR3 |
| 313 | AA | A02.34VL CDR2 |
| 314 | AA | A02.34VL FWR3 |
| 315 | AA | A02.35VL CDR2 |
| 316 | AA | A02.35VL FWR3 |
| 317 | AA | A02.36VL CDR2 |
| 318 | AA | A02.36VL FWR3 |
| 319 | AA | X02.16VL CDR1 |
| 320 | AA | X02.16VL FWR3 |
| 321 | AA | X02.16VL CDR3 |
| 322 | AA | X02.17VL CDR1 |
| 323 | AA | X02.17VL FWR3 |
| 324 | AA | X02.17VL CDR3 |
| 325 | AA | A02.52VL CDR1 |
| 326 | AA | A02.52VL CDR2 |
| 327 | AA | A02.52VL FWR3 |
| 328 | AA | A02.52VL CDR3 |
| 329 | AA | A02.53VL CDR1 |
| 330 | AA | A02.53VL CDR2 |
| 331 | AA | A02.53VL FWR3 |
| 332 | AA | A02.53VL CDR3 |
| 333 | AA | A02.54VL CDR1 |
| 334 | AA | A02.54VL CDR2 |
| 335 | AA | A02.54VL FWR3 |
| 336 | AA | A02.54VL CDR3 |
| 337 | AA | A02.55VL CDR1 |
| 338 | AA | A02.55VL CDR2 |
| 339 | AA | A02.55VL FWR3 |
| 340 | AA | A02.55VL CDR3 |
| 341 | AA | A02.56VL CDR1 |
| 342 | AA | A02.56VL CDR2 |
| 343 | AA | A02.56VL FWR3 |
| 344 | AA | A02.56VL CDR3 |
| 345 | AA | A02.57VL CDR1 |
| 346 | AA | A02.57VL CDR2 |
| 347 | AA | A02.57VL FWR3 |
| 348 | AA | A02.57VL CDR3 |
| 349 | AA | A02.58VL CDR1 |
| 350 | AA | A02.58VL CDR2 |
| 351 | AA | A02.58VL FWR3 |
| 352 | AA | A02.58VL CDR3 |
| 353 | AA | A02.59VL CDR1 |
| 354 | AA | A02.59VL CDR2 |
| 355 | AA | A02.59VL FWR3 |
| 356 | AA | A02.59VL CDR3 |
| 357 | AA | A02.60VL CDR1 |
| 358 | AA | A02.60VL CDR2 |
| 359 | AA | A02.60VL FWR3 |
| 360 | AA | A02.60VL CDR3 |
| 361 | AA | A02.61VL CDR1 |
| 362 | AA | A02.61VL CDR2 |
| 363 | AA | A02.61VL FWR3 |
| 364 | AA | A02.61VL CDR3 |
| 365 | AA | A02.62VL CDR1 |
| 366 | AA | A02.62VL CDR2 |
| 367 | AA | A02.62VL FWR3 |
| 368 | AA | A02.62VL CDR3 |
| 369 | AA | A02.63VL CDR1 |
| 370 | AA | A02.63VL CDR2 |
| 371 | AA | A02.63VL FWR3 |
| 372 | AA | A02.63VL CDR3 |
| 373 | AA | A02.64VL CDR1 |
| 374 | AA | A02.64VL CDR2 |
| 375 | AA | A02.64VL FWR3 |
| 376 | AA | A02.64VL CDR3 |
| 377 | AA | A02.65VL CDR1 |
| 378 | AA | A02.65VL CDR2 |
| 379 | AA | A02.65VL FWR3 |
| 380 | AA | A02.65VL CDR3 |
| 381 | AA | A02.66VL CDR1 |
| 382 | AA | A02.66VL CDR2 |
| 383 | AA | A02.66VL FWR3 |
| 384 | AA | A02.66VL CDR3 |
| 385 | AA | A02.67VL CDR1 |
| 386 | AA | A02.67VL CDR2 |
| 387 | AA | A02.67VL FWR3 |
| 388 | AA | A02.67VL CDR3 |
| 389 | AA | 5D1.1VH FWR1 |
| 390 | AA | 5D1.1VH CDR1 |
| 391 | AA | 5D1.1VH FWR2 |
| 392 | AA | 5D1.1VH CDR2 |
| 393 | AA | 5D1.1VH FWR3 |
| 394 | AA | 5D1.1VH CDR3 |
| 395 | AA | 5D1.1VH FWR4 |
| 396 | AA | 5D1.2VH FWR1 |
| 397 | AA | 5D1.2VH FWR2 |
| 398 | AA | 5D1.2VH CDR2 |
| 399 | AA | 5D1.2VH FWR3 |
| 400 | AA | 5D1.3VH FWR1 |
| 401 | AA | 5D1.3VH FWR2 |
| 402 | AA | 5D1.3VH CDR2 |
| 403 | AA | 5D1.3VH FWR3 |
| 404 | AA | 5D1.4VH FWR1 |
| 405 | AA | 5D1.4VH FWR2 |
| 406 | AA | 5D1.4VH CDR2 |
| 407 | AA | 5D1.4VH FWR3 |
| 408 | AA | 5D1.5VH FWR1 |
| 409 | AA | 5D1.5VH FWR2 |
| 410 | AA | 5D1.5VH CDR2 |
| 411 | AA | 5D1.5VH FWR3 |
| 412 | AA | 5D1.6VH FWR1 |
| 413 | AA | 5D1.6VH FWR2 |
| 414 | AA | 5D1.6VH CDR2 |
| 415 | AA | 5D1.6VH FWR3 |
| 416 | AA | 5D1.7VH FWR1 |
| 417 | AA | 5D1.7VH FWR2 |
| 418 | AA | 5D1.7VH CDR2 |
| 419 | AA | 5D1.7VH FWR3 |
| 420 | AA | 5D1.8VH FWR1 |
| 421 | AA | 5D1.8VH FWR2 |
| 422 | AA | 5D1.8VH CDR2 |
| 423 | AA | 5D1.8VH FWR3 |
| 424 | AA | 5D1.9VH FWR1 |
| 425 | AA | 5D1.9VH FWR2 |
| 426 | AA | 5D1.9VH CDR2 |
| 427 | AA | 5D1.9VH FWR3 |
| 428 | AA | 5D1.10VH FWR1 |
| 429 | AA | 5D1.10VH FWR2 |
| 430 | AA | 5D1.10VH CDR2 |
| 431 | AA | 5D1.10VH FWR3 |
| 432 | AA | 5D1.11VH FWR1 |
| 433 | AA | 5D1.11VH FWR2 |
| 434 | AA | 5D1.11VH CDR2 |
| 435 | AA | 5D1.11VH FWR3 |
| 436 | AA | 5D1.1VL FWR1 |
| 437 | AA | 5D1.1VL CDR1 |
| 438 | AA | 5D1.1VL FWR2 |
| 439 | AA | 5D1.1VL CDR2 |
| 440 | AA | 5D1.1VL FWR3 |
| 441 | AA | 5D1.1VL CDR3 |
| 442 | AA | 5D1.1VL FWR4 |
| 443 | AA | 5D1.2VL FWR1 |
| 444 | AA | 5D1.2VL FWR2 |
| 445 | AA | 5D1.2VL FWR3 |
| 446 | AA | 5D1.2VL FWR4 |
| 447 | AA | 5D1.3VL FWR1 |
| 448 | AA | 5D1.3VL FWR2 |
| 449 | AA | 5D1.3VL FWR3 |
| 450 | AA | 5D1.3VL FWR4 |
| 451 | AA | 5D1.4VL FWR1 |
| 452 | AA | 5D1.4VL FWR2 |
| 453 | AA | 5D1.4VL FWR3 |
| 454 | AA | 5D1.4VL FWR4 |
| 455 | AA | 5D1.5VL FWR1 |
| 456 | AA | 5D1.5VL FWR2 |
| 457 | AA | 5D1.5VL FWR3 |
| 458 | AA | 5D1.5VL FWR4 |
| 459 | AA | 5D1.6VL FWR1 |
| 460 | AA | 5D1.6VL FWR2 |
| 461 | AA | 5D1.6VL FWR3 |
| 462 | AA | 5D1.6VL FWR4 |
| 463 | AA | 5D1.7VL FWR1 |
| 464 | AA | 5D1.7VL FWR2 |
| 465 | AA | 5D1.7VL FWR3 |
| 466 | AA | 5E8.1VH FWR1 |
| 467 | AA | 5E8.1VH CDR2 |
| 468 | AA | 5E8.1VH FWR3 |
| 469 | AA | 5E8.1VH CDR3 |
| 470 | AA | 5E8.2VH FWR1 |
| 471 | AA | 5E8.2VH CDR2 |
| 472 | AA | 5E8.3VH FWR1 |
| 473 | AA | 5E8.3VH CDR2 |
| 474 | AA | 5E8.4VH FWR1 |
| 475 | AA | 5E8.4VH CDR2 |
| 476 | AA | 5E8.5VH FWR1 |
| 477 | AA | 5E8.5VH CDR2 |
| 478 | AA | 5E8.6VH FWR1 |
| 479 | AA | 5E8.6VH CDR2 |
| 480 | AA | 5E8.7VH FWR1 |
| 481 | AA | 5E8.7VH CDR2 |
| 482 | AA | 5E8.8VH FWR1 |
| 483 | AA | 5E8.8VH CDR2 |
| 484 | AA | 5E8.9VH FWR1 |
| 485 | AA | 5E8.9VH CDR2 |
| 486 | AA | 5E8.10VH FWR1 |
| 487 | AA | 58E.10VH CDR2 |
| 488 | AA | 5E8.11VH FWR1 |
| 489 | AA | 58E.11VH CDR2 |
| 490 | AA | 5E8.1VL FWR1 |
| 491 | AA | 5E8.1VL CDR1 |
| 492 | AA | 5E8.1VL FWR2 |
| 493 | AA | 5E8.1VL CDR2 |
| 494 | AA | 5E8.1VL FWR3 |
| 495 | AA | 5E8.1VL CDR3 |
| 496 | AA | 5E8.1VL FWR4 |
| 497 | AA | 5E8.2VL FWR1 |
| 498 | AA | 5E8.2VL FWR2 |
| 499 | AA | 5E8.2VL FWR3 |
| 500 | AA | 5E8.2VL FWR4 |
| 501 | AA | 5E8.3VL FWR1 |
| 502 | AA | 5E8.3VL FWR2 |
| 503 | AA | 5E8.3VL FWR3 |
| 504 | AA | 5E8.3VL FWR4 |
| 505 | AA | 5E8.4VL FWR1 |
| 506 | AA | 5E8.4VL FWR2 |
| 507 | AA | 5E8.4VL FWR3 |
| 508 | AA | 5E8.4VL FWR4 |
| 509 | AA | 5E8.5VL FWR1 |
| 510 | AA | 5E8.5VL FWR2 |
| 511 | AA | 5E8.5VL FWR3 |
| 512 | AA | 5E8.5VL FWR4 |
| 513 | AA | 10A2.1VH FWR1 |
| 514 | AA | 10A2.1VH CDR1 |
| 515 | AA | 10A2.1VH FWR2 |
| 516 | AA | 10A2.1VH CDR2 |
| 517 | AA | 10A2.1VH FWR3 |
| 518 | AA | 10A2.1VH CDR3 |
| 519 | AA | 10A2.1VH FWR4 |
| 520 | AA | 10A2.2VH FWR2 |
| 521 | AA | 10A2.3VH FWR2 |
| 522 | AA | 10A2.4VH FWR2 |
| 523 | AA | 10A2.5VH FWR2 |
| 524 | AA | 10A2.6VH FWR2 |
| 525 | AA | 10A2.7VH FWR2 |
| 526 | AA | 10A2.8VH CDR1 |
| 527 | AA | 10A2.9VH CDR1 |
| 528 | AA | 10A2.10VH CDR1 |
| 529 | AA | 10A2.11VH CDR1 |
| 530 | AA | 10A2.12VH FWR3 |
| 531 | AA | 10A2.13VH FWR3 |
| 532 | AA | 10A2.14VH FWR3 |
| 533 | AA | 10A2.15VH FWR3 |
| 534 | AA | 10A2.16VH CDR3 |
| 535 | AA | 10A2.17VH CDR3 |
| 536 | AA | 10A2.18VH CDR3 |
| 537 | AA | 10A2.19VH FWR1 |
| 538 | AA | 10A2.19VH FWR2 |
| 539 | AA | 10A2.19VH CDR2 |
| 540 | AA | 10A2.19VH FWR3 |
| 541 | AA | 10A2.19VH FWR4 |
| 542 | AA | 10A2.20VH FWR1 |
| 543 | AA | 10A2.20VH FWR2 |
| 544 | AA | 10A2.20VH CDR2 |
| 545 | AA | 10A2.20VH FWR3 |
| 546 | AA | 10A2.20VH FWR4 |
| 547 | AA | 10A2.21VH FWR1 |
| 548 | AA | 10A2.21VH FWR2 |
| 549 | AA | 10A2.21VH CDR2 |
| 550 | AA | 10A2.21VH FWR3 |
| 551 | AA | 10A2.21VH FWR4 |
| 552 | AA | 10A2.22VH FWR1 |
| 553 | AA | 10A2.22VH FWR2 |
| 554 | AA | 10A2.22VH CDR2 |
| 555 | AA | 10A2.22VH FWR3 |
| 556 | AA | 10A2.22VH FWR4 |
| 557 | AA | 10A2.23VH FWR1 |
| 558 | AA | 10A2.23VH FWR2 |
| 559 | AA | 10A2.23VH CDR2 |
| 560 | AA | 10A2.23VH FWR3 |
| 561 | AA | 10A2.23VH FWR4 |
| 562 | AA | 10A2.24VH FWR1 |
| 563 | AA | 10A2.24VH FWR2 |
| 564 | AA | 10A2.24VH CDR2 |
| 565 | AA | 10A2.24VH FWR3 |
| 566 | AA | 10A2.24VH FWR4 |
| 567 | AA | 10A2.25VH FWR1 |
| 568 | AA | 10A2.25VH FWR2 |
| 569 | AA | 10A2.25VH CDR2 |
| 570 | AA | 10A2.25VH FWR3 |
| 571 | AA | 10A2.25VH FWR4 |
| 572 | AA | 10A2.26VH FWR1 |
| 573 | AA | 10A2.26VH FWR2 |
| 574 | AA | 10A2.26VH CDR2 |
| 575 | AA | 10A2.26VH FWR3 |
| 576 | AA | 10A2.26VH FWR4 |
| 577 | AA | 10A2.27VH FWR1 |
| 578 | AA | 10A2.27VH FWR2 |
| 579 | AA | 10A2.27VH CDR2 |
| 580 | AA | 10A2.27VH FWR3 |
| 581 | AA | 10A2.27VH FWR4 |
| 582 | AA | 10A2.1VL FWR1 |
| 583 | AA | 10A2.1VL CDR1 |
| 584 | AA | 10A2.1VL FWR2 |
| 585 | AA | 10A2.1VL CDR2 |
| 586 | AA | 10A2.1VL FWR3 |
| 587 | AA | 10A2.1VL CDR3 |
| 588 | AA | 10A2.1VL FWR4 |
| 589 | AA | 10A2.2VL CDR1 |
| 590 | AA | 10A2.3VL CDR1 |
| 591 | AA | 10A2.4VL CDR2 |
| 592 | AA | 10A2.5VL FWR2 |
| 593 | AA | 10A2.6VL FWR2 |
| 594 | AA | 10A2.7VL CDR3 |
| 595 | AA | 10A2.8VL CDR3 |
| 596 | AA | 10A2.9VL CDR3 |
| 597 | AA | 10A2.10VL CDR3 |
| 598 | AA | 10A2.11VL CDR3 |
| 599 | AA | 10A2.12VL CDR3 |
| 600 | AA | 10A2.13VL CDR3 |
| 601 | AA | 10A2.14VL CDR3 |
| 602 | AA | 10A2.15VL CDR3 |
| 603 | AA | 10A2 16VL CDR3 |
| 604 | AA | 10A2.17VL CDR3 |
| 605 | AA | 10A2.18VL CDR2 |
| 606 | AA | 10A2.19VL CDR3 |
| 607 | AA | 10A2.20VL FWR1 |
| 608 | AA | 10A2.20VL CDR1 |
| 609 | AA | 10A2,20VL FWR2 |
| 610 | AA | 10A2.20VL CDR2 |
| 611 | AA | 10A2.20VL FWR3 |
| 612 | AA | 10A2.20VL CDR3 |
| 613 | AA | 10A2.20VL FWR4 |
| 614 | AA | 10A2.21VL FWR1 |
| 615 | AA | 10A2.21VL FWR2 |
| 616 | AA | 10A2.21VL FWR3 |
| 617 | AA | 10A2.21VL FWR4 |
| 618 | AA | 10A2.22VL FWR1 |
| 619 | AA | 10A2.22VL FWR2 |
| 620 | AA | 10A2.22VL FWR3 |
| 621 | AA | 10A2.22VL FWR4 |
| 622 | AA | 10A2.23VL FWR1 |
| 623 | AA | 10A2.23VL FWR2 |
| 624 | AA | 10A2.23VL FWR3 |
| 625 | AA | 10A2.23VL FWR4 |
| 626 | AA | 10A2.24VL FWR1 |
| 627 | AA | 10A2.24VL FWR2 |
| 628 | AA | 10A2.24VL FWR3 |
| 629 | AA | 10A2.24VL FWR4 |
| 630 | AA | 10A2.25VL FWR1 |
| 631 | AA | 10A2.25VL FWR2 |
| 632 | AA | 10A2.25VL FWR3 |
| 633 | AA | 10A2.25VL FWR4 |
| 634 | AA | 10A2.26VL FWR1 |
| 635 | AA | 10A2.26VL FWR2 |
| 636 | AA | 10A2.26VL FWR3 |
| 637 | AA | 10A2.26VL FWR4 |
| 638 | AA | 10A2.27VL FWR1 |
| 639 | AA | 10A2.27 VL FWR2 |
| 640 | AA | 10A2.27VL FWR3 |
| 641 | AA | 10A2.27VL FWR4 |
| 642 | AA | Gly4Ser1 |
| 643 | AA | Gly4Ser1 x 2 |
| 644 | AA | Gly4Ser1 x 3 |
| 645 | AA | Gly4Ser1 x 4 |
| 646 | AA | Gly4Ser1 x 5 |
| 647 | AA | IFN-alpha2b A145D |
| 648 | AA | Trunc IFN-alpha2b |
| 649 | AA | Trunc IFN-alpha2b A145D |
| 650 | AA | IFN-alpha2b A145G |
| 651 | AA | Trunc IFN-alpha2b A145G |
| 652 | AA | IgG4 IFN-alpha2b A145D |
| 653 | AA | IgG4 IFN-alpha2b A145G |
| 654 | AA | IgG4 S228P IFN-alpha2b A145G |
| 655 | AA | IgG1 IFN-alpha2b A145G |
| 656 | AA | IgG1 YTE IFN-alpha2b A145D |
| 657 | AA | IgG1 YTE IFN-alpha2b A145G |
| 658 | AA | IgG4 YTE IFN-alpha2b A145D |
| 659 | AA | A02 consensus variable heavy |
| 660 | AA | X02.114VL |
| 661 | AA | X02.115VL |
| 662 | AA | X02.116VL |
| 663 | AA | X02.117VL |
| 664 | AA | A02 consensus variable light |
| 665 | AA | A10 consensus variable heavy |
| 666 | AA | A10 consensus variable light |
| 667 | DNA | A02.12VH |
| 668 | DNA | X02.9VH |
| 669 | DNA | X02.107VL |
| 670 | DNA | A02.47VL |
| 671 | DNA | A02.31VL |
| 672 | DNA | A02.33VL |
| 673 | DNA | X02.16VL |
| 674 | DNA | X02.17VL |
| 675 | DNA | X02.114VL |
| 676 | DNA | X02.115VL |
| 677 | DNA | X02.116VL |
| 678 | DNA | X02.117VL |
| 679 | DNA | 10A2VH + A40E |
| 680 | DNA | 10A2VH + A40G |
| 681 | DNA | 10A2VH + A40H |
| 682 | DNA | 10A2VH + A40Q |
| 683 | DNA | 10A2VH + K96G |
| 684 | DNA | 10A2VH + K96T |
| 685 | DNA | 10A2_1-f^{∗}01VH94R |
| 686 | DNA | 10A2VH (AQ) + N98Q |
| 687 | DNA | 10A2-1-24^{∗}0171A94R |
| 688 | DNA | 10A2_1-33^{∗}01Vk |
| 689 | DNA | 10A2VK + K24G |
| 690 | DNA | 10A2VK + K24Q |
| 691 | DNA | 10A2VK + R54D |
| 692 | DNA | 10A2VK + M89A |
| 693 | DNA | 10A2VK (AV) + N53Q |
| 694 | AA | IgG4 YTE IFN-alpha2b A145G |
| 695 | DNA | 910VH variable heavy chain |
| 696 | AA | 10A2VK + K24G CDR1 |
| 697 | AA | 910VH CDR1 |
| 698 | AA | 910VH CDR2 |
| 699 | AA | 910VH CDR3 |
| 700 | AA | X02.118 variable light chain |
| 701 | AA | X02.119 variable light chain |
| 702 | DNA | X02.118 variable light chain |
| 703 | DNA | X02.119 variable light chain |
| 704 | AA | X10.60 variable light chain |
| 705 | AA | X10.61 variable light chain |
| 706 | AA | X10.62 variable light chain |
| 707 | AA | X10.63 variable light chain |
| 708 | AA | X10.64 variable light chain |
| 709 | AA | X10.65 variable light chain |
| 710 | AA | X10.66 variable light chain |
| 711 | AA | X10.67 variable light chain |
| 712 | DNA | X10.60 variable light chain |
| 713 | DNA | X10.61 variable light chain |
| 714 | DNA | X10.62 variable light chain |
| 715 | DNA | X10.63 variable light chain |
| 716 | DNA | X10.64 variable light chain |
| 717 | DNA | X10.65 variable light chain |
| 718 | DNA | X10.66 variable light chain |
| 719 | DNA | X10.67 variable light chain |
| 720 | AA | X10.68 variable heavy chain |
| 721 | AA | X10.69 variable heavy chain |
| 722 | AA | X10.70 variable heavy chain |
| 723 | AA | X10.71 variable heavy chain |
| 724 | DNA | X10.68 variable heavy chain |
| 725 | DNA | X10.69 variable heavy chain |
| 726 | DNA | X10.70 variable heavy chain |
| 727 | DNA | X10.71 variable heavy chain |
| 728 | AA | X02.120 variable heavy chain |
| 729 | AA | X02.121 variable heavy chain |
| 730 | AA | X02.122 variable heavy chain |
| 731 | AA | X02.123 variabel heavy chain |
| 732 | DNA | X02.120 variable heavy chain |
| 733 | DNA | X02.121 variable heavy chain |
| 734 | DNA | X02.122 variable heavy chain |
| 735 | DNA | X02.123 variable heavy chain |
| 736 | AA | 910 variable heavy consensus |
| 737 | AA | X02.122VH CDR2 |
| 738 | AA | X02.123VH CDR2 |
| 739 | AA | X10.72 variable heavy chain |
| 740 | AA | X10.73 variable heavy chain |
| 741 | AA | X10.74 variable heavy chain |
| 742 | AA | X10.75 variable heavy chain |
| 743 | DNA | X10.72 variable heavy chain |
| 744 | DNA | X10.73 variable heavy chain |
| 745 | DNA | X10.74 variable heavy chain |
| 746 | DNA | X10.75 variable heavy chain |
| 747 | AA | X10.64VL CDR2 |
| 748 | AA | 910 VH FRW1 |
| 749 | AA | 910 VH FRW2 |
| 750 | AA | X10.120VH FRW2 |
| 751 | AA | 910 VH FRW3 |
| 752 | AA | X10.121VH FRW3 |
| 753 | AA | 910VH FRW4 |

The disclosure is not limited to the embodiments described and exemplified above, but is capable of variation and modification within the scope of the appended claims.

## Claims

1. An antibody that specifically binds to CD38, the antibody comprising a heavy chain variable region that comprises the amino acid sequence of SEQ ID NO: 156 and a light chain variable region that comprises the amino acid sequence of SEQ ID NO: 185.

2. The antibody according to claim 1, wherein the antibody comprises a human IgG1 heavy chain constant region.

3. The antibody according to claim 1, wherein the antibody comprises a human IgG4 heavy chain constant region.

4. The antibody according to claim 3, wherein the human IgG4 heavy chain constant region comprises a proline at position 228 according to the EU numbering system.

5. The antibody according to claim 3 or claim 4, wherein the human IgG4 heavy chain constant region comprises a tyrosine at position 252, a threonine at position 254, and a glutamic acid at position 256 of the constant region according to the EU numbering system.

6. An anti-CD38 antibody-interferon alpha-2b fusion construct, comprising:
an antibody according to any one of claims 1 to 5; and
an interferon alpha-2b comprising an alanine to aspartic acid or alanine to glycine substitution at the position corresponding to position 168 of the amino acid sequence of SEQ ID NO. 7.

7. The construct according to claim 6, wherein the interferon alpha-2b is an N-terminally truncated interferon alpha-2b, optionally wherein the N-terminally truncated interferon alpha-2b does not have the twenty three N-terminal amino acids.

8. The construct according to claim 6 or claim 7, wherein the interferon alpha-2b comprises the amino acid sequence of SEQ ID NO: 649.

9. The construct according to claim 6 or 7, wherein the interferon alpha-2b comprises the amino acid sequence of SEQ ID NO: 651.

10. The construct according to claim 6, wherein the interferon alpha-2b comprises the amino acid sequence of SEQ ID NO: 647 or SEQ ID NO: 650.

11. A composition, comprising the antibody according to any one of claims 1 to 5 or the construct according to any one of claims 6 to 10 and a pharmaceutically acceptable carrier.

12. The antibody according to any one of claims 1 to 5, the construct according to any one of claims 6 to 10 or the composition according to claim 11 for use in medicine.

13. The antibody according to any one of claims 1 to 5, the construct according to any one of claims 6 to 10 or the composition according to claim 11 for use in the treatment of B-cell lymphoma, multiple myeloma, non-Hodgkin's lymphoma, chronic myelogenous leukemia, chronic lymphocytic leukemia or acute myelogenous leukemia.

## Patentansprüche

1. Antikörper, der an CD38 spezifisch bindet, wobei der Antikörper eine Schwere-Kette-variable-Region, die die Aminosäuresequenz unter SEQ ID NO: 156 umfasst, und eine Leichte-Kette-variable-Region, die die Aminosäuresequenz unter SEQ ID NO: 185 umfasst, umfasst.

2. Antikörper nach Anspruch 1, wobei der Antikörper eine menschliche IgG1-schwere-Kette-konstante-Region umfasst.

3. Antikörper nach Anspruch 1, wobei der Antikörper eine menschliche IgG4-schwere-Kette-konstante-Region umfasst.

4. Antikörper nach Anspruch 3, wobei die menschliche IgG4-schwere-Kette-konstante-Region ein Prolin an Position 228 gemäß dem EU-Nummerierungssystem umfasst.

5. Antikörper nach Anspruch 3 oder Anspruch 4, wobei die menschliche IgG4-schwere-Kette-konstante-Region ein Tyrosin an Position 252, ein Threonin an Position 254 und eine Glutaminsäure an Position 256 der konstanten Region gemäß dem EU-Nummerierungssystem umfasst.

6. Anti-CD38-Antikörper-Interferon-alpha-2b-Fusions-konstrukt, umfassend:
einen Antikörper nach einem der Ansprüche 1 bis 5; und
ein Interferon-alpha-2b, das eine Alanin-zu-Asparaginsäure- oder Alanin-zu-Glycin-Substitution an der Position 168 der Aminosäuresequenz unter SEQ ID NO: 7 entsprechenden Position umfasst.

7. Konstrukt nach Anspruch 6, wobei es sich bei dem Interferon-alpha-2b um ein N-terminal verkürztes Interferon-alpha-2b handelt, gegebenenfalls wobei das N-terminal verkürzte Interferon-alpha-2b nicht die dreiundzwanzig N-terminalen Aminosäuren umfasst.

8. Konstrukt nach Anspruch 6 oder Anspruch 7, wobei das Interferon-alpha-2b die Aminosäuresequenz unter SEQ ID NO: 649 umfasst.

9. Konstrukt nach Anspruch 6 oder 7, wobei das Interferon-alpha-2b die Aminosäuresequenz unter SEQ ID NO: 651 umfasst.

10. Konstrukt nach Anspruch 6, wobei das Interferon-alpha-2b die Aminosäuresequenz unter SEQ ID NO: 647 oder SEQ ID NO: 650 umfasst.

11. Zusammensetzung, umfassend den Antikörper nach einem der Ansprüche 1 bis 5 oder das Konstrukt nach einem der Ansprüche 6 bis 10 und einen pharmazeutisch unbedenklichen Träger.

12. Antikörper nach einem der Ansprüche 1 bis 5, Konstrukt nach einem der Ansprüche 6 bis 10 oder Zusammensetzung nach Anspruch 11 zur medizinischen Verwendung.

13. Antikörper nach einem der Ansprüche 1 bis 5, Konstrukt nach einem der Ansprüche 6 bis 10 oder Zusammensetzung nach Anspruch 11 zur Verwendung bei der Behandlung von B-Zell-Lymphom, multiplem Myelom, Non-Hodgkin-Lymphom, chronischer myeloischer Leukämie, chronischer lymphatischer Leukämie oder akuter myeloischer Leukämie.

## Revendications

1. Anticorps qui se lie spécifiquement au CD38, l'anticorps comprenant une région variable de chaîne lourde qui comprend la séquence d'acides aminés de SEQ ID n° : 156 et une région variable de chaîne légère qui comprend la séquence d'acides aminés de SEQ ID n° : 185.

2. Anticorps selon la revendication 1, l'anticorps comprenant une région constante de chaîne lourde d'une IgG1 humaine.

3. Anticorps selon la revendication 1, l'anticorps comprenant une région constante de chaîne lourde d'une IgG4 humaine.

4. Anticorps selon la revendication 3, dans lequel la région constante de chaîne lourde d'une IgG4 humaine comprend une proline en position 228, selon le système de numérotation de l'UE.

5. Anticorps selon la revendication 3 ou la revendication 4, dans lequel la région constante de chaîne lourde d'une IgG4 humaine comprend une tyrosine en position 252, une thréonine en position 254 et un acide glutamique en position 256 de la région constante, selon le système de numérotation de l'UE.

6. Construction d'une fusion d'anticorps anti-CD38-interféron alpha-2b, comprenant :
un anticorps selon l'une quelconque des revendications 1 à 5 ; et
un interféron alpha-2b comprenant une substitution d'une alanine en acide aspartique ou d'une alanine en glycine en position correspondant à la position 168 de la séquence d'acides aminés de SEQ ID n° : 7.

7. Construction selon la revendication 6, dans laquelle l'interféron alpha-2b est un interféron alpha-2b tronqué au niveau N-terminal, en option dans laquelle l'interféron alpha-2b tronqué au niveau N-terminal n'a pas les vingt-trois acides aminés N-terminaux.

8. Construction selon la revendication 6 ou la revendication 7, dans laquelle l'interféron alpha-2b comprend la séquence d'acides aminés de SEQ ID n° : 649.

9. Construction selon la revendication 6 ou 7, dans laquelle l'interféron alpha-2b comprend la séquence d'acides aminés de SEQ ID n° : 651.

10. Construction selon la revendication 6, dans laquelle l'interféron alpha-2b comprend la séquence d'acides aminés de SEQ ID n° : 647 ou de SEQ ID n° : 650.

11. Composition comprenant l'anticorps selon l'une quelconque des revendications 1 à 5, ou la construction, selon l'une quelconque des revendications 6 à 10, et un véhicule acceptable d'un point de vue pharmaceutique.

12. Anticorps selon l'une quelconque des revendications 1 à 5, construction selon l'une quelconque des revendications 6 à 10, ou composition selon la revendication 11, destiné(e) à être utilisé(e) en médecine.

13. Anticorps selon l'une quelconque des revendications 1 à 5, construction selon l'une quelconque des revendications 6 à 10, ou composition selon la revendication 11, destiné(e) à être utilisé(e) dans le traitement d'un lymphome à lymphocytes B, d'un myélome multiple, d'un lymphome non Hodgkinien, d'une leucémie myéloïde chronique, d'une leucémie lymphoïde chronique ou d'une leucémie myéloïde aiguë.


### SEQUENCE LISTING

<110> Cephalon Australia Pty Ltd
<120> ANTI-CD38 ANTIBODIES, ANTI-CD38 ANTIBODY CONSTRUCTS AND USES THEREOF
<130> 185704-3015 (CAP572 WO)
<160> 753
<170> PatentIn version 3.5
<210> 1
   <211> 258
   <212> PRT
   <213> Homo sapiens
<400> 1
   Val Pro Arg Trp Arg Gln Gln Trp Ser Gly Pro Gly Thr Thr Lys Arg
   1               5                   10                  15
   Phe Pro Glu Thr Val Leu Ala Arg Cys Val Lys Tyr Thr Glu Ile His
               20                  25                  30
   Pro Glu Met Arg His Val Asp Cys Gln Ser Val Trp Asp Ala Phe Lys
           35                  40                  45
   Gly Ala Phe Ile Ser Lys His Pro Cys Asn Ile Thr Glu Glu Asp Tyr
       50                  55                  60
   Gln Pro Leu Met Lys Leu Gly Thr Gln Thr Val Pro Cys Asn Lys Ile
   65                  70                  75                  80
   Leu Leu Trp Ser Arg Ile Lys Asp Leu Ala His Gln Phe Thr Gln Val
                   85                  90                  95
   Gln Arg Asp Met Phe Thr Leu Glu Asp Thr Leu Leu Gly Tyr Leu Ala
               100                 105                 110
   Asp Asp Leu Thr Trp Cys Gly Glu Phe Asn Thr Ser Lys Ile Asn Tyr
           115                 120                 125
   Gln Ser Cys Pro Asp Trp Arg Lys Asp Cys Ser Asn Asn Pro Val Ser
       130                 135                 140
   Val Phe Trp Lys Thr Val Ser Arg Arg Phe Ala Glu Ala Ala Cys Asp
   145                 150                 155                 160
   Val Val His Val Met Leu Asn Gly Ser Arg Ser Lys Ile Phe Asp Lys
                   165                 170                 175
   Asn Ser Thr Phe Gly Ser Val Glu Val His Asn Leu Gln Pro Glu Lys
               180                 185                 190
   Val Gln Thr Leu Glu Ala Trp Val Ile His Gly Gly Arg Glu Asp Ser
           195                 200                 205
   Arg Asp Leu Cys Gln Asp Pro Thr Ile Lys Glu Leu Glu Ser Ile Ile
       210                 215                 220
   Ser Lys Arg Asn Ile Gln Phe Ser Cys Lys Asn Ile Tyr Arg Pro Asp
   225                 230                 235                 240
   Lys Phe Leu Gln Cys Val Lys Asn Pro Glu Asp Ser Ser Cys Thr Ser
                   245                 250                 255
   Glu Ile
<210> 2
   <211> 258
   <212> PRT
   <213> Macaca fascicularis
<400> 2
   Leu Pro Arg Trp Arg Gln Gln Trp Ser Gly Ser Gly Thr Thr Ser Arg
   1               5                   10                  15
   Phe Pro Glu Thr Val Leu Ala Arg Cys Val Lys Tyr Thr Glu Val His
               20                  25                  30
   Pro Glu Met Arg His Val Asp Cys Gln Ser Val Trp Asp Ala Phe Lys
           35                  40                  45
   Gly Ala Phe Ile Ser Lys Tyr Pro Cys Asn Ile Thr Glu Glu Asp Tyr
       50                  55                  60
   Gln Pro Leu Val Lys Leu Gly Thr Gln Thr Val Pro Cys Asn Lys Thr
   65                  70                  75                  80
   Leu Leu Trp Ser Arg Ile Lys Asp Leu Ala His Gln Phe Thr Gln Val
                   85                  90                  95
   Gln Arg Asp Met Phe Thr Leu Glu Asp Met Leu Leu Gly Tyr Leu Ala
               100                 105                 110
   Asp Asp Leu Thr Trp Cys Gly Glu Phe Asn Thr Phe Glu Ile Asn Tyr
           115                 120                 125
   Gln Ser Cys Pro Asp Trp Arg Lys Asp Cys Ser Asn Asn Pro Val Ser
       130                 135                 140
   Val Phe Trp Lys Thr Val Ser Arg Arg Phe Ala Glu Thr Ala Cys Gly
   145                 150                 155                 160
   Val Val His Val Met Leu Asn Gly Ser Arg Ser Lys Ile Phe Asp Lys
                   165                 170                 175
   Asn Ser Thr Phe Gly Ser Val Glu Val His Asn Leu Gln Pro Glu Lys
               180                 185                 190
   Val Gln Ala Leu Glu Ala Trp Val Ile His Gly Gly Arg Glu Asp Ser
           195                 200                 205
   Arg Asp Leu Cys Gln Asp Pro Thr Ile Lys Glu Leu Glu Ser Ile Ile
       210                 215                 220
   Ser Lys Arg Asn Ile Arg Phe Phe Cys Lys Asn Ile Tyr Arg Pro Asp
   225                 230                 235                 240
   Lys Phe Leu Gln Cys Val Lys Asn Pro Glu Asp Ser Ser Cys Leu Ser
                   245                 250                 255
   Gly Ile
<210> 3
   <211> 327
   <212> PRT
   <213> Homo sapiens
<400> 3
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 4
   <211> 330
   <212> **PRT**
   <213> Homo sapiens
<400> 4
<210> 5
   <211> 106
   <212> PRT
   <213> Homo sapiens
<400> 5
   Thr Val Ala Ala Pro Ser Val Phe Ile Phe Pro Pro Ser Asp Glu Gln
   1               5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Cys
               100                 105
<210> 6
   <211> 105
   <212> PRT
   <213> Homo sapiens
<400> 6
   Gln Pro Lys Ala Ala Pro Ser Val Thr Leu Phe Pro Pro Ser Ser Glu
   1               5                   10                  15
   Glu Leu Gln Ala Asn Lys Ala Thr Leu Val Cys Leu Ile Ser Asp Phe
               20                  25                  30
   Tyr Pro Gly Ala Val Thr Val Ala Trp Lys Ala Asp Ser Ser Pro Val
           35                  40                  45
   Lys Ala Gly Val Glu Thr Thr Thr Pro Ser Lys Gln Ser Asn Asn Lys
       50                  55                  60
   Tyr Ala Ala Ser Ser Tyr Leu Ser Leu Thr Pro Glu Gln Trp Lys Ser
   65                  70                  75                  80
   His Arg Ser Tyr Ser Cys Gln Val Thr His Glu Gly Ser Thr Val Glu
                   85                  90                  95
   Lys Thr Val Ala Pro Thr Glu Cys Ser
               100                 105
<210> 7
   <211> 188
   <212> PRT
   <213> Homo sapiens
<400> 7
<210> 8
   <211> 165
   <212> PRT
   <213> Homo sapiens
<400> 8
   Cys Asp Leu Pro Gln Thr His Ser Leu Gly Ser Arg Arg Thr Leu Met
   1               5                   10                  15
   Leu Leu Ala Gln Met Arg Arg Ile Ser Leu Phe Ser Cys Leu Lys Asp
               20                  25                  30
   Arg His Asp Phe Gly Phe Pro Gln Glu Glu Phe Gly Asn Gln Phe Gln
           35                  40                  45
   Lys Ala Glu Thr Ile Pro Val Leu His Glu Met Ile Gln Gln Ile Phe
       50                  55                  60
   Asn Leu Phe Ser Thr Lys Asp Ser Ser Ala Ala Trp Asp Glu Thr Leu
   65                  70                  75                  80
   Leu Asp Lys Phe Tyr Thr Glu Leu Tyr Gln Gln Leu Asn Asp Leu Glu
                   85                  90                  95
   Ala Cys Val Ile Gln Gly Val Gly Val Thr Glu Thr Pro Leu Met Asn
               100                 105                 110
   Glu Asp Ser Ile Leu Ala Val Arg Lys Tyr Phe Gln Arg Ile Thr Leu
   115 120 125
   Tyr Leu Lys Glu Lys Lys Tyr Ser Pro Cys Ala Trp Glu Val Val Arg
   130 135 140
   Ala Glu Ile Met Arg Ser Phe Ser Leu Ser Thr Asn Leu Gln Glu Ser
   145 150 155 160
   Leu Arg Ser Lys Glu
   165
<210> 9
   <211> 492
   <212> PRT
   <213> Homo sapiens
<400> 9
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Lys Thr
   65 70 75 80
   Tyr Thr Cys Asn Val Asp His Lys Pro Ser Asn Thr Lys Val Asp Lys
   85 90 95
   Arg Val Glu Ser Lys Tyr Gly Pro Pro Cys Pro Pro Cys Pro Ala Pro
   100 105 110
   Glu Phe Leu Gly Gly Pro Ser Val Phe Leu Phe Pro Pro Lys Pro Lys
   115 120 125
   Asp Thr Leu Met Ile Ser Arg Thr Pro Glu Val Thr Cys Val Val Val
   130 135 140
   Asp Val Ser Gln Glu Asp Pro Glu Val Gln Phe Asn Trp Tyr Val Asp
   145 150 155 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys Cys Asp Leu Pro Gln Thr His Ser Leu
                   325                 330                 335
   Gly Ser Arg Arg Thr Leu Met Leu Leu Ala Gln Met Arg Arg Ile Ser
               340                 345                 350
   Leu Phe Ser Cys Leu Lys Asp Arg His Asp Phe Gly Phe Pro Gln Glu
           355                 360                 365
   Glu Phe Gly Asn Gln Phe Gln Lys Ala Glu Thr Ile Pro Val Leu His
       370                 375                 380
   Glu Met Ile Gln Gln Ile Phe Asn Leu Phe Ser Thr Lys Asp Ser Ser
   385                 390                 395                 400
   Ala Ala Trp Asp Glu Thr Leu Leu Asp Lys Phe Tyr Thr Glu Leu Tyr
                   405                 410                 415
   Gln Gln Leu Asn Asp Leu Glu Ala Cys Val Ile Gln Gly Val Gly Val
   420 425 430
   Thr Glu Thr Pro Leu Met Lys Glu Asp Ser Ile Leu Ala Val Arg Lys
   435 440 445
   Tyr Phe Gln Arg Ile Thr Leu Tyr Leu Lys Glu Lys Lys Tyr Ser Pro
   450 455 460
   Cys Ala Trp Glu Val Val Arg Asp Glu Ile Met Arg Ser Phe Ser Leu
   465 470 475 480
   Ser Thr Asn Leu Gln Glu Ser Leu Arg Ser Lys Glu
   485 490
<210> 10
   <211> 495
   <212> PRT
   <213> Homo sapiens
<400> 10
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys Asp Lys Thr His Thr Cys Pro Pro Cys
   100 105 110
   Pro Ala Pro Glu Leu Leu Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys Cys Asp Leu Pro Gln Thr
                   325                 330                 335
   His Ser Leu Gly Ser Arg Arg Thr Leu Met Leu Leu Ala Gln Met Arg
               340                 345                 350
   Arg Ile Ser Leu Phe Ser Cys Leu Lys Asp Arg His Asp Phe Gly Phe
           355                 360                 365
   Pro Gln Glu Glu Phe Gly Asn Gln Phe Gln Lys Ala Glu Thr Ile Pro
       370                 375                 380
   Val Leu His Glu Met Ile Gln Gln Ile Phe Asn Leu Phe Ser Thr Lys
   385 390 395 400
   Asp Ser Ser Ala Ala Trp Asp Glu Thr Leu Leu Asp Lys Phe Tyr Thr
   405 410 415
   Glu Leu Tyr Gln Gln Leu Asn Asp Leu Glu Ala Cys Val Ile Gln Gly
   420 425 430
   Val Gly Val Thr Glu Thr Pro Leu Met Lys Glu Asp Ser Ile Leu Ala
   435 440 445
   Val Arg Lys Tyr Phe Gln Arg Ile Thr Leu Tyr Leu Lys Glu Lys Lys
   450 455 460
   Tyr Ser Pro Cys Ala Trp Glu Val Val Arg Asp Glu Ile Met Arg Ser
   465 470 475 480
   Phe Ser Leu Ser Thr Asn Leu Gln Glu Ser Leu Arg Ser Lys Glu
   485 490 495
<210> 11
   <211> 121
   <212> PRT
   <213> Homo sapiens
<400> 11
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
                       115                 120
<210> 12
   <211> 222
   <212> PRT
   <213> Homo sapiens
<400> 12
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly Gln Pro Lys Ala Ala Pro Ser Val Thr Leu Phe
           115                 120                 125
   Pro Pro Ser Ser Glu Glu Leu Gln Ala Asn Lys Ala Thr Leu Val Cys
       130                 135                 140
   Leu Ile Ser Asp Phe Tyr Pro Gly Ala Val Thr Val Ala Trp Lys Ala
   145                 150                 155                 160
   Asp Ser Ser Pro Val Lys Ala Gly Val Glu Thr Thr Thr Pro Ser Lys
                   165                 170                 175
   Gln Ser Asn Asn Lys Tyr Ala Ala Ser Ser Tyr Leu Ser Leu Thr Pro
               180                 185                 190
   Glu Gln Trp Lys Ser His Arg Ser Tyr Ser Cys Gln Val Thr His Glu
           195                 200                 205
   Gly Ser Thr Val Glu Lys Thr Val Ala Pro Thr Glu Cys Ser
             210                 215                 220
<210> 13
   <211> 121
   <212> PRT
   <213> Homo sapiens
<400> 13
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 14
   <211> 117
   <212> PRT
   <213> Homo sapiens
<400> 14
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 15
   <211> 121
   <212> PRT
   <213> Homo sapiens
<400> 15
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 16
   <211> 99
   <212> PRT
   <213> Homo sapiens
<400> 16
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Val Ser Ser Gly
               20                  25                  30
   Ser Tyr Tyr Trp Ser Trp Ile Arg Gln Pro Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg
<210> 17
   <211> 121
   <212> PRT
   <213> Artificial Sequence
<220>
   <223> Completely synthesized.
<400> 17
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Ser Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
   100 105 110
   Gln Gly Thr Thr Val Thr Val Ser Ser
   115 120
<210> 18
   <211> 121
   <212> PRT
   <213> Artificial Sequence
<220>
   <223> Completely synthesized
<400> 18
   Gln Leu Gln Leu Gln Glu Ser Gly Pro Gly Leu Val Lys Pro Ser Glu
   1 5 10 15
   Thr Leu Ser Leu Thr Cys Thr Val Ser Gly Gly Ser Ile Ser Ser Ser
   20 25 30
   Ser Tyr Tyr Trp Ser Trp Ile Arg Gln Pro Pro Gly Lys Gly Leu Glu
   35 40 45
   Trp Ile Gly Tyr Ile Tyr Tyr Ser Gly Ser Thr Asn Tyr Asn Pro Ser
   50 55 60
   Leu Lys Ser Arg Val Thr Ile Ser Val Asp Thr Leu Lys Asn Gln Ile
   65 70 75 80
   Ser Leu Arg Leu Thr Ser Val Thr Ala Ala Asp Thr Ala Val Tyr Tyr
   85 90 95
   Cys Ala Arg Val Gly Gly Ala Gly Gly Trp Pro Met Asp Val Trp Gly
   100 105 110
   Gln Gly Thr Thr Val Thr Val Ser Ser
   115 120
<210> 19
   <211> 121
   <212> PRT
   <213> Artificial Sequence
<220>
   <223> Completely synthesized.
<400> 19
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Ser Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 20
   <211> 121
   <212> PRT
   <213> Artificial Sequence
<220>
   <223> Completely synthesized.
<400> 20
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 21
   <211> 121
   <212> PRT
   <213> Artificial Sequence
<220>
   <223> Completely synthesized.
<400> 21
   Gln Leu Gln Leu Gln Glu Ser Gly Pro Gly Leu Val Lys Pro Ser Glu
   1 5 10 15
   Thr Leu Ser Leu Thr Cys Lys Val Ser Gly Gly Ser Ile Ser Ser Ser
   20 25 30
   Ser Tyr Tyr Trp Ser Trp Ile Arg Gln His Pro Gly Lys Gly Leu Glu
   35 40 45
   Trp Ile Gly Tyr Ile Tyr Tyr Ser Gly Ser Thr Asn Tyr Asn Pro Ser
   50 55 60
   Leu Lys Ser Arg Val Thr Ile Ser Val Asp Thr Leu Lys Asn Gln Ile
   65 70 75 80
   Ser Leu Arg Leu Thr Ser Val Thr Ala Ala Asp Thr Ala Val Tyr Tyr
   85 90 95
   Cys Ala Arg Val Gly Gly Ala Gly Gly Trp Pro Met Asp Val Trp Gly
   100 105 110
   Gln Gly Thr Thr Val Thr Val Ser Ser
   115 120
<210> 22
   <211> 121
   <212> PRT
   <213> Artificial Sequence
<220>
   <223> Completely synthesized.
<400> 22
   Gln Val Gln Leu Gln Glu Ser Gly Pro Gly Leu Val Lys Pro Ser Glu
   1 5 10 15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 23
   <211> 121
   <212> PRT
   <213> Artificial Sequence
<220>
   <223> Completely synthesized.
<400> 23
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Arg Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
   100 105 110
   Gln Gly Thr Thr Val Thr Val Ser Ser
   115 120
<210> 24
   <211> 121
   <212> PRT
   <213> Artificial Sequence
<220>
   <223> Completely synthesized.
<400> 24
   Gln Leu Gln Leu Gln Glu Ser Gly Pro Gly Leu Val Lys Pro Ser Glu
   1 5 10 15
   Thr Leu Ser Leu Thr Cys Thr Val Ser Gly Gly Ser Val Ser Ser Ser
   20 25 30
   Ser Tyr Tyr Trp Ser Trp Ile Arg Gln His Pro Gly Lys Gly Leu Glu
   35 40 45
   Trp Ile Gly Tyr Ile Tyr Tyr Ser Gly Ser Thr Asn Tyr Asn Pro Ser
   50 55 60
   Leu Lys Ser Arg Val Thr Ile Ser Val Asp Thr Leu Lys Asn Gln Ile
   65 70 75 80
   Ser Leu Arg Leu Thr Ser Val Thr Ala Ala Asp Thr Ala Val Tyr Tyr
   85 90 95
   Cys Ala Arg Val Gly Gly Ala Gly Gly Trp Pro Met Asp Val Trp Gly
   100 105 110
   Gln Gly Thr Thr Val Thr Val Ser Ser
   115 120
<210> 25
   <211> 121
   <212> PRT
   <213> Artificial Sequence
<220>
   <223> Completely synthesized.
<400> 25
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Arg Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 26
   <211> 121
   <212> PRT
   <213> Artificial Sequence
<220>
   <223> Completely synthesized.
<400> 26
   Gln Leu Gln Leu Gln Glu Ser Gly Pro Gly Leu Val Lys Pro Ser Glu
   1               5                   10                  15
   Thr Leu Lys Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 27
   <211> 121
   <212> PRT
   <213> Artificial Sequence
<220>
   <223> Completely synthesized.
<400> 27
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Leu Glu Val Trp Gly
               100                 105                 110
   Gln Gly Thr Thr Val Thr Val Ser Ser
           115                 120
<210> 28
   <211> 121
   <212> PRT
   <213> Artificial Sequence
<220>
   <223> Completely synthesized.
<400> 28
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Lys Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 29
   <211> 121
   <212> PRT
   <213> Artificial Sequence
<220>
   <223> Completely synthesized.
<400> 29
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Leu Asp Val Trp Gly
               100                 105                 110
   Gln Gly Thr Thr Val Thr Val Ser Ser
           115                 120
<210> 30
   <211> 121
   <212> PRT
   <213> Artificial Sequence
<220>
   <223> Completely synthesized.
<400> 30
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Glu Val Trp Gly
               100                 105                 110
   Gln Gly Thr Thr Val Thr Val Ser Ser
           115                 120
<210> 31
   <211> 121
   <212> PRT
   <213> Artificial Sequence
<220>
   <223> Completely synthesized.
<400> 31
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Gly
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 32
   <211> 121
   <212> PRT
   <213> Artificial Sequence
<220>
   <223> Completely synthesized.
<400> 32
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Phe
   65                  70                  75                  80
   Ser Leu Arg Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 33
   <211> 121
   <212> PRT
   <213> Artificial Sequence
<220>
   <223> Completely synthesized.
<400> 33
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Leu Lys Asn Gln Ile
   65                  70                  75                  80
   Ser Leu Lys Leu Thr Ser Val Thr Ala Ala Asp Thr Ala Val Tyr Tyr
                   85                  90                  95
   Cys Ala Arg Val Gly Gly Ala Gly Gly Trp Pro Met Asp Val Trp Gly
               100                 105                 110
   Gln Gly Thr Thr Val Thr Val Ser Ser
           115                 120
<210> 34
   <211> 121
   <212> PRT
   <213> Artificial Sequence
<220>
   <223> Completely synthesized.
<400> 34
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
   20 25 30
   Ser Tyr Tyr Trp Ser Trp Ile Arg Gln His Pro Gly Lys Gly Leu Glu
   35 40 45
   Trp Ile Gly Tyr Ile Tyr Tyr Ser Gly Ser Thr Asn Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Val Gly Gly Ala Gly Gly Trp Pro Leu Asp Val Trp Gly
   100 105 110
   Gln Gly Thr Thr Val Thr Val Ser Ser
   115 120
<210> 35
   <211> 121
   <212> PRT
   <213> Artificial Sequence
<220>
   <223> Completely synthesized.
<400> 35
   Gln Leu Gln Leu Gln Glu Ser Gly Pro Gly Leu Val Lys Pro Ser Glu
   1 5 10 15
   Thr Leu Ser Leu Thr Cys Thr Val Ser Gly Gly Ser Ile Ser Ser Ser
   20 25 30
   Ser Tyr Tyr Trp Ser Trp Ile Arg Gln Pro Pro Gly Lys Gly Leu Glu
   35 40 45
   Trp Ile Gly Tyr Ile Tyr Tyr Ser Gly Ser Thr Asn Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Val Gly Gly Ala Gly Gly Trp Pro Leu Asp Val Trp Gly
               100                 105                 110
   Gln Gly Thr Thr Val Thr Val Ser Ser
           115                 120
<210> 36
   <211> 117
   <212> PRT
   <213> Homo sapiens
<400> 36
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 37
   <211> 117
   <212> PRT
   <213> Homo sapiens
<400> 37
   Gln Pro Val Leu Thr Gln Pro Pro Ser Ser Ser Ala Ser Pro Gly Glu
   1 5 10 15
   Ser Ala Arg Leu Thr Cys Thr Leu Pro Ser Asp Ile Asn Val Gly Ser
   20 25 30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser Asp Lys Gly Gln Gly Ser Gly Val
   50 55 60
   Pro Ser Arg Phe Ser Gly Ser Lys Asp Ala Ser Ala Asn Thr Gly Ile
   65 70 75 80
   Leu Leu Ile Ser Gly Leu Gln Ser Glu Asp Glu Ala Asp Tyr Tyr Cys
   85 90 95
   Met Ile Trp Pro Ser Asn Ala Ser Gly Val Phe Gly Gly Gly Thr Gln
   100 105 110
   Leu Thr Val Leu Gly
   115
<210> 38
   <211> 117
   <212> PRT
   <213> Artificial Sequence
<220>
   <223> Completely synthesized.
<400> 38
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Gln Gly Ser Gly Val Phe Gly Gly Gly Thr Gln
               100                 105                 110
   Leu Thr Val Leu Gly
           115
<210> 39
   <211> 117
   <212> PRT
   <213> Artificial Sequence
<220>
   <223> Completely synthesized.
<400> 39
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Ala Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 40
   <211> 117
   <212> PRT
   <213> Artificial Sequence
<220>
   <223> Completely synthesized.
<400> 40
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
   35 40 45
   Leu Leu Arg Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Glu Ala Asp Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 41
   <211> 117
   <212> PRT
   <213> Artificial Sequence
<220>
   <223> Completely synthesized.
<400> 41
   Gln Ala Val Leu Thr Gln Pro Ala Ser Leu Ser Ala Ser Pro Gly Ala
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 42
   <211> 117
   <212> PRT
   <213> Artificial Sequence
<220>
   <223> Completely synthesized.
<400> 42
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Arg Tyr Arg Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 43
   <211> 115
   <212> PRT
   <213> Artificial Sequence
<220>
   <223> Completely synthesized.
<400> 43
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Ser Thr Asn Ser Gly Ile Leu Leu
   65 70 75 80
   Ile Ser Gly Leu Gln Ser Glu Asp Glu Ala Asp Tyr Tyr Cys Met Thr
   85 90 95
   Trp Ser Ser Asn Gly Ser Gly Val Phe Gly Gly Gly Thr Gln Leu Thr
   100 105 110
   Val Leu Gly
   115
<210> 44
   <211> 117
   <212> PRT
   <213> Artificial Sequence
<220>
   <223> Completely synthesized.
<400> 44
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Gly Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 45
   <211> 117
   <212> PRT
   <213> Artificial Sequence
<220>
   <223> Completely synthesized.
<400> 45
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ala Gly Val Phe Gly Gly Gly Thr Gln
               100                 105                 110
   Leu Thr Val Leu Gly
           115
<210> 46
   <211> 117
   <212> PRT
   <213> Artificial Sequence
<220>
   <223> Completely synthesized.
<400> 46
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Arg Ile Tyr Trp Tyr Gln Gln Lys Pro Gly Ser Pro Pro Arg Tyr
               35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 47
   <211> 117
   <212> PRT
   <213> Artificial Sequence
<220>
   <223> Completely synthesized.
<400> 47
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Arg Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 48
   <211> 117
   <212> PRT
   <213> Artificial Sequence
<220>
   <223> Completely synthesized.
<400> 48
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Thr Gly Ser Gly Val Phe Gly Gly Gly Thr Gln
               100                 105                 110
   Leu Thr Val Leu Gly
           115
<210> 49
   <211> 117
   <212> PRT
   <213> Artificial Sequence
<220>
   <223> Completely synthesized.
<400> 49
   Gln Ala Val Leu Thr Gln Pro Ala Ser Leu Ser Ala Ser Pro Gly Glu
   1               5                   10                  15
   Ser Ala Arg Leu Thr Cys Thr Leu Arg Ser Asp Ile Asn Val Arg Tyr
               20                  25                  30
   Tyr Asn Ile Tyr Trp Tyr Gln Gln Lys Pro Gly Ser Pro Pro Arg Tyr
                   35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 50
   <211> 117
   <212> PRT
   <213> Artificial Sequence
<220>
   <223> Completely synthesized.
<400> 50
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Glu Ala Asp Tyr Tyr Cys
   85 90 95
   Met Thr Trp Ser Ser Asn Pro Ser Gly Val Phe Gly Gly Gly Thr Gln
   100 105 110
   Leu Thr Val Leu Gly
   115
<210> 51
   <211> 117
   <212> PRT
   <213> Artificial Sequence
<220>
   <223> Completely synthesized.
<400> 51
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Ile Thr Trp Ser Ser Asn Gly Ser Gly Val Phe Gly Gly Gly Thr Gln
               100                 105                 110
   Leu Thr Val Leu Gly
           115
<210> 52
   <211> 117
   <212> PRT
   <213> Artificial Sequence
<220>
   <223> Completely synthesized.
<400> 52
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Glu Ala Asp Tyr Tyr Cys
   85 90 95
   Leu Thr Trp Ser Ser Asn Gly Ser Gly Val Phe Gly Gly Gly Thr Gln
   100 105 110
   Leu Thr Val Leu Gly
   115
<210> 53
   <211> 117
   <212> PRT
   <213> Artificial Sequence
<220>
   <223> Completely synthesized.
<400> 53
   Gln Pro Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 54
   <211> 117
   <212> PRT
   <213> Artificial Sequence
<220>
   <223> Completely synthesized.
<400> 54
   Gln Ala Val Leu Thr Gln Pro Pro Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 55
   <211> 117
   <212> PRT
   <213> Artificial Sequence
<220>
   <223> Completely synthesized.
<400> 55
   Gln Ala Val Leu Thr Gln Pro Ala Ser Ser Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Glu Ala Asp Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 56
   <211> 117
   <212> PRT
   <213> Artificial Sequence
<220>
   <223> Completely synthesized.
<400> 56
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 57
   <211> 117
   <212> PRT
   <213> Artificial Sequence
<220>
   <223> Completely synthesized.
<400> 57
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Ser
   20 25 30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Glu Ala Asp Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 58
   <211> 117
   <212> PRT
   <213> Artificial Sequence
<220>
   <223> Completely synthesized.
<400> 58
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
               35                  40                  45
   Leu Leu Tyr Tyr Tyr Ser Asp Ser Asp Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 59
   <211> 117
   <212> PRT
   <213> Artificial Sequence
<220>
   <223> Completely synthesized.
<400> 59
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Ala Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 60
   <211> 117
   <212> PRT
   <213> Artificial Sequence
<220>
   <223> Completely synthesized.
<400> 60
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Ala Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 61
   <211> 117
   <212> PRT
   <213> Artificial Sequence
<220>
   <223> Completely synthesized.
<400> 61
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Thr Gly Ile
   65 70 75 80
   Leu Leu Ile Ser Gly Leu Gln Ser Glu Asp Glu Ala Asp Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 62
   <211> 117
   <212> PRT
   <213> Artificial Sequence
<220>
   <223> Completely synthesized.
<400> 62
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Ile Trp Ser Ser Asn Gly Ser Gly Val Phe Gly Gly Gly Thr Gln
               100                 105                 110
   Leu Thr Val Leu Gly
           115
<210> 63
   <211> 117
   <212> PRT
   <213> Artificial Sequence
<220>
   <223> Completely synthesized.
<400> 63
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Glu Ala Asp Tyr Tyr Cys
                   85                  90                  95
   Met Thr Trp Pro Ser Asn Gly Ser Gly Val Phe Gly Gly Gly Thr Gln
               100                 105                 110
   Leu Thr Val Leu Gly
           115
<210> 64
   <211> 117
   <212> PRT
   <213> Artificial Sequence
<220>
   <223> Completely synthesized.
<400> 64
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Glu Ala Asp Tyr Tyr Cys
   85 90 95
   Met Thr Trp Ser Ser Asn Ala Ser Gly Val Phe Gly Gly Gly Thr Gln
   100 105 110
   Leu Thr Val Leu Gly
   115
<210> 65
   <211> 117
   <212> PRT
   <213> Artificial Sequence
<220>
   <223> Completely synthesized.
<400> 65
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 66
   <211> 117
   <212> PRT
   <213> Artificial Sequence
<220>
   <223> Completely synthesized.
<400> 66
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Ile Ala Thr Tyr Tyr Cys
   85 90 95
   Leu Thr Trp Ser Ser Asn Gly Ser Gly Val Phe Gly Gly Gly Thr Gln
   100 105 110
   Leu Thr Val Leu Gly
   115
<210> 67
   <211> 117
   <212> PRT
   <213> Artificial Sequence
<220>
   <223> Completely synthesized.
<400> 67
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Gln Ser Gly Ile
   65 70 75 80
   Leu Leu Ile Ser Gly Leu Gln Ser Glu Asp Ile Ala Thr Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 68
   <211> 117
   <212> PRT
   <213> Artificial Sequence
<220>
   <223> Completely synthesized.
<400> 68
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Thr Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 69
   <211> 117
   <212> PRT
   <213> Artificial Sequence
<220>
   <223> Completely synthesized.
<400> 69
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Gly Ser Gly Ile
   65 70 75 80
   Leu Leu Ile Ser Gly Leu Gln Ser Glu Asp Ile Ala Thr Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 70
   <211> 117
   <212> PRT
   <213> Artificial Sequence
<220>
   <223> Completely synthesized.
<400> 70
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr His Ser Gly Ile
   65 70 75 80
   Leu Leu Ile Ser Gly Leu Gln Ser Glu Asp Ile Ala Thr Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 71
   <211> 117
   <212> PRT
   <213> Artificial Sequence
<220>
   <223> Completely synthesized.
<400> 71
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Lys Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 72
   <211> 117
   <212> PRT
   <213> Artificial Sequence
<220>
   <223> Completely synthesized.
<400> 72
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Pro Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 73
   <211> 117
   <212> PRT
   <213> Artificial Sequence
<220>
   <223> Completely synthesized.
<400> 73
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
               35                  40                  45
   Glu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 74
   <211> 117
   <212> PRT
   <213> Artificial Sequence
<220>
   <223> Completely synthesized.
<400> 74
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Gly Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 75
   <211> 117
   <212> PRT
   <213> Artificial Sequence
<220>
   <223> Completely synthesized.
<400> 75
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Asn Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Ile Ala Thr Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 76
   <211> 117
   <212> PRT
   <213> Artificial Sequence
<220>
   <223> Completely synthesized.
<400> 76
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Pro Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Ile Ala Thr Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 77
   <211> 117
   <212> PRT
   <213> Homo sapiens
<400> 77
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Ser Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Ile Ala Thr Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 78
   <211> 117
   <212> PRT
   <213> Artificial Sequence
<220>
   <223> Completely synthesized.
<400> 78
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Glu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 79
   <211> 117
   <212> PRT
   <213> Artificial Sequence
<220>
   <223> Completely synthesized.
<400> 79
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Pro Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 80
   <211> 117
   <212> PRT
   <213> Artificial Sequence
<220>
   <223> Completely synthesized.
<400> 80
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Glu Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 81
   <211> 117
   <212> PRT
   <213> Artificial Sequence
<220>
   <223> Completely synthesized.
<400> 81
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Gln Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 82
   <211> 117
   <212> PRT
   <213> Artificial Sequence
<220>
   <223> Completely synthesized.
<400> 82
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Pro Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Ile Ala Thr Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 83
   <211> 117
   <212> PRT
   <213> Artificial Sequence
<220>
   <223> Completely synthesized.
<400> 83
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Leu Leu Tyr Asn Tyr Ser Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Ile Ala Thr Tyr Tyr Cys
   85 90 95
   Met Thr Trp Ser Ser Asn Gly Ser Gly Val Phe Gly Gly Gly Thr Gln
   100 105 110
   Leu Thr Val Leu Gly
   115
<210> 84
   <211> 117
   <212> PRT
   <213> Artificial Sequence
<220>
   <223> Completely synthesized.
<400> 84
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Thr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 85
   <211> 117
   <212> PRT
   <213> Artificial Sequence
<220>
   <223> Completely synthesized.
<400> 85
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Asp Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 86
   <211> 117
   <212> PRT
   <213> Artificial Sequence
<220>
   <223> Completely synthesized.
<400> 86
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Glu Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 87
   <211> 117
   <212> PRT
   <213> Artificial Sequence
<220>
   <223> Completely synthesized.
<400> 87
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr His Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 88
   <211> 117
   <212> PRT
   <213> Artificial Sequence
<220>
   <223> Completely synthesized.
<400> 88
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 89
   <211> 117
   <212> PRT
   <213> Artificial Sequence
<220>
   <223> Completely synthesized.
<400> 89
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser Asn Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 90
   <211> 117
   <212> PRT
   <213> Artificial Sequence
<220>
   <223> Completely synthesized.
<400> 90
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser Pro Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 91
   <211> 117
   <212> PRT
   <213> Artificial Sequence
<220>
   <223> Completely synthesized.
<400> 91
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Asp Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 92
   <211> 117
   <212> PRT
   <213> Artificial Sequence
<220>
   <223> Completely synthesized.
<400> 92
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Gln Gly Ser Gly Val Phe Gly Gly Gly Thr Gln
               100                 105                 110
   Leu Thr Val Leu Gly
           115
<210> 93
   <211> 117
   <212> PRT
   <213> Artificial Sequence
<220>
   <223> Completely synthesized.
<400> 93
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 94
   <211> 117
   <212> PRT
   <213> Artificial Sequence
<220>
   <223> Completely synthesized.
<400> 94
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 95
   <211> 117
   <212> PRT
   <213> Artificial Sequence
<220>
   <223> Completely synthesized.
<400> 95
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 96
   <211> 117
   <212> PRT
   <213> Artificial Sequence
<220>
   <223> Completely synthesized.
<400> 96
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Gln Gly Ser Gly Val Phe Gly Gly Gly Thr Gln
               100                 105                 110
   Leu Thr Val Leu Gly
           115
<210> 97
   <211> 117
   <212> PRT
   <213> Artificial Sequence
<220>
   <223> Completely synthesized.
<400> 97
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Ser
   20 25 30
   Tyr Asn Ile Tyr Trp Tyr Gln Gln Lys Pro Gly Ser Pro Pro Arg Tyr
   35 40 45
   Leu Leu Tyr Tyr Tyr Gln Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Ile Ala Thr Tyr Tyr Cys
   85 90 95
   Leu Thr Trp Ser Ser Gln Gly Ser Gly Val Phe Gly Gly Gly Thr Gln
   100 105 110
   Leu Thr Val Leu Gly
   115
<210> 98
   <211> 117
   <212> PRT
   <213> Artificial Sequence
<220>
   <223> Completely synthesized.
<400> 98
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Glu Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 99
   <211> 117
   <212> PRT
   <213> Synthetic
<400> 99
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Glu Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 100
   <211> 117
   <212> PRT
   <213> Synthetic
<400> 100
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Glu Asp Ser His Lys Gly Gln Gly Ser Gly Val
       50                  55                  60
   Pro Ser Arg Phe Ser Gly Ser Lys Asp Val Ser Thr Asn Ser Gly Ile
   65                  70                  75                  80
   Leu Leu Ile Ser Gly Leu Gln Ser Glu Asp Ile Ala Thr Tyr Tyr Cys
   85 90 95
   Leu Thr Trp Ser Ser Gln Gly Ser Gly Val Phe Gly Gly Gly Thr Gln
   100 105 110
   Leu Thr Val Leu Gly
   115
<210> 101
   <211> 117
   <212> PRT
   <213> Artificial Sequence
<220>
   <223> Completely synthesized.
<400> 101
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Ser
   20 25 30
   Tyr Asn Ile Tyr Trp Tyr Gln Gln Lys Pro Gly Ser Pro Pro Arg Tyr
   35 40 45
   Leu Leu Tyr Tyr Tyr Glu Asp Ser His Lys Gly Gln Gly Ser Gly Val
   50 55 60
   Pro Ser Arg Phe Ser Gly Ser Lys Asp Val Ser Thr Asn Ser Gly Ile
   65 70 75 80
   Leu Leu Ile Ser Gly Leu Gln Ser Glu Asp Ile Ala Thr Tyr Tyr Cys
   85 90 95
   Leu Thr Trp Ser Ser Gln Gly Ser Gly Val Phe Gly Gly Gly Thr Gln
   100 105 110
   Leu Thr Val Leu Gly
   115
<210> 102
   <211> 117
   <212> PRT
   <213> Artificial Sequence
<220>
   <223> Completely synthesized.
<400> 102
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Gln Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 103
   <211> 117
   <212> PRT
   <213> Artificial Sequence
<220>
   <223> Completely synthesized.
<400> 103
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Gln Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 104
   <211> 117
   <212> PRT
   <213> Artificial Sequence
<220>
   <223> Completely synthesized.
<400> 104
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Gln Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Gln Gly Ser Gly Val Phe Gly Gly Gly Thr Gln
               100                 105                 110
   Leu Thr Val Leu Gly
           115
<210> 105
   <211> 117
   <212> PRT
   <213> Artificial Sequence
<220>
   <223> Completely synthesized.
<400> 105
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Gln Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Gln Gly Ser Gly Val Phe Gly Gly Gly Thr Gln
               100                 105                 110
   Leu Thr Val Leu Gly
           115
<210> 106
   <211> 117
   <212> PRT
   <213> Artificial Sequence
<220>
   <223> Completely synthesized.
<400> 106
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Glu Asp Ser His Lys Gly Gln Gly Ser Gly Val
       50                  55                  60
   Pro Ser Arg Phe Ser Gly Ser Lys Asp Val Ser Thr Gln Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 107
   <211> 117
   <212> PRT
   <213> Artificial Sequence
<220>
   <223> Completely synthesized.
<400> 107
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Glu Asp Ser His Lys Gly Gln Gly Ser Gly Val
       50                  55                  60
   Pro Ser Arg Phe Ser Gly Ser Lys Asp Val Ser Thr Gln Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 108
   <211> 117
   <212> PRT
   <213> Artificial Sequence
<220>
   <223> Completely synthesized.
<400> 108
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Glu Asp Ser His Lys Gly Gln Gly Ser Gly Val
       50                  55                  60
   Pro Ser Arg Phe Ser Gly Ser Lys Asp Val Ser Thr Gln Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Gln Gly Ser Gly Val Phe Gly Gly Gly Thr Gln
               100                 105                 110
   Leu Thr Val Leu Gly
           115
<210> 109
   <211> 117
   <212> PRT
   <213> Artificial Sequence
<220>
   <223> Completely synthesized.
<400> 109
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Glu Asp Ser His Lys Gly Gln Gly Ser Gly Val
       50                  55                  60
   Pro Ser Arg Phe Ser Gly Ser Lys Asp Val Ser Thr Gln Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Gln Gly Ser Gly Val Phe Gly Gly Gly Thr Gln
               100                 105                 110
   Leu Thr Val Leu Gly
           115
<210> 110
   <211> 122
   <212> PRT
   <213> Homo sapiens
<400> 110
   Glu Val Gln Leu Val Gln Ser Gly Ala Glu Val Lys Lys Ser Gly Ala
   1 5 10 15
   Ser Val Lys Val Ser Cys Lys Ala Ser Gly Tyr Thr Phe Thr Asp Tyr
   20 25 30
   Tyr Ile His Trp Val Arg Gln Ala Pro Gly Gln Gly Leu Glu Trp Met
   35 40 45
   Gly Trp Ile Asn Pro Asn Asn Gly Gly Val Thr Phe Ala Gln Lys Phe
   50 55 60
   Gln Gly Arg Val Thr Met Thr Arg Asp Thr Ser Ile Ser Thr Ala Tyr
   65 70 75 80
   Met Asp Leu Ser Ser Leu Arg Ser Asp Asp Thr Ala Val Tyr Phe Cys
   85 90 95
   Ala Arg Asp Ile Arg Met Ser Gly Trp Leu Ala Pro Phe Asp Tyr Trp
   100 105 110
   Gly Gln Gly Thr Leu Val Thr Val Ser Ser
   115 120
<210> 111
   <211> 120
   <212> **PRT**
   <213> Homo sapiens
<400> 111
   Gln Val Gln Leu Val Glu Ser Gly Gly Gly Val Val Gln Gly Gly Gly
   1 5 10 15
   Ser Leu Arg Leu Ser Cys Ala Ala Ser Gly Phe Thr Val Arg Ser Asn
   20 25 30
   Tyr Met Ser Trp Val Arg Gln Ala Pro Gly Lys Gly Leu Glu Trp Val
   35 40 45
   Ser Ala Ile Ser Gly Ser Gly Asp Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Val Ala Val Thr Thr Gly Trp Tyr Phe Asp Leu Trp Gly Arg
   100 105 110
   Gly Thr Leu Val Thr Val Ser Ser
   115 120
<210> 112
   <211> 117
   <212> PRT
   <213> Homo sapiens
<400> 112
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Arg Tyr
   20 25 30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Asp Gln Gly Ser Gly Val
   50 55 60
   Pro Ser Arg Phe Ser Gly Ser Lys Asp Thr Ser Ala Asn Thr Gly Ile
   65 70 75 80
   Leu Leu Ile Ser Gly Leu Gln Ser Glu Asp Glu Ala Asp Tyr Tyr Cys
   85 90 95
   Met Ile Trp Ala Ser Asn Gly Ser Gly Val Leu Gly Gly Gly Thr Gln
   100 105 110
   Leu Thr Val Leu Gly
   115
<210> 113
   <211> 117
   <212> PRT
   <213> Homo sapiens
<400> 113
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Arg Tyr
               20                  25                  30
   His Asn Ile Tyr Trp Tyr Gln Glu Lys Pro Gly Ser Pro Pro Arg Tyr
           35                  40                  45
   Leu Leu Tyr Tyr Tyr Ser Asp Ser Ser Lys Gly Gln Gly Ser Gly Val
       50                  55                  60
   Pro Ser Arg Phe Ser Gly Ser Lys Asp Val Ser Thr Asn Thr Gly Ile
   65                  70                  75                  80
   Leu Val Ile Ser Gly Leu Gln Ser Glu Asp Glu Ala Glu Tyr Tyr Cys
                   85                  90                  95
   Met Thr Trp Ser Ser Asn Gly Ser Gly Val Phe Gly Gly Gly Thr Gln
               100                 105                 110
   Leu Thr Val Leu Gly
           115
<210> 114
   <211> 120
   <212> PRT
   <213> Rattus norvegicus
<400> 114
   Glu Val Gln Leu Gln Gln Ser Gly Pro Glu Val Gly Arg Pro Gly Ser
   1               5                   10                  15
   Ser Val Lys Ile Ser Cys Lys Ala Ser Gly Tyr Thr Phe Thr Asp Tyr
               20                  25                  30
   Ile Met His Trp Val Lys Gln Ser Pro Gly Gln Gly Leu Glu Trp Ile
           35                  40                  45
   Gly Trp Ile Asp Pro Glu Tyr Gly Arg Thr Asp Tyr Ala Glu Lys Phe
       50                  55                  60
   Lys Lys Lys Ala Thr Leu Thr Ala Asp Thr Ser Ser Ser Thr Ala Tyr
   65                  70                  75                  80
   Ile Gln Leu Ser Ser Leu Ala Ser Glu Asp Thr Ala Thr Tyr Phe Cys
                   85                  90                  95
   Ala Arg Ser Ala His Thr Thr Gly Phe Gly Phe Ala Tyr Trp Gly Gln
               100                 105                 110
   Gly Thr Leu Val Thr Val Ser Ser
           115                 120
<210> 115
   <211> 120
   <212> PRT
   <213> Artificial Sequence
<220>
   <223> Completely synthesized.
<400> 115
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Tyr
               20                  25                  30
   Ile Met His Trp Val Gln Gln Ala Pro Gly Lys Gly Leu Glu Trp Met
           35                  40                  45
   Gly Trp Ile Asp Pro Glu Tyr Gly Arg Thr Asp Tyr Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Thr Ser Ala His Thr Thr Gly Phe Gly Phe Ala Tyr Trp Gly Gln
               100                 105                 110
   Gly Thr Leu Val Thr Val Ser Ser
           115                 120
<210> 116
   <211> 120
   <212> PRT
   <213> Artificial Sequence
<220>
   <223> Completely synthesized.
<400> 116
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Tyr
               20                  25                  30
   Ile Met His Trp Val Gln Gln Ala Pro Gly Lys Gly Leu Glu Trp Met
           35                  40                  45
   Gly Trp Ile Asp Pro Glu Tyr Gly Arg Thr Asp Tyr Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Ala His Thr Thr Gly Phe Gly Phe Ala Tyr Trp Gly Gln
               100                 105                 110
   Gly Thr Leu Val Thr Val Ser Ser
           115                 120
<210> 117
   <211> 120
   <212> PRT
   <213> Artificial Sequence
<220>
   <223> Completely synthesized.
<400> 117
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Ile Met His Trp Val Arg Gln Ala Pro Gly Gln Gly Leu Glu Trp Met
   35 40 45
   Gly Trp Ile Asp Pro Glu Tyr Gly Arg Thr Asp Tyr Ala Gln Lys Leu
   50 55 60
   Gln Gly Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Ser Ala His Thr Thr Gly Phe Gly Phe Ala Tyr Trp Gly Gln
   100 105 110
   Gly Thr Leu Val Thr Val Ser Ser
   115 120
<210> 118
   <211> 120
   <212> PRT
   <213> Artificial Sequence
<220>
   <223> Completely synthesized.
<400> 118
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Ile Met His Trp Val Arg Gln Ala Pro Gly Gln Gly Leu Glu Trp Met
           35                  40                  45
   Gly Trp Ile Asp Pro Glu Tyr Gly Arg Thr Asp Tyr Ala Gln Lys Leu
       50                  55                  60
   Gln Gly Arg Val Thr Met Thr Ala Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Ser Ala His Thr Thr Gly Phe Gly Phe Ala Tyr Trp Gly Gln
               100                 105                 110
   Gly Thr Leu Val Thr Val Ser Ser
           115                 120
<210> 119
   <211> 120
   <212> PRT
   <213> Artificial Sequence
<220>
   <223> Completely synthesized.
<400> 119
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr Asp Tyr
   20 25 30
   Ile Met His Trp Val Arg Gln Ala Pro Gly Lys Gly Leu Glu Trp Met
   35 40 45
   Gly Trp Ile Asp Pro Glu Tyr Gly Arg Thr Asp Tyr Ala Gln Lys Phe
   50 55 60
   Gln Gly Arg Val Thr Met Thr Glu Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Thr Ser Ala His Thr Thr Gly Phe Gly Phe Ala Tyr Trp Gly Gln
   100 105 110
   Gly Thr Leu Val Thr Val Ser Ser
   115 120
<210> 120
   <211> 120
   <212> PRT
   <213> Artificial Sequence
<220>
   <223> Completely synthesized.
<400> 120
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr Asp Tyr
   20 25 30
   Ile Met His Trp Val Arg Gln Ala Pro Gly Lys Gly Leu Glu Trp Met
   35 40 45
   Gly Trp Ile Asp Pro Glu Tyr Gly Arg Thr Asp Tyr Ala Gln Lys Phe
   50 55 60
   Gln Gly Arg Val Thr Met Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Thr Ser Ala His Thr Thr Gly Phe Gly Phe Ala Tyr Trp Gly Gln
   100 105 110
   Gly Thr Leu Val Thr Val Ser Ser
   115 120
<210> 121
   <211> 120
   <212> PRT
   <213> Artificial Sequence
<220>
   <223> Completely synthesized.
<400> 121
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Phe Thr Asp Tyr
   20 25 30
   Ile Met His Trp Val Arg Gln Ala Pro Gly Lys Gly Leu Glu Trp Met
   35 40 45
   Gly Trp Ile Asp Pro Glu Tyr Gly Arg Thr Asp Tyr Ala Gln Lys Phe
   50 55 60
   Gln Gly Arg Val Thr Met Thr Glu Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Thr Ser Ala His Thr Thr Gly Phe Gly Phe Ala Tyr Trp Gly Gln
   100 105 110
   Gly Thr Leu Val Thr Val Ser Ser
   115 120
<210> 122
   <211> 120
   <212> PRT
   <213> Artificial Sequence
<220>
   <223> Completely synthesized.
<400> 122
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr Asp Tyr
               20                  25                  30
   Ile Met His Trp Val Arg Gln Ala Pro Gly Lys Gly Leu Glu Trp Met
           35                  40                  45
   Gly Trp Ile Asp Pro Glu Tyr Gly Arg Thr Asp Tyr Ala Gln Lys Phe
       50                  55                  60
   Gln Gly Arg Val Thr Met Thr Glu Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Ala His Thr Thr Gly Phe Gly Phe Ala Tyr Trp Gly Gln
               100                 105                 110
   Gly Thr Leu Val Thr Val Ser Ser
           115                 120
<210> 123
   <211> 120
   <212> PRT
   <213> Artificial Sequence
<220>
   <223> Completely synthesized.
<400> 123
   Gln Met Gln Leu Val Gln Ser Gly Ala Glu Val Lys Lys Thr Gly Ser
   1 5 10 15
   Ser Val Lys Val Ser Cys Lys Ala Ser Gly Tyr Thr Phe Thr Asp Tyr
   20 25 30
   Ile Met His Trp Val Arg Gln Ala Pro Gly Gln Ala Leu Glu Trp Met
   35 40 45
   Gly Trp Ile Asp Pro Glu Tyr Gly Arg Thr Asp Tyr Ala Gln Lys Phe
   50 55 60
   Gln Asp Arg Val Thr Ile Thr Arg Asp Arg Ser Met Ser Thr Ala Tyr
   65 70 75 80
   Met Glu Leu Ser Ser Leu Arg Ser Glu Asp Thr Ala Met Tyr Tyr Cys
   85 90 95
   Ala Arg Ser Ala His Thr Thr Gly Phe Gly Phe Ala Tyr Trp Gly Gln
   100 105 110
   Gly Thr Leu Val Thr Val Ser Ser
   115 120
<210> 124
   <211> 120
   <212> PRT
   <213> Artificial Sequence
<220>
   <223> Completely synthesized.
<400> 124
   Gln Met Gln Leu Val Gln Ser Gly Ala Glu Val Lys Lys Thr Gly Ser
   1               5                   10                  15
   Ser Val Lys Val Ser Cys Lys Ala Ser Gly Tyr Thr Phe Thr Asp Tyr
               20                  25                  30
   Ile Met His Trp Val Arg Gln Ala Pro Gly Gln Ala Leu Glu Trp Met
           35                  40                  45
   Gly Trp Ile Asp Pro Glu Tyr Gly Arg Thr Asp Tyr Ala Gln Lys Phe
       50                  55                  60
   Gln Asp Arg Val Thr Ile Thr Ala Asp Arg Ser Met Ser Thr Ala Tyr
   65                  70                  75                  80
   Met Glu Leu Ser Ser Leu Arg Ser Glu Asp Thr Ala Met Tyr Tyr Cys
                   85                  90                  95
   Ala Arg Ser Ala His Thr Thr Gly Phe Gly Phe Ala Tyr Trp Gly Gln
               100                 105                 110
   Gly Thr Leu Val Thr Val Ser Ser
           115                 120
<210> 125
   <211> 108
   <212> PRT
   <213> Rattus norvegicus
<400> 125
   Asp Ile Gln Met Thr Gln Ser Pro Ala Ser Leu Ser Ala Ser Leu Gly
   1               5                   10                  15
   Glu Thr Val Thr Ile Glu Cys Arg Ala Ser Glu Asp Ile Tyr Ser Asn
               20                  25                  30
   Leu Ala Trp Tyr Gln Gln Lys Pro Gly Asn Ser Pro Gln Leu Leu Ile
           35                  40                  45
   Tyr Asp Ala Asn Ser Leu Ala Asp Gly Val Pro Ser Arg Phe Ser Ala
       50                  55                  60
   Ser Gly Ser Gly Thr Gln Phe Ser Leu Lys Ile Asn Ser Leu Arg Ser
   65                  70                  75                  80
   Glu Asp Val Ala Ser Tyr Phe Cys Gln Gln Tyr Asn Asn Tyr Pro Tyr
                   85                  90                  95
   Thr Phe Gly Ala Gly Thr Lys Leu Glu Leu Lys Arg
               100                 105
<210> 126
   <211> 108
   <212> PRT
   <213> Artificial Sequence
<220>
   <223> Completely synthesized.
<400> 126
   Asp Ile Gln Met Thr Gln Ser Pro Ser Thr Leu Ser Ala Ser Val Gly
   1               5                   10                  15
   Asp Arg Val Thr Ile Thr Cys Arg Ala Ser Glu Asp Ile Tyr Ser Asn
               20                  25                  30
   Leu Ala Trp Tyr Gln Gln Lys Pro Gly Lys Ala Pro Lys Leu Leu Ile
           35                  40                  45
   Tyr Asp Ala Asn Ser Leu Ala Asp Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Asn Tyr Pro Tyr
   85 90 95
   Thr Phe Gly Gln Gly Thr Lys Leu Glu Ile Lys Arg
   100 105
<210> 127
   <211> 108
   <212> PRT
   <213> Artificial Sequence
<220>
   <223> Completely synthesized.
<400> 127
   Asp Ile Gln Leu Thr Gln Ser Pro Ser Phe Leu Ser Ala Ser Val Gly
   1 5 10 15
   Asp Arg Val Thr Ile Thr Cys Arg Ala Ser Glu Asp Ile Tyr Ser Asn
   20 25 30
   Leu Ala Trp Tyr Gln Gln Lys Pro Gly Lys Ala Pro Lys Leu Leu Ile
   35 40 45
   Tyr Asp Ala Asn Ser Leu Ala Asp Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Glu Asp Phe Ala Thr Tyr Tyr Cys Gln Gln Tyr Asn Asn Tyr Pro Tyr
   85 90 95
   Thr Phe Gly Gln Gly Thr Lys Leu Glu Ile Lys Arg
   100 105
<210> 128
   <211> 108
   <212> PRT
   <213> Artificial Sequence
<220>
   <223> Completely synthesized.
<400> 128
   Asp Ile Gln Met Thr Gln Ser Pro Ser Ser Val Ser Ala Ser Val Gly
   1               5                   10                  15
   Asp Arg Val Thr Ile Thr Cys Arg Ala Ser Glu Asp Ile Tyr Ser Asn
               20                  25                  30
   Leu Ala Trp Tyr Gln Gln Lys Pro Gly Lys Ala Pro Lys Leu Leu Ile
           35                  40                  45
   Tyr Asp Ala Asn Ser Leu Ala Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Asn Tyr Pro Tyr
                   85                  90                  95
   Thr Phe Gly Gln Gly Thr Lys Leu Glu Ile Lys Arg
               100                 105
<210> 129
   <211> 108
   <212> PRT
   <213> Artificial Sequence
<220>
   <223> Completely synthesized.
<400> 129
   Ala Ile Gln Leu Thr Gln Ser Pro Ser Ser Leu Ser Ala Ser Val Gly
   1               5                   10                  15
   Asp Arg Val Thr Ile Thr Cys Arg Ala Ser Glu Asp Ile Tyr Ser Asn
               20                  25                  30
   Leu Ala Trp Tyr Gln Gln Lys Pro Gly Lys Ala Pro Lys Leu Leu Ile
           35                  40                  45
   Tyr Asp Ala Asn Ser Leu Ala Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Asn Tyr Pro Tyr
                   85                  90                  95
   Thr Phe Gly Gln Gly Thr Lys Leu Glu Ile Lys Arg
               100                 105
<210> 130
   <211> 108
   <212> PRT
   <213> Artificial Sequence
<220>
   <223> Completely synthesized.
<400> 130
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Glu Asp Ile Tyr Ser Asn
               20                  25                  30
   Leu Ala Trp Tyr Gln Gln Lys Pro Glu Lys Ala Pro Lys Ser Leu Ile
           35                  40                  45
   Tyr Asp Ala Asn Ser Leu Ala Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Asn Tyr Pro Tyr
                   85                  90                  95
   Thr Phe Gly Gln Gly Thr Lys Leu Glu Ile Lys Arg
               100                 105
<210> 131
   <211> 108
   <212> PRT
   <213> Artificial Sequence
<220>
   <223> Completely synthesized.
<400> 131
   Glu Ile Val Met Thr Gln Ser Pro Ala Thr Leu Ser Val Ser Pro Gly
   1               5                   10                  15
   Glu Arg Ala Thr Leu Ser Cys Arg Ala Ser Glu Asp Ile Tyr Ser Asn
               20                  25                  30
   Leu Ala Trp Tyr Gln Gln Lys Pro Gly Gln Ala Pro Arg Leu Leu Ile
           35                  40                  45
   Tyr Asp Ala Asn Ser Leu Ala Asp Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Tyr Pro Tyr
   85 90 95
   Thr Phe Gly Gln Gly Thr Lys Leu Glu Ile Lys Arg
   100 105
<210> 132
   <211> 123
   <212> PRT
   <213> Rattus norvegicus
<400> 132
   Glu Val Gln Leu Gln Gln Ser Gly Pro Glu Val Gly Arg Pro Gly Ser
   1 5 10 15
   Ser Val Lys Ile Ser Cys Lys Ala Ser Gly Tyr Ile Phe Thr Asp Tyr
   20 25 30
   Ile Met His Trp Val Lys Gln Ser Pro Gly Gln Gly Leu Glu Trp Ile
   35 40 45
   Gly Trp Ile Asp Pro Glu Tyr Gly Ser Thr Asp Tyr Ala Glu Lys Phe
   50 55 60
   Lys Lys Lys Ala Thr Leu Thr Ala Asp Thr Ser Ser Ser Thr Ala Tyr
   65 70 75 80
   Ile Gln Leu Ser Ser Leu Thr Ser Glu Asp Thr Ala Thr Tyr Phe Cys
   85 90 95
   Ala Arg Val Ala Ile Ile Thr Thr Val Ala Ser Gly Gly Phe Ala Tyr
   100 105 110
   Trp Gly Gln Gly Thr Leu Val Thr Val Ser Ser
   115 120
<210> 133
   <211> 123
   <212> PRT
   <213> Artificial Sequence
<220>
   <223> Completely synthesized.
<400> 133
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Tyr
   20 25 30
   Ile Met His Trp Val Gln Gln Ala Pro Gly Lys Gly Leu Glu Trp Met
   35 40 45
   Gly Trp Ile Asp Pro Glu Tyr Gly Ser Thr Asp Tyr Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Thr Val Ala Ile Ile Thr Thr Val Ala Ser Gly Gly Phe Ala Tyr
   100 105 110
   Trp Gly Gln Gly Thr Leu Val Thr Val Ser Ser
   115 120
<210> 134
   <211> 123
   <212> PRT
   <213> Artificial Sequence
<220>
   <223> Completely synthesized.
<400> 134
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Ile Phe Thr Asp Tyr
               20                  25                  30
   Ile Met His Trp Val Gln Gln Ala Pro Gly Lys Gly Leu Glu Trp Met
           35                  40                  45
   Gly Trp Ile Asp Pro Glu Tyr Gly Ser Thr Asp Tyr Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Thr Val Ala Ile Ile Thr Thr Val Ala Ser Gly Gly Phe Ala Tyr
                   100                 105                 110
   Trp Gly Gln Gly Thr Leu Val Thr Val Ser Ser
           115                 120
<210> 135
   <211> 123
   <212> PRT
   <213> Artificial Sequence
<220>
   <223> Completely synthesized.
<400> 135
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Tyr
   20 25 30
   Ile Met His Trp Val Gln Gln Ala Pro Gly Lys Gly Leu Glu Trp Met
   35 40 45
   Gly Trp Ile Asp Pro Glu Tyr Gly Ser Thr Asp Tyr Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Val Ala Ile Ile Thr Thr Val Ala Ser Gly Gly Phe Ala Tyr
   100 105 110
   Trp Gly Gln Gly Thr Leu Val Thr Val Ser Ser
   115 120
<210> 136
   <211> 123
   <212> PRT
   <213> Artificial Sequence
<220>
   <223> Completely synthesized.
<400> 136
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Ile Met His Trp Val Arg Gln Ala Pro Gly Gln Gly Leu Glu Trp Met
   35 40 45
   Gly Trp Ile Asp Pro Glu Tyr Gly Ser Thr Asp Tyr Ala Gln Lys Leu
   50 55 60
   Gln Gly Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Val Ala Ile Ile Thr Thr Val Ala Ser Gly Gly Phe Ala Tyr
   100 105 110
   Trp Gly Gln Gly Thr Leu Val Thr Val Ser Ser
   115 120
<210> 137
   <211> 123
   <212> PRT
   <213> Artificial Sequence
<220>
   <223> Completely synthesized.
<400> 137
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Ile Met His Trp Val Arg Gln Ala Pro Gly Gln Gly Leu Glu Trp Met
           35                  40                  45
   Gly Trp Ile Asp Pro Glu Tyr Gly Ser Thr Asp Tyr Ala Gln Lys Leu
       50                  55                  60
   Gln Gly Arg Val Thr Met Thr Ala Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Val Ala Ile Ile Thr Thr Val Ala Ser Gly Gly Phe Ala Tyr
                   100                 105                 110
   Trp Gly Gln Gly Thr Leu Val Thr Val Ser Ser
           115                 120
<210> 138
   <211> 123
   <212> PRT
   <213> Artificial Sequence
<220>
   <223> Completely synthesized.
<400> 138
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr Asp Tyr
   20 25 30
   Ile Met His Trp Val Arg Gln Ala Pro Gly Lys Gly Leu Glu Trp Met
   35 40 45
   Gly Trp Ile Asp Pro Glu Tyr Gly Ser Thr Asp Tyr Ala Gln Lys Phe
   50 55 60
   Gln Gly Arg Val Thr Met Thr Glu Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Thr Val Ala Ile Ile Thr Thr Val Ala Ser Gly Gly Phe Ala Tyr
   100 105 110
   Trp Gly Gln Gly Thr Leu Val Thr Val Ser Ser
   115 120
<210> 139
   <211> 123
   <212> PRT
   <213> Artificial Sequence
<220>
   <223> Completely synthesized.
<400> 139
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr Asp Tyr
               20                  25                  30
   Ile Met His Trp Val Arg Gln Ala Pro Gly Lys Gly Leu Glu Trp Met
           35                  40                  45
   Gly Trp Ile Asp Pro Glu Tyr Gly Ser Thr Asp Tyr Ala Gln Lys Phe
       50                  55                  60
   Gln Gly Arg Val Thr Met Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Thr Val Ala Ile Ile Thr Thr Val Ala Ser Gly Gly Phe Ala Tyr
               100                 105                 110
   Trp Gly Gln Gly Thr Leu Val Thr Val Ser Ser
           115                 120
<210> 140
   <211> 123
   <212> PRT
   <213> Artificial Sequence
<220>
   <223> Completely synthesized.
<400> 140
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr Asp Tyr
               20                  25                  30
   Ile Met His Trp Val Arg Gln Ala Pro Gly Lys Gly Leu Glu Trp Met
           35                  40                  45
   Gly Trp Ile Asp Pro Glu Tyr Gly Ser Thr Asp Tyr Ala Gln Lys Phe
       50                  55                  60
   Gln Gly Arg Val Thr Met Thr Glu Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Val Ala Ile Ile Thr Thr Val Ala Ser Gly Gly Phe Ala Tyr
                   100                 105                 110
   Trp Gly Gln Gly Thr Leu Val Thr Val Ser Ser
           115                 120
<210> 141
   <211> 123
   <212> PRT
   <213> Artificial Sequence
<220>
   <223> Completely synthesized.
<400> 141
   Gln Met Gln Leu Val Gln Ser Gly Ala Glu Val Lys Lys Thr Gly Ser
   1               5                   10                  15
   Ser Val Lys Val Ser Cys Lys Ala Ser Gly Tyr Thr Phe Thr Asp Tyr
               20                  25                  30
   Ile Met His Trp Val Arg Gln Ala Pro Gly Gln Ala Leu Glu Trp Met
           35                  40                  45
   Gly Trp Ile Asp Pro Glu Tyr Gly Ser Thr Asp Tyr Ala Gln Lys Phe
       50                  55                  60
   Gln Asp Arg Val Thr Ile Thr Arg Asp Arg Ser Met Ser Thr Ala Tyr
   65                  70                  75                  80
   Met Glu Leu Ser Ser Leu Arg Ser Glu Asp Thr Ala Met Tyr Tyr Cys
                   85                  90                  95
   Ala Arg Val Ala Ile Ile Thr Thr Val Ala Ser Gly Gly Phe Ala Tyr
               100                 105                 110
   Trp Gly Gln Gly Thr Leu Val Thr Val Ser Ser
           115                 120
<210> 142
   <211> 123
   <212> PRT
   <213> Artificial Sequence
<220>
   <223> Completely synthesized.
<400> 142
   Gln Met Gln Leu Val Gln Ser Gly Ala Glu Val Lys Lys Thr Gly Ser
   1               5                   10                  15
   Ser Val Lys Val Ser Cys Lys Ala Ser Gly Tyr Thr Phe Thr Asp Tyr
               20                  25                  30
   Ile Met His Trp Val Arg Gln Ala Pro Gly Gln Ala Leu Glu Trp Met
           35                  40                  45
   Gly Trp Ile Asp Pro Glu Tyr Gly Ser Thr Asp Tyr Ala Gln Lys Phe
       50                  55                  60
   Gln Asp Arg Val Thr Ile Thr Ala Asp Arg Ser Met Ser Thr Ala Tyr
   65                  70                  75                  80
   Met Glu Leu Ser Ser Leu Arg Ser Glu Asp Thr Ala Met Tyr Tyr Cys
                   85                  90                  95
   Ala Arg Val Ala Ile Ile Thr Thr Val Ala Ser Gly Gly Phe Ala Tyr
               100                 105                 110
   Trp Gly Gln Gly Thr Leu Val Thr Val Ser Ser
           115                 120
<210> 143
   <211> 113
   <212> PRT
   <213> Rattus norvegicus
<400> 143
   Asp Ile Val Met Thr Gln Gly Ala Leu Pro Asn Pro Val Pro Ser Gly
   1               5                   10                  15
   Glu Ser Ala Ser Ile Thr Cys Gln Ser Ser Glu Ser Leu Leu His Ser
               20                  25                  30
   Asn Gly Lys Thr Tyr Leu Asn Trp Tyr Leu Gln Arg Pro Gly Gln Ser
           35                  40                  45
   Pro Gln Leu Leu Ile Tyr Trp Met Ser Thr Arg Ala Ala Gly Val Ser
       50                  55                  60
   Asp Arg Phe Ser Gly Ser Gly Ser Gly Thr Asp Phe Thr Leu Thr Ile
   65                  70                  75                  80
   Ser Gly Val Glu Ala Glu Asp Val Gly Val Tyr Tyr Cys Gln Gln Phe
                   85                  90                  95
   Leu Glu Tyr Pro Pro Thr Phe Gly Ser Gly Thr Lys Leu Glu Ile Lys
               100                 105                 110
   Arg
<210> 144
   <211> 113
   <212> PRT
   <213> Artificial Sequence
<220>
   <223> Completely synthesized.
<400> 144
   Asp Ile Val Met Thr Gln Thr Pro Leu Ser Ser Pro Val Thr Leu Gly
   1               5                   10                  15
   Gln Pro Ala Ser Ile Ser Cys Gln Ser Ser Glu Ser Leu Leu His Ser
               20                  25                  30
   Asn Gly Lys Thr Tyr Leu Asn Trp Leu Gln Gln Arg Pro Gly Gln Pro
           35                  40                  45
   Pro Arg Leu Leu Ile Tyr Trp Met Ser Thr Arg Ala Ala Gly Val Pro
       50                  55                  60
   Asp Arg Phe Ser Gly Ser Gly Ala Gly Thr Asp Phe Thr Leu Lys Ile
   65                  70                  75                  80
   Ser Arg Val Glu Ala Glu Asp Val Gly Val Tyr Tyr Cys Gln Gln Phe
                   85                  90                  95
   Leu Glu Tyr Pro Pro Thr Phe Gly Gln Gly Thr Lys Leu Glu Ile Lys
               100                 105                 110
   Arg
<210> 145
   <211> 113
   <212> PRT
   <213> Artificial Sequence
<220>
   <223> Completely synthesized.
<400> 145
   Asp Ile Val Met Thr Gln Ser Pro Leu Ser Leu Pro Val Thr Pro Gly
   1               5                   10                  15
   Glu Pro Ala Ser Ile Ser Cys Gln Ser Ser Glu Ser Leu Leu His Ser
               20                  25                  30
   Asn Gly Lys Thr Tyr Leu Asn Trp Tyr Leu Gln Lys Pro Gly Gln Ser
           35                  40                  45
   Pro Gln Leu Leu Ile Tyr Trp Met Ser Thr Arg Ala Ala Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Gln Gln Phe
                   85                  90                  95
   Leu Glu Tyr Pro Pro Thr Phe Gly Gln Gly Thr Lys Leu Glu Ile Lys
               100                 105                 110
   Arg
<210> 146
   <211> 113
   <212> **PRT**
   <213> **Artificial** Sequence
<220>
   <223> Completely synthesized.
<400> 146
   Asp Ile Val Met Thr Gln Thr Pro Leu Ser Leu Ser Val Thr Pro Gly
   1               5                   10                  15
   Gln Pro Ala Ser Ile Ser Cys Gln Ser Ser Glu Ser Leu Leu His Ser
               20                  25                  30
   Asn Gly Lys Thr Tyr Leu Asn Trp Tyr Leu Gln Lys Pro Gly Gln Pro
           35                  40                  45
   Pro Gln Leu Leu Ile Tyr Trp Met Ser Thr Arg Ala Ala Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Gln Gln Phe
                   85                  90                  95
   Leu Glu Tyr Pro Pro Thr Phe Gly Gln Gly Thr Lys Leu Glu Ile Lys
               100                 105                 110
   Arg
<210> 147
   <211> 113
   <212> PRT
   <213> Artificial Sequence
<220>
   <223> Completely synthesized.
<400> 147
   Asp Val Val Met Thr Gln Ser Pro Leu Ser Leu Pro Val Thr Leu Gly
   1               5                   10                  15
   Gln Pro Ala Ser Ile Ser Cys Gln Ser Ser Glu Ser Leu Leu His Ser
               20                  25                  30
   Asn Gly Lys Thr Tyr Leu Asn Trp Phe Gln Gln Arg Pro Gly Gln Ser
           35                  40                  45
   Pro Arg Arg Leu Ile Tyr Trp Met Ser Thr Arg Ala Ala Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Gln Gln Phe
                   85                  90                  95
   Leu Glu Tyr Pro Pro Thr Phe Gly Gln Gly Thr Lys Leu Glu Ile Lys
               100                 105                 110
   Arg
<210> 148
   <211> 120
   <212> PRT
   <213> Rattus norvegicus
<400> 148
   Glu Val Gln Leu Gln Gln Ser Gly Pro Glu Val Gly Arg Pro Gly Ser
   1               5                   10                  15
   Ser Val Lys Ile Ser Cys Lys Ala Ser Gly Tyr Thr Phe Thr Asp Ser
               20                  25                  30
   Val Met Asn Trp Val Lys Gln Ser Pro Gly Gln Gly Leu Glu Trp Ile
           35                  40                  45
   Gly Trp Ile Asp Pro Glu Tyr Gly Arg Thr Asp Val Ala Glu Lys Phe
   50 55 60
   Lys Lys Lys Ala Thr Leu Thr Ala Asp Ser Ser Ser Ser Thr Ala Tyr
   65 70 75 80
   Ile Tyr Leu Ser Gly Leu Thr Ser Glu Asp Thr Ala Thr Tyr Phe Cys
   85 90 95
   Ala Arg Thr Lys Tyr Asn Ser Gly Tyr Gly Phe Pro Tyr Trp Gly Gln
   100 105 110
   Gly Ser Leu Val Thr Val Ser Ser
   115 120
<210> 149
   <211> 120
   <212> PRT
   <213> Artificial Sequence
<220>
   <223> Completely synthesized.
<400> 149
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr Asp Ser
   20 25 30
   Val Met Asn Trp Val Arg Gln Ala Pro Gly Lys Gly Leu Glu Trp Met
   35 40 45
   Gly Trp Ile Asp Pro Glu Tyr Gly Arg Thr Asp Val Ala Gln Lys Phe
   50 55 60
   Gln Gly Arg Val Thr Met Thr Glu Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Thr Thr Lys Tyr Asn Ser Gly Tyr Gly Phe Pro Tyr Trp Gly Gln
   100 105 110
   Gly Thr Thr Val Thr Val Ser Ser
   115 120
<210> 150
   <211> 120
   <212> PRT
   <213> Artificial Sequence
<220>
   <223> Completely synthesized.
<400> 150
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr Asp Ser
               20                  25                  30
   Val Met Asn Trp Val Arg Gln Ala Pro Gly Lys Gly Leu Glu Trp Met
           35                  40                  45
   Gly Trp Ile Asp Pro Glu Tyr Gly Arg Thr Asp Val Ala Gln Lys Phe
       50                  55                  60
   Gln Gly Arg Val Thr Met Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Thr Thr Lys Tyr Asn Ser Gly Tyr Gly Phe Pro Tyr Trp Gly Gln
               100                 105                 110
   Gly Thr Thr Val Thr Val Ser Ser
           115                 120
<210> 151
   <211> 120
   <212> PRT
   <213> Artificial Sequence
<220>
   <223> Completely synthesized.
<400> 151
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr Asp Ser
               20                  25                  30
   Val Met Asn Trp Val Arg Gln Ala Pro Gly Lys Gly Leu Glu Trp Met
           35                  40                  45
   Gly Trp Ile Asp Pro Glu Tyr Gly Arg Thr Asp Val Ala Gln Lys Phe
       50                  55                  60
   Gln Gly Arg Val Thr Met Thr Glu Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Lys Tyr Asn Ser Gly Tyr Gly Phe Pro Tyr Trp Gly Gln
               100                 105                 110
   Gly Thr Thr Val Thr Val Ser Ser
           115                 120
<210> 152
   <211> 120
   <212> PRT
   <213> Artificial Sequence
<220>
   <223> Completely synthesized.
<400> 152
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr Asp Ser
               20                  25                  30
   Val Met Asn Trp Val Arg Gln Ala Pro Gly Lys Gly Leu Glu Trp Met
           35                  40                  45
   Gly Trp Ile Asp Pro Glu Tyr Gly Arg Thr Asp Val Ala Gln Lys Phe
       50                  55                  60
   Gln Gly Arg Val Thr Met Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Lys Tyr Asn Ser Gly Tyr Gly Phe Pro Tyr Trp Gly Gln
               100                 105                 110
   Gly Thr Thr Val Thr Val Ser Ser
           115                 120
<210> 153
   <211> 120
   <212> PRT
   <213> Artificial Sequence
<220>
   <223> Completely synthesized.
<400> 153
   Gln Met Gln Leu Val Gln Ser Gly Ala Glu Val Lys Lys Thr Gly Ser
   1 5 10 15
   Ser Val Lys Val Ser Cys Lys Ala Ser Gly Tyr Thr Phe Thr Asp Ser
   20 25 30
   Val Met Asn Trp Val Arg Gln Ala Pro Gly Gln Ala Leu Glu Trp Met
   35 40 45
   Gly Trp Ile Asp Pro Glu Tyr Gly Arg Thr Asp Val Ala Gln Lys Phe
   50 55 60
   Gln Asp Arg Val Thr Ile Thr Arg Asp Arg Ser Met Ser Thr Ala Tyr
   65 70 75 80
   Met Glu Leu Ser Ser Leu Arg Ser Glu Asp Thr Ala Met Tyr Tyr Cys
   85 90 95
   Ala Arg Thr Lys Tyr Asn Ser Gly Tyr Gly Phe Pro Tyr Trp Gly Gln
   100 105 110
   Gly Thr Thr Val Thr Val Ser Ser
   115 120
<210> 154
   <211> 120
   <212> PRT
   <213> Artificial Sequence
<220>
   <223> Completely synthesized.
<400> 154
   Gln Met Gln Leu Val Gln Ser Gly Ala Glu Val Lys Lys Thr Gly Ser
   1 5 10 15
   Ser Val Lys Val Ser Cys Lys Ala Ser Gly Tyr Thr Phe Thr Asp Ser
   20 25 30
   Val Met Asn Trp Val Arg Gln Ala Pro Gly Gln Ala Leu Glu Trp Met
   35 40 45
   Gly Trp Ile Asp Pro Glu Tyr Gly Arg Thr Asp Val Ala Gln Lys Phe
       50                  55                  60
   Gln Asp Arg Val Thr Ile Thr Ala Asp Arg Ser Met Ser Thr Ala Tyr
   65                  70                  75                  80
   Met Glu Leu Ser Ser Leu Arg Ser Glu Asp Thr Ala Met Tyr Tyr Cys
                   85                  90                  95
   Ala Arg Thr Lys Tyr Asn Ser Gly Tyr Gly Phe Pro Tyr Trp Gly Gln
               100                 105                 110
   Gly Thr Thr Val Thr Val Ser Ser
           115                 120
<210> 155
   <211> 120
   <212> PRT
   <213> Artificial Sequence
<220>
   <223> Completely synthesized.
<400> 155
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Asn Trp Val Gln Gln Ala Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Thr Thr Lys Tyr Asn Ser Gly Tyr Gly Phe Pro Tyr Trp Gly Gln
               100                 105                 110
   Gly Thr Thr Val Thr Val Ser Ser
           115                 120
<210> 156
   <211> 120
   <212> PRT
   <213> Artificial Sequence
<220>
   <223> Completely synthesized.
<400> 156
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Asn Trp Val Gln Gln Ala Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Lys Tyr Asn Ser Gly Tyr Gly Phe Pro Tyr Trp Gly Gln
               100                 105                 110
   Gly Thr Thr Val Thr Val Ser Ser
           115                 120
<210> 157
   <211> 108
   <212> PRT
   <213> Rattus norvegicus
<400> 157
   Asp Ile Val Met Thr Gln Ser Pro Thr Ser Ile Ser Ile Ser Val Gly
   1               5                   10                  15
   Glu Arg Val Thr Met Asn Cys Lys Ala Ser Gln Asn Val Asp Ser Asp
               20                  25                  30
   Val Asp Trp Tyr Gln Gln Lys Thr Gly Gln Ser Pro Lys Leu Leu Ile
           35                  40                  45
   Tyr Lys Ala Ser Asn Arg Tyr Thr Gly Val Pro Asp Arg Phe Thr Gly
       50                  55                  60
   Ser Gly Ser Gly Thr Asp Phe Thr Phe Thr Ile Ser Asn Met Gln Ala
   65                  70                  75                  80
   Glu Asp Leu Ala Val Tyr Tyr Cys Met Gln Ser Asn Thr His Pro Arg
                   85                  90                  95
   Thr Phe Gly Gly Gly Thr Lys Leu Glu Leu Lys Arg
               100                 105
<210> 158
   <211> 108
   <212> PRT
   <213> Artificial Sequence
<220>
   <223> Completely synthesized.
<400> 158
   Asp Ile Gln Leu Thr Gln Ser Pro Ser Phe Leu Ser Ala Ser Val Gly
   1 5 10 15
   Asp Arg Val Thr Ile Thr Cys Lys Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Asn Arg Tyr Thr Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Glu Asp Phe Ala Thr Tyr Tyr Cys Met Gln Ser Asn Thr His Pro Arg
   85 90 95
   Thr Phe Gly Gly Gly Thr Lys Val Glu Ile Lys Arg
   100 105
<210> 159
   <211> 108
   <212> PRT
   <213> Artificial Sequence
<220>
   <223> Completely synthesized.
<400> 159
   Asp Ile Gln Met Thr Gln Ser Pro Ser Ser Val Ser Ala Ser Val Gly
   1               5                   10                  15
   Asp Arg Val Thr Ile Thr Cys Lys Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Met Gln Ser Asn Thr His Pro Arg
                   85                  90                  95
   Thr Phe Gly Gly Gly Thr Lys Val Glu Ile Lys Arg
               100                 105
<210> 160
   <211> 108
   <212> PRT
   <213> Artificial Sequence
<220>
   <223> Completely synthesized.
<400> 160
   Ala Ile Gln Leu Thr Gln Ser Pro Ser Ser Leu Ser Ala Ser Val Gly
   1 5 10 15
   Asp Arg Val Thr Ile Thr Cys Lys Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Asn Arg Tyr Thr Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Met Gln Ser Asn Thr His Pro Arg
   85 90 95
   Thr Phe Gly Gly Gly Thr Lys Val Glu Ile Lys Arg
   100 105
<210> 161
   <211> 108
   <212> PRT
   <213> Artificial Sequence
<220>
   <223> Completely synthesized.
<400> 161
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Met Gln Ser Asn Thr His Pro Arg
                   85                  90                  95
   Thr Phe Gly Gly Gly Thr Lys Val Glu Ile Lys Arg
               100                 105
<210> 162
   <211> 108
   <212> PRT
   <213> Artificial Sequence
<220>
   <223> Completely synthesized.
<400> 162
   Glu Ile Val Leu Thr Gln Ser Pro Ala Thr Leu Ser Leu Ser Pro Gly
   1               5                   10                  15
   Glu Arg Ala Thr Leu Ser Cys Lys Ala Ser Gln Asn Val Asp Ser Asp
               20                  25                  30
   Val Asp Trp Tyr Gln Gln Lys Pro Gly Gln Ala Pro Arg Leu Leu Ile
           35                  40                  45
   Tyr Lys Ala Ser Asn Arg Tyr Thr Gly Ile Pro Ala Arg Phe Ser Gly
       50                  55                  60
   Ser Gly Ser Gly Arg Asp Phe Thr Leu Thr Ile Ser Ser Leu Glu Pro
   65                  70                  75                  80
   Glu Asp Phe Ala Val Tyr Tyr Cys Met Gln Ser Asn Thr His Pro Arg
                   85                  90                  95
   Thr Phe Gly Gly Gly Thr Lys Val Glu Ile Lys Arg
               100                 105
<210> 163
   <211> 108
   <212> PRT
   <213> Artificial Sequence
<220>
   <223> Completely synthesized.
<400> 163
   Glu Ile Val Met Thr Gln Ser Pro Ala Thr Leu Ser Val Ser Pro Gly
   1               5                   10                  15
   Glu Arg Ala Thr Leu Ser Cys Lys Ala Ser Gln Asn Val Asp Ser Asp
               20                  25                  30
   Val Asp Trp Tyr Gln Gln Lys Pro Gly Gln Ala Pro Arg Leu Leu Ile
           35                  40                  45
   Tyr Lys Ala Ser Asn Arg Tyr Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Met Gln Ser Asn Thr His Pro Arg
                   85                  90                  95
   Thr Phe Gly Gly Gly Thr Lys Val Glu Ile Lys Arg
               100                 105
<210> 164
   <211> 108
   <212> PRT
   <213> Artificial Sequence
<220>
   <223> Completely synthesized.
<400> 164
   Glu Ile Val Leu Thr Gln Ser Pro Asp Phe Gln Ser Val Thr Pro Lys
   1               5                   10                  15
   Glu Lys Val Thr Ile Thr Cys Lys Ala Ser Gln Asn Val Asp Ser Asp
               20                  25                  30
   Val Asp Trp Tyr Gln Gln Lys Pro Asp Gln Ser Pro Lys Leu Leu Ile
           35                  40                  45
   Lys Lys Ala Ser Asn Arg Tyr Thr Gly Val Pro Ser Arg Phe Ser Gly
       50                  55                  60
   Ser Gly Ser Gly Thr Asp Phe Thr Leu Thr Ile Asn Ser Leu Glu Ala
   65                  70                  75                  80
   Glu Asp Ala Ala Thr Tyr Tyr Cys Met Gln Ser Asn Thr His Pro Arg
                   85                  90                  95
   Thr Phe Gly Gly Gly Thr Lys Val Glu Ile Lys Arg
               100                 105
<210> 165
   <211> 120
   <212> PRT
   <213> Artificial Sequence
<220>
   <223> Completely synthesized.
<400> 165
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Asn Trp Val Gln Gln Glu Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Lys Tyr Asn Ser Gly Tyr Gly Phe Pro Tyr Trp Gly Gln
               100                 105                 110
   Gly Thr Thr Val Thr Val Ser Ser
           115                 120
<210> 166
   <211> 120
   <212> PRT
   <213> Artificial Sequence
<220>
   <223> Completely synthesized.
<400> 166
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Asn Trp Val Gln Gln Gly Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Lys Tyr Asn Ser Gly Tyr Gly Phe Pro Tyr Trp Gly Gln
               100                 105                 110
   Gly Thr Thr Val Thr Val Ser Ser
           115                 120
<210> 167
   <211> 120
   <212> PRT
   <213> Artificial Sequence
<220>
   <223> Completely synthesized.
<400> 167
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
   20 25 30
   Val Met Asn Trp Val Gln Gln His Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Lys Tyr Asn Ser Gly Tyr Gly Phe Pro Tyr Trp Gly Gln
   100 105 110
   Gly Thr Thr Val Thr Val Ser Ser
   115 120
<210> 168
   <211> 120
   <212> PRT
   <213> Artificial Sequence
<220>
   <223> Completely synthesized.
<400> 168
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Asn Trp Val Gln Gln Gln Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Lys Tyr Asn Ser Gly Tyr Gly Phe Pro Tyr Trp Gly Gln
   100 105 110
   Gly Thr Thr Val Thr Val Ser Ser
   115 120
<210> 169
   <211> 120
   <212> PRT
   <213> Artificial Sequence
<220>
   <223> Completely synthesized.
<400> 169
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Asn Trp Val Gln Gln Ser Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Lys Tyr Asn Ser Gly Tyr Gly Phe Pro Tyr Trp Gly Gln
   100 105 110
   Gly Thr Thr Val Thr Val Ser Ser
   115 120
<210> 170
   <211> 120
   <212> PRT
   <213> Artificial Sequence
<220>
   <223> Completely synthesized.
<400> 170
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Asn Trp Val Gln Gln Val Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Lys Tyr Asn Ser Gly Tyr Gly Phe Pro Tyr Trp Gly Gln
               100                 105                 110
   Gly Thr Thr Val Thr Val Ser Ser
           115                 120
<210> 171
   <211> 120
   <212> PRT
   <213> Artificial Sequence
<220>
   <223> Completely synthesized.
<400> 171
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Glu Trp Val Gln Gln Ala Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Lys Tyr Asn Ser Gly Tyr Gly Phe Pro Tyr Trp Gly Gln
               100                 105                 110
   Gly Thr Thr Val Thr Val Ser Ser
           115                 120
<210> 172
   <211> 120
   <212> PRT
   <213> Artificial Sequence
<220>
   <223> Completely synthesized.
<400> 172
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Pro Trp Val Gln Gln Ala Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Lys Tyr Asn Ser Gly Tyr Gly Phe Pro Tyr Trp Gly Gln
               100                 105                 110
   Gly Thr Thr Val Thr Val Ser Ser
           115                 120
<210> 173
   <211> 120
   <212> PRT
   <213> Artificial Sequence
<220>
   <223> Completely synthesized.
<400> 173
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
   20 25 30
   Val Met Gln Trp Val Gln Gln Ala Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Lys Tyr Asn Ser Gly Tyr Gly Phe Pro Tyr Trp Gly Gln
   100 105 110
   Gly Thr Thr Val Thr Val Ser Ser
   115 120
<210> 174
   <211> 120
   <212> PRT
   <213> Artificial Sequence
<220>
   <223> Completely synthesized.
<400> 174
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Ser Trp Val Gln Gln Ala Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Lys Tyr Asn Ser Gly Tyr Gly Phe Pro Tyr Trp Gly Gln
   100 105 110
   Gly Thr Thr Val Thr Val Ser Ser
   115 120
<210> 175
   <211> 120
   <212> PRT
   <213> Artificial Sequence
<220>
   <223> Completely synthesized.
<400> 175
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Asn Trp Val Gln Gln Ala Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Glu Thr Lys Tyr Asn Ser Gly Tyr Gly Phe Pro Tyr Trp Gly Gln
   100 105 110
   Gly Thr Thr Val Thr Val Ser Ser
   115 120
<210> 176
   <211> 120
   <212> PRT
   <213> Artificial Sequence
<220>
   <223> Completely synthesized.
<400> 176
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Asn Trp Val Gln Gln Ala Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Gly Thr Lys Tyr Asn Ser Gly Tyr Gly Phe Pro Tyr Trp Gly Gln
               100                 105                 110
   Gly Thr Thr Val Thr Val Ser Ser
           115                 120
<210> 177
   <211> 120
   <212> PRT
   <213> Artificial Sequence
<220>
   <223> Completely synthesized.
<400> 177
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Asn Trp Val Gln Gln Ala Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Pro Thr Lys Tyr Asn Ser Gly Tyr Gly Phe Pro Tyr Trp Gly Gln
               100                 105                 110
   Gly Thr Thr Val Thr Val Ser Ser
           115                 120
<210> 178
   <211> 120
   <212> PRT
   <213> Artificial Sequence
<220>
   <223> Completely synthesized.
<400> 178
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Asn Trp Val Gln Gln Ala Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Thr Thr Lys Tyr Asn Ser Gly Tyr Gly Phe Pro Tyr Trp Gly Gln
   100 105 110
   Gly Thr Thr Val Thr Val Ser Ser
   115 120
<210> 179
   <211> 120
   <212> PRT
   <213> Artificial Sequence
<220>
   <223> Completely synthesized.
<400> 179
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Asn Trp Val Gln Gln Ala Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Gly Tyr Asn Ser Gly Tyr Gly Phe Pro Tyr Trp Gly Gln
   100 105 110
   Gly Thr Thr Val Thr Val Ser Ser
   115 120
<210> 180
   <211> 120
   <212> PRT
   <213> Artificial Sequence
<220>
   <223> Completely synthesized.
<400> 180
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Asn Trp Val Gln Gln Ala Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Thr Tyr Asn Ser Gly Tyr Gly Phe Pro Tyr Trp Gly Gln
   100 105 110
   Gly Thr Thr Val Thr Val Ser Ser
   115 120
<210> 181
   <211> 108
   <212> PRT
   <213> Artificial Sequence
<220>
   <223> Completely synthesized.
<400> 181
   Asp Ile Gln Met Thr Gln Ser Pro Ser Ser Leu Ser Ala Ser Val Gly
   1 5 10 15
   Asp Arg Val Thr Ile Thr Cys Glu Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Asn Arg Tyr Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Met Gln Ser Asn Thr His Pro Arg
   85 90 95
   Thr Phe Gly Gly Gly Thr Lys Val Glu Ile Lys Arg
   100 105
<210> 182
   <211> 108
   <212> PRT
   <213> Artificial Sequence
<220>
   <223> Completely synthesized.
<400> 182
   Asp Ile Gln Met Thr Gln Ser Pro Ser Ser Leu Ser Ala Ser Val Gly
   1               5                   10                  15
   Asp Arg Val Thr Ile Thr Cys Gly Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Met Gln Ser Asn Thr His Pro Arg
                   85                  90                  95
   Thr Phe Gly Gly Gly Thr Lys Val Glu Ile Lys Arg
               100                 105
<210> 183
   <211> 108
   <212> PRT
   <213> Artificial Sequence
<220>
   <223> Completely synthesized.
<400> 183
   Asp Ile Gln Met Thr Gln Ser Pro Ser Ser Leu Ser Ala Ser Val Gly
   1               5                   10                  15
   Asp Arg Val Thr Ile Thr Cys Pro Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Met Gln Ser Asn Thr His Pro Arg
                   85                  90                  95
   Thr Phe Gly Gly Gly Thr Lys Val Glu Ile Lys Arg
               100                 105
<210> 184
   <211> 108
   <212> PRT
   <213> Artificial Sequence
<220>
   <223> Completely synthesized.
<400> 184
   Asp Ile Gln Met Thr Gln Ser Pro Ser Ser Leu Ser Ala Ser Val Gly
   1 5 10 15
   Asp Arg Val Thr Ile Thr Cys Gln Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Asn Arg Tyr Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Met Gln Ser Asn Thr His Pro Arg
   85 90 95
   Thr Phe Gly Gly Gly Thr Lys Val Glu Ile Lys Arg
   100 105
<210> 185
   <211> 108
   <212> PRT
   <213> Artificial Sequence
<220>
   <223> Completely synthesized.
<400> 185
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Asn Asp Tyr Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Met Gln Ser Asn Thr His Pro Arg
   85 90 95
   Thr Phe Gly Gly Gly Thr Lys Val Glu Ile Lys Arg
   100 105
<210> 186
   <211> 108
   <212> PRT
   <213> Artificial Sequence
<220>
   <223> Completely synthesized.
<400> 186
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asn Val Asp Ser Asp
   20 25 30
   Val Asp Trp Tyr Gln Gln Lys Pro Gly Lys Ala Pro Lys Leu Leu Asp
   35 40 45
   Tyr Lys Ala Ser Asn Arg Tyr Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Met Gln Ser Asn Thr His Pro Arg
   85 90 95
   Thr Phe Gly Gly Gly Thr Lys Val Glu Ile Lys Arg
   100 105
<210> 187
   <211> 108
   <212> PRT
   <213> Artificial Sequence
<220>
   <223> Completely synthesized.
<400> 187
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Glu Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Met Gln Ser Asn Thr His Pro Arg
                   85                  90                  95
   Thr Phe Gly Gly Gly Thr Lys Val Glu Ile Lys Arg
               100                 105
<210> 188
   <211> 108
   <212> PRT
   <213> Artificial Sequence
<220>
   <223> Completely synthesized.
<400> 188
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Ala Gln Ser Asn Thr His Pro Arg
                   85                  90                  95
   Thr Phe Gly Gly Gly Thr Lys Val Glu Ile Lys Arg
               100                 105
<210> 189
   <211> 108
   <212> PRT
   <213> Artificial Sequence
<220>
   <223> Completely synthesized.
<400> 189
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Glu Gln Ser Asn Thr His Pro Arg
                   85                  90                  95
   Thr Phe Gly Gly Gly Thr Lys Val Glu Ile Lys Arg
               100                 105
<210> 190
   <211> 108
   <212> PRT
   <213> Artificial Sequence
<220>
   <223> Completely synthesized.
<400> 190
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys His Gln Ser Asn Thr His Pro Arg
   85 90 95
   Thr Phe Gly Gly Gly Thr Lys Val Glu Ile Lys Arg
   100 105
<210> 191
   <211> 108
   <212> PRT
   <213> Artificial Sequence
<220>
   <223> Completely synthesized.
<400> 191
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Asn Arg Tyr Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Lys Gln Ser Asn Thr His Pro Arg
   85 90 95
   Thr Phe Gly Gly Gly Thr Lys Val Glu Ile Lys Arg
   100 105
<210> 192
   <211> 108
   <212> PRT
   <213> Artificial Sequence
<220>
   <223> Completely synthesized.
<400> 192
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Asn Arg Tyr Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Pro Gln Ser Asn Thr His Pro Arg
   85 90 95
   Thr Phe Gly Gly Gly Thr Lys Val Glu Ile Lys Arg
   100 105
<210> 193
   <211> 108
   <212> PRT
   <213> Artificial Sequence
<220>
   <223> Completely synthesized.
<400> 193
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Asn Arg Tyr Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Gln Gln Ser Asn Thr His Pro Arg
   85 90 95
   Thr Phe Gly Gly Gly Thr Lys Val Glu Ile Lys Arg
   100 105
<210> 194
   <211> 108
   <212> PRT
   <213> Artificial Sequence
<220>
   <223> Completely synthesized.
<400> 194
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Ser Gln Ser Asn Thr His Pro Arg
                   85                  90                  95
   Thr Phe Gly Gly Gly Thr Lys Val Glu Ile Lys Arg
               100                 105
<210> 195
   <211> 108
   <212> PRT
   <213> Artificial Sequence
<220>
   <223> Completely synthesized.
<400> 195
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Val Gln Ser Asn Thr His Pro Arg
                   85                  90                  95
   Thr Phe Gly Gly Gly Thr Lys Val Glu Ile Lys Arg
               100                 105
<210> 196
   <211> 108
   <212> PRT
   <213> Artificial Sequence
<220>
   <223> Completely synthesized.
<400> 196
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Met Asp Ser Asn Thr His Pro Arg
                   85                  90                  95
   Thr Phe Gly Gly Gly Thr Lys Val Glu Ile Lys Arg
               100                 105
<210> 197
   <211> 120
   <212> PRT
   <213> Artificial Sequence
<220>
   <223> Completely synthesized.
<400> 197
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Asn Trp Val Gln Gln Ala Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Lys Tyr Gln Ser Gly Tyr Gly Phe Pro Tyr Trp Gly Gln
               100                 105                 110
   Gly Thr Thr Val Thr Val Ser Ser
           115                 120
<210> 198
   <211> 108
   <212> PRT
   <213> Artificial Sequence
<220>
   <223> Completely synthesized.
<400> 198
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Gln Arg Tyr Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Met Gln Ser Asn Thr His Pro Arg
   85 90 95
   Thr Phe Gly Gly Gly Thr Lys Val Glu Ile Lys Arg
   100 105
<210> 199
   <211> 30
   <212> PRT
   <213> Homo sapiens
<400> 199
   Gln Leu Gln Leu Gln Glu Ser Gly Pro Gly Leu Val Lys Pro Ser Glu
   1 5 10 15
   Thr Leu Ser Leu Thr Cys Thr Val Ser Gly Gly Ser Ile Ser
   20 25 30
<210> 200
   <211> 7
   <212> PRT
   <213> Homo sapiens
<400> 200
   Ser Ser Ser Tyr Tyr Trp Ser
   1 5
<210> 201
   <211> 14
   <212> PRT
   <213> Homo sapiens
<400> 201
   Trp Ile Arg Gln His Pro Gly Lys Gly Leu Glu Trp Ile Gly
   1 5 10
<210> 202
   <211> 16
   <212> PRT
   <213> Homo sapiens
<400> 202
   Tyr Ile Tyr Tyr Ser Gly Ser Thr Asn Tyr Asn Pro Ser Leu Lys Ser
   1 5 10 15
<210> 203
   <211> 32
   <212> PRT
   <213> Homo sapiens
<400> 203
   Arg Val Thr Ile Ser Val Asp Thr Leu Lys Asn Gln Ile Ser Leu Arg
   1 5 10 15
   Leu Thr Ser Val Thr Ala Ala Asp Thr Ala Val Tyr Tyr Cys Ala Arg
   20 25 30
<210> 204
   <211> 11
   <212> PRT
   <213> Homo sapiens
<400> 204
   Val Gly Gly Ala Gly Gly Trp Pro Met Asp Val
   1 5 10
<210> 205
   <211> 11
   <212> PRT
   <213> Homo sapiens
<400> 205
   Trp Gly Gln Gly Thr Thr Val Thr Val Ser Ser
   1 5 10
<210> 206
   <211> 30
   <212> PRT
   <213> Homo sapiens
<400> 206
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Val Ser
               20                  25                  30
<210> 207
   <211> 7
   <212> PRT
   <213> Homo sapiens
<400> 207
   Ser Gly Ser Tyr Tyr Trp Ser
   1               5
<210> 208
   <211> 14
   <212> PRT
   <213> Homo sapiens
<400> 208
   Trp Ile Arg Gln Pro Pro Gly Lys Gly Leu Glu Trp Ile Gly
   1               5                   10
<210> 209
   <211> 32
   <212> PRT
   <213> Homo sapiens
<400> 209
   Arg Val Thr Ile Ser Val Asp Thr Ser Lys Asn Gln Phe Ser Leu Lys
   1               5                   10                  15
   Leu Ser Ser Val Thr Ala Ala Asp Thr Ala Val Tyr Tyr Cys Ala Arg
               20                  25                  30
<210> 210
   <211> 32
   <212> PRT
   <213> Artificial Sequence
<220>
   <223> Completely synthesized.
<400> 210
   Arg Val Thr Ile Ser Val Asp Thr Ser Lys Asn Gln Ile Ser Leu Arg
   1               5                   10                  15
   Leu Thr Ser Val Thr Ala Ala Asp Thr Ala Val Tyr Tyr Cys Ala Arg
               20                  25                  30
<210> 211
   <211> 14
   <212> PRT
   <213> Artificial Sequence
<220>
   <223> Completely synthesized.
<400> 211
   Trp Ile Arg Gln Pro Pro Gly Lys Gly Leu Glu Trp Ile Gly
   1               5                   10
<210> 212
   <211> 32
   <212> PRT
   <213> Artificial Sequence
<220>
   <223> Completely synthesized.
<400> 212
   Arg Val Thr Ile Ser Val Asp Thr Leu Lys Asn Gln Ile Ser Leu Arg
   1               5                   10                  15
   Leu Ser Ser Val Thr Ala Ala Asp Thr Ala Val Tyr Tyr Cys Ala Arg
               20                  25                  30
<210> 213
   <211> 32
   <212> PRT
   <213> Artificial Sequence
<220>
   <223> Completely synthesized.
<400> 213
   Arg Val Thr Ile Ser Val Asp Thr Ser Lys Asn Gln Phe Ser Leu Lys
   1               5                   10                  15
   Leu Ser Ser Val Thr Ala Ala Asp Thr Ala Val Tyr Tyr Cys Ala Arg
               20                  25                  30
<210> 214
   <211> 30
   <212> PRT
   <213> Artificial Sequence
<220>
   <223> Completely synthesized.
<400> 214
   Gln Leu Gln Leu Gln Glu Ser Gly Pro Gly Leu Val Lys Pro Ser Glu
   1               5                   10                  15
   Thr Leu Ser Leu Thr Cys Lys Val Ser Gly Gly Ser Ile Ser
               20                  25                  30
<210> 215
   <211> 30
   <212> PRT
   <213> Artificial Sequence
<220>
   <223> Completely synthesized.
<400> 215
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser
               20                  25                  30
<210> 216
   <211> 32
   <212> PRT
   <213> Artificial Sequence
<220>
   <223> Completely synthesized.
<400> 216
   Arg Val Thr Ile Ser Arg Asp Thr Leu Lys Asn Gln Ile Ser Leu Arg
   1               5                   10                  15
   Leu Thr Ser Val Thr Ala Ala Asp Thr Ala Val Tyr Tyr Cys Ala Arg
               20                  25                  30
<210> 217
   <211> 30
   <212> PRT
   <213> Artificial Sequence
<220>
   <223> Completely synthesized.
<400> 217
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Val Ser
               20                  25                  30
<210> 218
   <211> 32
   <212> PRT
   <213> Artificial Sequence
<220>
   <223> Completely synthesized.
<400> 218
   Arg Val Thr Ile Ser Val Asp Thr Leu Lys Asn Gln Ile Ser Leu Arg
   1               5                   10                  15
   Leu Thr Ser Val Arg Ala Ala Asp Thr Ala Val Tyr Tyr Cys Ala Arg
               20                  25                  30
<210> 219
   <211> 30
   <212> PRT
   <213> Artificial Sequence
<220>
   <223> Completely synthesized.
<400> 219
   Gln Leu Gln Leu Gln Glu Ser Gly Pro Gly Leu Val Lys Pro Ser Glu
   1               5                   10                  15
   Thr Leu Lys Leu Thr Cys Thr Val Ser Gly Gly Ser Ile Ser
               20                  25                  30
<210> 220
   <211> 11
   <212> PRT
   <213> Artificial Sequence
<220>
   <223> Completely synthesized.
<400> 220
   Val Gly Gly Ala Gly Gly Trp Pro Leu Glu Val
   1               5                   10
<210> 221
   <211> 32
   <212> PRT
   <213> Artificial Sequence
<220>
   <223> Completely synthesized.
<400> 221
   Arg Val Thr Ile Ser Val Asp Lys Leu Lys Asn Gln Ile Ser Leu Arg
   1 5 10 15
   Leu Thr Ser Val Thr Ala Ala Asp Thr Ala Val Tyr Tyr Cys Ala Arg
   20 25 30
<210> 222
   <211> 11
   <212> PRT
   <213> Artificial Sequence
<220>
   <223> Completely synthesized.
<400> 222
   Val Gly Gly Ala Gly Gly Trp Pro Leu Asp Val
   1 5 10
<210> 223
   <211> 11
   <212> PRT
   <213> Artificial Sequence
<220>
   <223> Completely synthesized.
<400> 223
   Val Gly Gly Ala Gly Gly Trp Pro Met Glu Val
   1 5 10
<210> 224
   <211> 7
   <212> PRT
   <213> Artificial Sequence
<220>
   <223> Completely synthesized.
<400> 224
   Ser Gly Ser Tyr Tyr Trp Ser
   1 5
<210> 225
   <211> 32
   <212> PRT
   <213> Artificial Sequence
<220>
   <223> Completely synthesized.
<400> 225
   Arg Val Thr Ile Ser Val Asp Thr Leu Lys Asn Gln Phe Ser Leu Arg
   1               5                   10                  15
   Leu Thr Ser Val Thr Ala Ala Asp Thr Ala Val Tyr Tyr Cys Ala Arg
               20                  25                  30
<210> 226
   <211> 32
   <212> PRT
   <213> Artificial Sequence
<220>
   <223> Completely synthesized.
<400> 226
   Arg Val Thr Ile Ser Val Asp Thr Leu Lys Asn Gln Ile Ser Leu Lys
   1               5                   10                  15
   Leu Thr Ser Val Thr Ala Ala Asp Thr Ala Val Tyr Tyr Cys Ala Arg
               20                  25                  30
<210> 227
   <211> 32
   <212> PRT
   <213> Artificial Sequence
<220>
   <223> Completely synthesized.
<400> 227
   Arg Val Thr Ile Ser Val Asp Thr Ser Lys Asn Gln Phe Ser Leu Lys
   1               5                   10                  15
   Leu Ser Ser Val Thr Ala Ala Asp Thr Ala Val Tyr Tyr Cys Ala Arg
               20                  25                  30
<210> 228
   <211> 11
   <212> PRT
   <213> Artificial Sequence
<220>
   <223> Completely synthesized.
<400> 228
   Val Gly Gly Ala Gly Gly Trp Pro Leu Asp Val
   1               5                   10
<210> 229
   <211> 14
   <212> PRT
   <213> Artificial Sequence
<220>
   <223> Completely synthesized.
<400> 229
   Trp Ile Arg Gln Pro Pro Gly Lys Gly Leu Glu Trp Ile Gly
   1 5 10
<210> 230
   <211> 32
   <212> PRT
   <213> Artificial Sequence
<220>
   <223> Completely synthesized.
<400> 230
   Arg Val Thr Ile Ser Val Asp Thr Ser Lys Asn Gln Phe Ser Leu Lys
   1 5 10 15
   Leu Ser Ser Val Thr Ala Ala Asp Thr Ala Val Tyr Tyr Cys Ala Arg
   20 25 30
<210> 231
   <211> 11
   <212> PRT
   <213> Artificial Sequence
<220>
   <223> Completely synthesized.
<400> 231
   Val Gly Gly Ala Gly Gly Trp Pro Met Asp Val
   1 5 10
<210> 232
   <211> 22
   <212> PRT
   <213> Artificial Sequence
<220>
   <223> Completely synthesized.
<400> 232
   Gln Ala Val Leu Thr Gln Pro Ala Ser Leu Ser Ala Ser Pro Gly Glu
   1 5 10 15
   Ser Ala Arg Leu Thr Cys
   20
<210> 233
   <211> 14
   <212> PRT
   <213> Artificial Sequence
<220>
   <223> Completely synthesized.
<400> 233
   Thr Leu Pro Ser Asp Ile Asn Val Arg Tyr Tyr Asn Ile Tyr
   1 5 10
<210> 234
   <211> 15
   <212> PRT
   <213> Artificial Sequence
<220>
   <223> Completely synthesized.
<400> 234
   Trp Tyr Gln Gln Lys Pro Gly Ser Pro Pro Arg Tyr Leu Leu Tyr
   1 5 10 15
<210> 235
   <211> 11
   <212> PRT
   <213> Artificial Sequence
<220>
   <223> Completely synthesized.
<400> 235
   Tyr Tyr Ser Asp Ser His Lys Gly Gln Gly Ser
   1 5 10
<210> 236
   <211> 34
   <212> PRT
   <213> Artificial Sequence
<220>
   <223> Completely synthesized.
<400> 236
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Glu Ala Asp Tyr
   20 25 30
   Tyr Cys
<210> 237
   <211> 10
   <212> PRT
   <213> Artificial Sequence
<220>
   <223> Completely synthesized.
<400> 237
   Met Thr Trp Ser Ser Asn Gly Ser Gly Val
   1               5                   10
<210> 238
   <211> 11
   <212> PRT
   <213> Artificial Sequence
<220>
   <223> Completely synthesized.
<400> 238
   Phe Gly Gly Gly Thr Gln Leu Thr Val Leu Gly
   1               5                   10
<210> 239
   <211> 22
   <212> PRT
   <213> Homo sapiens
<400> 239
   Gln Pro Val Leu Thr Gln Pro Pro Ser Ser Ser Ala Ser Pro Gly Glu
   1               5                   10                  15
   Ser Ala Arg Leu Thr Cys
               20
<210> 240
   <211> 14
   <212> PRT
   <213> Homo sapiens
<400> 240
   Thr Leu Pro Ser Asp Ile Asn Val Gly Ser Tyr Asn Ile Tyr
   1               5                   10
<210> 241
   <211> 11
   <212> PRT
   <213> Homo sapiens
<400> 241
   Tyr Tyr Ser Asp Ser Asp Lys Gly Gln Gly Ser
   1 5 10
<210> 242
   <211> 34
   <212> PRT
   <213> Homo sapiens
<400> 242
   Gly Val Pro Ser Arg Phe Ser Gly Ser Lys Asp Ala Ser Ala Asn Thr
   1 5 10 15
   Gly Ile Leu Leu Ile Ser Gly Leu Gln Ser Glu Asp Glu Ala Asp Tyr
   20 25 30
   Tyr Cys
<210> 243
   <211> 8
   <212> PRT
   <213> Homo sapiens
<400> 243
   Met Ile Trp Pro Ser Asn Ala Ser
   1 5
<210> 244
   <211> 10
   <212> PRT
   <213> Artificial Sequence
<220>
   <223> Completely synthesized.
<400> 244
   Met Thr Trp Ser Ser Gln Gly Ser Gly Val
   1 5 10
<210> 245
   <211> 34
   <212> PRT
   <213> Artificial Sequence
<220>
   <223> Completely synthesized.
<400> 245
   Gly Val Pro Ser Arg Phe Ser Gly Ser Lys Asp Val Ser Thr Ala Ser
   1               5                   10                  15
   Gly Ile Leu Leu Ile Ser Gly Leu Gln Ser Glu Asp Glu Ala Asp Tyr
               20                  25                  30
   Tyr Cys
<210> 246
   <211> 15
   <212> PRT
   <213> Artificial Sequence
<220>
   <223> Completely synthesized.
<400> 246
   Trp Tyr Gln Gln Lys Pro Gly Ser Pro Pro Arg Tyr Leu Leu Arg
   1               5                   10                  15
<210> 247
   <211> 22
   <212> PRT
   <213> Artificial Sequence
<220>
   <223> Completely synthesized.
<400> 247
   Gln Ala Val Leu Thr Gln Pro Ala Ser Leu Ser Ala Ser Pro Gly Ala
   1               5                   10                  15
   Ser Ala Arg Leu Thr Cys
               20
<210> 248
   <211> 15
   <212> PRT
   <213> Artificial Sequence
<220>
   <223> Completely synthesized.
<400> 248
   Trp Tyr Gln Gln Lys Pro Gly Ser Pro Pro Arg Tyr Leu Leu Arg
   1               5                   10                  15
<210> 249
   <211> 11
   <212> PRT
   <213> Artificial Sequence
<220>
   <223> Completely synthesized.
<400> 249
   Tyr Arg Ser Asp Ser His Lys Gly Gln Gly Ser
   1               5                   10
<210> 250
   <211> 14
   <212> PRT
   <213> Artificial Sequence
<220>
   <223> Completely synthesized.
<400> 250
   Thr Leu Pro Ser Gly Ile Asn Val Arg Tyr Tyr Asn Ile Tyr
   1               5                   10
<210> 251
   <211> 10
   <212> PRT
   <213> Artificial Sequence
<220>
   <223> Completely synthesized.
<400> 251
   Met Thr Trp Ser Ser Asn Gly Ala Gly Val
   1               5                   10
<210> 252
   <211> 14
   <212> PRT
   <213> Artificial Sequence
<220>
   <223> Completely synthesized.
<400> 252
   Thr Leu Pro Ser Asp Ile Asn Val Arg Tyr Tyr Arg Ile Tyr
   1               5                   10
<210> 253
   <211> 11
   <212> PRT
   <213> Artificial Sequence
<220>
   <223> Completely synthesized.
<400> 253
   Tyr Arg Ser Asp Ser His Lys Gly Gln Gly Ser
   1 5 10
<210> 254
   <211> 10
   <212> PRT
   <213> Artificial Sequence
<220>
   <223> Completely synthesized.
<400> 254
   Met Thr Trp Ser Ser Thr Gly Ser Gly Val
   1 5 10
<210> 255
   <211> 14
   <212> PRT
   <213> Artificial Sequence
<220>
   <223> Completely synthesized.
<400> 255
   Thr Leu Arg Ser Asp Ile Asn Val Arg Tyr Tyr Asn Ile Tyr
   1 5 10
<210> 256
   <211> 10
   <212> PRT
   <213> Artificial Sequence
<220>
   <223> Completely synthesized.
<400> 256
   Met Thr Trp Ser Ser Asn Pro Ser Gly Val
   1 5 10
<210> 257
   <211> 10
   <212> PRT
   <213> Artificial Sequence
<220>
   <223> Completely synthesized.
<400> 257
   Ile Thr Trp Ser Ser Asn Gly Ser Gly Val
   1 5 10
<210> 258
   <211> 10
   <212> PRT
   <213> Artificial Sequence
<220>
   <223> Completely synthesized.
<400> 258
   Leu Thr Trp Ser Ser Asn Gly Ser Gly Val
   1 5 10
<210> 259
   <211> 22
   <212> PRT
   <213> Artificial Sequence
<220>
   <223> Completely synthesized.
<400> 259
   Gln Pro Val Leu Thr Gln Pro Ala Ser Leu Ser Ala Ser Pro Gly Glu
   1 5 10 15
   Ser Ala Arg Leu Thr Cys
   20
<210> 260
   <211> 22
   <212> PRT
   <213> Artificial Sequence
<220>
   <223> Completely synthesized.
<400> 260
   Gln Ala Val Leu Thr Gln Pro Pro Ser Leu Ser Ala Ser Pro Gly Glu
   1 5 10 15
   Ser Ala Arg Leu Thr Cys
   20
<210> 261
   <211> 22
   <212> PRT
   <213> Artificial Sequence
<220>
   <223> Completely synthesized.
<400> 261
   Gln Ala Val Leu Thr Gln Pro Ala Ser Ser Ser Ala Ser Pro Gly Glu
   1 5 10 15
   Ser Ala Arg Leu Thr Cys
   20
<210> 262
   <211> 14
   <212> PRT
   <213> Synthetic
<400> 262
<210> 263
   <211> 14
   <212> PRT
   <213> Artificial Sequence
<220>
   <223> Completely synthesized.
<400> 263
   Thr Leu Pro Ser Asp Ile Asn Val Arg Ser Tyr Asn Ile Tyr
   1 5 10
<210> 264
   <211> 11
   <212> PRT
   <213> Artificial Sequence
<220>
   <223> Completely synthesized.
<400> 264
   Tyr Tyr Ser Asp Ser Asp Lys Gly Gln Gly Ser
   1 5 10
<210> 265
   <211> 34
   <212> PRT
   <213> Artificial Sequence
<220>
   <223> Completely synthesized.
<400> 265
   Gly Val Pro Ser Arg Phe Ser Gly Ser Lys Asp Ala Ser Thr Asn Ser
   1 5 10 15
   Gly Ile Leu Leu Ile Ser Gly Leu Gln Ser Glu Asp Glu Ala Asp Tyr
   20 25 30
   Tyr Cys
<210> 266
   <211> 34
   <212> PRT
   <213> Artificial Sequence
<220>
   <223> Completely synthesized.
<400> 266
   Gly Val Pro Ser Arg Phe Ser Gly Ser Lys Asp Val Ser Ala Asn Ser
   1 5 10 15
   Gly Ile Leu Leu Ile Ser Gly Leu Gln Ser Glu Asp Glu Ala Asp Tyr
   20 25 30
   Tyr Cys
<210> 267
   <211> 34
   <212> PRT
   <213> Artificial Sequence
<220>
   <223> Completely synthesized.
<400> 267
   Gly Val Pro Ser Arg Phe Ser Gly Ser Lys Asp Val Ser Thr Asn Thr
   1 5 10 15
   Gly Ile Leu Leu Ile Ser Gly Leu Gln Ser Glu Asp Glu Ala Asp Tyr
   20 25 30
   Tyr Cys
<210> 268
   <211> 10
   <212> PRT
   <213> Artificial Sequence
<220>
   <223> Completely synthesized.
<400> 268
   Met Ile Trp Ser Ser Asn Gly Ser Gly Val
   1 5 10
<210> 269
   <211> 10
   <212> PRT
   <213> Artificial Sequence
<220>
   <223> Completely synthesized.
<400> 269
   Met Thr Trp Pro Ser Asn Gly Ser Gly Val
   1 5 10
<210> 270
   <211> 10
   <212> PRT
   <213> Artificial Sequence
<220>
   <223> Completely synthesized.
<400> 270
   Met Thr Trp Ser Ser Asn Ala Ser Gly Val
   1 5 10
<210> 271
   <211> 34
   <212> PRT
   <213> Artificial Sequence
<220>
   <223> Completely synthesized.
<400> 271
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 272
   <211> 34
   <212> PRT
   <213> Artificial Sequence
<220>
   <223> Completely synthesized.
<400> 272
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 273
   <211> 10
   <212> PRT
   <213> Artificial Sequence
<220>
   <223> Completely synthesized.
<400> 273
   Leu Thr Trp Ser Ser Asn Gly Ser Gly Val
   1 5 10
<210> 274
   <211> 34
   <212> PRT
   <213> Artificial Sequence
<220>
   <223> Completely synthesized.
<400> 274
   Gly Val Pro Ser Arg Phe Ser Gly Ser Lys Asp Val Ser Thr Gln Ser
   1 5 10 15
   Gly Ile Leu Leu Ile Ser Gly Leu Gln Ser Glu Asp Ile Ala Thr Tyr
   20 25 30
   Tyr Cys
<210> 275
   <211> 10
   <212> PRT
   <213> Artificial Sequence
<220>
   <223> Completely synthesized.
<400> 275
   Met Thr Trp Ser Ser Asn Gly Ser Gly Val
   1 5 10
<210> 276
   <211> 34
   <212> PRT
   <213> Synthetic
<400> 276
   Gly Val Pro Ser Arg Phe Ser Gly Ser Lys Asp Val Ser Thr Thr Ser
   1               5                   10                  15
   Gly Ile Leu Leu Ile Ser Gly Leu Gln Ser Glu Asp Ile Ala Thr Tyr
               20                  25                  30
   Tyr Cys
<210> 277
   <211> 34
   <212> PRT
   <213> Artificial Sequence
<220>
   <223> Completely synthesized.
<400> 277
   Gly Val Pro Ser Arg Phe Ser Gly Ser Lys Asp Val Ser Thr Gly Ser
   1               5                   10                  15
   Gly Ile Leu Leu Ile Ser Gly Leu Gln Ser Glu Asp Ile Ala Thr Tyr
               20                  25                  30
   Tyr Cys
<210> 278
   <211> 34
   <212> PRT
   <213> Artificial Sequence
<220>
   <223> Completely synthesized.
<400> 278
   Gly Val Pro Ser Arg Phe Ser Gly Ser Lys Asp Val Ser Thr His Ser
   1               5                   10                  15
   Gly Ile Leu Leu Ile Ser Gly Leu Gln Ser Glu Asp Ile Ala Thr Tyr
               20                  25                  30
   Tyr Cys
<210> 279
   <211> 34
   <212> PRT
   <213> Artificial Sequence
<220>
   <223> Completely synthesized.
<400> 279
   Gly Val Pro Ser Arg Phe Ser Gly Ser Lys Asp Val Ser Thr Lys Ser
   1 5 10 15
   Gly Ile Leu Leu Ile Ser Gly Leu Gln Ser Glu Asp Ile Ala Thr Tyr
   20 25 30
   Tyr Cys
<210> 280
   <211> 34
   <212> PRT
   <213> Artificial Sequence
<220>
   <223> Completely synthesized.
<400> 280
   Gly Val Pro Ser Arg Phe Ser Gly Ser Lys Asp Val Ser Thr Pro Ser
   1 5 10 15
   Gly Ile Leu Leu Ile Ser Gly Leu Gln Ser Glu Asp Ile Ala Thr Tyr
   20 25 30
   Tyr Cys
<210> 281
   <211> 15
   <212> PRT
   <213> Artificial Sequence
<220>
   <223> Completely synthesized.
<400> 281
   Trp Tyr Gln Gln Lys Pro Gly Ser Pro Pro Arg Tyr Glu Leu Tyr
   1 5 10 15
<210> 282
   <211> 34
   <212> PRT
   <213> Artificial Sequence
<220>
   <223> Completely synthesized.
<400> 282
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 283
   <211> 15
   <212> PRT
   <213> Artificial Sequence
<220>
   <223> Completely synthesized.
<400> 283
   Trp Tyr Gln Gln Lys Pro Gly Ser Pro Pro Arg Tyr Gly Leu Tyr
   1               5                   10                  15
<210> 284
   <211> 34
   <212> PRT
   <213> Artificial Sequence
<220>
   <223> Completely synthesized.
<400> 284
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 285
   <211> 15
   <212> PRT
   <213> Artificial Sequence
<220>
   <223> Completely synthesized.
<400> 285
   Trp Tyr Gln Gln Lys Pro Gly Ser Pro Pro Arg Tyr Asn Leu Tyr
   1               5                   10                  15
<210> 286
   <211> 34
   <212> PRT
   <213> Artificial Sequence
<220>
   <223> Completely synthesized.
<400> 286
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 287
   <211> 15
   <212> PRT
   <213> Artificial Sequence
<220>
   <223> Completely synthesized.
<400> 287
   Trp Tyr Gln Gln Lys Pro Gly Ser Pro Pro Arg Tyr Pro Leu Tyr
   1 5 10 15
<210> 288
   <211> 34
   <212> PRT
   <213> Artificial Sequence
<220>
   <223> Completely synthesized.
<400> 288
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 289
   <211> 15
   <212> PRT
   <213> Artificial Sequence
<220>
   <223> Completely synthesized.
<400> 289
   Trp Tyr Gln Gln Lys Pro Gly Ser Pro Pro Arg Tyr Ser Leu Tyr
   1               5                   10                  15
<210> 290
   <211> 34
   <212> PRT
   <213> Artificial Sequence
<220>
   <223> Completely synthesized.
<400> 290
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 291
   <211> 15
   <212> PRT
   <213> Artificial Sequence
<220>
   <223> Completely synthesized.
<400> 291
   Trp Tyr Gln Gln Lys Pro Gly Ser Pro Pro Arg Tyr Leu Glu Tyr
   1               5                   10                  15
<210> 292
   <211> 34
   <212> PRT
   <213> Artificial Sequence
<220>
   <223> Completely synthesized.
<400> 292
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
   20 25 30
   Tyr Cys
<210> 293
   <211> 15
   <212> PRT
   <213> Artificial Sequence
<220>
   <223> Completely synthesized.
<400> 293
   Trp Tyr Gln Gln Lys Pro Gly Ser Pro Pro Arg Tyr Leu Pro Tyr
   1 5 10 15
<210> 294
   <211> 34
   <212> PRT
   <213> Artificial Sequence
<220>
   <223> Completely synthesized.
<400> 294
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 295
   <211> 15
   <212> PRT
   <213> Artificial Sequence
<220>
   <223> Completely synthesized.
<400> 295
   Trp Tyr Gln Gln Lys Pro Gly Ser Pro Pro Arg Tyr Leu Leu Glu
   1 5 10 15
<210> 296
   <211> 34
   <212> PRT
   <213> Artificial Sequence
<220>
   <223> Completely synthesized.
<400> 296
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 297
   <211> 15
   <212> PRT
   <213> Artificial Sequence
<220>
   <223> Completely synthesized.
<400> 297
   Trp Tyr Gln Gln Lys Pro Gly Ser Pro Pro Arg Tyr Leu Leu Gln
   1 5 10 15
<210> 298
   <211> 34
   <212> PRT
   <213> Artificial Sequence
<220>
   <223> Completely synthesized.
<400> 298
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 299
   <211> 11
   <212> PRT
   <213> Artificial Sequence
<220>
   <223> Completely synthesized.
<400> 299
   Pro Tyr Ser Asp Ser His Lys Gly Gln Gly Ser
   1 5 10
<210> 300
   <211> 34
   <212> PRT
   <213> Artificial Sequence
<220>
   <223> Completely synthesized.
<400> 300
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 301
   <211> 11
   <212> PRT
   <213> Artificial Sequence
<220>
   <223> Completely synthesized.
<400> 301
   Asn Tyr Ser Asp Ser His Lys Gly Gln Gly Ser
   1 5 10
<210> 302
   <211> 34
   <212> PRT
   <213> Artificial Sequence
<220>
   <223> Completely synthesized.
<400> 302
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 303
   <211> 11
   <212> PRT
   <213> Artificial Sequence
<220>
   <223> Completely synthesized.
<400> 303
   Thr Tyr Ser Asp Ser His Lys Gly Gln Gly Ser
   1               5                   10
<210> 304
   <211> 34
   <212> PRT
   <213> Artificial Sequence
<220>
   <223> Completely synthesized.
<400> 304
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 305
   <211> 11
   <212> PRT
   <213> Artificial Sequence
<220>
   <223> Completely synthesized.
<400> 305
   Tyr Asp Ser Asp Ser His Lys Gly Gln Gly Ser
   1               5                   10
<210> 306
   <211> 34
   <212> PRT
   <213> Artificial Sequence
<220>
   <223> Completely synthesized.
<400> 306
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
   20 25 30
   Tyr Cys
<210> 307
   <211> 11
   <212> PRT
   <213> Artificial Sequence
<220>
   <223> Completely synthesized.
<400> 307
   Tyr Tyr Glu Asp Ser His Lys Gly Gln Gly Ser
   1 5 10
<210> 308
   <211> 34
   <212> PRT
   <213> Artificial Sequence
<220>
   <223> Completely synthesized.
<400> 308
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 309
   <211> 11
   <212> PRT
   <213> Artificial Sequence
<220>
   <223> Completely synthesized.
<400> 309
   Tyr Tyr His Asp Ser His Lys Gly Gln Gly Ser
   1 5 10
<210> 310
   <211> 34
   <212> PRT
   <213> Artificial Sequence
<220>
   <223> Completely synthesized.
<400> 310
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 311
   <211> 11
   <212> PRT
   <213> Artificial Sequence
<220>
   <223> Completely synthesized.
<400> 311
   Tyr Tyr Gln Asp Ser His Lys Gly Gln Gly Ser
   1               5                   10
<210> 312
   <211> 34
   <212> PRT
   <213> Artificial Sequence
<220>
   <223> Completely synthesized.
<400> 312
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 313
   <211> 11
   <212> PRT
   <213> Artificial Sequence
<220>
   <223> Completely synthesized.
<400> 313
   Tyr Tyr Ser Asp Ser Asn Lys Gly Gln Gly Ser
   1 5 10
<210> 314
   <211> 34
   <212> PRT
   <213> Artificial Sequence
<220>
   <223> Completely synthesized.
<400> 314
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 315
   <211> 11
   <212> PRT
   <213> Artificial Sequence
<220>
   <223> Completely synthesized.
<400> 315
   Tyr Tyr Ser Asp Ser Pro Lys Gly Gln Gly Ser
   1 5 10
<210> 316
   <211> 34
   <212> PRT
   <213> Artificial Sequence
<220>
   <223> Completely synthesized.
<400> 316
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 317
   <211> 11
   <212> PRT
   <213> Artificial Sequence
<220>
   <223> Completely synthesized.
<400> 317
   Tyr Tyr Ser Asp Ser His Asp Gly Gln Gly Ser
   1               5                   10
<210> 318
   <211> 34
   <212> PRT
   <213> Artificial Sequence
<220>
   <223> Completely synthesized.
<400> 318
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 319
   <211> 14
   <212> PRT
   <213> Artificial Sequence
<220>
   <223> Completely synthesized.
<400> 319
   Thr Leu Pro Ser Asp Ile Asn Val Gly Ser Tyr Asn Ile Tyr
   1               5                   10
<210> 320
   <211> 34
   <212> PRT
   <213> Artificial Sequence
<220>
   <223> Completely synthesized.
<400> 320
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
   20 25 30
   Tyr Cys
<210> 321
   <211> 10
   <212> PRT
   <213> Artificial Sequence
<220>
   <223> Completely synthesized.
<400> 321
   Leu Thr Trp Ser Ser Gln Gly Ser Gly Val
   1 5 10
<210> 322
   <211> 14
   <212> PRT
   <213> Artificial Sequence
<220>
   <223> Completely synthesized.
<400> 322
   Thr Leu Pro Ser Asp Ile Asn Val Gly Ser Tyr Asn Ile Tyr
   1 5 10
<210> 323
   <211> 34
   <212> PRT
   <213> Artificial Sequence
<220>
   <223> Completely synthesized.
<400> 323
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 324
   <211> 10
   <212> PRT
   <213> Artificial Sequence
<220>
   <223> Completely synthesized.
<400> 324
   Leu Thr Trp Ser Ser Glu Gly Ser Gly Val
   1               5                   10
<210> 325
   <211> 14
   <212> PRT
   <213> Artificial Sequence
<220>
   <223> Completely synthesized.
<400> 325
   Thr Leu Pro Ser Asp Ile Asn Val Gly Ser Tyr Asn Ile Tyr
   1               5                   10
<210> 326
   <211> 11
   <212> PRT
   <213> Artificial Sequence
<220>
   <223> Completely synthesized.
<400> 326
   Tyr Tyr Gln Asp Ser His Lys Gly Gln Gly Ser
   1               5                   10
<210> 327
   <211> 34
   <212> PRT
   <213> Artificial Sequence
<220>
   <223> Completely synthesized.
<400> 327
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 328
   <211> 10
   <212> PRT
   <213> Artificial Sequence
<220>
   <223> Completely synthesized.
<400> 328
   Leu Thr Trp Ser Ser Glu Gly Ser Gly Val
   1               5                   10
<210> 329
   <211> 14
   <212> PRT
   <213> Artificial Sequence
<220>
   <223> Completely synthesized.
<400> 329
   Thr Leu Pro Ser Asp Ile Asn Val Arg Ser Tyr Asn Ile Tyr
   1               5                   10
<210> 330
   <211> 11
   <212> PRT
   <213> Artificial Sequence
<220>
   <223> Completely synthesized.
<400> 330
   Tyr Tyr Gln Asp Ser His Lys Gly Gln Gly Ser
   1               5                   10
<210> 331
   <211> 34
   <212> PRT
   <213> Artificial Sequence
<220>
   <223> Completely synthesized.
<400> 331
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 332
   <211> 10
   <212> PRT
   <213> Artificial Sequence
<220>
   <223> Completely synthesized.
<400> 332
   Leu Thr Trp Ser Ser Glu Gly Ser Gly Val
   1               5                   10
<210> 333
   <211> 14
   <212> PRT
   <213> Artificial Sequence
<220>
   <223> Completely synthesized.
<400> 333
   Thr Leu Pro Ser Asp Ile Asn Val Gly Ser Tyr Asn Ile Tyr
   1               5                   10
<210> 334
   <211> 11
   <212> PRT
   <213> Artificial Sequence
<220>
   <223> Completely synthesized.
<400> 334
   Tyr Tyr Gln Asp Ser His Lys Gly Gln Gly Ser
   1               5                   10
<210> 335
   <211> 34
   <212> PRT
   <213> Artificial Sequence
<220>
   <223> Completely synthesized.
<400> 335
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 336
   <211> 10
   <212> PRT
   <213> Artificial Sequence
<220>
   <223> Completely synthesized.
<400> 336
   Leu Thr Trp Ser Ser Gln Gly Ser Gly Val
   1               5                   10
<210> 337
   <211> 14
   <212> PRT
   <213> Artificial Sequence
<220>
   <223> Completely synthesized.
<400> 337
   Thr Leu Pro Ser Asp Ile Asn Val Arg Ser Tyr Asn Ile Tyr
   1               5                   10
<210> 338
   <211> 11
   <212> PRT
   <213> Artificial Sequence
<220>
   <223> Completely synthesized.
<400> 338
   Tyr Tyr Gln Asp Ser His Lys Gly Gln Gly Ser
   1               5                   10
<210> 339
   <211> 34
   <212> PRT
   <213> Artificial Sequence
<220>
   <223> Completely synthesized.
<400> 339
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 340
   <211> 10
   <212> PRT
   <213> Artificial Sequence
<220>
   <223> Completely synthesized.
<400> 340
   Leu Thr Trp Ser Ser Gln Gly Ser Gly Val
   1 5 10
<210> 341
   <211> 14
   <212> PRT
   <213> Artificial Sequence
<220>
   <223> Completely synthesized.
<400> 341
   Thr Leu Pro Ser Asp Ile Asn Val Gly Ser Tyr Asn Ile Tyr
   1 5 10
<210> 342
   <211> 11
   <212> PRT
   <213> Artificial Sequence
<220>
   <223> Completely synthesized.
<400> 342
   Tyr Tyr Glu Asp Ser His Lys Gly Gln Gly Ser
   1 5 10
<210> 343
   <211> 34
   <212> PRT
   <213> Artificial Sequence
<220>
   <223> Completely synthesized.
<400> 343
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 344
   <211> 10
   <212> PRT
   <213> Artificial Sequence
<220>
   <223> Completely synthesized.
<400> 344
   Leu Thr Trp Ser Ser Glu Gly Ser Gly Val
   1 5 10
<210> 345
   <211> 14
   <212> PRT
   <213> Artificial Sequence
<220>
   <223> Completely synthesized.
<400> 345
   Thr Leu Pro Ser Asp Ile Asn Val Arg Ser Tyr Asn Ile Tyr
   1 5 10
<210> 346
   <211> 11
   <212> PRT
   <213> Artificial Sequence
<220>
   <223> Completely synthesized.
<400> 346
   Tyr Tyr Glu Asp Ser His Lys Gly Gln Gly Ser
   1 5 10
<210> 347
   <211> 34
   <212> PRT
   <213> Artificial Sequence
<220>
   <223> Completely synthesized.
<400> 347
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 348
   <211> 10
   <212> PRT
   <213> Artificial Sequence
<220>
   <223> Completely synthesized.
<400> 348
   Leu Thr Trp Ser Ser Glu Gly Ser Gly Val
   1 5 10
<210> 349
   <211> 14
   <212> PRT
   <213> Artificial Sequence
<220>
   <223> Completely synthesized.
<400> 349
   Thr Leu Pro Ser Asp Ile Asn Val Gly Ser Tyr Asn Ile Tyr
   1 5 10
<210> 350
   <211> 11
   <212> PRT
   <213> Artificial Sequence
<220>
   <223> Completely synthesized.
<400> 350
   Tyr Tyr Glu Asp Ser His Lys Gly Gln Gly Ser
   1 5 10
<210> 351
   <211> 34
   <212> PRT
   <213> Artificial Sequence
<220>
   <223> Completely synthesized.
<400> 351
   Gly Val Pro Ser Arg Phe Ser Gly Ser Lys Asp Val Ser Thr Asn Ser
   1 5 10 15
   Gly Ile Leu Leu Ile Ser Gly Leu Gln Ser Glu Asp Ile Ala Thr Tyr
   20 25 30
   Tyr Cys
<210> 352
   <211> 10
   <212> PRT
   <213> Artificial Sequence
<220>
   <223> Completely synthesized.
<400> 352
   Leu Thr Trp Ser Ser Gln Gly Ser Gly Val
   1               5                   10
<210> 353
   <211> 14
   <212> PRT
   <213> Artificial Sequence
<220>
   <223> Completely synthesized.
<400> 353
   Thr Leu Pro Ser Asp Ile Asn Val Arg Ser Tyr Asn Ile Tyr
   1               5                   10
<210> 354
   <211> 11
   <212> PRT
   <213> Artificial Sequence
<220>
   <223> Completely synthesized.
<400> 354
   Tyr Tyr Glu Asp Ser His Lys Gly Gln Gly Ser
   1               5                   10
<210> 355
   <211> 34
   <212> PRT
   <213> Artificial Sequence
<220>
   <223> Completely synthesized.
<400> 355
   Gly Val Pro Ser Arg Phe Ser Gly Ser Lys Asp Val Ser Thr Asn Ser
   1               5                   10                  15
   Gly Ile Leu Leu Ile Ser Gly Leu Gln Ser Glu Asp Ile Ala Thr Tyr
               20                  25                  30
   Tyr Cys
<210> 356
   <211> 10
   <212> PRT
   <213> Artificial Sequence
<220>
   <223> Completely synthesized.
<400> 356
   Leu Thr Trp Ser Ser Gln Gly Ser Gly Val
   1               5                   10
<210> 357
   <211> 14
   <212> PRT
   <213> Artificial Sequence
<220>
   <223> Completely synthesized.
<400> 357
   Thr Leu Pro Ser Asp Ile Asn Val Gly Ser Tyr Asn Ile Tyr
   1               5                   10
<210> 358
   <211> 11
   <212> PRT
   <213> Artificial Sequence
<220>
   <223> Completely synthesized.
<400> 358
   Tyr Tyr Gln Asp Ser His Lys Gly Gln Gly Ser
   1               5                   10
<210> 359
   <211> 34
   <212> PRT
   <213> Artificial Sequence
<220>
   <223> Completely synthesized.
<400> 359
   Gly Val Pro Ser Arg Phe Ser Gly Ser Lys Asp Val Ser Thr Gln Ser
   1               5                   10                  15
   Gly Ile Leu Leu Ile Ser Gly Leu Gln Ser Glu Asp Ile Ala Thr Tyr
               20                  25                  30
   Tyr Cys
<210> 360
   <211> 10
   <212> PRT
   <213> Artificial Sequence
<220>
   <223> Completely synthesized.
<400> 360
   Leu Thr Trp Ser Ser Glu Gly Ser Gly Val
   1               5                   10
<210> 361
   <211> 14
   <212> PRT
   <213> Artificial Sequence
<220>
   <223> Completely synthesized.
<400> 361
   Thr Leu Pro Ser Asp Ile Asn Val Arg Ser Tyr Asn Ile Tyr
   1               5                   10
<210> 362
   <211> 11
   <212> PRT
   <213> Artificial Sequence
<220>
   <223> Completely synthesized.
<400> 362
   Tyr Tyr Gln Asp Ser His Lys Gly Gln Gly Ser
   1               5                   10
<210> 363
   <211> 34
   <212> PRT
   <213> Artificial Sequence
<220>
   <223> Completely synthesized.
<400> 363
   Gly Val Pro Ser Arg Phe Ser Gly Ser Lys Asp Val Ser Thr Gln Ser
   1               5                   10                  15
   Gly Ile Leu Leu Ile Ser Gly Leu Gln Ser Glu Asp Ile Ala Thr Tyr
               20                  25                  30
   Tyr Cys
<210> 364
   <211> 10
   <212> PRT
   <213> Artificial Sequence
<220>
   <223> Completely synthesized.
<400> 364
   Leu Thr Trp Ser Ser Glu Gly Ser Gly Val
   1 5 10
<210> 365
   <211> 14
   <212> PRT
   <213> Artificial Sequence
<220>
   <223> Completely synthesized.
<400> 365
   Thr Leu Pro Ser Asp Ile Asn Val Gly Ser Tyr Asn Ile Tyr
   1 5 10
<210> 366
   <211> 11
   <212> PRT
   <213> Artificial Sequence
<220>
   <223> Completely synthesized.
<400> 366
   Tyr Tyr Gln Asp Ser His Lys Gly Gln Gly Ser
   1 5 10
<210> 367
   <211> 34
   <212> PRT
   <213> Artificial Sequence
<220>
   <223> Completely synthesized.
<400> 367
   Gly Val Pro Ser Arg Phe Ser Gly Ser Lys Asp Val Ser Thr Gln Ser
   1 5 10 15
   Gly Ile Leu Leu Ile Ser Gly Leu Gln Ser Glu Asp Ile Ala Thr Tyr
   20 25 30
   Tyr Cys
<210> 368
   <211> 10
   <212> PRT
   <213> Artificial Sequence
<220>
   <223> Completely synthesized.
<400> 368
   Leu Thr Trp Ser Ser Gln Gly Ser Gly Val
   1 5 10
<210> 369
   <211> 14
   <212> PRT
   <213> Artificial Sequence
<220>
   <223> Completely synthesized.
<400> 369
   Thr Leu Pro Ser Asp Ile Asn Val Arg Ser Tyr Asn Ile Tyr
   1 5 10
<210> 370
   <211> 11
   <212> PRT
   <213> Artificial Sequence
<220>
   <223> Completely synthesized.
<400> 370
   Tyr Tyr Gln Asp Ser His Lys Gly Gln Gly Ser
   1 5 10
<210> 371
   <211> 34
   <212> PRT
   <213> Artificial Sequence
<220>
   <223> Completely synthesized.
<400> 371
   Gly Val Pro Ser Arg Phe Ser Gly Ser Lys Asp Val Ser Thr Gln Ser
   1 5 10 15
   Gly Ile Leu Leu Ile Ser Gly Leu Gln Ser Glu Asp Ile Ala Thr Tyr
   20 25 30
   Tyr Cys
<210> 372
   <211> 10
   <212> PRT
   <213> Artificial Sequence
<220>
   <223> Completely synthesized.
<400> 372
   Leu Thr Trp Ser Ser Gln Gly Ser Gly Val
   1               5                   10
<210> 373
   <211> 14
   <212> PRT
   <213> Artificial Sequence
<220>
   <223> Completely synthesized.
<400> 373
   Thr Leu Pro Ser Asp Ile Asn Val Gly Ser Tyr Asn Ile Tyr
   1               5                   10
<210> 374
   <211> 11
   <212> PRT
   <213> Artificial Sequence
<220>
   <223> Completely synthesized.
<400> 374
   Tyr Tyr Glu Asp Ser His Lys Gly Gln Gly Ser
   1               5                   10
<210> 375
   <211> 34
   <212> PRT
   <213> Artificial Sequence
<220>
   <223> Completely synthesized.
<400> 375
   Gly Val Pro Ser Arg Phe Ser Gly Ser Lys Asp Val Ser Thr Gln Ser
   1               5                   10                  15
   Gly Ile Leu Leu Ile Ser Gly Leu Gln Ser Glu Asp Ile Ala Thr Tyr
               20                  25                  30
   Tyr Cys
<210> 376
   <211> 10
   <212> PRT
   <213> Artificial Sequence
<220>
   <223> Completely synthesized.
<400> 376
   Leu Thr Trp Ser Ser Glu Gly Ser Gly Val
   1 5 10
<210> 377
   <211> 14
   <212> PRT
   <213> Artificial Sequence
<220>
   <223> Completely synthesized.
<400> 377
   Thr Leu Pro Ser Asp Ile Asn Val Arg Ser Tyr Asn Ile Tyr
   1 5 10
<210> 378
   <211> 11
   <212> PRT
   <213> Artificial Sequence
<220>
   <223> Completely synthesized.
<400> 378
   Tyr Tyr Glu Asp Ser His Lys Gly Gln Gly Ser
   1 5 10
<210> 379
   <211> 34
   <212> PRT
   <213> Artificial Sequence
<220>
   <223> Completely synthesized.
<400> 379
   Gly Val Pro Ser Arg Phe Ser Gly Ser Lys Asp Val Ser Thr Gln Ser
   1 5 10 15
   Gly Ile Leu Leu Ile Ser Gly Leu Gln Ser Glu Asp Ile Ala Thr Tyr
   20 25 30
   Tyr Cys
<210> 380
   <211> 10
   <212> PRT
   <213> Artificial Sequence
<220>
   <223> Completely synthesized.
<400> 380
   Leu Thr Trp Ser Ser Glu Gly Ser Gly Val
   1               5                   10
<210> 381
   <211> 14
   <212> PRT
   <213> Artificial Sequence
<220>
   <223> Completely synthesized.
<400> 381
   Thr Leu Pro Ser Asp Ile Asn Val Gly Ser Tyr Asn Ile Tyr
   1               5                   10
<210> 382
   <211> 11
   <212> PRT
   <213> Artificial Sequence
<220>
   <223> Completely synthesized.
<400> 382
   Tyr Tyr Glu Asp Ser His Lys Gly Gln Gly Ser
   1               5                   10
<210> 383
   <211> 34
   <212> PRT
   <213> Artificial Sequence
<220>
   <223> Completely synthesized.
<400> 383
   Gly Val Pro Ser Arg Phe Ser Gly Ser Lys Asp Val Ser Thr Gln Ser
   1               5                   10                  15
   Gly Ile Leu Leu Ile Ser Gly Leu Gln Ser Glu Asp Ile Ala Thr Tyr
               20                  25                  30
   Tyr Cys
<210> 384
   <211> 10
   <212> PRT
   <213> Artificial Sequence
<220>
   <223> Completely synthesized.
<400> 384
   Leu Thr Trp Ser Ser Gln Gly Ser Gly Val
   1               5                   10
<210> 385
   <211> 14
   <212> PRT
   <213> Artificial Sequence
<220>
   <223> Completely synthesized.
<400> 385
   Thr Leu Pro Ser Asp Ile Asn Val Arg Ser Tyr Asn Ile Tyr
   1               5                   10
<210> 386
   <211> 11
   <212> PRT
   <213> Artificial Sequence
<220>
   <223> Completely synthesized.
<400> 386
   Tyr Tyr Glu Asp Ser His Lys Gly Gln Gly Ser
   1               5                   10
<210> 387
   <211> 34
   <212> PRT
   <213> Artificial Sequence
<220>
   <223> Completely synthesized.
<400> 387
   Gly Val Pro Ser Arg Phe Ser Gly Ser Lys Asp Val Ser Thr Gln Ser
   1               5                   10                  15
   Gly Ile Leu Leu Ile Ser Gly Leu Gln Ser Glu Asp Ile Ala Thr Tyr
               20                  25                  30
   Tyr Cys
<210> 388
   <211> 10
   <212> PRT
   <213> Artificial Sequence
<220>
   <223> Completely synthesized.
<400> 388
   Leu Thr Trp Ser Ser Gln Gly Ser Gly Val
   1               5                   10
<210> 389
   <211> 30
   <212> PRT
   <213> Rattus norvegicus
<400> 389
   Glu Val Gln Leu Gln Gln Ser Gly Pro Glu Val Gly Arg Pro Gly Ser
   1               5                   10                  15
   Ser Val Lys Ile Ser Cys Lys Ala Ser Gly Tyr Thr Phe Thr
               20                  25                  30
<210> 390
   <211> 5
   <212> PRT
   <213> Rattus norvegicus
<400> 390
   Asp Tyr Ile Met His
   1               5
<210> 391
   <211> 14
   <212> PRT
   <213> Rattus norvegicus
<400> 391
   Trp Val Lys Gln Ser Pro Gly Gln Gly Leu Glu Trp Ile Gly
   1               5                   10
<210> 392
   <211> 17
   <212> PRT
   <213> Rattus norvegicus
<400> 392
   Trp Ile Asp Pro Glu Tyr Gly Arg Thr Asp Tyr Ala Glu Lys Phe Lys
   1               5                   10                  15
   Lys
<210> 393
   <211> 32
   <212> PRT
   <213> Rattus norvegicus
<400> 393
   Lys Ala Thr Leu Thr Ala Asp Thr Ser Ser Ser Thr Ala Tyr Ile Gln
   1               5                   10                  15
   Leu Ser Ser Leu Ala Ser Glu Asp Thr Ala Thr Tyr Phe Cys Ala Arg
               20                  25                  30
<210> 394
   <211> 11
   <212> PRT
   <213> Rattus norvegicus
<400> 394
   Ser Ala His Thr Thr Gly Phe Gly Phe Ala Tyr
   1               5                   10
<210> 395
   <211> 11
   <212> PRT
   <213> Rattus norvegicus
<400> 395
   Trp Gly Gln Gly Thr Leu Val Thr Val Ser Ser
   1               5                   10
<210> 396
   <211> 30
   <212> PRT
   <213> Artificial Sequence
<220>
   <223> Completely synthesized.
<400> 396
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr
               20                  25                  30
<210> 397
   <211> 14
   <212> PRT
   <213> Artificial Sequence
<220>
   <223> Completely synthesized.
<400> 397
   Trp Val Gln Gln Ala Pro Gly Lys Gly Leu Glu Trp Met Gly
   1 5 10
<210> 398
   <211> 17
   <212> PRT
   <213> Synthetic
<400> 398
   Trp Ile Asp Pro Glu Tyr Gly Arg Thr Asp Tyr Ala Glu Lys Phe Gln
   1 5 10 15
   Gly
<210> 399
   <211> 32
   <212> PRT
   <213> Artificial Sequence
<220>
   <223> Completely synthesized.
<400> 399
   Arg Val Thr Ile Thr Ala Asp Thr Ser Thr Asp Thr Ala Tyr Met Glu
   1 5 10 15
   Leu Ser Ser Leu Arg Ser Glu Asp Thr Ala Val Tyr Tyr Cys Ala Thr
   20 25 30
<210> 400
   <211> 30
   <212> PRT
   <213> Artificial Sequence
<220>
   <223> Completely synthesized.
<400> 400
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr
   20 25 30
<210> 401
   <211> 14
   <212> PRT
   <213> Artificial Sequence
<220>
   <223> Completely synthesized.
<400> 401
   Trp Val Gln Gln Ala Pro Gly Lys Gly Leu Glu Trp Met Gly
   1 5 10
<210> 402
   <211> 17
   <212> PRT
   <213> Artificial Sequence
<220>
   <223> Completely synthesized.
<400> 402
   Trp Ile Asp Pro Glu Tyr Gly Arg Thr Asp Tyr Ala Glu Lys Phe Gln
   1 5 10 15
   Gly
<210> 403
   <211> 32
   <212> PRT
   <213> Artificial Sequence
<220>
   <223> Completely synthesized.
<400> 403
   Arg Val Thr Ile Thr Ala Asp Thr Ser Thr Asp Thr Ala Tyr Met Glu
   1 5 10 15
   Leu Ser Ser Leu Arg Ser Glu Asp Thr Ala Val Tyr Tyr Cys Ala Arg
   20 25 30
<210> 404
   <211> 30
   <212> PRT
   <213> Artificial Sequence
<220>
   <223> Completely synthesized.
<400> 404
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr
               20                  25                  30
<210> 405
   <211> 14
   <212> PRT
   <213> Artificial Sequence
<220>
   <223> Completely synthesized.
<400> 405
   Trp Val Arg Gln Ala Pro Gly Gln Gly Leu Glu Trp Met Gly
   1               5                   10
<210> 406
   <211> 17
   <212> PRT
   <213> Artificial Sequence
<220>
   <223> Completely synthesized.
<400> 406
   Trp Ile Asp Pro Glu Tyr Gly Arg Thr Asp Tyr Ala Gln Lys Leu Gln
   1               5                   10                  15
   Gly
<210> 407
   <211> 32
   <212> PRT
   <213> Artificial Sequence
<220>
   <223> Completely synthesized.
<400> 407
   Arg Val Thr Met Thr Thr Asp Thr Ser Thr Ser Thr Ala Tyr Met Glu
   1               5                   10                  15
   Leu Arg Ser Leu Arg Ser Asp Asp Thr Ala Val Tyr Tyr Cys Ala Arg
               20                  25                  30
<210> 408
   <211> 30
   <212> PRT
   <213> Artificial Sequence
<220>
   <223> Completely synthesized.
<400> 408
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr
               20                  25                  30
<210> 409
   <211> 14
   <212> PRT
   <213> Artificial Sequence
<220>
   <223> Completely synthesized.
<400> 409
   Trp Val Arg Gln Ala Pro Gly Gln Gly Leu Glu Trp Met Gly
   1               5                   10
<210> 410
   <211> 17
   <212> PRT
   <213> Artificial Sequence
<220>
   <223> Completely synthesized.
<400> 410
   Trp Ile Asp Pro Glu Tyr Gly Arg Thr Asp Tyr Ala Gln Lys Leu Gln
   1               5                   10                  15
   Gly
<210> 411
   <211> 32
   <212> PRT
   <213> Artificial Sequence
<220>
   <223> Completely synthesized.
<400> 411
   Arg Val Thr Met Thr Ala Asp Thr Ser Thr Ser Thr Ala Tyr Met Glu
   1               5                   10                  15
   Leu Arg Ser Leu Arg Ser Asp Asp Thr Ala Val Tyr Tyr Cys Ala Arg
   20 25 30
<210> 412
   <211> 30
   <212> PRT
   <213> Artificial Sequence
<220>
   <223> Completely synthesized.
<400> 412
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr
   20 25 30
<210> 413
   <211> 14
   <212> PRT
   <213> Artificial Sequence
<220>
   <223> Completely synthesized.
<400> 413
   Trp Val Arg Gln Ala Pro Gly Lys Gly Leu Glu Trp Met Gly
   1 5 10
<210> 414
   <211> 17
   <212> PRT
   <213> Artificial Sequence
<220>
   <223> Completely synthesized.
<400> 414
   Trp Ile Asp Pro Glu Tyr Gly Arg Thr Asp Tyr Ala Gln Lys Phe Gln
   1 5 10 15
   Gly
<210> 415
   <211> 32
   <212> PRT
   <213> Artificial Sequence
<220>
   <223> Completely synthesized.
<400> 415
   Arg Val Thr Met Thr Glu Asp Thr Ser Thr Asp Thr Ala Tyr Met Glu
   1               5                   10                  15
   Leu Ser Ser Leu Arg Ser Glu Asp Thr Ala Val Tyr Tyr Cys Ala Thr
               20                  25                  30
<210> 416
   <211> 30
   <212> PRT
   <213> Artificial Sequence
<220>
   <223> Completely synthesized.
<400> 416
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr
               20                  25                  30
<210> 417
   <211> 14
   <212> PRT
   <213> Artificial Sequence
<220>
   <223> Completely synthesized.
<400> 417
   Trp Val Arg Gln Ala Pro Gly Lys Gly Leu Glu Trp Met Gly
   1               5                   10
<210> 418
   <211> 17
   <212> PRT
   <213> Artificial Sequence
<220>
   <223> Completely synthesized.
<400> 418
   Trp Ile Asp Pro Glu Tyr Gly Arg Thr Asp Tyr Ala Gln Lys Phe Gln
   1               5                   10                  15
   Gly
<210> 419
   <211> 32
   <212> PRT
   <213> Artificial Sequence
<220>
   <223> Completely synthesized.
<400> 419
   Arg Val Thr Met Thr Ala Asp Thr Ser Thr Asp Thr Ala Tyr Met Glu
   1               5                   10                  15
   Leu Ser Ser Leu Arg Ser Glu Asp Thr Ala Val Tyr Tyr Cys Ala Thr
               20                  25                  30
<210> 420
   <211> 30
   <212> PRT
   <213> Artificial Sequence
<220>
   <223> Completely synthesized.
<400> 420
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Phe Thr
               20                  25                  30
<210> 421
   <211> 14
   <212> PRT
   <213> Artificial Sequence
<220>
   <223> Completely synthesized.
<400> 421
   Trp Val Arg Gln Ala Pro Gly Lys Gly Leu Glu Trp Met Gly
   1               5                   10
<210> 422
   <211> 17
   <212> PRT
   <213> Artificial Sequence
<220>
   <223> Completely synthesized.
<400> 422
   Trp Ile Asp Pro Glu Tyr Gly Arg Thr Asp Tyr Ala Gln Lys Phe Gln
   1               5                   10                  15
   Gly
<210> 423
   <211> 32
   <212> PRT
   <213> Artificial Sequence
<220>
   <223> Completely synthesized.
<400> 423
   Arg Val Thr Met Thr Glu Asp Thr Ser Thr Asp Thr Ala Tyr Met Glu
   1 5 10 15
   Leu Ser Ser Leu Arg Ser Glu Asp Thr Ala Val Tyr Tyr Cys Ala Thr
   20 25 30
<210> 424
   <211> 30
   <212> PRT
   <213> Artificial Sequence
<220>
   <223> Completely synthesized.
<400> 424
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr
   20 25 30
<210> 425
   <211> 14
   <212> PRT
   <213> Artificial Sequence
<220>
   <223> Completely synthesized.
<400> 425
   Trp Val Arg Gln Ala Pro Gly Lys Gly Leu Glu Trp Met Gly
   1 5 10
<210> 426
   <211> 17
   <212> PRT
   <213> Artificial Sequence
<220>
   <223> Completely synthesized.
<400> 426
   Trp Ile Asp Pro Glu Tyr Gly Arg Thr Asp Tyr Ala Gln Lys Phe Gln
   1               5                   10                  15
   Gly
<210> 427
   <211> 32
   <212> PRT
   <213> Artificial Sequence
<220>
   <223> Completely synthesized.
<400> 427
   Arg Val Thr Met Thr Glu Asp Thr Ser Thr Asp Thr Ala Tyr Met Glu
   1               5                   10                  15
   Leu Ser Ser Leu Arg Ser Glu Asp Thr Ala Val Tyr Tyr Cys Ala Arg
               20                  25                  30
<210> 428
   <211> 30
   <212> PRT
   <213> Artificial Sequence
<220>
   <223> Completely synthesized.
<400> 428
   Gln Met Gln Leu Val Gln Ser Gly Ala Glu Val Lys Lys Thr Gly Ser
   1               5                   10                  15
   Ser Val Lys Val Ser Cys Lys Ala Ser Gly Tyr Thr Phe Thr
               20                  25                  30
<210> 429
   <211> 14
   <212> PRT
   <213> Artificial Sequence
<220>
   <223> Completely synthesized.
<400> 429
   Trp Val Arg Gln Ala Pro Gly Gln Ala Leu Glu Trp Met Gly
   1               5                   10
<210> 430
   <211> 17
   <212> PRT
   <213> Artificial Sequence
<220>
   <223> Completely synthesized.
<400> 430
   Trp Ile Asp Pro Glu Tyr Gly Arg Thr Asp Tyr Ala Gln Lys Phe Gln
   1               5                   10                  15
   Asp
<210> 431
   <211> 32
   <212> PRT
   <213> Artificial Sequence
<220>
   <223> Completely synthesized.
<400> 431
   Arg Val Thr Ile Thr Arg Asp Arg Ser Met Ser Thr Ala Tyr Met Glu
   1               5                   10                  15
   Leu Ser Ser Leu Arg Ser Glu Asp Thr Ala Met Tyr Tyr Cys Ala Arg
               20                  25                  30
<210> 432
   <211> 30
   <212> PRT
   <213> Artificial Sequence
<220>
   <223> Completely synthesized.
<400> 432
   Gln Met Gln Leu Val Gln Ser Gly Ala Glu Val Lys Lys Thr Gly Ser
   1               5                   10                  15
   Ser Val Lys Val Ser Cys Lys Ala Ser Gly Tyr Thr Phe Thr
               20                  25                  30
<210> 433
   <211> 14
   <212> PRT
   <213> Artificial Sequence
<220>
   <223> Completely synthesized.
<400> 433
   Trp Val Arg Gln Ala Pro Gly Gln Ala Leu Glu Trp Met Gly
   1               5                   10
<210> 434
   <211> 17
   <212> PRT
   <213> Artificial Sequence
<220>
   <223> Completely synthesized.
<400> 434
   Trp Ile Asp Pro Glu Tyr Gly Arg Thr Asp Tyr Ala Gln Lys Phe Gln
   1               5                   10                  15
   Asp
<210> 435
   <211> 32
   <212> PRT
   <213> Artificial Sequence
<220>
   <223> Completely synthesized.
<400> 435
   Arg Val Thr Ile Thr Ala Asp Arg Ser Met Ser Thr Ala Tyr Met Glu
   1               5                   10                  15
   Leu Ser Ser Leu Arg Ser Glu Asp Thr Ala Met Tyr Tyr Cys Ala Arg
               20                  25                  30
<210> 436
   <211> 23
   <212> PRT
   <213> Artificial Sequence
<220>
   <223> Completely synthesized.
<400> 436
   Asp Ile Gln Met Thr Gln Ser Pro Ala Ser Leu Ser Ala Ser Leu Gly
   1               5                   10                  15
   Glu Thr Val Thr Ile Glu Cys
               20
<210> 437
   <211> 11
   <212> PRT
   <213> Artificial Sequence
<220>
   <223> Completely synthesized.
<400> 437
   Arg Ala Ser Glu Asp Ile Tyr Ser Asn Leu Ala
   1               5                   10
<210> 438
   <211> 15
   <212> PRT
   <213> Artificial Sequence
<220>
   <223> Completely synthesized.
<400> 438
   Trp Tyr Gln Gln Lys Pro Gly Asn Ser Pro Gln Leu Leu Ile Tyr
   1               5                   10                  15
<210> 439
   <211> 7
   <212> PRT
   <213> Artificial Sequence
<220>
   <223> Completely synthesized.
<400> 439
   Asp Ala Asn Ser Leu Ala Asp
   1               5
<210> 440
   <211> 32
   <212> PRT
   <213> Artificial Sequence
<220>
   <223> Completely synthesized.
<400> 440
   Gly Val Pro Ser Arg Phe Ser Ala Ser Gly Ser Gly Thr Gln Phe Ser
   1               5                   10                  15
   Leu Lys Ile Asn Ser Leu Arg Ser Glu Asp Val Ala Ser Tyr Phe Cys
               20                  25                  30
<210> 441
   <211> 9
   <212> PRT
   <213> Artificial Sequence
<220>
   <223> Completely synthesized.
<400> 441
   Gln Gln Tyr Asn Asn Tyr Pro Tyr Thr
   1 5
<210> 442
   <211> 11
   <212> PRT
   <213> Artificial Sequence
<220>
   <223> Completely synthesized.
<400> 442
   Phe Gly Ala Gly Thr Lys Leu Glu Leu Lys Arg
   1 5 10
<210> 443
   <211> 23
   <212> PRT
   <213> Artificial Sequence
<220>
   <223> Completely synthesized.
<400> 443
   Asp Ile Gln Met Thr Gln Ser Pro Ser Thr Leu Ser Ala Ser Val Gly
   1 5 10 15
   Asp Arg Val Thr Ile Thr Cys
   20
<210> 444
   <211> 15
   <212> PRT
   <213> Artificial Sequence
<220>
   <223> Completely synthesized.
<400> 444
   Trp Tyr Gln Gln Lys Pro Gly Lys Ala Pro Lys Leu Leu Ile Tyr
   1 5 10 15
<210> 445
   <211> 32
   <212> PRT
   <213> Artificial Sequence
<220>
   <223> Completely synthesized.
<400> 445
   Gly Val Pro Ser Arg Phe Ser Gly Ser Gly Ser Gly Thr Glu Phe Thr
   1               5                   10                  15
   Leu Thr Ile Ser Ser Leu Gln Pro Asp Asp Phe Ala Thr Tyr Tyr Cys
   20                  25                  30
<210> 446
   <211> 11
   <212> PRT
   <213> Artificial Sequence
<220>
   <223> Completely synthesized.
<400> 446
   Phe Gly Gln Gly Thr Lys Leu Glu Ile Lys Arg
   1               5                   10
<210> 447
   <211> 23
   <212> PRT
   <213> Artificial Sequence
<220>
   <223> Completely synthesized.
<400> 447
   Asp Ile Gln Leu Thr Gln Ser Pro Ser Phe Leu Ser Ala Ser Val Gly
   1               5                   10                  15
   Asp Arg Val Thr Ile Thr Cys
               20
<210> 448
   <211> 15
   <212> PRT
   <213> Artificial Sequence
<220>
   <223> Completely synthesized.
<400> 448
   Trp Tyr Gln Gln Lys Pro Gly Lys Ala Pro Lys Leu Leu Ile Tyr
   1               5                   10                  15
<210> 449
   <211> 32
   <212> PRT
   <213> Artificial Sequence
<220>
   <223> Completely synthesized.
<400> 449
   Gly Val Pro Ser Arg Phe Ser Gly Ser Gly Ser Gly Thr Glu Phe Thr
   1 5 10 15
   Leu Thr Ile Ser Ser Leu Gln Pro Glu Asp Phe Ala Thr Tyr Tyr Cys
   20 25 30
<210> 450
   <211> 11
   <212> PRT
   <213> Artificial Sequence
<220>
   <223> Completely synthesized.
<400> 450
   Phe Gly Gln Gly Thr Lys Leu Glu Ile Lys Arg
   1 5 10
<210> 451
   <211> 23
   <212> PRT
   <213> Artificial Sequence
<220>
   <223> Completely synthesized.
<400> 451
   Asp Ile Gln Met Thr Gln Ser Pro Ser Ser Val Ser Ala Ser Val Gly
   1 5 10 15
   Asp Arg Val Thr Ile Thr Cys
   20
<210> 452
   <211> 15
   <212> PRT
   <213> Artificial Sequence
<220>
   <223> Completely synthesized.
<400> 452
   Trp Tyr Gln Gln Lys Pro Gly Lys Ala Pro Lys Leu Leu Ile Tyr
   1 5 10 15
<210> 453
   <211> 32
   <212> PRT
   <213> Artificial Sequence
<220>
   <223> Completely synthesized.
<400> 453
   Gly Val Pro Ser Arg Phe Ser Gly Ser Gly Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 454
   <211> 11
   <212> PRT
   <213> Artificial Sequence
<220>
   <223> Completely synthesized.
<400> 454
   Phe Gly Gln Gly Thr Lys Leu Glu Ile Lys Arg
   1               5                   10
<210> 455
   <211> 23
   <212> PRT
   <213> Artificial Sequence
<220>
   <223> Completely synthesized.
<400> 455
   Ala Ile Gln Leu Thr Gln Ser Pro Ser Ser Leu Ser Ala Ser Val Gly
   1               5                   10                  15
   Asp Arg Val Thr Ile Thr Cys
               20
<210> 456
   <211> 15
   <212> PRT
   <213> Artificial Sequence
<220>
   <223> Completely synthesized.
<400> 456
   Trp Tyr Gln Gln Lys Pro Gly Lys Ala Pro Lys Leu Leu Ile Tyr
   1               5                   10                  15
<210> 457
   <211> 32
   <212> PRT
   <213> Artificial Sequence
<220>
   <223> Completely synthesized.
<400> 457
   Gly Val Pro Ser Arg Phe Ser Gly Ser Gly Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 458
   <211> 11
   <212> PRT
   <213> Artificial Sequence
<220>
   <223> Completely synthesized.
<400> 458
   Phe Gly Gln Gly Thr Lys Leu Glu Ile Lys Arg
   1               5                   10
<210> 459
   <211> 23
   <212> PRT
   <213> Artificial Sequence
<220>
   <223> Completely synthesized.
<400> 459
   Asp Ile Gln Met Thr Gln Ser Pro Ser Ser Leu Ser Ala Ser Val Gly
   1               5                   10                  15
   Glu Asp Arg Thr Ile Thr Cys
               20
<210> 460
   <211> 15
   <212> PRT
   <213> Artificial Sequence
<220>
   <223> Completely synthesized.
<400> 460
   Trp Tyr Gln Gln Lys Pro Glu Lys Ala Pro Lys Ser Leu Ile Tyr
   1               5                   10                  15
<210> 461
   <211> 32
   <212> PRT
   <213> Artificial Sequence
<220>
   <223> Completely synthesized.
<400> 461
   Gly Val Pro Ser Arg Phe Ser Gly Ser Gly Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 462
   <211> 11
   <212> PRT
   <213> Artificial Sequence
<220>
   <223> Completely synthesized.
<400> 462
   Phe Gly Gln Gly Thr Lys Leu Glu Ile Lys Arg
   1               5                   10
<210> 463
   <211> 23
   <212> PRT
   <213> Artificial Sequence
<220>
   <223> Completely synthesized.
<400> 463
   Glu Ile Val Met Thr Gln Ser Pro Ala Thr Leu Ser Val Ser Pro Gly
   1               5                   10                  15
   Glu Arg Ala Thr Leu Ser Cys
               20
<210> 464
   <211> 15
   <212> PRT
   <213> Artificial Sequence
<220>
   <223> Completely synthesized.
<400> 464
   Trp Tyr Gln Gln Lys Pro Gly Gln Ala Pro Arg Leu Leu Ile Tyr
   1               5                   10                  15
<210> 465
   <211> 32
   <212> PRT
   <213> Artificial Sequence
<220>
   <223> Completely synthesized.
<400> 465
   Gly Ile Pro Ala Arg Phe Ser Gly Ser Gly Ser Gly Thr Glu Phe Thr
   1               5                   10                  15
   Leu Thr Ile Ser Ser Leu Gln Ser Glu Asp Phe Ala Val Tyr Tyr Cys
               20                  25                  30
<210> 466
   <211> 30
   <212> PRT
   <213> Rattus norvegicus
<400> 466
   Glu Val Gln Leu Gln Gln Ser Gly Pro Glu Val Gly Arg Pro Gly Ser
   1               5                   10                  15
   Ser Val Lys Ile Ser Cys Lys Ala Ser Gly Tyr Ile Phe Thr
               20                  25                  30
<210> 467
   <211> 17
   <212> PRT
   <213> Rattus norvegicus
<400> 467
   Trp Ile Asp Pro Glu Tyr Gly Ser Thr Asp Tyr Ala Glu Lys Phe Lys
   1               5                   10                  15
   Lys
<210> 468
   <211> 32
   <212> PRT
   <213> Rattus norvegicus
<400> 468
   Lys Ala Thr Leu Thr Ala Asp Thr Ser Ser Ser Thr Ala Tyr Ile Gln
   1               5                   10                  15
   Leu Ser Ser Leu Thr Ser Glu Asp Thr Ala Thr Tyr Phe Cys Ala Arg
               20                  25                  30
<210> 469
   <211> 14
   <212> PRT
   <213> Rattus norvegicus
<400> 469
   Val Ala Ile Ile Thr Thr Val Ala Ser Gly Gly Phe Ala Tyr
   1               5                   10
<210> 470
   <211> 30
   <212> PRT
   <213> Artificial Sequence
<220>
   <223> Completely synthesized.
<400> 470
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr
               20                  25                  30
<210> 471
   <211> 17
   <212> PRT
   <213> Artificial Sequence
<220>
   <223> Completely synthesized.
<400> 471
   Trp Ile Asp Pro Glu Tyr Gly Ser Thr Asp Tyr Ala Glu Lys Phe Gln
   1               5                   10                  15
   Gly
<210> 472
   <211> 30
   <212> PRT
   <213> Artificial Sequence
<220>
   <223> Completely synthesized.
<400> 472
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Ile Phe Thr
               20                  25                  30
<210> 473
   <211> 17
   <212> PRT
   <213> Artificial Sequence
<220>
   <223> Completely synthesized.
<400> 473
   Trp Ile Asp Pro Glu Tyr Gly Ser Thr Asp Tyr Ala Glu Lys Phe Gln
   1               5                   10                  15
   Gly
<210> 474
   <211> 30
   <212> PRT
   <213> Artificial Sequence
<220>
   <223> Completely synthesized.
<400> 474
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr
               20                  25                  30
<210> 475
   <211> 17
   <212> PRT
   <213> Artificial Sequence
<220>
   <223> Completely synthesized.
<400> 475
   Trp Ile Asp Pro Glu Tyr Gly Ser Thr Asp Tyr Ala Glu Lys Phe Gln
   1               5                   10                  15
   Gly
<210> 476
   <211> 30
   <212> PRT
   <213> Artificial Sequence
<220>
   <223> Completely synthesized.
<400> 476
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr
               20                  25                  30
<210> 477
   <211> 17
   <212> PRT
   <213> Synthetic
<400> 477
   Trp Ile Asp Pro Glu Tyr Gly Ser Thr Asp Tyr Ala Gln Lys Leu Gln
   1               5                   10                  15
   Gly
<210> 478
   <211> 30
   <212> PRT
   <213> Synthetic
<400> 478
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr
               20                  25                  30
<210> 479
   <211> 17
   <212> PRT
   <213> Artificial Sequence
<220>
   <223> Completely synthesized.
<400> 479
   Trp Ile Asp Pro Glu Tyr Gly Ser Thr Asp Tyr Ala Gln Lys Leu Gln
   1               5                   10                  15
   Gly
<210> 480
   <211> 30
   <212> PRT
   <213> Artificial Sequence
<220>
   <223> Completely synthesized.
<400> 480
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr
               20                  25                  30
<210> 481
   <211> 17
   <212> PRT
   <213> Artificial Sequence
<220>
   <223> Completely synthesized.
<400> 481
   Trp Ile Asp Pro Glu Tyr Gly Ser Thr Asp Tyr Ala Gln Lys Phe Gln
   1               5                   10                  15
   Gly
<210> 482
   <211> 30
   <212> PRT
   <213> Artificial Sequence
<220>
   <223> Completely synthesized.
<400> 482
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr
               20                  25                  30
<210> 483
   <211> 17
   <212> PRT
   <213> Synthetic
<400> 483
   Trp Ile Asp Pro Glu Tyr Gly Ser Thr Asp Tyr Ala Gln Lys Phe Gln
   1               5                   10                  15
   Gly
<210> 484
   <211> 30
   <212> PRT
   <213> Artificial Sequence
<220>
   <223> Completely synthesized.
<400> 484
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr
   20 25 30
<210> 485
   <211> 17
   <212> PRT
   <213> Artificial Sequence
<220>
   <223> Completely synthesized.
<400> 485
   Trp Ile Asp Pro Glu Tyr Gly Ser Thr Asp Tyr Ala Gln Lys Phe Gln
   1 5 10 15
   Gly
<210> 486
   <211> 30
   <212> PRT
   <213> Artificial Sequence
<220>
   <223> Completely synthesized.
<400> 486
   Gln Met Gln Leu Val Gln Ser Gly Ala Glu Val Lys Lys Thr Gly Ser
   1 5 10 15
   Ser Val Lys Val Ser Cys Lys Ala Ser Gly Tyr Thr Phe Thr
   20 25 30
<210> 487
   <211> 17
   <212> PRT
   <213> Artificial Sequence
<220>
   <223> Completely synthesized.
<400> 487
   Trp Ile Asp Pro Glu Tyr Gly Ser Thr Asp Tyr Ala Gln Lys Phe Gln
   1 5 10 15
   Asp
<210> 488
   <211> 30
   <212> PRT
   <213> Artificial Sequence
<220>
   <223> Completely synthesized.
<400> 488
   Gln Met Gln Leu Val Gln Ser Gly Ala Glu Val Lys Lys Thr Gly Ser
   1 5 10 15
   Ser Val Lys Val Ser Cys Lys Ala Ser Gly Tyr Thr Phe Thr
   20 25 30
<210> 489
   <211> 17
   <212> PRT
   <213> Artificial Sequence
<220>
   <223> Completely synthesized.
<400> 489
   Trp Ile Asp Pro Glu Tyr Gly Ser Thr Asp Tyr Ala Gln Lys Phe Gln
   1 5 10 15
   Asp
<210> 490
   <211> 23
   <212> PRT
   <213> Rattus norvegicus
<400> 490
   Asp Ile Val Met Thr Gln Gly Ala Leu Pro Asn Pro Val Pro Ser Gly
   1 5 10 15
   Glu Ser Ala Ser Ile Thr Cys
   20
<210> 491
   <211> 16
   <212> PRT
   <213> Rattus norvegicus
<400> 491
   Gln Ser Ser Glu Ser Leu Leu His Ser Asn Gly Lys Thr Tyr Leu Asn
   1               5                   10                  15
<210> 492
   <211> 15
   <212> PRT
   <213> Rattus norvegicus
<400> 492
   Trp Tyr Leu Gln Arg Pro Gly Gln Ser Pro Gln Leu Leu Ile Tyr
   1               5                   10                  15
<210> 493
   <211> 7
   <212> PRT
   <213> Rattus norvegicus
<400> 493
   Trp Met Ser Thr Arg Ala Ala
   1               5
<210> 494
   <211> 32
   <212> PRT
   <213> Rattus norvegicus
<400> 494
   Gly Val Ser Asp Arg Phe Ser Gly Ser Gly Ser Gly Thr Asp Phe Thr
   1               5                   10                  15
   Leu Thr Ile Ser Gly Val Glu Ala Glu Asp Val Gly Val Tyr Tyr Cys
               20                  25                  30
<210> 495
   <211> 9
   <212> PRT
   <213> Rattus norvegicus
<400> 495
   Gln Gln Phe Leu Glu Tyr Pro Pro Thr
   1               5
<210> 496
   <211> 11
   <212> PRT
   <213> Rattus norvegicus
<400> 496
   Phe Gly Ser Gly Thr Lys Leu Glu Ile Lys Arg
   1               5                   10
<210> 497
   <211> 23
   <212> PRT
   <213> Artificial Sequence
<220>
   <223> Completely synthesized.
<400> 497
   Asp Ile Val Met Thr Gln Thr Pro Leu Ser Ser Pro Val Thr Leu Gly
   1               5                   10                  15
   Gln Pro Ala Ser Ile Ser Cys
               20
<210> 498
   <211> 15
   <212> PRT
   <213> Artificial Sequence
<220>
   <223> Completely synthesized.
<400> 498
   Trp Leu Gln Gln Arg Pro Gly Gln Pro Pro Arg Leu Leu Ile Tyr
   1               5                   10                  15
<210> 499
   <211> 32
   <212> PRT
   <213> Artificial Sequence
<220>
   <223> Completely synthesized.
<400> 499
   Gly Val Pro Asp Arg Phe Ser Gly Ser Gly Ala Gly Thr Asp Phe Thr
   1               5                   10                  15
   Leu Lys Ile Ser Arg Val Glu Ala Glu Asp Val Gly Val Tyr Tyr Cys
               20                  25                  30
<210> 500
   <211> 11
   <212> PRT
   <213> Artificial Sequence
<220>
   <223> Completely synthesized.
<400> 500
   Phe Gly Gln Gly Thr Lys Leu Glu Ile Lys Arg
   1 5 10
<210> 501
   <211> 23
   <212> PRT
   <213> Artificial Sequence
<220>
   <223> Completely synthesized.
<400> 501
   Asp Ile Val Met Thr Gln Ser Pro Leu Ser Leu Pro Val Thr Pro Gly
   1 5 10 15
   Glu Pro Ala Ser Ile Ser Cys
   20
<210> 502
   <211> 15
   <212> PRT
   <213> Artificial Sequence
<220>
   <223> Completely synthesized.
<400> 502
   Trp Tyr Leu Gln Lys Pro Gly Gln Ser Pro Gln Leu Leu Ile Tyr
   1 5 10 15
<210> 503
   <211> 32
   <212> PRT
   <213> Artificial Sequence
<220>
   <223> Completely synthesized.
<400> 503
   Gly Val Pro Asp Arg Phe Ser Gly Ser Gly Ser Gly Thr Asp Phe Thr
   1 5 10 15
   Leu Lys Ile Ser Arg Val Glu Ala Glu Asp Val Gly Val Tyr Tyr Cys
   20 25 30
<210> 504
   <211> 11
   <212> PRT
   <213> Artificial Sequence
<220>
   <223> Completely synthesized.
<400> 504
   Phe Gly Gln Gly Thr Lys Leu Glu Ile Lys Arg
   1 5 10
<210> 505
   <211> 23
   <212> PRT
   <213> Artificial Sequence
<220>
   <223> Completely synthesized.
<400> 505
   Asp Ile Val Met Thr Gln Thr Pro Leu Ser Leu Ser Val Thr Pro Gly
   1 5 10 15
   Gln Pro Ala Ser Ile Ser Cys
   20
<210> 506
   <211> 15
   <212> PRT
   <213> Artificial Sequence
<220>
   <223> Completely synthesized.
<400> 506
   Trp Tyr Leu Gln Lys Pro Gly Gln Pro Pro Gln Leu Leu Ile Tyr
   1 5 10 15
<210> 507
   <211> 32
   <212> PRT
   <213> Artificial Sequence
<220>
   <223> Completely synthesized.
<400> 507
   Gly Val Pro Asp Arg Phe Ser Gly Ser Gly Ser Gly Thr Asp Phe Thr
   1 5 10 15
   Leu Lys Ile Ser Arg Val Glu Ala Glu Asp Val Gly Val Tyr Tyr Cys
   20 25 30
<210> 508
   <211> 11
   <212> PRT
   <213> Artificial Sequence
<220>
   <223> Completely synthesized.
<400> 508
   Phe Gly Gln Gly Thr Lys Leu Glu Ile Lys Arg
   1 5 10
<210> 509
   <211> 23
   <212> PRT
   <213> Artificial Sequence
<220>
   <223> Completely synthesized.
<400> 509
   Asp Val Val Met Thr Gln Ser Pro Leu Ser Leu Pro Val Thr Leu Gly
   1 5 10 15
   Gln Pro Ala Ser Ile Ser Cys
   20
<210> 510
   <211> 15
   <212> PRT
   <213> Artificial Sequence
<220>
   <223> Completely synthesized.
<400> 510
   Trp Phe Gln Gln Arg Pro Gly Gln Ser Pro Arg Arg Leu Ile Tyr
   1 5 10 15
<210> 511
   <211> 32
   <212> PRT
   <213> Artificial Sequence
<220>
   <223> Completely synthesized.
<400> 511
   Gly Val Pro Asp Arg Phe Ser Gly Ser Gly Ser Gly Thr Asp Phe Thr
   1 5 10 15
   Leu Lys Ile Ser Arg Val Glu Ala Glu Asp Val Gly Val Tyr Tyr Cys
   20 25 30
<210> 512
   <211> 11
   <212> PRT
   <213> Artificial Sequence
<220>
   <223> Completely synthesized.
<400> 512
   Phe Gly Gln Gly Thr Lys Leu Glu Ile Lys Arg
   1               5                   10
<210> 513
   <211> 30
   <212> PRT
   <213> Rattus norvegicus
<400> 513
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr
               20                  25                  30
<210> 514
   <211> 5
   <212> PRT
   <213> Rattus norvegicus
<400> 514
   Asp Ser Val Met Asn
   1               5
<210> 515
   <211> 14
   <212> PRT
   <213> Rattus norvegicus
<400> 515
   Trp Val Gln Gln Ala Pro Gly Lys Gly Leu Glu Trp Met Gly
   1               5                   10
<210> 516
   <211> 17
   <212> PRT
   <213> Rattus norvegicus
<400> 516
   Trp Ile Asp Pro Glu Tyr Gly Arg Thr Asp Val Ala Glu Lys Phe Gln
   1               5                   10                  15
   Gly
<210> 517
   <211> 32
   <212> PRT
   <213> Rattus norvegicus
<400> 517
   Arg Val Thr Ile Thr Ala Asp Thr Ser Thr Asp Thr Ala Tyr Met Glu
   1               5                   10                  15
   Leu Ser Ser Leu Arg Ser Glu Asp Thr Ala Val Tyr Tyr Cys Ala Arg
               20                  25                  30
<210> 518
   <211> 11
   <212> PRT
   <213> Rattus norvegicus
<400> 518
   Thr Lys Tyr Asn Ser Gly Tyr Gly Phe Pro Tyr
   1               5                   10
<210> 519
   <211> 11
   <212> PRT
   <213> Rattus norvegicus
<400> 519
   Trp Gly Gln Gly Thr Thr Val Thr Val Ser Ser
   1               5                   10
<210> 520
   <211> 14
   <212> PRT
   <213> Artificial Sequence
<220>
   <223> Completely synthesized.
<400> 520
   Trp Val Gln Gln Glu Pro Gly Lys Gly Leu Glu Trp Met Gly
   1               5                   10
<210> 521
   <211> 14
   <212> PRT
   <213> Artificial Sequence
<220>
   <223> Completely synthesized.
<400> 521
   Trp Val Gln Gln Gly Pro Gly Lys Gly Leu Glu Trp Met Gly
   1               5                   10
<210> 522
   <211> 14
   <212> PRT
   <213> Artificial Sequence
<220>
   <223> Completely synthesized.
<400> 522
   Trp Val Gln Gln His Pro Gly Lys Gly Leu Glu Trp Met Gly
   1               5                   10
<210> 523
   <211> 14
   <212> PRT
   <213> Artificial Sequence
<220>
   <223> Completely synthesized.
<400> 523
   Trp Val Gln Gln Gln Pro Gly Lys Gly Leu Glu Trp Met Gly
   1               5                   10
<210> 524
   <211> 14
   <212> PRT
   <213> Artificial Sequence
<220>
   <223> Completely synthesized.
<400> 524
   Trp Val Gln Gln Ser Pro Gly Lys Gly Leu Glu Trp Met Gly
   1               5                   10
<210> 525
   <211> 14
   <212> PRT
   <213> Artificial Sequence
<220>
   <223> Completely synthesized.
<400> 525
   Trp Val Gln Gln Val Pro Gly Lys Gly Leu Glu Trp Met Gly
   1               5                   10
<210> 526
   <211> 5
   <212> PRT
   <213> Artificial Sequence
<220>
   <223> Completely synthesized.
<400> 526
   Asp Ser Val Met Glu
   1 5
<210> 527
   <211> 5
   <212> PRT
   <213> Artificial Sequence
<220>
   <223> Completely synthesized.
<400> 527
   Asp Ser Val Met Pro
   1 5
<210> 528
   <211> 5
   <212> PRT
   <213> Artificial Sequence
<220>
   <223> Completely synthesized.
<400> 528
   Asp Ser Val Met Gln
   1 5
<210> 529
   <211> 5
   <212> PRT
   <213> Artificial Sequence
<220>
   <223> Completely synthesized.
<400> 529
   Asp Ser Val Met Ser
   1 5
<210> 530
   <211> 32
   <212> PRT
   <213> Artificial Sequence
<220>
   <223> Completely synthesized.
<400> 530
   Arg Val Thr Ile Thr Ala Asp Thr Ser Thr Asp Thr Ala Tyr Met Glu
   1 5 10 15
   Leu Ser Ser Leu Arg Ser Glu Asp Thr Ala Val Tyr Tyr Cys Ala Glu
   20 25 30
<210> 531
   <211> 32
   <212> PRT
   <213> Artificial Sequence
<220>
   <223> Completely synthesized.
<400> 531
   Arg Val Thr Ile Thr Ala Asp Thr Ser Thr Asp Thr Ala Tyr Met Glu
   1 5 10 15
   Leu Ser Ser Leu Arg Ser Glu Asp Thr Ala Val Tyr Tyr Cys Ala Gly
   20 25 30
<210> 532
   <211> 32
   <212> PRT
   <213> Artificial Sequence
<220>
   <223> Completely synthesized.
<400> 532
   Arg Val Thr Ile Thr Ala Asp Thr Ser Thr Asp Thr Ala Tyr Met Glu
   1 5 10 15
   Leu Ser Ser Leu Arg Ser Glu Asp Thr Ala Val Tyr Tyr Cys Ala Pro
   20 25 30
<210> 533
   <211> 32
   <212> PRT
   <213> Artificial Sequence
<220>
   <223> Completely synthesized.
<400> 533
   Arg Val Thr Ile Thr Ala Asp Thr Ser Thr Asp Thr Ala Tyr Met Glu
   1 5 10 15
   Leu Ser Ser Leu Arg Ser Glu Asp Thr Ala Val Tyr Tyr Cys Ala Thr
   20 25 30
<210> 534
   <211> 11
   <212> PRT
   <213> Artificial Sequence
<220>
   <223> Completely synthesized.
<400> 534
   Thr Gly Tyr Asn Ser Gly Tyr Gly Phe Pro Tyr
   1               5                   10
<210> 535
   <211> 11
   <212> PRT
   <213> Artificial Sequence
<220>
   <223> Completely synthesized.
<400> 535
   Thr Thr Tyr Asn Ser Gly Tyr Gly Phe Pro Tyr
   1               5                   10
<210> 536
   <211> 11
   <212> PRT
   <213> Artificial Sequence
<220>
   <223> Completely synthesized.
<400> 536
   Thr Lys Tyr Gln Ser Gly Tyr Gly Phe Pro Tyr
   1               5                   10
<210> 537
   <211> 30
   <212> PRT
   <213> Artificial Sequence
<220>
   <223> Completely synthesized.
<400> 537
   Glu Val Gln Leu Gln Gln Ser Gly Pro Glu Val Gly Arg Pro Gly Ser
   1               5                   10                  15
   Ser Val Lys Ile Ser Cys Lys Ala Ser Gly Tyr Thr Phe Thr
               20                  25                  30
<210> 538
   <211> 14
   <212> PRT
   <213> Synthetic
<400> 538
   Trp Val Lys Gln Ser Pro Gly Gln Gly Leu Glu Trp Ile Gly
   1 5 10
<210> 539
   <211> 17
   <212> PRT
   <213> Artificial Sequence
<220>
   <223> Completely synthesized.
<400> 539
   Trp Ile Asp Pro Glu Tyr Gly Arg Thr Asp Val Ala Glu Lys Phe Lys
   1 5 10 15
   Lys
<210> 540
   <211> 32
   <212> PRT
   <213> Artificial Sequence
<220>
   <223> Completely synthesized.
<400> 540
   Lys Ala Thr Leu Thr Ala Asp Ser Ser Ser Ser Thr Ala Tyr Ile Tyr
   1 5 10 15
   Leu Ser Gly Leu Thr Ser Glu Asp Thr Ala Thr Tyr Phe Cys Ala Arg
   20 25 30
<210> 541
   <211> 11
   <212> PRT
   <213> Artificial Sequence
<220>
   <223> Completely synthesized.
<400> 541
   Trp Gly Gln Gly Ser Leu Val Thr Val Ser Ser
   1 5 10
<210> 542
   <211> 30
   <212> PRT
   <213> Artificial Sequence
<220>
   <223> Completely synthesized.
<400> 542
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr
               20                  25                  30
<210> 543
   <211> 14
   <212> PRT
   <213> Artificial Sequence
<220>
   <223> Completely synthesized.
<400> 543
   Trp Val Arg Gln Ala Pro Gly Lys Gly Leu Glu Trp Met Gly
   1               5                   10
<210> 544
   <211> 17
   <212> PRT
   <213> Artificial Sequence
<220>
   <223> Completely synthesized.
<400> 544
   Trp Ile Asp Pro Glu Tyr Gly Arg Thr Asp Val Ala Gln Lys Phe Gln
   1               5                   10                  15
   Gly
<210> 545
   <211> 33
   <212> PRT
   <213> Artificial Sequence
<220>
   <223> Completely synthesized.
<400> 545
   Gly Arg Val Thr Met Thr Glu Asp Thr Ser Thr Asp Thr Ala Tyr Met
   1 5 10 15
   Glu Leu Ser Ser Leu Arg Ser Glu Asp Thr Ala Val Tyr Tyr Cys Ala
   20 25 30
   Thr
<210> 546
   <211> 11
   <212> PRT
   <213> Artificial Sequence
<220>
   <223> Completely synthesized.
<400> 546
   Trp Gly Gln Gly Thr Thr Val Thr Val Ser Ser
   1 5 10
<210> 547
   <211> 30
   <212> PRT
   <213> Artificial Sequence
<220>
   <223> Completely synthesized.
<400> 547
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr
   20 25 30
<210> 548
   <211> 14
   <212> PRT
   <213> Artificial Sequence
<220>
   <223> Completely synthesized.
<400> 548
   Trp Val Arg Gln Ala Pro Gly Lys Gly Leu Glu Trp Met Gly
   1 5 10
<210> 549
   <211> 17
   <212> PRT
   <213> Artificial Sequence
<220>
   <223> Completely synthesized.
<400> 549
   Trp Ile Asp Pro Glu Tyr Gly Arg Thr Asp Val Ala Gln Lys Phe Gln
   1 5 10 15
   Gly
<210> 550
   <211> 32
   <212> PRT
   <213> Artificial Sequence
<220>
   <223> Completely synthesized.
<400> 550
   Arg Val Thr Met Thr Ala Asp Thr Ser Thr Asp Thr Ala Tyr Met Glu
   1 5 10 15
   Leu Ser Ser Leu Arg Ser Glu Asp Thr Ala Val Tyr Tyr Cys Ala Thr
   20 25 30
<210> 551
   <211> 11
   <212> PRT
   <213> Artificial Sequence
<220>
   <223> Completely synthesized.
<400> 551
   Trp Gly Gln Gly Thr Thr Val Thr Val Ser Ser
   1 5 10
<210> 552
   <211> 30
   <212> PRT
   <213> Artificial Sequence
<220>
   <223> Completely synthesized.
<400> 552
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr
   20 25 30
<210> 553
   <211> 14
   <212> PRT
   <213> Artificial Sequence
<220>
   <223> Completely synthesized.
<400> 553
   Trp Val Arg Gln Ala Pro Gly Lys Gly Leu Glu Trp Met Gly
   1               5                   10
<210> 554
   <211> 17
   <212> PRT
   <213> Artificial Sequence
<220>
   <223> Completely synthesized.
<400> 554
   Trp Ile Asp Pro Glu Tyr Gly Arg Thr Asp Val Ala Gln Lys Phe Gln
   1               5                   10                  15
   Gly
<210> 555
   <211> 32
   <212> PRT
   <213> Artificial Sequence
<220>
   <223> Completely synthesized.
<400> 555
   Arg Val Thr Met Thr Glu Asp Thr Ser Thr Asp Thr Ala Tyr Met Glu
   1               5                   10                  15
   Leu Ser Ser Leu Arg Ser Glu Asp Thr Ala Val Tyr Tyr Cys Ala Arg
               20                  25                  30
<210> 556
   <211> 11
   <212> PRT
   <213> Artificial Sequence
<220>
   <223> Completely synthesized.
<400> 556
   Trp Gly Gln Gly Thr Thr Val Thr Val Ser Ser
   1               5                   10
<210> 557
   <211> 30
   <212> PRT
   <213> Artificial Sequence
<220>
   <223> Completely synthesized.
<400> 557
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr
               20                  25                  30
<210> 558
   <211> 14
   <212> PRT
   <213> Artificial Sequence
<220>
   <223> Completely synthesized.
<400> 558
   Trp Val Arg Gln Ala Pro Gly Lys Gly Leu Glu Trp Met Gly
   1               5                   10
<210> 559
   <211> 17
   <212> PRT
   <213> Artificial Sequence
<220>
   <223> Completely synthesized.
<400> 559
   Trp Ile Asp Pro Glu Tyr Gly Arg Thr Asp Val Ala Gln Lys Phe Gln
   1               5                   10                  15
   Gly
<210> 560
   <211> 33
   <212> PRT
   <213> Artificial Sequence
<220>
   <223> Completely synthesized.
<400> 560
   Gly Arg Val Thr Met Thr Ala Asp Thr Ser Thr Asp Thr Ala Tyr Met
   1               5                   10                  15
   Glu Leu Ser Ser Leu Arg Ser Glu Asp Thr Ala Val Tyr Tyr Cys Ala
               20                  25                  30
   Arg
<210> 561
   <211> 11
   <212> PRT
   <213> Artificial Sequence
<220>
   <223> Completely synthesized.
<400> 561
   Trp Gly Gln Gly Thr Thr Val Thr Val Ser Ser
   1               5                   10
<210> 562
   <211> 30
   <212> PRT
   <213> Artificial Sequence
<220>
   <223> Completely synthesized.
<400> 562
   Gln Met Gln Leu Val Gln Ser Gly Ala Glu Val Lys Lys Thr Gly Ser
   1               5                   10                  15
   Ser Val Lys Val Ser Cys Lys Ala Ser Gly Tyr Thr Phe Thr
               20                  25                  30
<210> 563
   <211> 14
   <212> PRT
   <213> Artificial Sequence
<220>
   <223> Completely synthesized.
<400> 563
   Trp Val Arg Gln Ala Pro Gly Gln Ala Leu Glu Trp Met Gly
   1               5                   10
<210> 564
   <211> 17
   <212> PRT
   <213> Artificial Sequence
<220>
   <223> Completely synthesized.
<400> 564
   Trp Ile Asp Pro Glu Tyr Gly Arg Thr Asp Val Ala Gln Lys Phe Gln
   1               5                   10                  15
   Asp
<210> 565
   <211> 32
   <212> PRT
   <213> Artificial Sequence
<220>
   <223> Completely synthesized.
<400> 565
   Arg Val Thr Ile Thr Arg Asp Arg Ser Met Ser Thr Ala Tyr Met Glu
   1               5                   10                  15
   Leu Ser Ser Leu Arg Ser Glu Asp Thr Ala Met Tyr Tyr Cys Ala Arg
               20                  25                  30
<210> 566
   <211> 11
   <212> PRT
   <213> Artificial Sequence
<220>
   <223> Completely synthesized.
<400> 566
   Trp Gly Gln Gly Thr Thr Val Thr Val Ser Ser
   1               5                   10
<210> 567
   <211> 30
   <212> PRT
   <213> Artificial Sequence
<220>
   <223> Completely synthesized.
<400> 567
   Gln Met Gln Leu Val Gln Ser Gly Ala Glu Val Lys Lys Thr Gly Ser
   1 5 10 15
   Ser Val Lys Val Ser Cys Lys Ala Ser Gly Tyr Thr Phe Thr
   20 25 30
<210> 568
   <211> 14
   <212> PRT
   <213> Artificial Sequence
<220>
   <223> Completely synthesized.
<400> 568
   Trp Val Arg Gln Ala Pro Gly Gln Ala Leu Glu Trp Met Gly
   1 5 10
<210> 569
   <211> 17
   <212> PRT
   <213> Artificial Sequence
<220>
   <223> Completely synthesized.
<400> 569
   Trp Ile Asp Pro Glu Tyr Gly Arg Thr Asp Val Ala Gln Lys Phe Gln
   1 5 10 15
   Asp
<210> 570
   <211> 32
   <212> PRT
   <213> Artificial Sequence
<220>
   <223> Completely synthesized.
<400> 570
   Arg Val Thr Ile Thr Ala Asp Arg Ser Met Ser Thr Ala Tyr Met Glu
   1 5 10 15
   Leu Ser Ser Leu Arg Ser Glu Asp Thr Ala Met Tyr Tyr Cys Ala Arg
   20 25 30
<210> 571
   <211> 11
   <212> PRT
   <213> Artificial Sequence
<220>
   <223> Completely synthesized.
<400> 571
   Trp Gly Gln Gly Thr Thr Val Thr Val Ser Ser
   1 5 10
<210> 572
   <211> 30
   <212> PRT
   <213> Artificial Sequence
<220>
   <223> Completely synthesized.
<400> 572
   Glu Val Gln Leu Val Gln Ser Gly Pro Glu Val Lys Lys Pro Gly Ala
   1 5 10 15
   Thr Val Lys Ile Ser Cys Lys Val Ser Gly Tyr Thr Phe Thr
   20 25 30
<210> 573
   <211> 14
   <212> PRT
   <213> Artificial Sequence
<220>
   <223> Completely synthesized.
<400> 573
   Trp Val Gln Gln Ala Pro Gly Lys Gly Leu Glu Trp Met Gly
   1 5 10
<210> 574
   <211> 17
   <212> PRT
   <213> Artificial Sequence
<220>
   <223> Completely synthesized.
<400> 574
   Trp Ile Asp Pro Glu Tyr Gly Arg Thr Asp Val Ala Glu Lys Phe Gln
   1 5 10 15
   Gly
<210> 575
   <211> 32
   <212> PRT
   <213> Artificial Sequence
<220>
   <223> Completely synthesized.
<400> 575
   Arg Val Thr Ile Thr Ala Asp Thr Ser Thr Asp Thr Ala Tyr Met Glu
   1 5 10 15
   Leu Ser Ser Leu Arg Ser Glu Asp Thr Ala Val Tyr Tyr Cys Ala Thr
   20 25 30
<210> 576
   <211> 11
   <212> PRT
   <213> Artificial Sequence
<220>
   <223> Completely synthesized.
<400> 576
   Trp Gly Gln Gly Thr Thr Val Thr Val Ser Ser
   1 5 10
<210> 577
   <211> 30
   <212> PRT
   <213> Artificial Sequence
<220>
   <223> Completely synthesized.
<400> 577
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr
   20 25 30
<210> 578
   <211> 14
   <212> PRT
   <213> Artificial Sequence
<220>
   <223> Completely synthesized.
<400> 578
   Trp Val Gln Gln Ala Pro Gly Lys Gly Leu Glu Trp Met Gly
   1 5 10
<210> 579
   <211> 17
   <212> PRT
   <213> Artificial Sequence
<220>
   <223> Completely synthesized.
<400> 579
   Trp Ile Asp Pro Glu Tyr Gly Arg Thr Asp Val Ala Glu Lys Phe Gln
   1               5                   10                  15
   Gly
<210> 580
   <211> 32
   <212> PRT
   <213> Artificial Sequence
<220>
   <223> Completely synthesized.
<400> 580
   Arg Val Thr Ile Thr Ala Asp Thr Ser Thr Asp Thr Ala Tyr Met Glu
   1               5                   10                  15
   Leu Ser Ser Leu Arg Ser Glu Asp Thr Ala Val Tyr Tyr Cys Ala Arg
               20                  25                  30
<210> 581
   <211> 11
   <212> PRT
   <213> Artificial Sequence
<220>
   <223> Completely synthesized.
<400> 581
   Trp Gly Gln Gly Thr Thr Val Thr Val Ser Ser
   1               5                   10
<210> 582
   <211> 23
   <212> PRT
   <213> Rattus norvegicus
<400> 582
   Asp Ile Gln Met Thr Gln Ser Pro Ser Ser Leu Ser Ala Ser Val Gly
   1               5                   10                  15
   Asp Arg Val Thr Ile Thr Cys
               20
<210> 583
   <211> 11
   <212> PRT
   <213> Rattus norvegicus
<400> 583
   Lys Ala Ser Gln Asn Val Asp Ser Asp Val Asp
   1 5 10
<210> 584
   <211> 15
   <212> PRT
   <213> Rattus norvegicus
<400> 584
   Trp Tyr Gln Gln Lys Pro Gly Lys Ala Pro Lys Leu Leu Ile Tyr
   1 5 10 15
<210> 585
   <211> 7
   <212> PRT
   <213> Rattus norvegicus
<400> 585
   Lys Ala Ser Asn Arg Tyr Thr
   1 5
<210> 586
   <211> 32
   <212> PRT
   <213> Rattus norvegicus
<400> 586
   Gly Val Pro Ser Arg Phe Ser Gly Ser Gly Ser Gly Thr Asp Phe Thr
   1 5 10 15
   Phe Thr Ile Ser Ser Leu Gln Pro Glu Asp Ile Ala Thr Tyr Tyr Cys
   20 25 30
<210> 587
   <211> 9
   <212> PRT
   <213> Rattus norvegicus
<400> 587
   Met Gln Ser Asn Thr His Pro Arg Thr
   1 5
<210> 588
   <211> 11
   <212> PRT
   <213> Rattus norvegicus
<400> 588
   Phe Gly Gly Gly Thr Lys Val Glu Ile Lys Arg
   1 5 10
<210> 589
   <211> 11
   <212> PRT
   <213> Artificial Sequence
<220>
   <223> Completely synthesized.
<400> 589
   Pro Ala Ser Gln Asn Val Asp Ser Asp Val Asp
   1 5 10
<210> 590
   <211> 11
   <212> PRT
   <213> Artificial Sequence
<220>
   <223> Completely synthesized.
<400> 590
   Gln Ala Ser Gln Asn Val Asp Ser Asp Val Asp
   1 5 10
<210> 591
   <211> 7
   <212> PRT
   <213> Artificial Sequence
<220>
   <223> Completely synthesized.
<400> 591
   Lys Ala Ser Asn Asp Tyr Thr
   1 5
<210> 592
   <211> 15
   <212> PRT
   <213> Artificial Sequence
<220>
   <223> Completely synthesized.
<400> 592
   Trp Tyr Gln Gln Lys Pro Gly Lys Ala Pro Lys Leu Leu Asp Tyr
   1 5 10 15
<210> 593
   <211> 15
   <212> PRT
   <213> Artificial Sequence
<220>
   <223> Completely synthesized.
<400> 593
   Trp Tyr Gln Gln Lys Pro Gly Lys Ala Pro Lys Leu Leu Ile Glu
   1               5                   10                  15
<210> 594
   <211> 9
   <212> PRT
   <213> Artificial Sequence
<220>
   <223> Completely synthesized.
<400> 594
   Ala Gln Ser Asn Thr His Pro Arg Thr
   1               5
<210> 595
   <211> 9
   <212> PRT
   <213> Artificial Sequence
<220>
   <223> Completely synthesized.
<400> 595
   Glu Gln Ser Asn Thr His Pro Arg Thr
   1               5
<210> 596
   <211> 9
   <212> PRT
   <213> Artificial Sequence
<220>
   <223> Completely synthesized.
<400> 596
   His Gln Ser Asn Thr His Pro Arg Thr
   1               5
<210> 597
   <211> 9
   <212> PRT
   <213> Artificial Sequence
<220>
   <223> Completely synthesized.
<400> 597
   Lys Gln Ser Asn Thr His Pro Arg Thr
   1               5
<210> 598
   <211> 9
   <212> PRT
   <213> Artificial Sequence
<220>
   <223> Completely synthesized.
<400> 598
   Pro Gln Ser Asn Thr His Pro Arg Thr
   1               5
<210> 599
   <211> 9
   <212> PRT
   <213> Artificial Sequence
<220>
   <223> Completely synthesized.
<400> 599
   Gln Gln Ser Asn Thr His Pro Arg Thr
   1               5
<210> 600
   <211> 9
   <212> PRT
   <213> Artificial Sequence
<220>
   <223> Completely synthesized.
<400> 600
   Ser Gln Ser Asn Thr His Pro Arg Thr
   1               5
<210> 601
   <211> 9
   <212> PRT
   <213> Artificial Sequence
<220>
   <223> Completely synthesized.
<400> 601
   Val Gln Ser Asn Thr His Pro Arg Thr
   1               5
<210> 602
   <211> 9
   <212> PRT
   <213> Artificial Sequence
<220>
   <223> Completely synthesized.
<400> 602
   Met Asp Ser Asn Thr His Pro Arg Thr
   1               5
<210> 603
   <211> 9
   <212> PRT
   <213> Artificial Sequence
<220>
   <223> Completely synthesized.
<400> 603
   Met Gln Glu Asn Thr His Pro Arg Thr
   1               5
<210> 604
   <211> 9
   <212> PRT
   <213> Artificial Sequence
<220>
   <223> Completely synthesized.
<400> 604
   Met Gln Ser Thr Thr His Pro Arg Thr
   1               5
<210> 605
   <211> 7
   <212> PRT
   <213> Artificial Sequence
<220>
   <223> Completely synthesized.
<400> 605
   Lys Ala Ser Gln Arg Tyr Thr
   1               5
<210> 606
   <211> 9
   <212> PRT
   <213> Artificial Sequence
<220>
   <223> Completely synthesized.
<400> 606
   Met Gln Ser Gln Thr His Pro Arg Thr
   1 5
<210> 607
   <211> 23
   <212> PRT
   <213> Artificial Sequence
<220>
   <223> Completely synthesized.
<400> 607
   Asp Ile Val Met Thr Gln Ser Pro Thr Ser Ile Ser Ile Ser Val Gly
   1 5 10 15
   Glu Arg Val Thr Met Asn Cys
   20
<210> 608
   <211> 11
   <212> PRT
   <213> Artificial Sequence
<220>
   <223> Completely synthesized.
<400> 608
   Lys Ala Ser Gln Asn Val Asp Ser Asp Val Asp
   1 5 10
<210> 609
   <211> 15
   <212> PRT
   <213> Artificial Sequence
<220>
   <223> Completely synthesized.
<400> 609
   Trp Tyr Gln Gln Lys Thr Gly Gln Ser Pro Lys Leu Leu Ile Tyr
   1 5 10 15
<210> 610
   <211> 7
   <212> PRT
   <213> Artificial Sequence
<220>
   <223> Completely synthesized.
<400> 610
   Lys Ala Ser Asn Arg Tyr Thr
   1 5
<210> 611
   <211> 32
   <212> PRT
   <213> Artificial Sequence
<220>
   <223> Completely synthesized.
<400> 611
   Gly Val Pro Asp Arg Phe Thr Gly Ser Gly Ser Gly Thr Asp Phe Thr
   1 5 10 15
   Phe Thr Ile Ser Asn Met Gln Ala Glu Asp Leu Ala Val Tyr Tyr Cys
   20 25 30
<210> 612
   <211> 9
   <212> PRT
   <213> Artificial Sequence
<220>
   <223> Completely synthesized.
<400> 612
   Met Gln Ser Asn Thr His Pro Arg Thr
   1 5
<210> 613
   <211> 11
   <212> PRT
   <213> Artificial Sequence
<220>
   <223> Completely synthesized.
<400> 613
   Phe Gly Gly Gly Thr Lys Leu Glu Leu Lys Arg
   1 5 10
<210> 614
   <211> 23
   <212> PRT
   <213> Artificial Sequence
<220>
   <223> Completely synthesized.
<400> 614
   Asp Ile Gln Leu Thr Gln Ser Pro Ser Phe Leu Ser Ala Ser Val Gly
   1 5 10 15
   Asp Arg Val Thr Ile Thr Cys
   20
<210> 615
   <211> 15
   <212> PRT
   <213> Artificial Sequence
<220>
   <223> Completely synthesized.
<400> 615
   Trp Tyr Gln Gln Lys Pro Gly Lys Ala Pro Lys Leu Leu Ile Tyr
   1 5 10 15
<210> 616
   <211> 32
   <212> PRT
   <213> Artificial Sequence
<220>
   <223> Completely synthesized.
<400> 616
   Gly Val Pro Ser Arg Phe Ser Gly Ser Gly Ser Gly Thr Glu Phe Thr
   1 5 10 15
   Leu Thr Ile Ser Ser Leu Gln Pro Glu Asp Phe Ala Thr Tyr Tyr Cys
   20 25 30
<210> 617
   <211> 11
   <212> PRT
   <213> Artificial Sequence
<220>
   <223> Completely synthesized.
<400> 617
   Phe Gly Gly Gly Thr Lys Val Glu Ile Lys Arg
   1 5 10
<210> 618
   <211> 23
   <212> PRT
   <213> Artificial Sequence
<220>
   <223> Completely synthesized.
<400> 618
   Asp Ile Gln Met Thr Gln Ser Pro Ser Ser Val Ser Ala Ser Val Gly
   1               5                   10                  15
   Asp Arg Val Thr Ile Thr Cys
               20
<210> 619
   <211> 15
   <212> PRT
   <213> Artificial Sequence
<220>
   <223> Completely synthesized.
<400> 619
   Trp Tyr Gln Gln Lys Pro Gly Lys Ala Pro Lys Leu Leu Ile Tyr
   1               5                   10                  15
<210> 620
   <211> 32
   <212> PRT
   <213> Artificial Sequence
<220>
   <223> Completely synthesized.
<400> 620
   Gly Val Pro Ser Arg Phe Ser Gly Ser Gly Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 621
   <211> 11
   <212> PRT
   <213> Artificial Sequence
<220>
   <223> Completely synthesized.
<400> 621
   Phe Gly Gly Gly Thr Lys Val Glu Ile Lys Arg
   1               5                   10
<210> 622
   <211> 23
   <212> PRT
   <213> Artificial Sequence
<220>
   <223> Completely synthesized.
<400> 622
   Ala Ile Gln Leu Thr Gln Ser Pro Ser Ser Leu Ser Ala Ser Val Gly
   1               5                   10                  15
   Asp Arg Val Thr Ile Thr Cys
               20
<210> 623
   <211> 15
   <212> PRT
   <213> Artificial Sequence
<220>
   <223> Completely synthesized.
<400> 623
   Trp Tyr Gln Gln Lys Pro Gly Lys Ala Pro Lys Leu Leu Ile Tyr
   1               5                   10                  15
<210> 624
   <211> 32
   <212> PRT
   <213> Artificial Sequence
<220>
   <223> Completely synthesized.
<400> 624
   Gly Val Pro Ser Arg Phe Ser Gly Ser Gly Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 625
   <211> 11
   <212> PRT
   <213> Artificial Sequence
<220>
   <223> Completely synthesized.
<400> 625
   Phe Gly Gly Gly Thr Lys Val Glu Ile Lys Arg
   1               5                   10
<210> 626
   <211> 23
   <212> PRT
   <213> Artificial Sequence
<220>
   <223> Completely synthesized.
<400> 626
   Asp Ile Gln Met Thr Gln Ser Pro Ser Ser Leu Ser Ala Ser Val Gly
   1               5                   10                  15
   Asp Arg Val Thr Ile Thr Cys
               20
<210> 627
   <211> 15
   <212> PRT
   <213> Artificial Sequence
<220>
   <223> Completely synthesized.
<400> 627
   Trp Tyr Gln Gln Lys Pro Gly Lys Ala Pro Lys Leu Leu Ile Tyr
   1               5                   10                  15
<210> 628
   <211> 32
   <212> PRT
   <213> Artificial Sequence
<220>
   <223> Completely synthesized.
<400> 628
   Gly Val Pro Ser Arg Phe Ser Gly Ser Gly Ser Gly Thr Asp Phe Thr
   1               5                   10                  15
   Phe Thr Ile Ser Ser Leu Gln Pro Glu Asp Ile Ala Thr Tyr Tyr Cys
               20                  25                  30
<210> 629
   <211> 11
   <212> PRT
   <213> Artificial Sequence
<220>
   <223> Completely synthesized.
<400> 629
   Phe Gly Gly Gly Thr Lys Val Glu Ile Lys Arg
   1               5                   10
<210> 630
   <211> 23
   <212> PRT
   <213> Artificial Sequence
<220>
   <223> Completely synthesized.
<400> 630
   Glu Ile Val Leu Thr Gln Ser Pro Ala Thr Leu Ser Leu Ser Pro Gly
   1 5 10 15
   Glu Arg Ala Thr Leu Ser Cys
   20
<210> 631
   <211> 15
   <212> PRT
   <213> Artificial Sequence
<220>
   <223> Completely synthesized.
<400> 631
   Trp Tyr Gln Gln Lys Pro Gly Gln Ala Pro Arg Leu Leu Ile Tyr
   1 5 10 15
<210> 632
   <211> 32
   <212> PRT
   <213> Artificial Sequence
<220>
   <223> Completely synthesized.
<400> 632
   Gly Ile Pro Ala Arg Phe Ser Gly Ser Gly Ser Gly Arg Asp Phe Thr
   1 5 10 15
   Leu Thr Ile Ser Ser Leu Glu Pro Glu Asp Phe Ala Val Tyr Tyr Cys
   20 25 30
<210> 633
   <211> 11
   <212> PRT
   <213> Artificial Sequence
<220>
   <223> Completely synthesized.
<400> 633
   Phe Gly Gly Gly Thr Lys Val Glu Ile Lys Arg
   1 5 10
<210> 634
   <211> 23
   <212> PRT
   <213> Artificial Sequence
<220>
   <223> Completely synthesized.
<400> 634
   Glu Ile Val Met Thr Gln Ser Pro Ala Thr Leu Ser Val Ser Pro Gly
   1 5 10 15
   Glu Arg Ala Thr Leu Ser Cys
   20
<210> 635
   <211> 15
   <212> PRT
   <213> Artificial Sequence
<220>
   <223> Completely synthesized.
<400> 635
   Trp Tyr Gln Gln Lys Pro Gly Gln Ala Pro Arg Leu Leu Ile Tyr
   1 5 10 15
<210> 636
   <211> 32
   <212> PRT
   <213> Artificial Sequence
<220>
   <223> Completely synthesized.
<400> 636
   Gly Ile Pro Ala Arg Phe Ser Gly Ser Gly Ser Gly Thr Glu Phe Thr
   1 5 10 15
   Leu Thr Ile Ser Ser Leu Gln Ser Glu Asp Phe Ala Val Tyr Tyr Cys
   20 25 30
<210> 637
   <211> 11
   <212> PRT
   <213> Artificial Sequence
<220>
   <223> Completely synthesized.
<400> 637
   Phe Gly Gly Gly Thr Lys Val Glu Ile Lys Arg
   1 5 10
<210> 638
   <211> 23
   <212> PRT
   <213> Artificial Sequence
<220>
   <223> Completely synthesized.
<400> 638
   Glu Ile Val Leu Thr Gln Ser Pro Asp Phe Gln Ser Val Thr Pro Lys
   1               5                   10                  15
   Glu Lys Val Thr Ile Thr Cys
               20
<210> 639
   <211> 15
   <212> PRT
   <213> Artificial Sequence
<220>
   <223> Completely synthesized.
<400> 639
   Trp Tyr Gln Gln Lys Pro Asp Gln Ser Pro Lys Leu Leu Ile Lys
   1               5                   10                  15
<210> 640
   <211> 32
   <212> PRT
   <213> Artificial Sequence
<220>
   <223> Completely synthesized.
<400> 640
   Gly Val Pro Ser Arg Phe Ser Gly Ser Gly Ser Gly Thr Asp Phe Thr
   1               5                   10                  15
   Leu Thr Ile Asn Ser Leu Glu Ala Glu Asp Ala Ala Thr Tyr Tyr Cys
               20                  25                  30
<210> 641
   <211> 11
   <212> PRT
   <213> Artificial Sequence
<220>
   <223> Completely synthesized.
<400> 641
   Phe Gly Gly Gly Thr Lys Val Glu Ile Lys Arg
   1               5                   10
<210> 642
   <211> 5
   <212> PRT
   <213> Artificial Sequence
<220>
   <223> Completely synthesized
<400> 642
   Gly Gly Gly Gly Ser
   1               5
<210> 643
   <211> 10
   <212> PRT
   <213> Artificial Sequence
<220>
   <223> Completely synthesized
<400> 643
   Gly Gly Gly Gly Ser Gly Gly Gly Gly Ser
   1               5                   10
<210> 644
   <211> 15
   <212> PRT
   <213> Artificial Sequence
<220>
   <223> Completely synthesized
<400> 644
   Gly Gly Gly Gly Ser Gly Gly Gly Gly Ser Gly Gly Gly Gly Ser
   1               5                   10                  15
<210> 645
   <211> 20
   <212> PRT
   <213> Artificial Sequence
<220>
   <223> Completely synthesized
<400> 645
   Gly Gly Gly Gly Ser Gly Gly Gly Gly Ser Gly Gly Gly Gly Ser Gly
   1               5                   10                  15
   Gly Gly Gly Ser
               20
<210> 646
   <211> 25
   <212> PRT
   <213> Artificial Sequence
<220>
   <223> Completely synthesized
<400> 646
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser
               20                  25
<210> 647
   <211> 188
   <212> PRT
   <213> Artificial Sequence
<220>
   <223> Completely synthesized.
<400> 647
   Met Ala Leu Thr Phe Ala Leu Leu Val Ala Leu Leu Val Leu Ser Cys
   1               5                   10                  15
   Lys Ser Ser Cys Ser Val Gly Cys Asp Leu Pro Gln Thr His Ser Leu
               20                  25                  30
   Gly Ser Arg Arg Thr Leu Met Leu Leu Ala Gln Met Arg Arg Ile Ser
           35                  40                  45
   Leu Phe Ser Cys Leu Lys Asp Arg His Asp Phe Gly Phe Pro Gln Glu
       50                  55                  60
   Glu Phe Gly Asn Gln Phe Gln Lys Ala Glu Thr Ile Pro Val Leu His
   65                  70                  75                  80
   Glu Met Ile Gln Gln Ile Phe Asn Leu Phe Ser Thr Met Asp Ser Ser
                   85                  90                  95
   Ala Ala Trp Asp Glu Thr Leu Leu Asp Lys Phe Tyr Thr Glu Leu Tyr
               100                 105                 110
   Gln Gln Leu Asn Asp Leu Glu Ala Cys Val Ile Gln Gly Val Gly Val
           115                 120                 125
   Thr Glu Thr Pro Leu Met Lys Glu Asp Ser Ile Leu Ala Val Arg Lys
       130                 135                 140
   Tyr Phe Gln Arg Ile Thr Leu Tyr Leu Lys Glu Lys Lys Tyr Ser Pro
   145                 150                 155                 160
   Cys Ala Trp Glu Val Val Arg Asp Glu Ile Met Arg Ser Phe Ser Leu
                   165                 170                 175
   Ser Thr Asn Leu Gln Glu Ser Leu Arg Ser Lys Glu
               180                 185
<210> 648
   <211> 165
   <212> PRT
   <213> Homo sapiens
<400> 648
   Cys Asp Leu Pro Gln Thr His Ser Leu Gly Ser Arg Arg Thr Leu Met
   1               5                   10                  15
   Leu Leu Ala Gln Met Arg Arg Ile Ser Leu Phe Ser Cys Leu Lys Asp
               20                  25                  30
   Arg His Asp Phe Gly Phe Pro Gln Glu Glu Phe Gly Asn Gln Phe Gln
           35                  40                  45
   Lys Ala Glu Thr Ile Pro Val Leu His Glu Met Ile Gln Gln Ile Phe
       50                  55                  60
   Asn Leu Phe Ser Thr Met Asp Ser Ser Ala Ala Trp Asp Glu Thr Leu
   65                  70                  75                  80
   Leu Asp Lys Phe Tyr Thr Glu Leu Tyr Gln Gln Leu Asn Asp Leu Glu
                   85                  90                  95
   Ala Cys Val Ile Gln Gly Val Gly Val Thr Glu Thr Pro Leu Met Lys
   100 105 110
   Glu Asp Ser Ile Leu Ala Val Arg Lys Tyr Phe Gln Arg Ile Thr Leu
   115 120 125
   Tyr Leu Lys Glu Lys Lys Tyr Ser Pro Cys Ala Trp Glu Val Val Arg
   130 135 140
   Ala Glu Ile Met Arg Ser Phe Ser Leu Ser Thr Asn Leu Gln Glu Ser
   145 150 155 160
   Leu Arg Ser Lys Glu
   165
<210> 649
   <211> 165
   <212> PRT
   <213> Artificial Sequence
<220>
   <223> Completely synthesized.
<400> 649
   Cys Asp Leu Pro Gln Thr His Ser Leu Gly Ser Arg Arg Thr Leu Met
   1 5 10 15
   Leu Leu Ala Gln Met Arg Arg Ile Ser Leu Phe Ser Cys Leu Lys Asp
   20 25 30
   Arg His Asp Phe Gly Phe Pro Gln Glu Glu Phe Gly Asn Gln Phe Gln
   35 40 45
   Lys Ala Glu Thr Ile Pro Val Leu His Glu Met Ile Gln Gln Ile Phe
   50 55 60
   Asn Leu Phe Ser Thr Met Asp Ser Ser Ala Ala Trp Asp Glu Thr Leu
   65 70 75 80
   Leu Asp Lys Phe Tyr Thr Glu Leu Tyr Gln Gln Leu Asn Asp Leu Glu
   85 90 95
   Ala Cys Val Ile Gln Gly Val Gly Val Thr Glu Thr Pro Leu Met Lys
   100 105 110
   Glu Asp Ser Ile Leu Ala Val Arg Lys Tyr Phe Gln Arg Ile Thr Leu
   115 120 125
   Tyr Leu Lys Glu Lys Lys Tyr Ser Pro Cys Ala Trp Glu Val Val Arg
   130 135 140
   Asp Glu Ile Met Arg Ser Phe Ser Leu Ser Thr Asn Leu Gln Glu Ser
   145 150 155 160
   Leu Arg Ser Lys Glu
   165
<210> 650
   <211> 188
   <212> PRT
   <213> Artificial Sequence
<220>
   <223> Completely synthesized.
<400> 650
   Met Ala Leu Thr Phe Ala Leu Leu Val Ala Leu Leu Val Leu Ser Cys
   1 5 10 15
   Lys Ser Ser Cys Ser Val Gly Cys Asp Leu Pro Gln Thr His Ser Leu
   20 25 30
   Gly Ser Arg Arg Thr Leu Met Leu Leu Ala Gln Met Arg Arg Ile Ser
   35 40 45
   Leu Phe Ser Cys Leu Lys Asp Arg His Asp Phe Gly Phe Pro Gln Glu
   50 55 60
   Glu Phe Gly Asn Gln Phe Gln Lys Ala Glu Thr Ile Pro Val Leu His
   65 70 75 80
   Glu Met Ile Gln Gln Ile Phe Asn Leu Phe Ser Thr Met Asp Ser Ser
   85 90 95
   Ala Ala Trp Asp Glu Thr Leu Leu Asp Lys Phe Tyr Thr Glu Leu Tyr
   100 105 110
   Gln Gln Leu Asn Asp Leu Glu Ala Cys Val Ile Gln Gly Val Gly Val
   115 120 125
   Thr Glu Thr Pro Leu Met Lys Glu Asp Ser Ile Leu Ala Val Arg Lys
   130 135 140
   Tyr Phe Gln Arg Ile Thr Leu Tyr Leu Lys Glu Lys Lys Tyr Ser Pro
   145 150 155 160
   Cys Ala Trp Glu Val Val Arg Gly Glu Ile Met Arg Ser Phe Ser Leu
   165 170 175
   Ser Thr Asn Leu Gln Glu Ser Leu Arg Ser Lys Glu
   180 185
<210> 651
   <211> 165
   <212> PRT
   <213> Artificial Sequence
<220>
   <223> Completely synthesized.
<400> 651
   Cys Asp Leu Pro Gln Thr His Ser Leu Gly Ser Arg Arg Thr Leu Met
   1 5 10 15
   Leu Leu Ala Gln Met Arg Arg Ile Ser Leu Phe Ser Cys Leu Lys Asp
   20 25 30
   Arg His Asp Phe Gly Phe Pro Gln Glu Glu Phe Gly Asn Gln Phe Gln
   35 40 45
   Lys Ala Glu Thr Ile Pro Val Leu His Glu Met Ile Gln Gln Ile Phe
   50 55 60
   Asn Leu Phe Ser Thr Met Asp Ser Ser Ala Ala Trp Asp Glu Thr Leu
   65 70 75 80
   Leu Asp Lys Phe Tyr Thr Glu Leu Tyr Gln Gln Leu Asn Asp Leu Glu
   85 90 95
   Ala Cys Val Ile Gln Gly Val Gly Val Thr Glu Thr Pro Leu Met Lys
   100 105 110
   Glu Asp Ser Ile Leu Ala Val Arg Lys Tyr Phe Gln Arg Ile Thr Leu
   115 120 125
   Tyr Leu Lys Glu Lys Lys Tyr Ser Pro Cys Ala Trp Glu Val Val Arg
   130 135 140
   Gly Glu Ile Met Arg Ser Phe Ser Leu Ser Thr Asn Leu Gln Glu Ser
   145 150 155 160
   Leu Arg Ser Lys Glu
   165
<210> 652
   <211> 492
   <212> PRT
   <213> Artificial Sequence
<220>
   <223> Completely synthesized.
<400> 652
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Arg Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu Gly Lys Cys Asp Leu Pro Gln Thr His Ser Leu
   325 330 335
   Gly Ser Arg Arg Thr Leu Met Leu Leu Ala Gln Met Arg Arg Ile Ser
   340 345 350
   Leu Phe Ser Cys Leu Lys Asp Arg His Asp Phe Gly Phe Pro Gln Glu
   355 360 365
   Glu Phe Gly Asn Gln Phe Gln Lys Ala Glu Thr Ile Pro Val Leu His
   370 375 380
   Glu Met Ile Gln Gln Ile Phe Asn Leu Phe Ser Thr Lys Asp Ser Ser
   385 390 395 400
   Ala Ala Trp Asp Glu Thr Leu Leu Asp Lys Phe Tyr Thr Glu Leu Tyr
   405 410 415
   Gln Gln Leu Asn Asp Leu Glu Ala Cys Val Ile Gln Gly Val Gly Val
   420 425 430
   Thr Glu Thr Pro Leu Met Lys Glu Asp Ser Ile Leu Ala Val Arg Lys
   435 440 445
   Tyr Phe Gln Arg Ile Thr Leu Tyr Leu Lys Glu Lys Lys Tyr Ser Pro
   450 455 460
   Cys Ala Trp Glu Val Val Arg Asp Glu Ile Met Arg Ser Phe Ser Leu
   465 470 475 480
   Ser Thr Asn Leu Gln Glu Ser Leu Arg Ser Lys Glu
   485 490
<210> 653
   <211> 492
   <212> PRT
   <213> Artificial Sequence
<220>
   <223> Completely synthesized.
<400> 653
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys Cys Asp Leu Pro Gln Thr His Ser Leu
                   325                 330                 335
   Gly Ser Arg Arg Thr Leu Met Leu Leu Ala Gln Met Arg Arg Ile Ser
               340                 345                 350
   Leu Phe Ser Cys Leu Lys Asp Arg His Asp Phe Gly Phe Pro Gln Glu
           355                 360                 365
   Glu Phe Gly Asn Gln Phe Gln Lys Ala Glu Thr Ile Pro Val Leu His
       370                 375                 380
   Glu Met Ile Gln Gln Ile Phe Asn Leu Phe Ser Thr Lys Asp Ser Ser
   385                 390                 395                 400
   Ala Ala Trp Asp Glu Thr Leu Leu Asp Lys Phe Tyr Thr Glu Leu Tyr
                   405                 410                 415
   Gln Gln Leu Asn Asp Leu Glu Ala Cys Val Ile Gln Gly Val Gly Val
               420                 425                 430
   Thr Glu Thr Pro Leu Met Lys Glu Asp Ser Ile Leu Ala Val Arg Lys
   435 440 445
   Tyr Phe Gln Arg Ile Thr Leu Tyr Leu Lys Glu Lys Lys Tyr Ser Pro
   450 455 460
   Cys Ala Trp Glu Val Val Arg Gly Glu Ile Met Arg Ser Phe Ser Leu
   465 470 475 480
   Ser Thr Asn Leu Gln Glu Ser Leu Arg Ser Lys Glu
   485 490
<210> 654
   <211> 492
   <212> PRT
   <213> Artificial Sequence
<220>
   <223> Completely synthesized.
<400> 654
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys Cys Asp Leu Pro Gln Thr His Ser Leu
                   325                 330                 335
   Gly Ser Arg Arg Thr Leu Met Leu Leu Ala Gln Met Arg Arg Ile Ser
               340                 345                 350
   Leu Phe Ser Cys Leu Lys Asp Arg His Asp Phe Gly Phe Pro Gln Glu
           355                 360                 365
   Glu Phe Gly Asn Gln Phe Gln Lys Ala Glu Thr Ile Pro Val Leu His
       370                 375                 380
   Glu Met Ile Gln Gln Ile Phe Asn Leu Phe Ser Thr Lys Asp Ser Ser
   385                 390                 395                 400
   Ala Ala Trp Asp Glu Thr Leu Leu Asp Lys Phe Tyr Thr Glu Leu Tyr
   405 410 415
   Gln Gln Leu Asn Asp Leu Glu Ala Cys Val Ile Gln Gly Val Gly Val
   420 425 430
   Thr Glu Thr Pro Leu Met Lys Glu Asp Ser Ile Leu Ala Val Arg Lys
   435 440 445
   Tyr Phe Gln Arg Ile Thr Leu Tyr Leu Lys Glu Lys Lys Tyr Ser Pro
   450 455 460
   Cys Ala Trp Glu Val Val Arg Gly Glu Ile Met Arg Ser Phe Ser Leu
   465 470 475 480
   Ser Thr Asn Leu Gln Glu Ser Leu Arg Ser Lys Glu
   485 490
<210> 655
   <211> 495
   <212> PRT
   <213> Artificial Sequence
<220>
   <223> Completely synthesized.
<400> 655
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys Cys Asp Leu Pro Gln Thr
                   325                 330                 335
   His Ser Leu Gly Ser Arg Arg Thr Leu Met Leu Leu Ala Gln Met Arg
               340                 345                 350
   Arg Ile Ser Leu Phe Ser Cys Leu Lys Asp Arg His Asp Phe Gly Phe
           355                 360                 365
   Pro Gln Glu Glu Phe Gly Asn Gln Phe Gln Lys Ala Glu Thr Ile Pro
       370                 375                 380
   Val Leu His Glu Met Ile Gln Gln Ile Phe Asn Leu Phe Ser Thr Lys
   385                 390                 395                 400
   Asp Ser Ser Ala Ala Trp Asp Glu Thr Leu Leu Asp Lys Phe Tyr Thr
                   405                 410                 415
   Glu Leu Tyr Gln Gln Leu Asn Asp Leu Glu Ala Cys Val Ile Gln Gly
               420                 425                 430
   Val Gly Val Thr Glu Thr Pro Leu Met Lys Glu Asp Ser Ile Leu Ala
           435                 440                 445
   Val Arg Lys Tyr Phe Gln Arg Ile Thr Leu Tyr Leu Lys Glu Lys Lys
       450                 455                 460
   Tyr Ser Pro Cys Ala Trp Glu Val Val Arg Gly Glu Ile Met Arg Ser
   465                 470                 475                 480
   Phe Ser Leu Ser Thr Asn Leu Gln Glu Ser Leu Arg Ser Lys Glu
                   485                 490                 495
<210> 656
   <211> 495
   <212> PRT
   <213> Artificial Sequence
<220>
   <223> Completely synthesized.
<400> 656
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Tyr Ile Thr Arg Glu Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys Cys Asp Leu Pro Gln Thr
                   325                 330                 335
   His Ser Leu Gly Ser Arg Arg Thr Leu Met Leu Leu Ala Gln Met Arg
               340                 345                 350
   Arg Ile Ser Leu Phe Ser Cys Leu Lys Asp Arg His Asp Phe Gly Phe
           355                 360                 365
   Pro Gln Glu Glu Phe Gly Asn Gln Phe Gln Lys Ala Glu Thr Ile Pro
       370                 375                 380
   Val Leu His Glu Met Ile Gln Gln Ile Phe Asn Leu Phe Ser Thr Lys
   385                 390                 395                 400
   Asp Ser Ser Ala Ala Trp Asp Glu Thr Leu Leu Asp Lys Phe Tyr Thr
                   405                 410                 415
   Glu Leu Tyr Gln Gln Leu Asn Asp Leu Glu Ala Cys Val Ile Gln Gly
               420                 425                 430
   Val Gly Val Thr Glu Thr Pro Leu Met Lys Glu Asp Ser Ile Leu Ala
           435                 440                 445
   Val Arg Lys Tyr Phe Gln Arg Ile Thr Leu Tyr Leu Lys Glu Lys Lys
       450                 455                 460
   Tyr Ser Pro Cys Ala Trp Glu Val Val Arg Asp Glu Ile Met Arg Ser
   465                 470                 475                 480
   Phe Ser Leu Ser Thr Asn Leu Gln Glu Ser Leu Arg Ser Lys Glu
                   485                 490                 495
<210> 657
   <211> 495
   <212> PRT
   <213> Artificial Sequence
<220>
   <223> Completely synthesized.
<400> 657
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys Asp Lys Thr His Thr Cys Pro Pro Cys
   100 105 110
   Pro Ala Pro Glu Leu Leu Gly Gly Pro Ser Val Phe Leu Phe Pro Pro
   115 120 125
   Lys Pro Lys Asp Thr Leu Tyr Ile Thr Arg Glu Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Asn Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Asp Glu
   225 230 235 240
   Leu Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys Cys Asp Leu Pro Gln Thr
   325 330 335
   His Ser Leu Gly Ser Arg Arg Thr Leu Met Leu Leu Ala Gln Met Arg
   340 345 350
   Arg Ile Ser Leu Phe Ser Cys Leu Lys Asp Arg His Asp Phe Gly Phe
   355 360 365
   Pro Gln Glu Glu Phe Gly Asn Gln Phe Gln Lys Ala Glu Thr Ile Pro
   370 375 380
   Val Leu His Glu Met Ile Gln Gln Ile Phe Asn Leu Phe Ser Thr Lys
   385 390 395 400
   Asp Ser Ser Ala Ala Trp Asp Glu Thr Leu Leu Asp Lys Phe Tyr Thr
   405 410 415
   Glu Leu Tyr Gln Gln Leu Asn Asp Leu Glu Ala Cys Val Ile Gln Gly
   420 425 430
   Val Gly Val Thr Glu Thr Pro Leu Met Lys Glu Asp Ser Ile Leu Ala
   435 440 445
   Val Arg Lys Tyr Phe Gln Arg Ile Thr Leu Tyr Leu Lys Glu Lys Lys
   450 455 460
   Tyr Ser Pro Cys Ala Trp Glu Val Val Arg Gly Glu Ile Met Arg Ser
   465 470 475 480
   Phe Ser Leu Ser Thr Asn Leu Gln Glu Ser Leu Arg Ser Lys Glu
   485 490 495
<210> 658
   <211> 492
   <212> PRT
   <213> Artificial Sequence
<220>
   <223> Completely synthesized.
<400> 658
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Tyr Ile Thr Arg Glu Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
   275 280 285
   Arg Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu Gly Lys Cys Asp Leu Pro Gln Thr His Ser Leu
   325 330 335
   Gly Ser Arg Arg Thr Leu Met Leu Leu Ala Gln Met Arg Arg Ile Ser
   340 345 350
   Leu Phe Ser Cys Leu Lys Asp Arg His Asp Phe Gly Phe Pro Gln Glu
   355 360 365
   Glu Phe Gly Asn Gln Phe Gln Lys Ala Glu Thr Ile Pro Val Leu His
   370 375 380
   Glu Met Ile Gln Gln Ile Phe Asn Leu Phe Ser Thr Lys Asp Ser Ser
   385 390 395 400
   Ala Ala Trp Asp Glu Thr Leu Leu Asp Lys Phe Tyr Thr Glu Leu Tyr
   405 410 415
   Gln Gln Leu Asn Asp Leu Glu Ala Cys Val Ile Gln Gly Val Gly Val
   420 425 430
   Thr Glu Thr Pro Leu Met Lys Glu Asp Ser Ile Leu Ala Val Arg Lys
   435 440 445
   Tyr Phe Gln Arg Ile Thr Leu Tyr Leu Lys Glu Lys Lys Tyr Ser Pro
   450 455 460
   Cys Ala Trp Glu Val Val Arg Asp Glu Ile Met Arg Ser Phe Ser Leu
   465 470 475 480
   Ser Thr Asn Leu Gln Glu Ser Leu Arg Ser Lys Glu
   485 490
<210> 659
   <211> 121
   <212> PRT
   <213> Artificial Sequence
<220>
   <223> Completely synthesized
<220>
   <221> Variant
   <222> (42)..(42)
   <223> X = His or Pro
<220>
   <221> Variant
   <222> (76).. (76)
   <223> X = Ser or Leu
<220>
   <221> Variant
   <222> (80)..(80)
   <223> X = Phe or Ile
<220>
   <221> Variant
   <222> (83)..(83)
   <223> X = Lys or Arg
<220>
   <221> Variant
   <222> (85)..(85)
   <223> X = Ser or Thr
<220>
   <221> Variant
   <222> (108)..(108)
   <223> X = Leu or Met
<400> 659
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Ser Trp Ile Arg Gln Xaa Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Xaa Lys Asn Gln Xaa
   65                  70                  75                  80
   Ser Leu Xaa Leu Xaa Ser Val Thr Ala Ala Asp Thr Ala Val Tyr Tyr
                   85                  90                  95
   Cys Ala Arg Val Gly Gly Ala Gly Gly Trp Pro Xaa Asp Val Trp Gly
               100                 105                 110
   Gln Gly Thr Thr Val Thr Val Ser Ser
           115                 120
<210> 660
   <211> 117
   <212> PRT
   <213> Artificial Sequence
<220>
   <223> Completely synthesized.
<400> 660
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Glu Asp Ser His Lys Gly Gln Gly Ser Gly Val
       50                  55                  60
   Pro Ser Arg Phe Ser Gly Ser Lys Asp Val Ser Thr Thr Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Gln Gly Ser Gly Val Phe Gly Gly Gly Thr Gln
               100                 105                 110
   Leu Thr Val Leu Gly
           115
<210> 661
   <211> 117
   <212> PRT
   <213> Artificial Sequence
<220>
   <223> Completely synthesized.
<400> 661
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
   35 40 45
   Leu Leu Tyr Tyr Tyr Glu Asp Ser His Lys Gly Gln Gly Ser Gly Val
   50 55 60
   Pro Ser Arg Phe Ser Gly Ser Lys Asp Val Ser Thr Thr Ser Gly Ile
   65 70 75 80
   Leu Leu Ile Ser Gly Leu Gln Ser Glu Asp Ile Ala Thr Tyr Tyr Cys
   85 90 95
   Leu Thr Trp Ser Ser Glu Gly Ser Gly Val Phe Gly Gly Gly Thr Gln
   100 105 110
   Leu Thr Val Leu Gly
   115
<210> 662
   <211> 117
   <212> PRT
   <213> Artificial Sequence
<220>
   <223> Completely synthesized.
<400> 662
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Thr Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Gln Gly Ser Gly Val Phe Gly Gly Gly Thr Gln
               100                 105                 110
   Leu Thr Val Leu Gly
           115
<210> 663
   <211> 117
   <212> PRT
   <213> Artificial Sequence
<220>
   <223> Completely synthesized.
<400> 663
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Gln Asp Ser His Lys Gly Gln Gly Ser Gly Val
       50                  55                  60
   Pro Ser Arg Phe Ser Gly Ser Lys Asp Val Ser Thr Thr Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 664
   <211> 117
   <212> PRT
   <213> Artificial Sequence
<220>
   <223> Completely synthesized
<220>
   <221> Variant
   <222> (31)..(31)
   <223> X = Arg or Gly
<220>
   <221> Variant
   <222> (32)..(32)
   <223> X = Tyr or Ser
<220>
   <221> Variant
   <222> (54)..(54)
   <223> X = Ser or Glu or Gln
<220>
   <221> Variant
   <222> (77)..(77)
   <223> X = Asn or Thr
<220>
   <221> Variant
   <222> (97)..(97)
   <223> X = Met or Leu
<220>
   <221> Variant
   <222> (102)..(102)
   <223> X = Asn or Gln or Glu
<400> 664
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Xaa Xaa
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Xaa Asp Ser His Lys Gly Gln Gly Ser Gly Val
       50                  55                  60
   Pro Ser Arg Phe Ser Gly Ser Lys Asp Val Ser Thr Xaa Ser Gly Ile
   65                  70                  75                  80
   Leu Leu Ile Ser Gly Leu Gln Ser Glu Asp Ile Ala Thr Tyr Tyr Cys
                   85                  90                  95
   Xaa Thr Trp Ser Ser Xaa Gly Ser Gly Val Phe Gly Gly Gly Thr Gln
               100                 105                 110
   Leu Thr Val Leu Gly
           115
<210> 665
   <211> 120
   <212> PRT
   <213> Artificial Sequence
<220>
   <223> Completely synthesized.
<220>
   <221> Variant
   <222> (1)..(1)
   <223> X= Glu or Gln
<220>
   <221> Variant
   <222> (17)..(17)
   <223> X= Thr or Ser
<220>
   <221> Variant
   <222> (20)..(20)
   <223> X= Ile or Val
<220>
   <221> Variant
   <222> (29).. (29)
   <223> X= Phe or Leu
<220>
   <221> Variant
   <222> (38)..(38)
   <223> X= Gln or Arg
<220>
   <221> Variant
   <222> (40)..(40)
   <223> X=Glu or Gly or His or Ala
<220>
   <221> Variant
   <222> (62)..(62)
   <223> X=Glu or Gln
<220>
   <221> Variant
   <222> (70)..(70)
   <223> X=Ile or Met
<220>
   <221> Variant
   <222> (100)..(100)
   <223> X=Lys or Gly or Thr
<220>
   <221> Variant
   <222> (102)..(102)
   <223> X=Asn or Gln
<400> 665
   Xaa Val Gln Leu Val Gln Ser Gly Ala Glu Val Lys Lys Pro Gly Ala
   1               5                   10                  15
   Xaa Val Lys Xaa Ser Cys Lys Val Ser Gly Tyr Thr Xaa Thr Asp Ser
               20                  25                  30
   Val Met Asn Trp Val Xaa Gln Xaa Pro Gly Lys Gly Leu Glu Trp Met
           35                  40                  45
   Gly Trp Ile Asp Pro Glu Tyr Gly Arg Thr Asp Val Ala Xaa Lys Phe
       50                  55                  60
   Gln Gly Arg Val Thr Xaa Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Xaa Tyr Xaa Ser Gly Tyr Gly Phe Pro Tyr Trp Gly Gln
               100                 105                 110
   Gly Thr Thr Val Thr Val Ser Ser
           115                 120
<210> 666
   <211> 108
   <212> PRT
   <213> Artificial Sequence
<220>
   <223> Completely synthesized.
<220>
   <221> Variant
   <222> (24)..(24)
   <223> X = Lys or Gly or Gln
<220>
   <221> Variant
   <222> (53)..(53)
   <223> X = Asn or Gln
<220>
   <221> Variant
   <222> (54)..(54)
   <223> X = Arg or Asp
<220>
   <221> Variant
   <222> (89).. (89)
   <223> X = Met or Ala
<400> 666
   Asp Ile Gln Met Thr Gln Ser Pro Ser Ser Leu Ser Ala Ser Val Gly
   1               5                   10                  15
   Asp Arg Val Thr Ile Thr Cys Xaa Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Xaa Xaa Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Xaa Gln Ser Asn Thr His Pro Arg
                   85                  90                  95
   Thr Phe Gly Gly Gly Thr Lys Val Glu Ile Lys Arg
               100                 105
<210> 667
   <211> 363
   <212> DNA
   <213> Artificial Sequence
<220>
   <223> Completely synthesized.
<400> 667
   cagctgcagc tgcaggaatc cggccctggc ctggtcaagc cctccgagac actgagcctg      60
   acctgcaccg tgtccggcgg ctccatctcc tccagctcct actactggtc ctggatccgg     120
   cagcaccccg gcaagggcct ggaatggatc ggctacatct actactccgg ctccaccaac     180
   tacaacccca gcctgaagtc cagagtgacc atctccgtgg acacctccaa gaaccagttc     240
   tccctgaagc tgtcctccgt gaccgccgct gacaccgccg tgtactactg tgccagagtg     300
   ggcggagctg gcggctggcc tctggatgtg tggggccagg gcaccaccgt caccgtgtcc     360
   tca                                                                   363
<210> 668
   <211> 363
   <212> DNA
   <213> Artificial Sequence
<220>
   <223> Completely synthesized.
<400> 668
   cagctgcagc tgcaggaatc cggccctggc ctggtcaagc cctccgagac actgagcctg 60
   acctgcaccg tgtccggcgg ctccatctcc tccagctcct actactggtc ctggatccgg 120
   cagcctcccg gcaagggcct ggaatggatc ggctacatct actactccgg ctccaccaac 180
   tacaacccca gcctgaagtc cagagtgacc atctccgtgg acaccctgaa gaaccagatc 240
   tccctgcggc tgacctccgt gaccgccgct gacaccgccg tgtactactg tgccagagtg 300
   ggcggagctg gcggctggcc tatggatgtg tggggccagg gcaccaccgt caccgtgtcc 360
   tca 363
<210> 669
   <211> 351
   <212> DNA
   <213> Artificial Sequence
<220>
   <223> Completely synthesized.
<400> 669
   caggccgtgc tgacccagcc tgcctccctg tctgcctctc ctggcgagtc cgccagactg 60
   acctgcaccc tgccctccga catcaacgtg cggtactaca acatctactg gtatcagcag 120
   aagcccggca gcccccccag atacctgctg tactactact ccgactccca caagggccag 180
   ggctccggcg tgccctccag attctccggc tccaaggacg tgtccaccaa ctccggcatc 240
   ctgctgatct ccggcctgca gtccgaggac attgccacct actactgcat gacttggagc 300
   agcaacggca gcggcgtgtt cggcggaggc acccagctga ccgtcctagg t 351
<210> 670
   <211> 351
   <212> DNA
   <213> Artificial Sequence
<220>
   <223> Completely synthesized.
<400> 670
   caggccgtgc tgacccagcc tgcctccctg tctgcctctc ctggcgagtc cgccagactg 60
   acctgcaccc tgccctccga catcaacgtg cggtactaca acatctactg gtatcagcag 120
   aagcccggca gcccccccag atacctgctg tactactact ccgactccca caagggccag 180
   ggctccggcg tgccctccag attctccggc tccaaggacg tgtccaccac ctccggcatc 240
   ctgctgatct ccggcctgca gtccgaggac attgccacct actactgcat gacttggagc 300
   agcaacggca gcggcgtgtt cggcggaggc acccagctga ccgtcctagg t 351
<210> 671
   <211> 351
   <212> DNA
   <213> Artificial Sequence
<220>
   <223> Completely synthesized.
<400> 671
   caggccgtgc tgacccagcc tgcctccctg tctgcctctc ctggcgagtc cgccagactg 60
   acctgcaccc tgccctccga catcaacgtg cggtactaca acatctactg gtatcagcag 120
   aagcccggca gcccccccag atacctgctg tactactacg aggactccca caagggccag 180
   ggctccggcg tgccctccag attctccggc tccaaggacg tgtccaccaa ctccggcatc 240
   ctgctgatct ccggcctgca gtccgaggac attgccacct actactgcat gacttggagc 300
   agcaacggca gcggcgtgtt cggcggaggc acccagctga ccgtcctagg t 351
<210> 672
   <211> 351
   <212> DNA
   <213> Artificial Sequence
<220>
   <223> Completely synthesized.
<400> 672
   caggccgtgc tgacccagcc tgcctccctg tctgcctctc ctggcgagtc cgccagactg 60
   acctgcaccc tgccctccga catcaacgtg cggtactaca acatctactg gtatcagcag 120
   aagcccggca gcccccccag atacctgctg tactactacc aggactccca caagggccag 180
   ggctccggcg tgccctccag attctccggc tccaaggacg tgtccaccaa ctccggcatc 240
   ctgctgatct ccggcctgca gtccgaggac attgccacct actactgcat gacttggagc 300
   agcaacggca gcggcgtgtt cggcggaggc acccagctga ccgtcctagg t 351
<210> 673
   <211> 351
   <212> DNA
   <213> Artificial Sequence
<220>
   <223> Completely synthesized.
<400> 673
   caggctgtgc tgacgcagcc ggccagcctc tccgcgtccc ctggcgaatc cgcacggctg 60
   acttgcaccc tgccgagcga cattaacgtc ggtagctaca atatctactg gtaccaacaa 120
   aagccgggga gcccaccccg ctacttactg tattactact cggattccca taaaggacag 180
   ggatcgggag tgccatcacg cttcagcgga tcgaaggatg tgtcgaccaa ttcgggaatc 240
   ctgctcatct caggcttgca gagcgaggac atcgccacct actactgtct gacttggtca 300
   tcccaggggt caggcgtgtt tggaggaggt acccagctga ctgtcctagg t 351
<210> 674
   <211> 351
   <212> DNA
   <213> Artificial Sequence
<220>
   <223> Completely synthesized.
<400> 674
   caggctgtgc tgacgcagcc ggccagcctc tccgcgtccc ctggcgaatc cgcacggctg 60
   acttgcaccc tgccgagcga cattaacgtc ggtagctaca atatctactg gtaccaacaa 120
   aagccgggga gcccaccccg ctacttactg tattactact cggattccca taaaggacag 180
   ggatcgggag tgccatcacg cttcagcgga tcgaaggatg tgtcgaccaa ttcgggaatc 240
   ctgctcatct caggcttgca gagcgaggac atcgccacct actactgtct gacttggtca 300
   tccgaagggt caggcgtgtt tggaggaggt acccagctga ctgtcctagg t 351
<210> 675
   <211> 351
   <212> DNA
   <213> Artificial Sequence
<220>
   <223> Completely synthesized.
<400> 675
   caggctgtgc tgacgcagcc ggccagcctc tccgcgtccc ctggcgaatc cgcacggctg 60
   acttgcaccc tgccgagcga cattaacgtc ggtagctaca atatctactg gtaccaacaa 120
   aagccgggga gcccaccccg ctacttactg tattactacg aggattccca taaaggacag 180
   ggatcgggag tgccatcacg cttcagcgga tcgaaggatg tgtcgaccac ctcgggaatc 240
   ctgctcatct caggcttgca gagcgaggac atcgccacct actactgtct gacttggtca 300
   tcccaggggt caggcgtgtt tggaggaggt acccagctga ctgtcctagg t 351
<210> 676
   <211> 351
   <212> DNA
   <213> Artificial Sequence
<220>
   <223> Completely synthesized.
<400> 676
   caggctgtgc tgacgcagcc ggccagcctc tccgcgtccc ctggcgaatc cgcacggctg 60
   acttgcaccc tgccgagcga cattaacgtc ggtagctaca atatctactg gtaccaacaa 120
   aagccgggga gcccaccccg ctacttactg tattactacg aggattccca taaaggacag 180
   ggatcgggag tgccatcacg cttcagcgga tcgaaggatg tgtcgaccac ctcgggaatc 240
   ctgctcatct caggcttgca gagcgaggac atcgccacct actactgtct gacttggtca 300
   tccgaagggt caggcgtgtt tggaggaggt acccagctga ctgtcctagg t 351
<210> 677
   <211> 351
   <212> DNA
   <213> Artificial Sequence
<220>
   <223> Completely synthesized.
<400> 677
   caggctgtgc tgacgcagcc ggccagcctc tccgcgtccc ctggcgaatc cgcacggctg 60
   acttgcaccc tgccgagcga cattaacgtc ggtagctaca atatctactg gtaccaacaa 120
   aagccgggga gcccaccccg ctacttactg tattactacc aggattccca taaaggacag 180
   ggatcgggag tgccatcacg cttcagcgga tcgaaggatg tgtcgaccac ctcgggaatc 240
   ctgctcatct caggcttgca gagcgaggac atcgccacct actactgtct gacttggtca 300
   tcccaggggt caggcgtgtt tggaggaggt acccagctga ctgtcctagg t 351
<210> 678
   <211> 351
   <212> DNA
   <213> Artificial Sequence
<220>
   <223> Completely synthesized.
<400> 678
   caggctgtgc tgacgcagcc ggccagcctc tccgcgtccc ctggcgaatc cgcacggctg 60
   acttgcaccc tgccgagcga cattaacgtc ggtagctaca atatctactg gtaccaacaa 120
   aagccgggga gcccaccccg ctacttactg tattactacc aggattccca taaaggacag 180
   ggatcgggag tgccatcacg cttcagcgga tcgaaggatg tgtcgaccac ctcgggaatc 240
   ctgctcatct caggcttgca gagcgaggac atcgccacct actactgtct gacttggtca 300
   tccgaagggt caggcgtgtt tggaggaggt acccagctga ctgtcctagg t 351
<210> 679
   <211> 360
   <212> DNA
   <213> Artificial Sequence
<220>
   <223> Completely synthesized.
<400> 679
   gaggtgcagc tggtgcagtc cggcgctgaa gtgaagaaac ccggcgctac cgtgaagatc 60
   tcctgcaagg tgtccggcta caccttcacc gactccgtga tgaactgggt gcagcaggag 120
   cctggcaagg gcctggaatg gatgggctgg atcgaccccg agtacggcag aaccgacgtg 180
   gccgagaagt tccagggcag agtgaccatc accgccgaca cctccaccga caccgcctac 240
   atggaactgt cctccctgcg gagcgaggac accgccgtgt actactgcgc ccggaccaag 300
   tacaactccg gctacggctt cccctactgg ggccagggca ccaccgtgac agtgtcctca 360
<210> 680
   <211> 360
   <212> DNA
   <213> Artificial Sequence
<220>
   <223> Completely synthesized.
<400> 680
   gaggtgcagc tggtgcagtc cggcgctgaa gtgaagaaac ccggcgctac cgtgaagatc 60
   tcctgcaagg tgtccggcta caccttcacc gactccgtga tgaactgggt gcagcagggc 120
   cctggcaagg gcctggaatg gatgggctgg atcgaccccg agtacggcag aaccgacgtg 180
   gccgagaagt tccagggcag agtgaccatc accgccgaca cctccaccga caccgcctac 240
   atggaactgt cctccctgcg gagcgaggac accgccgtgt actactgcgc ccggaccaag 300
   tacaactccg gctacggctt cccctactgg ggccagggca ccaccgtgac agtgtcctca 360
<210> 681
   <211> 360
   <212> DNA
   <213> Artificial Sequence
<220>
   <223> Completely synthesized.
<400> 681
   gaggtgcagc tggtgcagtc cggcgctgaa gtgaagaaac ccggcgctac cgtgaagatc        60
   tcctgcaagg tgtccggcta caccttcacc gactccgtga tgaactgggt gcagcagcac       120
   cctggcaagg gcctggaatg gatgggctgg atcgaccccg agtacggcag aaccgacgtg       180
   gccgagaagt tccagggcag agtgaccatc accgccgaca cctccaccga caccgcctac       240
   atggaactgt cctccctgcg gagcgaggac accgccgtgt actactgcgc ccggaccaag       300
   tacaactccg gctacggctt cccctactgg ggccagggca ccaccgtgac agtgtcctca       360
<210> 682
   <211> 360
   <212> DNA
   <213> Artificial Sequence
<220>
   <223> Completely synthesized.
<400> 682
   gaggtgcagc tggtgcagtc cggcgctgaa gtgaagaaac ccggcgctac cgtgaagatc        60
   tcctgcaagg tgtccggcta caccttcacc gactccgtga tgaactgggt gcagcagcag       120
   cctggcaagg gcctggaatg gatgggctgg atcgaccccg agtacggcag aaccgacgtg       180
   gccgagaagt tccagggcag agtgaccatc accgccgaca cctccaccga caccgcctac       240
   atggaactgt cctccctgcg gagcgaggac accgccgtgt actactgcgc ccggaccaag       300
   tacaactccg gctacggctt cccctactgg ggccagggca ccaccgtgac agtgtcctca       360
<210> 683
   <211> 360
   <212> DNA
   <213> Artificial Sequence
<220>
   <223> Completely synthesized.
<400> 683
   gaggtgcagc tggtgcagtc cggcgctgaa gtgaagaaac ccggcgctac cgtgaagatc 60
   tcctgcaagg tgtccggcta caccttcacc gactccgtga tgaactgggt gcagcaggcc 120
   cctggcaagg gcctggaatg gatgggctgg atcgaccccg agtacggcag aaccgacgtg 180
   gccgagaagt tccagggcag agtgaccatc accgccgaca cctccaccga caccgcctac 240
   atggaactgt cctccctgcg gagcgaggac accgccgtgt actactgcgc ccggaccggc 300
   tacaactccg gctacggctt cccctactgg ggccagggca ccaccgtgac agtgtcctca 360
<210> 684
   <211> 360
   <212> DNA
   <213> Artificial Sequence
<220>
   <223> Completely synthesized.
<400> 684
   gaggtgcagc tggtgcagtc cggcgctgaa gtgaagaaac ccggcgctac cgtgaagatc 60
   tcctgcaagg tgtccggcta caccttcacc gactccgtga tgaactgggt gcagcaggcc 120
   cctggcaagg gcctggaatg gatgggctgg atcgaccccg agtacggcag aaccgacgtg 180
   gccgagaagt tccagggcag agtgaccatc accgccgaca cctccaccga caccgcctac 240
   atggaactgt cctccctgcg gagcgaggac accgccgtgt actactgcgc ccggaccacc 300
   tacaactccg gctacggctt cccctactgg ggccagggca ccaccgtgac agtgtcctca 360
<210> 685
   <211> 360
   <212> DNA
   <213> Artificial Sequence
<220>
   <223> Completely synthesized.
<400> 685
   gaggtgcagc tggtgcagtc cggcgctgaa gtgaagaaac ccggcgctac cgtgaagatc 60
   tcctgcaagg tgtccggcta caccttcacc gactccgtga tgaactgggt gcagcaggcc 120
   cctggcaagg gcctggaatg gatgggctgg atcgaccccg agtacggcag aaccgacgtg 180
   gccgagaagt tccagggcag agtgaccatc accgccgaca cctccaccga caccgcctac 240
   atggaactgt cctccctgcg gagcgaggac accgccgtgt actactgcgc ccggaccaag 300
   tacaactccg gctacggctt cccctactgg ggccagggca ccaccgtgac agtgtcctca 360
<210> 686
   <211> 360
   <212> DNA
   <213> Artificial Sequence
<220>
   <223> Completely synthesized.
<400> 686
   gaggtgcagc tggtgcagtc cggcgctgaa gtgaagaaac ccggcgctac cgtgaagatc 60
   tcctgcaagg tgtccggcta caccttcacc gactccgtga tgaactgggt gcagcaggcc 120
   cctggcaagg gcctggaatg gatgggctgg atcgaccccg agtacggcag aaccgacgtg 180
   gccgagaagt tccagggcag agtgaccatc accgccgaca cctccaccga caccgcctac 240
   atggaactgt cctccctgcg gagcgaggac accgccgtgt actactgcgc ccctaccaag 300
   tacaactccg gctacggctt cccctactgg ggccagggca ccaccgtgac agtgtcctca 360
<210> 687
   <211> 360
   <212> DNA
   <213> Artificial Sequence
<220>
   <223> Completely synthesized.
<400> 687
   caggtgcagc tggtgcagtc cggcgctgaa gtgaagaaac ccggcgcttc cgtgaaggtg 60
   tcctgcaagg tgtccggcta caccctgacc gactccgtga tgaactgggt gcgacaggcc 120
   cctggcaagg gcctggaatg gatgggctgg atcgaccccg agtacggcag aaccgacgtg 180
   gcccagaaat tccagggcag agtgaccatg accgccgaca cctccaccga caccgcctac 240
   atggaactgt cctccctgcg gagcgaggac accgccgtgt actactgcgc ccggaccaag 300
   tacaactccg gctacggctt cccctactgg ggccagggca ccaccgtgac cgtgtcctca 360
<210> 688
   <211> 324
   <212> DNA
   <213> Artificial Sequence
<220>
   <223> Completely synthesized.
<400> 688
   gacatccaga tgacccagag cccttccagc ctgtccgcct ctgtgggcga cagagtgacc    60
   atcacatgca aggcctccca gaacgtggac agcgacgtgg actggtatca gcagaagccc   120
   ggcaaggccc ctaagctgct gatctacaag gccagcaaca gatacaccgg cgtgccctcc   180
   agattctccg gctctggatc tggcaccgac ttcaccttca ccatcagctc cctgcagccc   240
   gaggacattg ccacctacta ctgtatgcag tccaacaccc acccccggac cttcggcgga   300
   ggcaccaagg tggaaatcaa gcgt                                          324
<210> 689
   <211> 324
   <212> DNA
   <213> Artificial Sequence
<220>
   <223> Completely synthesized.
<400> 689
   gacatccaga tgacccagag cccttccagc ctgtccgcct ctgtgggcga cagagtgacc    60
   atcacatgcg gcgcctccca gaacgtggac agcgacgtgg actggtatca gcagaagccc   120
   ggcaaggccc ctaagctgct gatctacaag gccagcaaca gatacaccgg cgtgccctcc   180
   agattctccg gctctggatc tggcaccgac ttcaccttca ccatcagctc cctgcagccc   240
   gaggacattg ccacctacta ctgtatgcag tccaacaccc acccccggac cttcggcgga   300
   ggcaccaagg tggaaatcaa gcgt                                          324
<210> 690
   <211> 324
   <212> DNA
   <213> Artificial Sequence
<220>
   <223> Completely synthesized.
<400> 690
   gacatccaga tgacccaatc cccttcctca ttgtccgctt cggtcggcga ccgcgtgact    60
   atcacttgcc aggcatcaca aaacgtggat tcggatgtgg attggtacca gcagaagccc   120
   ggaaaggccc cgaaactgct gatctacaag gcgtccaacc ggtacactgg cgtcccgtcg   180
   agattcagcg gaagcgggtc gggaaccgac tttaccttca ctatcagctc actccagcca   240
   gaggacattg ccacctatta ctgtatgcag agcaataccc acccgcgcac cttcggagga   300
   ggtactaaag tggaaatcaa gcgt                                          324
<210> 691
   <211> 324
   <212> DNA
   <213> Artificial Sequence
<220>
   <223> Completely synthesized.
<400> 691
   gacatccaga tgacccagag cccttccagc ctgtccgcct ctgtgggcga cagagtgacc      60
   atcacatgca aggcctccca gaacgtggac agcgacgtgg actggtatca gcagaagccc     120
   ggcaaggccc ctaagctgct gatctacaag gccagcaacg actacaccgg cgtgccctcc     180
   agattctccg gctctggatc tggcaccgac ttcaccttca ccatcagctc cctgcagccc     240
   gaggacattg ccacctacta ctgtatgcag tccaacaccc acccccggac cttcggcgga     300
   ggcaccaagg tggaaatcaa gcgt                                            324
<210> 692
   <211> 324
   <212> DNA
   <213> Artificial Sequence
<220>
   <223> Completely synthesized.
<400> 692
   gacatccaga tgacccagag cccttccagc ctgtccgcct ctgtgggcga cagagtgacc      60
   atcacatgca aggcctccca gaacgtggac agcgacgtgg actggtatca gcagaagccc     120
   ggcaaggccc ctaagctgct gatctacaag gccagcaaca gatacaccgg cgtgccctcc     180
   agattctccg gctctggatc tggcaccgac ttcaccttca ccatcagctc cctgcagccc     240
   gaggacattg ccacctacta ctgtgcgcag tccaacaccc acccccggac cttcggcgga     300
   ggcaccaagg tggaaatcaa gcgt                                            324
<210> 693
   <211> 324
   <212> DNA
   <213> Artificial Sequence
<220>
   <223> Completely synthesized.
<400> 693
   gacatccaga tgacccaatc cccttcctca ttgtccgctt cggtcggcga ccgcgtgact 60
   atcacttgca aagcatcaca aaacgtggat tcggatgtgg attggtacca gcagaagccc 120
   ggaaaggccc cgaaactgct gatctacaag gcgtcccagc ggtacactgg cgtcccgtcg 180
   agattcagcg gaagcgggtc gggaaccgac tttaccttca ctatcagctc actccagcca 240
   gaggacattg ccacctatta ctgtatgcag agcaataccc acccgcgcac cttcggagga 300
   ggtactaaag tggaaatcaa gcgt 324
<210> 694
   <211> 492
   <212> PRT
   <213> Artificial Sequence
<220>
   <223> Completely synthesized.
<400> 694
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Lys Thr
   65 70 75 80
   Tyr Thr Cys Asn Val Asp His Lys Pro Ser Asn Thr Lys Val Asp Lys
   85 90 95
   Arg Val Glu Ser Lys Tyr Gly Pro Pro Cys Pro Pro Cys Pro Ala Pro
   100 105 110
   Glu Phe Leu Gly Gly Pro Ser Val Phe Leu Phe Pro Pro Lys Pro Lys
   115 120 125
   Asp Thr Leu Tyr Ile Thr Arg Glu Pro Glu Val Thr Cys Val Val Val
   130 135 140
   Asp Val Ser Gln Glu Asp Pro Glu Val Gln Phe Asn Trp Tyr Val Asp
   145 150 155 160
   Gly Val Glu Val His Asn Ala Lys Thr Lys Pro Arg Glu Glu Gln Phe
   165 170 175
   Asn Ser Thr Tyr Arg Val Val Ser Val Leu Thr Val Leu His Gln Asp
   180 185 190
   Trp Leu Asn Gly Lys Glu Tyr Lys Cys Lys Val Ser Asn Lys Gly Leu
   195 200 205
   Pro Ser Ser Ile Glu Lys Thr Ile Ser Lys Ala Lys Gly Gln Pro Arg
   210 215 220
   Glu Pro Gln Val Tyr Thr Leu Pro Pro Ser Gln Glu Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys Cys Asp Leu Pro Gln Thr His Ser Leu
                   325                 330                 335
   Gly Ser Arg Arg Thr Leu Met Leu Leu Ala Gln Met Arg Arg Ile Ser
               340                 345                 350
   Leu Phe Ser Cys Leu Lys Asp Arg His Asp Phe Gly Phe Pro Gln Glu
           355                 360                 365
   Glu Phe Gly Asn Gln Phe Gln Lys Ala Glu Thr Ile Pro Val Leu His
       370                 375                 380
   Glu Met Ile Gln Gln Ile Phe Asn Leu Phe Ser Thr Lys Asp Ser Ser
   385                 390                 395                 400
   Ala Ala Trp Asp Glu Thr Leu Leu Asp Lys Phe Tyr Thr Glu Leu Tyr
                   405                 410                 415
   Gln Gln Leu Asn Asp Leu Glu Ala Cys Val Ile Gln Gly Val Gly Val
               420                 425                 430
   Thr Glu Thr Pro Leu Met Lys Glu Asp Ser Ile Leu Ala Val Arg Lys
           435                 440                 445
   Tyr Phe Gln Arg Ile Thr Leu Tyr Leu Lys Glu Lys Lys Tyr Ser Pro
       450                 455                 460
   Cys Ala Trp Glu Val Val Arg Gly Glu Ile Met Arg Ser Phe Ser Leu
   465                 470                 475                 480
   Ser Thr Asn Leu Gln Glu Ser Leu Arg Ser Lys Glu
                   485                 490
<210> 695
   <211> 366
   <212> DNA
   <213> Homo sapiens
<400> 695
   gaggtccagc tggtacagtc tggggctgag gtgaagaagt ctggggcctc agtgaaggtc 60
   tcctgcaagg cttctggata caccttcacc gactactata tacactgggt gcgacaggcc 120
   cctggacaag ggcttgagtg gatgggatgg atcaacccta acaatggtgg cgtaaccttt 180
   gcccagaagt ttcagggcag ggtcaccatg accagggaca cgtccatcag cacagcctac 240
   atggacctga gcagcctgag atctgacgac acggccgtct acttctgtgc gagagatatt 300
   cgaatgagcg ggtggctggc gccatttgac tactggggcc agggaaccct ggtcaccgtc 360
   tcctca 366
<210> 696
   <211> 11
   <212> PRT
   <213> Artificial Sequence
<220>
   <223> Completely synthesized
<400> 696
   Gly Ala Ser Gln Asn Val Asp Ser Asp Val Asp
   1 5 10
<210> 697
   <211> 5
   <212> PRT
   <213> Homo sapiens
<400> 697
   Asp Tyr Tyr Ile His
   1 5
<210> 698
   <211> 17
   <212> PRT
   <213> Homo sapiens
<400> 698
   Trp Ile Asn Pro Asn Asn Gly Gly Val Thr Phe Ala Gln Lys Phe Gln
   1 5 10 15
   Gly
<210> 699
   <211> 13
   <212> PRT
   <213> Homo sapiens
<400> 699
   Asp Ile Arg Met Ser Gly Trp Leu Ala Pro Phe Asp Tyr
   1 5 10
<210> 700
   <211> 117
   <212> PRT
   <213> Artificial Sequence
<220>
   <223> Completely synthesized
<400> 700
   Gln Ala Val Leu Thr Gln Pro Ala Ser Leu Ser Ala Ser Pro Gly Glu
   1 5 10 15
   Ser Ala Arg Leu Thr Cys Thr Leu Pro Ser Asp Ile Asn Val Gly Ser
   20 25 30
   Tyr Asn Ile Tyr Trp Tyr Gln Gln Lys Pro Gly Ser Pro Pro Arg Tyr
   35 40 45
   Leu Leu Tyr Tyr Tyr Ser Asp Ser His Lys Gly Gln Gly Ser Gly Val
   50 55 60
   Pro Ser Arg Phe Ser Gly Ser Lys Asp Val Ser Thr Thr Ser Gly Ile
   65 70 75 80
   Leu Leu Ile Ser Gly Leu Gln Ser Glu Asp Ile Ala Thr Tyr Tyr Cys
   85 90 95
   Leu Thr Trp Ser Ser Gln Gly Ser Gly Val Phe Gly Gly Gly Thr Gln
   100 105 110
   Leu Thr Val Leu Gly
   115
<210> 701
   <211> 117
   <212> PRT
   <213> Artificial Sequence
<220>
   <223> Completely synthesized
<400> 701
   Gln Ala Val Leu Thr Gln Pro Ala Ser Leu Ser Ala Ser Pro Gly Glu
   1               5                   10                  15
   Ser Ala Arg Leu Thr Cys Thr Leu Pro Ser Asp Ile Asn Val Gly Ser
               20                  25                  30
   Tyr Asn Ile Tyr Trp Tyr Gln Gln Lys Pro Gly Ser Pro Pro Arg Tyr
           35                  40                  45
   Leu Leu Tyr Tyr Tyr Ser Asp Ser His Lys Gly Gln Gly Ser Gly Val
       50                  55                  60
   Pro Ser Arg Phe Ser Gly Ser Lys Asp Val Ser Thr Thr Ser Gly Ile
   65                  70                  75                  80
   Leu Leu Ile Ser Gly Leu Gln Ser Glu Asp Ile Ala Thr Tyr Tyr Cys
                   85                  90                  95
   Leu Thr Trp Ser Ser Glu Gly Ser Gly Val Phe Gly Gly Gly Thr Gln
               100                 105                 110
   Leu Thr Val Leu Gly
           115
<210> 702
   <211> 351
   <212> DNA
   <213> Artificial Sequence
<220>
   <223> Completely synthesized
<400> 702
   caagccgtcc tgactcagcc agccagcctg tccgcgtcac cgggagaatc cgctcgcctg       60
   acttgcactc tgccctcaga catcaatgtc ggttcgtaca acatctactg gtaccaacaa      120
   aagccggggt cgcctccgcg gtatctgttg tactactact cggatagcca caagggacag      180
   ggctcgggag tgccatccag attttccggg tcaaaagatg tgagcacgac ctcgggcatc      240
   ctcctcatca gcggacttca gtccgaggac attgcgacct actactgcct cacttggtcg      300
   tcacagggaa gcggagtgtt cggaggaggc acccagctga ccgtcctagg t               351
<210> 703
   <211> 351
   <212> DNA
   <213> Artificial Sequence
<220>
   <223> Completely synthesized
<400> 703
   caagccgtcc tgactcagcc agccagcctg tccgcgtcac cgggagaatc cgctcgcctg     60
   acttgcactc tgccctcaga catcaatgtc ggttcgtaca acatctactg gtaccaacaa    120
   aagccggggt cgcctccgcg gtatctgttg tactactact cggatagcca caagggacag    180
   ggctcgggag tgccatccag attttccggg tcaaaagatg tgagcacgac ctcgggcatc    240
   ctcctcatca gcggacttca gtccgaggac attgcgacct actactgcct cacttggtcg    300
   tcagaaggaa gcggagtgtt cggaggaggc acccagctga ccgtcctagg t             351
<210> 704
   <211> 108
   <212> PRT
   <213> Artificial Sequence
<220>
   <223> Completely synthesized
<400> 704
   Asp Ile Gln Met Thr Gln Ser Pro Ser Ser Leu Ser Ala Ser Val Gly
   1               5                   10                  15
   Asp Arg Val Thr Ile Thr Cys Gly Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Asp Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Ala Gln Ser Asn Thr His Pro Arg
                   85                  90                  95
   Thr Phe Gly Gly Gly Thr Lys Val Glu Ile Lys Arg
               100                 105
<210> 705
   <211> 108
   <212> PRT
   <213> Artificial Sequence
<220>
   <223> Completely synthesized
<400> 705
   Asp Ile Gln Met Thr Gln Ser Pro Ser Ser Leu Ser Ala Ser Val Gly
   1               5                   10                  15
   Asp Arg Val Thr Ile Thr Cys Gly Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Ala Gln Ser Asn Thr His Pro Arg
                   85                  90                  95
   Thr Phe Gly Gly Gly Thr Lys Val Glu Ile Lys Arg
               100                 105
<210> 706
   <211> 108
   <212> PRT
   <213> Artificial Sequence
<220>
   <223> Completely synthesized
<400> 706
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Asp Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Ala Gln Ser Asn Thr His Pro Arg
                   85                  90                  95
   Thr Phe Gly Gly Gly Thr Lys Val Glu Ile Lys Arg
               100                 105
<210> 707
   <211> 108
   <212> PRT
   <213> Artificial Sequence
<220>
   <223> Completely synthesized
<400> 707
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Asn Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Ala Gln Ser Asn Thr His Pro Arg
                   85                  90                  95
   Thr Phe Gly Gly Gly Thr Lys Val Glu Ile Lys Arg
               100                 105
<210> 708
   <211> 108
   <212> PRT
   <213> Artificial Sequence
<220>
   <223> Completely synthesized
<400> 708
   Asp Ile Gln Met Thr Gln Ser Pro Ser Ser Leu Ser Ala Ser Val Gly
   1               5                   10                  15
   Asp Arg Val Thr Ile Thr Cys Gly Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Gln Asp Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Ala Gln Ser Asn Thr His Pro Arg
                   85                  90                  95
   Thr Phe Gly Gly Gly Thr Lys Val Glu Ile Lys Arg
   100 105
<210> 709
   <211> 108
   <212> PRT
   <213> Artificial Sequence
<220>
   <223> Completely synthesized
<400> 709
   Asp Ile Gln Met Thr Gln Ser Pro Ser Ser Leu Ser Ala Ser Val Gly
   1 5 10 15
   Asp Arg Val Thr Ile Thr Cys Gly Ala Ser Gln Asn Val Asp Ser Asp
   20 25 30
   Val Asp Trp Tyr Gln Gln Lys Pro Gly Lys Ala Pro Lys Leu Leu Ile
   35 40 45
   Tyr Lys Ala Ser Gln Arg Tyr Thr Gly Val Pro Ser Arg Phe Ser Gly
   50 55 60
   Ser Gly Ser Gly Thr Asp Phe Thr Phe Thr Ile Ser Ser Leu Gln Pro
   65 70 75 80
   Glu Asp Ile Ala Thr Tyr Tyr Cys Ala Gln Ser Asn Thr His Pro Arg
   85 90 95
   Thr Phe Gly Gly Gly Thr Lys Val Glu Ile Lys Arg
   100 105
<210> 710
   <211> 108
   <212> PRT
   <213> Artificial Sequence
<220>
   <223> Completely synthesized
<400> 710
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Gln Asp Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Ala Gln Ser Asn Thr His Pro Arg
                   85                  90                  95
   Thr Phe Gly Gly Gly Thr Lys Val Glu Ile Lys Arg
               100                 105
<210> 711
   <211> 108
   <212> PRT
   <213> Artificial Sequence
<220>
   <223> Completely synthesized
<400> 711
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asn Val Asp Ser Asp
               20                  25                  30
   Val Asp Trp Tyr Gln Gln Lys Pro Gly Lys Ala Pro Lys Leu Leu Ile
           35                  40                  45
   Tyr Lys Ala Ser Gln Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Ala Gln Ser Asn Thr His Pro Arg
                   85                  90                  95
   Thr Phe Gly Gly Gly Thr Lys Val Glu Ile Lys Arg
               100                 105
<210> 712
   <211> 324
   <212> DNA
   <213> Artificial Sequence
<220>
   <223> Completely synthesized
<400> 712
   gacatccaga tgactcagag cccgtcctcg ctttcggctt ccgtcggcga ccgcgtgacc       60
   atcacttgtg gcgcgtcgca gaacgtcgat tccgacgtgg actggtacca acagaagccg      120
   gggaaagcgc ccaagctgct gatctacaag gcctccaatg attacactgg agtgcctagc      180
   cggttcagcg gatcagggtc gggaacggac ttcactttta ccatctcaag cctccaacca      240
   gaagatattg ccacctatta ctgcgcacaa tcaaacaccc acccgagaac cttcggcgga      300
   ggaaccaagg tggagatcaa acgt                                             324
<210> 713
   <211> 324
   <212> DNA
   <213> Artificial Sequence
<220>
   <223> Completely synthesized
<400> 713
   gacatccaga tgactcagag cccgtcctcg ctttcggctt ccgtcggcga ccgcgtgacc 60
   atcacttgtg gcgcgtcgca gaacgtcgat tccgacgtgg actggtacca acagaagccg 120
   gggaaagcgc ccaagctgct gatctacaag gcctccaatc gctacactgg agtgcctagc 180
   cggttcagcg gatcagggtc gggaacggac ttcactttta ccatctcaag cctccaacca 240
   gaagatattg ccacctatta ctgcgcacaa tcaaacaccc acccgagaac cttcggcgga 300
   ggaaccaagg tggagatcaa acgt 324
<210> 714
   <211> 324
   <212> DNA
   <213> Artificial Sequence
<220>
   <223> Completely synthesized
<400> 714
   gacatccaga tgactcagag cccgtcctcg ctttcggctt ccgtcggcga ccgcgtgacc 60
   atcacttgtc aggcgtcgca gaacgtcgat tccgacgtgg actggtacca acagaagccg 120
   gggaaagcgc ccaagctgct gatctacaag gcctccaatg attacactgg agtgcctagc 180
   cggttcagcg gatcagggtc gggaacggac ttcactttta ccatctcaag cctccaacca 240
   gaagatattg ccacctatta ctgcgcacaa tcaaacaccc acccgagaac cttcggcgga 300
   ggaaccaagg tggagatcaa acgt 324
<210> 715
   <211> 324
   <212> DNA
   <213> Artificial Sequence
<220>
   <223> Completely synthesized
<400> 715
   gacatccaga tgactcagag cccgtcctcg ctttcggctt ccgtcggcga ccgcgtgacc 60
   atcacttgtc aggcgtcgca gaacgtcgat tccgacgtgg actggtacca acagaagccg 120
   gggaaagcgc ccaagctgct gatctacaag gcctccaatc gctacactgg agtgcctagc 180
   cggttcagcg gatcagggtc gggaacggac ttcactttta ccatctcaag cctccaacca 240
   gaagatattg ccacctatta ctgcgcacaa tcaaacaccc acccgagaac cttcggcgga 300
   ggaaccaagg tggagatcaa acgt 324
<210> 716
   <211> 324
   <212> DNA
   <213> Artificial Sequence
<220>
   <223> Completely synthesized
<400> 716
   gacatccaga tgactcagag cccgtcctcg ctttcggctt ccgtcggcga ccgcgtgacc        60
   atcacttgtg gcgcgtcgca gaacgtcgat tccgacgtgg actggtacca acagaagccg       120
   gggaaagcgc ccaagctgct gatctacaag gcctcccagg attacactgg agtgcctagc       180
   cggttcagcg gatcagggtc gggaacggac ttcactttta ccatctcaag cctccaacca       240
   gaagatattg ccacctatta ctgcgcacaa tcaaacaccc acccgagaac cttcggcgga       300
   ggaaccaagg tggagatcaa acgt                                              324
<210> 717
   <211> 324
   <212> DNA
   <213> Artificial Sequence
<220>
   <223> Completely synthesized
<400> 717
   gacatccaga tgactcagag cccgtcctcg ctttcggctt ccgtcggcga ccgcgtgacc        60
   atcacttgtg gcgcgtcgca gaacgtcgat tccgacgtgg actggtacca acagaagccg       120
   gggaaagcgc ccaagctgct gatctacaag gcctcccagc gctacactgg agtgcctagc       180
   cggttcagcg gatcagggtc gggaacggac ttcactttta ccatctcaag cctccaacca       240
   gaagatattg ccacctatta ctgcgcacaa tcaaacaccc acccgagaac cttcggcgga       300
   ggaaccaagg tggagatcaa acgt                                              324
<210> 718
   <211> 324
   <212> DNA
   <213> Artificial Sequence
<220>
   <223> Completely synthesized
<400> 718
   gacatccaga tgactcagag cccgtcctcg ctttcggctt ccgtcggcga ccgcgtgacc 60
   atcacttgtc aggcgtcgca gaacgtcgat tccgacgtgg actggtacca acagaagccg 120
   gggaaagcgc ccaagctgct gatctacaag gcctcccagg attacactgg agtgcctagc 180
   cggttcagcg gatcagggtc gggaacggac ttcactttta ccatctcaag cctccaacca 240
   gaagatattg ccacctatta ctgcgcacaa tcaaacaccc acccgagaac cttcggcgga 300
   ggaaccaagg tggagatcaa acgt 324
<210> 719
   <211> 324
   <212> DNA
   <213> Artificial Sequence
<220>
   <223> Completely synthesized
<400> 719
   gacatccaga tgactcagag cccgtcctcg ctttcggctt ccgtcggcga ccgcgtgacc 60
   atcacttgtc aggcgtcgca gaacgtcgat tccgacgtgg actggtacca acagaagccg 120
   gggaaagcgc ccaagctgct gatctacaag gcctcccagc gctacactgg agtgcctagc 180
   cggttcagcg gatcagggtc gggaacggac ttcactttta ccatctcaag cctccaacca 240
   gaagatattg ccacctatta ctgcgcacaa tcaaacaccc acccgagaac cttcggcgga 300
   ggaaccaagg tggagatcaa acgt 324
<210> 720
   <211> 120
   <212> PRT
   <213> Artificial Sequence
<220>
   <223> Completely synthesized
<400> 720
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr Asp Ser
               20                  25                  30
   Val Met Asn Trp Val Arg Gln Gly Pro Gly Lys Gly Leu Glu Trp Met
           35                  40                  45
   Gly Trp Ile Asp Pro Glu Tyr Gly Arg Thr Asp Val Ala Gln Lys Phe
   50 55 60
   Gln Gly Arg Val Thr Met Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Gly Tyr Asn Ser Gly Tyr Gly Phe Pro Tyr Trp Gly Gln
   100 105 110
   Gly Thr Thr Val Thr Val Ser Ser
   115 120
<210> 721
   <211> 120
   <212> PRT
   <213> Artificial Sequence
<220>
   <223> Completely synthesized
<400> 721
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr Asp Ser
   20 25 30
   Val Met Asn Trp Val Arg Gln His Pro Gly Lys Gly Leu Glu Trp Met
   35 40 45
   Gly Trp Ile Asp Pro Glu Tyr Gly Arg Thr Asp Val Ala Gln Lys Phe
   50 55 60
   Gln Gly Arg Val Thr Met Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Gly Tyr Asn Ser Gly Tyr Gly Phe Pro Tyr Trp Gly Gln
   100 105 110
   Gly Thr Thr Val Thr Val Ser Ser
   115 120
<210> 722
   <211> 120
   <212> PRT
   <213> Artificial Sequence
<220>
   <223> Completely synthesized
<400> 722
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr Asp Ser
               20                  25                  30
   Val Met Asn Trp Val Arg Gln Gly Pro Gly Lys Gly Leu Glu Trp Met
           35                  40                  45
   Gly Trp Ile Asp Pro Glu Tyr Gly Arg Thr Asp Val Ala Gln Lys Phe
       50                  55                  60
   Gln Gly Arg Val Thr Met Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Gly Tyr Gln Ser Gly Tyr Gly Phe Pro Tyr Trp Gly Gln
               100                 105                 110
   Gly Thr Thr Val Thr Val Ser Ser
           115                 120
<210> 723
   <211> 120
   <212> PRT
   <213> Artificial Sequence
<220>
   <223> Completely synthesized
<400> 723
   Gln Val Gln Leu Val Gln Ser Gly Ala Glu Val Lys Lys Pro Gly Ala
   1 5 10 15
   Ser Val Lys Val Ser Cys Lys Val Ser Gly Tyr Thr Leu Thr Asp Ser
   20 25 30
   Val Met Asn Trp Val Arg Gln His Pro Gly Lys Gly Leu Glu Trp Met
   35 40 45
   Gly Trp Ile Asp Pro Glu Tyr Gly Arg Thr Asp Val Ala Gln Lys Phe
   50 55 60
   Gln Gly Arg Val Thr Met Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Gly Tyr Gln Ser Gly Tyr Gly Phe Pro Tyr Trp Gly Gln
   100 105 110
   Gly Thr Thr Val Thr Val Ser Ser
   115 120
<210> 724
   <211> 360
   <212> DNA
   <213> Artificial Sequence
<220>
   <223> Completely synthesized
<400> 724
   caagtgcaac tggtgcagtc cggtgccgaa gtgaagaaac cgggtgcgtc cgtcaaagtg 60
   agctgcaagg tgtccgggta caccttgact gattcagtga tgaattgggt gcggcagggg 120
   ccaggaaagg gactggagtg gatgggatgg atcgaccctg aatacggaag gactgacgtc 180
   gcccagaagt ttcagggacg cgtcacgatg actgccgata cttcgaccga cacggcttac 240
   atggaactct cgtccctgag atcggaggac accgcagtct actactgtgc tcgcactggc 300
   tataactcag gctacggctt cccgtactgg ggacaaggaa ccaccgtgac agtgtcctca 360
<210> 725
   <211> 360
   <212> DNA
   <213> Artificial Sequence
<220>
   <223> Completely synthesized
<400> 725
   caagtgcaac tggtgcagtc cggtgccgaa gtgaagaaac cgggtgcgtc cgtcaaagtg 60
   agctgcaagg tgtccgggta caccttgact gattcagtga tgaattgggt gcggcagcac 120
   ccaggaaagg gactggagtg gatgggatgg atcgaccctg aatacggaag gactgacgtc 180
   gcccagaagt ttcagggacg cgtcacgatg actgccgata cttcgaccga cacggcttac 240
   atggaactct cgtccctgag atcggaggac accgcagtct actactgtgc tcgcactggc 300
   tataactcag gctacggctt cccgtactgg ggacaaggaa ccaccgtgac agtgtcctca 360
<210> 726
   <211> 360
   <212> DNA
   <213> Artificial Sequence
<220>
   <223> Completely synthesized
<400> 726
   caagtgcaac tggtgcagtc cggtgccgaa gtgaagaaac cgggtgcgtc cgtcaaagtg 60
   agctgcaagg tgtccgggta caccttgact gattcagtga tgaattgggt gcggcagggg 120
   ccaggaaagg gactggagtg gatgggatgg atcgaccctg aatacggaag gactgacgtc 180
   gcccagaagt ttcagggacg cgtcacgatg actgccgata cttcgaccga cacggcttac 240
   atggaactct cgtccctgag atcggaggac accgcagtct actactgtgc tcgcactggc 300
   tatcagtcag gctacggctt cccgtactgg ggacaaggaa ccaccgtgac agtgtcctca 360
<210> 727
   <211> 360
   <212> DNA
   <213> Artificial Sequence
<220>
   <223> Completely synthesized
<400> 727
   caagtgcaac tggtgcagtc cggtgccgaa gtgaagaaac cgggtgcgtc cgtcaaagtg 60
   agctgcaagg tgtccgggta caccttgact gattcagtga tgaattgggt gcggcagcac 120
   ccaggaaagg gactggagtg gatgggatgg atcgaccctg aatacggaag gactgacgtc 180
   gcccagaagt ttcagggacg cgtcacgatg actgccgata cttcgaccga cacggcttac 240
   atggaactct cgtccctgag atcggaggac accgcagtct actactgtgc tcgcactggc 300
   tatcagtcag gctacggctt cccgtactgg ggacaaggaa ccaccgtgac agtgtcctca 360
<210> 728
   <211> 122
   <212> PRT
   <213> Artificial Sequence
<220>
   <223> Completely synthesized
<400> 728
   Glu Val Gln Leu Val Gln Ser Gly Ala Glu Val Lys Lys Ser Gly Ala
   1               5                   10                  15
   Ser Val Lys Val Ser Cys Lys Ala Ser Gly Tyr Thr Phe Thr Asp Tyr
               20                  25                  30
   Tyr Ile His Trp Val Arg Gln Pro Pro Gly Gln Gly Leu Glu Trp Met
           35                  40                  45
   Gly Trp Ile Asn Pro Asn Asn Gly Gly Val Thr Phe Ala Gln Lys Phe
       50                  55                  60
   Gln Gly Arg Val Thr Met Thr Arg Asp Thr Ser Ile Ser Thr Ala Tyr
   65                  70                  75                  80
   Met Asp Leu Ser Ser Leu Arg Ser Asp Asp Thr Ala Val Tyr Phe Cys
                   85                  90                  95
   Ala Arg Asp Ile Arg Met Ser Gly Trp Leu Ala Pro Phe Asp Tyr Trp
               100                 105                 110
   Gly Gln Gly Thr Leu Val Thr Val Ser Ser
           115                 120
<210> 729
   <211> 122
   <212> PRT
   <213> Artificial Sequence
<220>
   <223> Completely synthesized
<400> 729
   Glu Val Gln Leu Val Gln Ser Gly Ala Glu Val Lys Lys Ser Gly Ala
   1               5                   10                  15
   Ser Val Lys Val Ser Cys Lys Ala Ser Gly Tyr Thr Phe Thr Asp Tyr
               20                  25                  30
   Tyr Ile His Trp Val Arg Gln Pro Pro Gly Gln Gly Leu Glu Trp Met
           35                  40                  45
   Gly Trp Ile Asn Pro Asn Asn Gly Gly Val Thr Phe Ala Gln Lys Phe
       50                  55                  60
   Gln Gly Arg Val Thr Met Thr Arg Asp Thr Ser Ile Ser Thr Ala Tyr
   65                  70                  75                  80
   Met Asp Leu Ser Ser Leu Arg Ser Asp Asp Thr Ala Val Tyr Tyr Cys
                   85                  90                  95
   Ala Arg Asp Ile Arg Met Ser Gly Trp Leu Ala Pro Phe Asp Tyr Trp
               100                 105                 110
   Gly Gln Gly Thr Leu Val Thr Val Ser Ser
           115                 120
<210> 730
   <211> 122
   <212> PRT
   <213> Artificial Sequence
<220>
   <223> Completely synthesized
<400> 730
   Glu Val Gln Leu Val Gln Ser Gly Ala Glu Val Lys Lys Ser Gly Ala
   1               5                   10                  15
   Ser Val Lys Val Ser Cys Lys Ala Ser Gly Tyr Thr Phe Thr Asp Tyr
               20                  25                  30
   Tyr Ile His Trp Val Arg Gln Pro Pro Gly Gln Gly Leu Glu Trp Met
           35                  40                  45
   Gly Trp Ile Asn Pro Asn Asn Gly Gly Thr Thr Phe Ala Gln Lys Phe
       50                  55                  60
   Gln Gly Arg Val Thr Met Thr Arg Asp Thr Ser Ile Ser Thr Ala Tyr
   65                  70                  75                  80
   Met Asp Leu Ser Ser Leu Arg Ser Asp Asp Thr Ala Val Tyr Phe Cys
                   85                  90                  95
   Ala Arg Asp Ile Arg Met Ser Gly Trp Leu Ala Pro Phe Asp Tyr Trp
               100                 105                 110
   Gly Gln Gly Thr Leu Val Thr Val Ser Ser
           115                 120
<210> 731
   <211> 122
   <212> PRT
   <213> Artificial Sequence
<220>
   <223> Completely synthesized
<400> 731
   Glu Val Gln Leu Val Gln Ser Gly Ala Glu Val Lys Lys Ser Gly Ala
   1               5                   10                  15
   Ser Val Lys Val Ser Cys Lys Ala Ser Gly Tyr Thr Phe Thr Asp Tyr
               20                  25                  30
   Tyr Ile His Trp Val Arg Gln Pro Pro Gly Gln Gly Leu Glu Trp Met
           35                  40                  45
   Gly Trp Ile Asn Pro Asn Asn Gly Gly Val Thr Phe Ala Gln Lys Phe
       50                  55                  60
   Gln Gly Arg Val Thr Met Thr Arg Asp Thr Ser Ile Ser Thr Ala Tyr
   65                  70                  75                  80
   Met Asp Leu Ser Ser Leu Arg Ser Asp Asp Thr Ala Val Tyr Phe Cys
                   85                  90                  95
   Ala Arg Asp Ile Arg Leu Ser Gly Trp Leu Ala Pro Phe Asp Tyr Trp
               100                 105                 110
   Gly Gln Gly Thr Leu Val Thr Val Ser Ser
           115                 120
<210> 732
   <211> 366
   <212> DNA
   <213> Artificial Sequence
<220>
   <223> Completely synthesized
<400> 732
   gaagtccaac tggtccagtc gggcgcagag gtgaagaagt caggggcgag cgtgaaagtg      60
   tcatgtaaag cctccggcta cacttttacc gattactaca ttcactgggt gagacagcct     120
   ccgggacaag gactcgaatg gatgggctgg atcaatccaa acaacggagg tgtcaccttc     180
   gctcagaagt tccaaggtcg ggtgaccatg acccgcgaca cgtcaatcag cactgcctac     240
   atggatttgt cgtccctgcg gtccgatgac actgcggtgt acttctgcgc aagggacatc     300
   cgcatgtcgg ggtggctggc cccgttcgac tattggggac agggaactct cgtgacagtg     360
   tcctca                                                                366
<210> 733
   <211> 366
   <212> DNA
   <213> Artificial Sequence
<220>
   <223> Completely synthesized
<400> 733
   gaagtccaac tggtccagtc gggcgcagag gtgaagaagt caggggcgag cgtgaaagtg 60
   tcatgtaaag cctccggcta cacttttacc gattactaca ttcactgggt gagacagcct 120
   ccgggacaag gactcgaatg gatgggctgg atcaatccaa acaacggagg tgtcaccttc 180
   gctcagaagt tccaaggtcg ggtgaccatg acccgcgaca cgtcaatcag cactgcctac 240
   atggatttgt cgtccctgcg gtccgatgac actgcggtgt actactgcgc aagggacatc 300
   cgcatgtcgg ggtggctggc cccgttcgac tattggggac agggaactct cgtgacagtg 360
   tcctca 366
<210> 734
   <211> 366
   <212> DNA
   <213> Artificial Sequence
<220>
   <223> Completely synthesized
<400> 734
   gaagtccaac tggtccagtc gggcgcagag gtgaagaagt caggggcgag cgtgaaagtg 60
   tcatgtaaag cctccggcta cacttttacc gattactaca ttcactgggt gagacagcct 120
   ccgggacaag gactcgaatg gatgggctgg atcaatccaa acaacggagg taccaccttc 180
   gctcagaagt tccaaggtcg ggtgaccatg acccgcgaca cgtcaatcag cactgcctac 240
   atggatttgt cgtccctgcg gtccgatgac actgcggtgt acttctgcgc aagggacatc 300
   cgcatgtcgg ggtggctggc cccgttcgac tattggggac agggaactct cgtgacagtg 360
   tcctca 366
<210> 735
   <211> 366
   <212> DNA
   <213> Artificial Sequence
<220>
   <223> Completely synthesized
<400> 735
   gaagtccaac tggtccagtc gggcgcagag gtgaagaagt caggggcgag cgtgaaagtg    60
   tcatgtaaag cctccggcta cacttttacc gattactaca ttcactgggt gagacagcct   120
   ccgggacaag gactcgaatg gatgggctgg atcaatccaa acaacggagg tgtcaccttc   180
   gctcagaagt tccaaggtcg ggtgaccatg acccgcgaca cgtcaatcag cactgcctac   240
   atggatttgt cgtccctgcg gtccgatgac actgcggtgt acttctgcgc aagggacatc   300
   cgcctgtcgg ggtggctggc cccgttcgac tattggggac agggaactct cgtgacagtg   360
   tcctca                                                              366
<210> 736
   <211> 122
   <212> PRT
   <213> Artificial Sequence
<220>
   <223> Completely synthesized
<220>
   <221> Variant
   <222> (40)..(40)
   <223> X = Ala or Pro
<220>
   <221> Variant
   <222> (58)..(58)
   <223> X = Val or Thr
<220>
   <221> Variant
   <222> (95)..(95)
   <223> X = Phe or Tyr
<220>
   <221> Variant
   <222> (102)..(102)
   <223> X = Met or Leu
<400> 736
   Glu Val Gln Leu Val Gln Ser Gly Ala Glu Val Lys Lys Ser Gly Ala
   1               5                   10                  15
   Ser Val Lys Val Ser Cys Lys Ala Ser Gly Tyr Thr Phe Thr Asp Tyr
               20                  25                  30
   Tyr Ile His Trp Val Arg Gln Xaa Pro Gly Gln Gly Leu Glu Trp Met
           35                  40                  45
   Gly Trp Ile Asn Pro Asn Asn Gly Gly Xaa Thr Phe Ala Gln Lys Phe
       50                  55                  60
   Gln Gly Arg Val Thr Met Thr Arg Asp Thr Ser Ile Ser Thr Ala Tyr
   65                  70                  75                  80
   Met Asp Leu Ser Ser Leu Arg Ser Asp Asp Thr Ala Val Tyr Xaa Cys
                   85                  90                  95
   Ala Arg Asp Ile Arg Xaa Ser Gly Trp Leu Ala Pro Phe Asp Tyr Trp
               100                 105                 110
   Gly Gln Gly Thr Leu Val Thr Val Ser Ser
           115                 120
<210> 737
   <211> 17
   <212> PRT
   <213> Artificial Sequence
<220>
   <223> Completely synthesized
<400> 737
   Trp Ile Asn Pro Asn Asn Gly Gly Thr Thr Phe Ala Gln Lys Phe Gln
   1               5                   10                  15
   Gly
<210> 738
   <211> 13
   <212> PRT
   <213> Artificial Sequence
<220>
   <223> Completely synthesized
<400> 738
   Asp Ile Arg Leu Ser Gly Trp Leu Ala Pro Phe Asp Tyr
   1               5                   10
<210> 739
   <211> 120
   <212> PRT
   <213> Artificial Sequence
<220>
   <223> Completely synthesized
<400> 739
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Asp Ser
               20                  25                  30
   Val Met Asn Trp Val Gln Gln Gly Pro Gly Lys Gly Leu Glu Trp Met
           35                  40                  45
   Gly Trp Ile Asp Pro Glu Tyr Gly Arg Thr Asp Val Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Thr Gly Tyr Asn Ser Gly Tyr Gly Phe Pro Tyr Trp Gly Gln
                   100                 105                 110
   Gly Thr Thr Val Thr Val Ser Ser
           115                 120
<210> 740
   <211> 120
   <212> PRT
   <213> Artificial Sequence
<220>
   <223> Completely synthesized
<400> 740
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Asn Trp Val Gln Gln His Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Gly Tyr Asn Ser Gly Tyr Gly Phe Pro Tyr Trp Gly Gln
   100 105 110
   Gly Thr Thr Val Thr Val Ser Ser
   115 120
<210> 741
   <211> 120
   <212> PRT
   <213> Artificial Sequence
<220>
   <223> Completely synthesized
<400> 741
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Asn Trp Val Gln Gln Gly Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Gly Tyr Gln Ser Gly Tyr Gly Phe Pro Tyr Trp Gly Gln
   100 105 110
   Gly Thr Thr Val Thr Val Ser Ser
   115 120
<210> 742
   <211> 120
   <212> PRT
   <213> Artificial Sequence
<220>
   <223> Completely synthesized
<400> 742
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Asp Ser
   20 25 30
   Val Met Asn Trp Val Gln Gln His Pro Gly Lys Gly Leu Glu Trp Met
   35 40 45
   Gly Trp Ile Asp Pro Glu Tyr Gly Arg Thr Asp Val Ala Glu Lys Phe
   50 55 60
   Gln Gly Arg Val Thr Ile Thr Ala Asp Thr Ser Thr Asp Thr Ala Tyr
   65 70 75 80
   Met Glu Leu Ser Ser Leu Arg Ser Glu Asp Thr Ala Val Tyr Tyr Cys
   85 90 95
   Ala Arg Thr Gly Tyr Gln Ser Gly Tyr Gly Phe Pro Tyr Trp Gly Gln
   100 105 110
   Gly Thr Thr Val Thr Val Ser Ser
   115 120
<210> 743
   <211> 360
   <212> DNA
   <213> Artificial Sequence
<220>
   <223> Completely synthesized
<400> 743
   gaagtgcagc tggtgcagtc gggtgcagag gtcaagaaac cgggagcgac cgtgaaaatc 60
   agctgcaagg tgtccgggta cactttcacg gactcggtga tgaattgggt ccaacaggga 120
   ccggggaagg gattggagtg gatgggatgg attgacccag aatacggccg caccgacgtc 180
   gcagaaaagt ttcaaggacg ggtcactatc accgcggaca cctcaaccga tactgcctac 240
   atggagctct cctcgctgag aagcgaagat actgccgtgt actactgtgc caggaccggt 300
   tacaactcgg gatacggctt cccttattgg ggacagggca ccactgtgac agtgtcctca 360
<210> 744
   <211> 360
   <212> DNA
   <213> Artificial Sequence
<220>
   <223> Completely synthesized
<400> 744
   gaggtgcagc tggtccagtc aggagcagaa gtcaaaaagc cgggagccac cgtcaagatt 60
   tcgtgtaagg tgtcgggcta cactttcact gattccgtca tgaattgggt gcagcaacac 120
   ccagggaaag ggttggaatg gatgggatgg atcgaccctg aatacggccg caccgacgtg 180
   gcggagaagt ttcaaggaag agtgacgatc actgccgata cctcgaccga caccgcatac 240
   atggaactga gctcgctccg gtccgaggac accgccgtgt actactgcgc gaggactggc 300
   tacaacagcg gatacggatt cccgtattgg ggccagggta ccactgtgac agtgtcctca 360
<210> 745
   <211> 360
   <212> DNA
   <213> Artificial Sequence
<220>
   <223> Completely synthesized
<400> 745
   gaggtccagc tggtgcaatc aggagccgaa gtgaaaaagc cgggagcaac cgtcaagatt 60
   agctgtaagg tgtcggggta caccttcact gactccgtga tgaactgggt gcagcaaggg 120
   ccaggaaagg gactggagtg gatgggctgg atcgaccctg aatacggcag gactgacgtc 180
   gccgagaaat tccagggtag agtgaccatc actgcggata cctcgaccga cactgcgtac 240
   atggaactct ccagcttgcg gtcggaagat accgccgtct actactgcgc acgcaccggt 300
   tatcagtcgg gctacggatt tccgtactgg ggacaaggaa ctacggtgac agtgtcctca 360
<210> 746
   <211> 360
   <212> DNA
   <213> Artificial Sequence
<220>
   <223> Completely synthesized
<400> 746
   gaagtccagc tcgtgcagtc gggagccgaa gtgaagaagc cgggagcgac tgtcaagatt        60
   tcgtgtaagg tcagcggata taccttcacc gattccgtga tgaactgggt gcagcagcac       120
   ccaggcaaag gccttgagtg gatggggtgg atcgacccgg agtacgggag aaccgacgtg       180
   gcagagaaat ttcaaggacg ggtcaccatc actgccgaca cttccactga cactgcatac       240
   atggaactgt catcgctgcg ctcggaagat accgccgtgt actactgcgc gaggacgggt       300
   taccaaagcg gatacggatt cccttactgg ggccaaggta ccaccgtgac agtgtcctca       360
<210> 747
   <211> 7
   <212> PRT
   <213> Artificial Sequence
<220>
   <223> Completely synthesized
<400> 747
   Lys Ala Ser Gln Asp Tyr Thr
   1               5
<210> 748
   <211> 30
   <212> PRT
   <213> Homo sapiens
<400> 748
   Glu Val Gln Leu Val Gln Ser Gly Ala Glu Val Lys Lys Ser Gly Ala
   1               5                   10                  15
   Ser Val Lys Val Ser Cys Lys Ala Ser Gly Tyr Thr Phe Thr
               20                  25                  30
<210> 749
   <211> 14
   <212> PRT
   <213> Homo sapiens
<400> 749
   Trp Val Arg Gln Ala Pro Gly Gln Gly Leu Glu Trp Met Gly
   1               5                   10
<210> 750
   <211> 14
   <212> PRT
   <213> Artificial Sequence
<220>
   <223> Completely synthesized
<400> 750
   Trp Val Arg Gln Pro Pro Gly Gln Gly Leu Glu Trp Met Gly
   1 5 10
<210> 751
   <211> 32
   <212> PRT
   <213> Homo sapiens
<400> 751
   Arg Val Thr Met Thr Arg Asp Thr Ser Ile Ser Thr Ala Tyr Met Asp
   1 5 10 15
   Leu Ser Ser Leu Arg Ser Asp Asp Thr Ala Val Tyr Phe Cys Ala Arg
   20 25 30
<210> 752
   <211> 32
   <212> PRT
   <213> Artificial Sequence
<220>
   <223> Completely synthesized
<400> 752
   Arg Val Thr Met Thr Arg Asp Thr Ser Ile Ser Thr Ala Tyr Met Asp
   1 5 10 15
   Leu Ser Ser Leu Arg Ser Asp Asp Thr Ala Val Tyr Tyr Cys Ala Arg
   20 25 30
<210> 753
   <211> 11
   <212> PRT
   <213> Homo sapiens
<400> 753
   Trp Gly Gln Gly Thr Leu Val Thr Val Ser Ser
   1 5 10